(19)

Europäisches
Patentamt
European
Patent Office
Office européen
des brevets

(11) **EP 4 559 477 A2**

(12) **EUROPEAN PATENT APPLICATION**

(43) Date of publication:
**28.05.2025 Bulletin 2025/22**

(51) International Patent Classification (IPC):
**A61K 39/00** (2006.01)

(21) Application number: **25155974.6**

(22) Date of filing: **13.05.2021**

(52) Cooperative Patent Classification (CPC):
**C07K 16/2878; A61P 35/00; C07K 16/2818;**
A61K 2039/505; A61K 2039/507; A61K 2039/545;
A61K 2039/55; C07K 2317/32; C07K 2317/75;
C07K 2317/76; C07K 2317/92

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **13.05.2020 PCT/CN2020/090073**
**04.06.2020 PCT/CN2020/094278**
**17.09.2020 PCT/CN2020/115795**

(62) Document number(s) of the earlier application(s) in
accordance with Art. 76 EPC:
**21803953.5 / 4 149 547**

(71) Applicant: **Adagene AG**
**4051 Basel (CH)**

(72) Inventors:
• **LIU, Guizhong**
**Suzhou, 215123 (CN)**
• **GONG, Changhua**
**San Diego, 92121 (US)**
• **LUO, Peter Peizhi**
**Suzhou, 215123 (CN)**

(74) Representative: **J A Kemp LLP**
**80 Turnmill Street**
**London EC1M 5QU (GB)**

Remarks:
•The complete document including Reference
Table(s) and the Sequence Listing(s) can be
downloaded from the EPO website
•This application was filed on 05-02-2025 as a
divisional application to the application mentioned
under INID code 62.
•Claims filed after the date of filing of the application /
after the date of receipt of the divisional application
(Rule 68(4) EPC).

(54) **COMPOSITIONS AND METHODS FOR TREATING CANCER**

(57) The present application provides compositions and methods for treating cancers, including follicular lymphoma, T cell lymphoma and adenoid cystic carcinoma, using an anti-CD137 antibody that specifically binds to an extracellular domain of human CD137. In some embodiment, combination therapies including the anti-CD137 antibody and an immune checkpoint inhibitor, and/or a chemotherapeutic agent are provided. Biomarkers such as total CD137, membrane bound CD137 (mCD137), soluble CD137 (sCD137), CD137 ligand, Ki67, CD8+ effector memory T ($T_{em}$) cells, regulatory T ($T_{reg}$) cells, and natural killer (NK) cell levels for the methods of treatment described herein are also provided.

**EP 4 559 477 A2**

**Description**

**CROSS REFERENCE TO RELATED APPLICATIONS**

This application claims the priority benefit of International Application No. PCT/CN2020/090073, filed on May 13, 2020, International Application No. PCT/CN2020/094278, filed on June 4, 2020, and International Application No. PCT/CN2020/115795, filed on September 17, 2020, which are incorporated herein by reference in its entirety.

**SUBMISSION OF SEQUENCE LISTING ON ASCII TEXT FILE**

The content of the following submission on ASCII text file is incorporated herein by reference in its entirety: a computer readable form (CRF) of the Sequence Listing (file name: 695402001245SEQLIST.TXT, date recorded: May 10, 2021, size: 86 KB).

**FIELD**

The present application is in the field of cancer therapeutics, and relates to compositions and methods for treating cancers using antibodies that bind to human CD137.

**BACKGROUND**

CD137 (also referred to as CD137 receptor, 4-1BB, TNFRSF9, *etc*.) is a transmembrane protein of the Tumor Necrosis Factor Receptor Superfamily (TNFRS). Current understanding of CD137 indicates that its expression is generally activation dependent and is present in a broad subset of immune cells including activated NK and NKT cells, regulatory T cells, dendritic cells (DC), stimulated mast cells, differentiating myeloid cells, monocytes, neutrophils, and eosinophils (Wang, 2009, Immunological Reviews 229: 192-215). CD137 expression has also been demonstrated on tumor vasculature (Broll, 2001, Amer. J. Clin. Pathol. 115(4):543-549; Seaman, 2007, Cancer Cell 11: 539-554) and at sites of inflamed or atherosclerotic endothelium (Drenkard, 2007 FASEB J. 21: 456-463; Olofsson, 2008, Circulation 117: 1292-1301). The ligand that stimulates CD137, *i.e.,* CD137 Ligand (CD137L), is expressed on activated antigen-presenting cells (APCs), myeloid progenitor cells, and hematopoietic stem cells.

Numerous studies of murine and human T cells indicate that CD137 promotes enhanced cellular proliferation, survival, and cytokine production (Croft, 2009, Nat Rev Immunol 9:271-285). Studies have indicated that some CD137 agonist mAbs increase costimulatory molecule expression and markedly enhance cytolytic T lymphocyte responses, resulting in anti-tumor efficacy in various models. CD137 agonist mAbs have demonstrated efficacy in prophylactic and therapeutic settings. Further, CD137 monotherapy and combination therapy tumor models have established durable anti-tumor protective T cell memory responses (Lynch, 2008, Immunol Rev. 22: 277-286). CD137 agonists also have been shown to inhibit autoimmune reactions in a variety of art-recognized autoimmunity models (Vinay, 2006, J Mol Med 84:726-736). This dual activity of CD137 offers the potential to provide anti-tumor activity while dampening autoimmune side effects that can be associated with immunotherapy approaches that break immune tolerance.

There is a long-felt unmet need for antibodies that bind human CD137, increase a CD137-mediated response, and thereby provide potential therapeutics for treatment of various diseases and conditions, including cancer and autoimmune diseases.

The disclosures of all publications, patents, patent applications and published patent applications cited throughout this disclosure are hereby incorporated herein by reference in their entirety.

**BRIEF SUMMARY**

The present application provides methods for treating cancer with an anti-CD137 antibody, and biomarkers (e.g., prognostic biomarkers) for the methods described herein.

The present invention in one aspect provides a method of treating a cancer in a subject, comprising administering to the subject an effective amount of an anti-CD137 antibody that specifically binds to an extracellular domain of human CD137, wherein the antibody binds to one or more amino acid residues within amino acid residues 51, 53, 62-73, 83, 89, 92, 95-104 and 112-116 of SEQ ID NO: 1, and wherein the anti-CD137 antibody is administered at a dose of no more than 500 mg (e.g., about 50 mg to about 200 mg, about 100 mg to about 200 mg, about 150 mg to about 500 mg, about 150 mg to about 300 mg, or about 300 mg to about 400 mg). In some embodiments, the anti-CD137 antibody is administered at a dose of about 50 mg, about 100 mg, about 200 mg, about 300 mg, or about 400 mg. In some embodiments, the anti-CD137 antibody is administered as a monotherapy at a dose of about 100 mg, about 200 mg, about 300 mg or about 400 mg. In some embodiments, the anti-CD137 antibody is administered in combination with an additional therapeutic agent (*e.g.,* an immune checkpoint inhibitor such as an anti-PD-1 antibody), wherein the anti-CD137 antibody is administered at a dose of about 50 mg, about 100 mg, or about 200 mg. In some embodiments, the subject has a body weight of no more than about 100 kg, such as about 60 kg to about 100 kg.

One aspect of the present application provides a method of treating a cancer in a subject, comprising administering to the subject an effective amount of an anti-CD137 antibody that specifically binds to an extracellular domain of human CD137, wherein the antibody binds to one or more amino acid residues within amino acid residues 51, 53, 62-73, 83, 89, 92, 95-104 and 112-116 of SEQ ID NO: 1, and wherein the cancer is resistant or refractory to a prior therapy (e.g., a prior immunotherapy). In some embodiments, the prior therapy is an anti-CD20 antibody. In some embodiments, the prior therapy is rituximab. In some embodiments, the anti-CD137 antibody is administered at a dose of no more than 500 mg (e.g., about 50 mg to about 200 mg, about 100 mg to about 200 mg, about 150 mg to about 500 mg, about 150 mg to about 300 mg, or about 300 mg to about 400 mg).

One aspect of the present application provides a method of treating a cancer in a subject, comprising administering to the subject an effective amount of an anti-CD137 antibody that specifically binds to an extracellular domain of human CD137, wherein the antibody binds to one or more amino acid residues selected from the group consisting of amino acid residues 51, 53, 62-73, 83, 89, 92, 95-104 and 112-116 of SEQ ID NO: 1; and wherein the subject has a high level in one or more biomarkers selected from the group consisting of total CD137, membrane bound CD137 (mCD137), CD137 ligand (CD137L) and PD-L1 and/or a low level of CD8+ effector memory T ($T_{em}$) cells or natural killer (NK) cells compared to a reference level. In some embodiments, the anti-CD137 antibody is administered at a dose of no more than 500 mg (e.g., about 50 mg to about 200 mg, about 100 mg to about 200 mg, about 150 mg to about 500 mg, about 150 mg to about 300 mg, or about 300 mg to about 400 mg). In some embodiments, the method further comprises administering to the subject an effective amount of an immune checkpoint inhibitor, such as an anti-PD-1 antibody (e.g., toripalimab).

One aspect of the present application provides a method of treating a cancer in a subject, comprising: (a) administering to the subject an effective amount of an anti-CD137 antibody that specifically binds to an extracellular domain of human CD137, wherein the antibody binds to one or more amino acid residues selected from the group consisting of amino acid residues 51, 53, 62-73, 83, 89, 92, 95-104 and 112-116 of SEQ ID NO: 1; and (b) subsequently determining a level of one or more biomarkers selected from the group consisting of total CD137, membrane bound (mCD137), soluble CD137 (sCD137), CD137L, Ki67, NK cells, CD8+ effector memory T ($T_{em}$) cells, regulatory T ($T_{reg}$) cells and NK cells in a sample of the subject. In some embodiments, an increased level of one or more biomarkers selected from the group consisting of total CD137, sCD137, Ki67, CD137L, NK cells, and CD8 $T_{em}$ cells, and/or a decreased level of one or more biomarkers selected from the group consisting of mCD137 and $T_{reg}$ cells after administration of the anti-CD137 antibody compared to the level of the one or more biomarkers before administration of the anti-CD137 antibody indicates that the subject may benefit from the administration of the ant-CD137 antibody. In some embodiments, the sample has an increased level of one or more biomarkers selected from the group consisting of total CD137, sCD137, Ki67, CD137L, NK cells, and CD8 $T_{em}$ cells, and/or a decreased level of one or more biomarkers selected from the group consisting of mCD137 and $T_{reg}$ cells after administration of the anti-CD137 antibody compared to the level of the one or more biomarkers before administration of the anti-CD137 antibody, the method further comprises administering to the subject an effective amount of the anti-CD137 antibody. In some embodiments, the level of one or more biomarkers comprises a level of CD137 (e.g., in a tumor biopsy sample). In some embodiments, the level of one or more biomarkers comprises a level of sCD137 in a blood sample, such as a plasma sample. In some embodiments, the level of one or more biomarkers comprises a level of mCD137 on CD8+ T cells. In some embodiments, the level of one or more biomarkers comprises a level of Ki67 on CD8+ T cells. In some embodiments, the CD8+ T cells are tumor infiltrating T cells. In some embodiments, the level of one or more biomarkers comprises a level of CD137L (e.g., in a tumor biopsy sample). In some embodiments, the level of one or more biomarkers comprises a level of NK cells in a blood sample. In some embodiments, the sample is a tumor biopsy sample. In some embodiments, the sample is a formalin-fixed paraffin-embedded (FFPE) sample. In some embodiments, the level of the one or more biomarkers is detected by immunohistochemistry (IHC). In some embodiments, the anti-CD137 antibody is administered at a dose of no more than 500 mg (e.g., about 50 mg to about 200 mg, about 100 mg to about 200 mg, about 150 mg to about 500 mg, about 150 mg to about 300 mg, or about 300 mg to about 400 mg). In some embodiments, the method further comprises administering to the subject an effective amount of an immune checkpoint inhibitor, such as an anti-PD-1 antibody (e.g., toripalimab).

One aspect of the present application provides a method of providing a prognosis for a subject who has been administered with an effective amount of an anti-CD137 antibody that specifically binds to an extracellular domain of human CD137, wherein the antibody binds to one or more amino acid residues selected from the group consisting of amino acid residues 51, 53, 62-73, 83, 89, 92, 95-104 and 112-116 of SEQ ID NO: 1; the method comprising determining a level of one or more biomarkers selected from the group consisting of total CD137, membrane bound (mCD137), soluble CD137 (sCD137), CD137L, Ki67, CD8+ effector memory T ($T_{em}$) cells, regulatory T ($T_{reg}$) cells and NK cells in a sample of the subject, wherein an increased level of one or more biomarkers selected from the group consisting of total CD137, sCD137, Ki67, CD137L, CD8 $T_{em}$ cells, and NK cells, and/or a decreased level of one or more biomarkers selected from the group consisting of mCD137 and $T_{reg}$ cells after administration of the anti-CD137 antibody compared to the level of the one or more biomarkers before administration of the anti-CD137 antibody identifies the subject as having a high likelihood of responding to the anti-CD137 antibody treatment. In some embodiments, the level of one or more biomarkers comprises a level of CD137 (*e.g.,* in a tumor biopsy sample). In some embodiments, the level of one or more biomarkers comprises a level of sCD137 in a blood sample (e.g., a plasma sample). In some embodiments, the level of one or more biomarkers comprises a level of mCD137 on CD8+ T cells. In some embodiments, the level of one or more biomarkers comprises a level of Ki67 on CD8+ T cells. In some embodiments, the CD8+ T cells are tumor infiltrating T cells. In some embodiments, the level of one or more biomarkers comprises a level of CD137L (*e.g.,* in a tumor biopsy sample). In some embodiments, the level of one or more biomarkers comprises a level of NK cells in a blood sample. In some embodiments, the sample is a tumor biopsy sample. In some embodiments, the sample is a formalin-fixed paraffin-embedded (FFPE) sample. In some embodiments, the level of the one or more biomarkers is detected by immunohistochemistry (IHC). In some embodiments, the sample is a tumor biopsy sample. In some embodiments, the anti-CD137 antibody is administered at a dose of no more than 500 mg (*e.g.,* about 50 mg to about 200 mg, about 100 mg to about 200 mg, about 150 mg to about 500 mg, about 150 mg to about 300 mg, or about 300 mg to about 400 mg). In some embodiments, the method further comprises administering to the subject an effective amount of an immune checkpoint inhibitor, such as an anti-PD-1 antibody (*e.g.,* toripalimab).

In some embodiments according to any one of the methods described above, the cancer is a solid cancer. In some embodiments, the cancer is selected from the group consisting of colon cancer (*e.g.,* Sigmoid colon cancer), breast cancer (*e.g.,* triple negative breast cancer or TNBC), lung cancer (*e.g.,* non-small cell lung cancer or NSCLC; or small cell lung cancer or SCLC), esophageal cancer, endometrial cancer, gastrointestinal cancer (*e.g.,* gastrointestinal neuroectodermal tumor), cholangiocarcinoma, nasopharyngeal cancer (NPC), adenoid cystic carcinoma (ACC), melanoma, mesothelioma (*e.g.,* malignant pleural mesothelioma or MPM), mantle cell lymphoma, anal cancer, T cell lymphoma (TCL), head and neck cancer *(e.g.,* head and neck squamous cell carcinoma or HNSCC) and appendiceal and sebaceous cancer. In some embodiments, the cancer is a liquid cancer. In some embodiments, the cancer is follicular lymphoma. In some embodiments, the cancer is non-Hodgkin's lymphoma (NHL). In some embodiments, the cancer is T cell lymphoma, such as angioimmunoblastic T-cell lymphoma (AITL) or Peripheral T-cell lymphoma (PTCL).

One aspect of the present application provides a method of treating a cancer in a subject, comprising administering to the subject an effective amount of an anti-CD137 antibody that specifically binds to an extracellular domain of human CD137, wherein the antibody binds to one or more amino acid residues within amino acid residues 51, 53, 62-73, 83, 89, 92, 95-104 and 112-116 of SEQ ID NO: 1, and wherein the cancer is selected from the group consisting of follicular lymphoma, T cell lymphoma, and ACC. In some embodiments, the cancer is follicular lymphoma. In some embodiments, the cancer is T cell lymphoma. In some embodiments, the cancer is AITL or PTCL. In some embodiments, the cancer is ACC. In some embodiments, the anti-CD137 antibody is administered at a dose of no more than 500 mg (*e.g.,* about 50 mg to about 200 mg, about 100 mg to about 200 mg, about 150 mg to about 500 mg, about 150 mg to about 300 mg, or about 300 mg to about 400 mg).

One aspect of the present application provides a method of treating a lung cancer in a subject, comprising administering to the subject: (a) an effective amount of an anti-CD137 antibody that specifically binds to an extracellular domain of human CD137, wherein the antibody binds to one or more amino acid residues selected from the group consisting of amino acid residues 51, 53, 62-73, 83, 89, 92, 95-104 and 112-116 of SEQ ID NO: 1; and (b) an effective amount of an immune checkpoint inhibitor. In some embodiments, the immune checkpoint inhibitor is an anti-PD-L1 antibody (*e.g.,* atezolizumab). In some embodiments, the immune checkpoint inhibitor is an anti-PD-1 antibody (*e.g.,* toripalimab). In some embodiments, the immune checkpoint inhibitor is an anti-CTLA-4 antibody (*e.g.,* ADG116). In some embodiments, the anti-CD137 antibody is administered at a dose of no more than 500 mg (*e.g.,* about 50 mg to about 200 mg, about 100 mg to about 200 mg, about 150 mg to about 500 mg, about 150 mg to about 300 mg, or about 300 mg to about 400 mg). In some embodiments, the anti-CD137 antibody is administered at a dose of about 50 mg, about 100 mg, or about 200 mg.

One aspect of the present application provides method of treating a breast cancer in a subject, comprising administering to the subject: (a) an effective amount of an anti-CD137 antibody that specifically binds to an extracellular domain of human CD137, wherein the antibody binds to one or more amino acid residues selected from the group consisting of amino acid residues 51, 53, 62-73, 83, 89, 92, 95-104 and 112-116 of SEQ ID NO: 1; and (b) an effective amount of an immune checkpoint inhibitor. In some embodiments, the breast cancer is triple-negative breast cancer. In some embodiments, the immune checkpoint inhibitor is an anti-PD-L1 antibody (*e.g.,* atezolizumab). In some embodiments, the immune checkpoint inhibitor is an anti-PD-1 antibody (*e.g.,* toripalimab). In some embodiments, the immune checkpoint inhibitor is an anti-CTLA-4 antibody (*e.g.,* ADG116). In some embodiments, the anti-CD137 antibody is administered at a dose of no more than 500 mg (*e.g.,* about 50 mg to about 200 mg, about 100 mg to about 200 mg, about 150 mg to about 500 mg, about 150 mg to about 300 mg, or about 300 mg to about 400 mg). In some embodiments, the anti-CD137 antibody is administered at a dose of about 50 mg, about 100 mg, or about 200 mg.

One aspect of the present application provides a method of treating a lung cancer in a subject, comprising administering to the subject: (a) an effective amount of an anti-CD137 antibody that specifically binds to an extracellular domain of human CD137, wherein the antibody binds to one or more amino acid residues selected from the group consisting of amino acid residues 51, 53, 62-73, 83, 89, 92, 95-104 and 112-116 of SEQ ID NO: 1; and (b) an effective amount of a chemotherapeutic agent. In some embodiments, the chemotherapeutic agent is docetaxel. In some embodiments, the chemotherapeutic agent is cisplatin. In some embodiments, the anti-CD137 antibody is administered at a dose of no more than 500 mg (*e.g.,* about 50 mg to about 200 mg, about 100 mg to about 200 mg, about 150 mg to about 500 mg, about 150 mg to about 300 mg, or about 300 mg to about 400 mg). In some embodiments, the chemotherapeutic agent is cisplatin, and the method does not comprise administering the subject paclitaxel or pemetrexed.

One aspect of the present application provides a method of treating a breast cancer in a subject, comprising administering to the subject: (a) an effective amount of an anti-CD137 antibody that specifically binds to an extracellular domain of human CD137, wherein the antibody binds to one or more amino acid residues selected from the group consisting of amino acid residues 51, 53, 62-73, 83, 89, 92, 95-104 and 112-116 of SEQ ID NO: 1; and (b) an effective amount of a chemotherapeutic agent. In some embodiments, the breast cancer is triple-negative breast cancer. In some embodiments, the chemotherapeutic agent is docetaxel. In some embodiments, the chemotherapeutic agent is cisplatin. In some embodiments, the anti-CD137 antibody is administered at a dose of no more than 500 mg (*e.g.,* about 50 mg to about 200 mg, about 100 mg to about 200 mg, about 150 mg to about 500 mg, about 150 mg to about 300 mg, or about 300 mg to about 400 mg).

One aspect of the present application provides a method of treating a lung cancer in a subject, comprising administering to the subject: (a) an effective amount of an anti-CD137 antibody that specifically binds to an extracellular domain of human CD137, wherein the antibody binds to one or more amino acid residues selected from the group consisting of amino acid residues 51, 53, 62-73, 83, 89, 92, 95-104 and 112-116 of SEQ ID NO: 1; and (b) an effective amount of an anti-CD20 antibody. In some embodiments, the anti-CD20 antibody is rituximab. In some embodiments, the anti-CD137 antibody is administered at a dose of no more than 500 mg (*e.g.,* about 50 mg to about 200 mg, about 100 mg to about 200 mg, about 150 mg to about 500 mg, about 150 mg to about 300 mg, or about 300 mg to about 400 mg).

One aspect of the present application provides a method of treating a colon cancer in a subject, comprising administering to the subject: (a) an effective amount of an anti-CD137 antibody that specifically binds to an extracellular domain of human CD137, wherein the antibody binds to one or more amino acid residues selected from the group consisting of amino acid residues 51, 53, 62-73, 83, 89, 92, 95-104 and 112-116 of SEQ ID NO: 1; and (b) an effective amount of a radiation therapy. In some embodiments, the radiation therapy is local radiation. In some embodiments, the anti-CD137 antibody is administered at a dose of no more than 500 mg (*e.g.,* about 50 mg to about 200 mg, about 100 mg to about 200 mg, about 150 mg to about 500 mg, about 150 mg to about 300 mg, or about 300 mg to about 400 mg).

In some embodiments according to any one of the methods described above, the anti-CD137 antibody is administered at a dose at a dose of about 300 mg to about 400 mg. In some embodiments, the anti-CD137 antibody is administered at a dose of about 50 mg, about 100 mg, about 200 mg, about 300 mg, or about 400 mg. In some embodiments, the subject has a body weight of about 60 kg to about 100 kg.

In some embodiments, the anti-CD 137 antibody is administered at a dose of about 0.1 mg/kg to about 10 mg/kg. In some embodiments, the anti-CD137 antibody is administered at a dose of about 3 mg/kg to about 8 mg/kg. In some embodiments, the anti-CD137 antibody is administered at a dose of about 1.5 mg/kg, about 3 mg/kg, about 5 mg/kg, or about 8 mg/kg. As used herein, a dose measured in mg/kg is with respect to the body weight of the subject in kilogram.

In some embodiments according to any one of the methods described above, the anti-CD137 antibody is administered intravenously. In some embodiments, the anti-CD137 antibody is administered about once every three weeks. In some embodiments, the subject receives at least 2 cycles of treatment with the anti-CD137 antibody.

In some embodiments according to any one of the methods described above, the cancer is advanced-stage cancer. In some embodiments, the cancer is metastatic cancer. In some embodiments, the cancer is resistant or refractory to a prior therapy. In some embodiments, the prior therapy is selected from the group consisting of viral gene therapy, immunotherapy, targeted therapy, radiation therapy, and chemotherapy.

In some embodiments according to any one of the methods described above, the anti-CD137 antibody is cross-reactive with a CD137 polypeptide from at least one non-human species selected from the group consisting of cynomolgus monkey, mouse, rat and dog.

In some embodiments according to any one of the methods described above, the anti-CD137 antibody binds to amino acid residues 51, 63-67, 69-73, 83, 89, 92, 98-104 and 112-114 of SEQ ID NO: 1.

In some embodiments, the anti-CD 137 antibody comprises a heavy chain variable region (VH) and a light chain variable region (VL), wherein the VH comprises a HVR-H1 comprising the amino acid sequence of SEQ ID NO: 2, a HVR-H2 comprising the amino acid sequence of SEQ ID NO: 3, and a HVR-H3 comprising the amino acid sequence of SEQ ID NO: 4; and wherein the VL comprises a HVR-L1 comprising the amino acid sequence of SEQ ID NO: 5, a HVR-L2 comprising the amino acid sequence of SEQ ID NO: 6, and a HVR-L3 comprising the amino acid sequence of SEQ ID NO: 7. In some embodiments, the VH comprises the amino acid sequence of SEQ ID NO: 8, and/or the VL comprises the amino acid sequence of SEQ ID NO: 9. In some embodiments, the antibody comprises a heavy chain and a light chain, and wherein the heavy chain comprises the amino acid sequence of SEQ ID NO: 10, and/or the light chain comprises the amino acid sequence of SEQ ID NO: 11.

In some embodiments, the anti-CD137 antibody comprises a VH and a VL, wherein the VH comprises a HVR-H1 comprising the amino acid sequence of SEQ ID NO: 12, a HVR-H2 comprising the amino acid sequence of SEQ ID NO: 13, and a HVR-H3 comprising the amino acid sequence of SEQ ID NO: 14; and wherein the VL comprises a HVR-L1 comprising the amino acid sequence of SEQ ID NO: 15, a HVR-L2 comprising the amino acid sequence of SEQ ID NO: 16, and a HVR-L3 comprising the amino acid sequence of SEQ ID NO: 17. In some embodiments, the VH comprises the amino acid sequence of SEQ ID NO: 18, and/or the VL, comprises the amino acid sequence of SEQ ID NO: 19. In some embodiments, the antibody comprises a heavy chain and a light chain, wherein the heavy chain comprises the amino acid sequence of SEQ ID NO: 20, and/or the light chain comprises the amino acid sequence of SEQ ID NO: 21.

In some embodiments, the anti-CD137 antibody comprises a VH and a VL, wherein the VH comprises a HVR-H1 comprising the amino acid sequence of SEQ ID NO: 22, a HVR-H2 comprising the amino acid sequence of SEQ ID NO: 23, and a HVR-H3 comprising the amino acid sequence of SEQ ID NO: 24; and wherein the VL comprises a HVR-L1 comprising the amino acid sequence of SEQ ID NO: 25, a HVR-L2 comprising the amino acid sequence of SEQ ID NO: 26, and a HVR-L3 comprising the amino acid sequence of SEQ ID NO: 27. In some embodiments, the VH comprises the amino acid sequence of SEQ ID NO: 28, and/or the VL comprises the amino acid sequence of SEQ ID NO: 29. In some embodiments, the antibody comprises a heavy chain and a light chain, wherein the heavy chain comprises the amino acid sequence of SEQ ID NO: 30, and/or the light chain comprises the amino acid sequence of SEQ ID NO: 31.

In some embodiments according to any one of the methods described above, the anti-CD137 antibody comprises a human IgG4 Fc region. In some embodiments, the human IgG4 Fc region comprises an S241P mutation, wherein numbering is according to Kabat.

In some embodiments according to any one of the methods described above, the subject is a human subject. In some embodiments, the method further comprises administering to the subject a therapeutically effective amount of at least one additional therapeutic agent. In some embodiments, the at least one additional therapeutic agent is selected from the group consisting of viral gene therapy, immune checkpoint inhibitors, targeted therapies, radiation therapies, and chemotherapies.

Also provided are compositions, kits, and articles of manufacture for use in any one of the methods described herein.

It is to be understood that one, some, or all of the properties of the various embodiments described above and herein may be combined to form other embodiments of the present disclosure. These and other aspects of the present disclosure will become apparent to one of skill in the art. These and other embodiments of the present disclosure are further described by the detailed description that follows.

**BRIEF DESCRIPTION OF THE DRAWINGS**

FIGs. 1A-1D show the alignment of epitopes on CD137 bound by CD137 ligand, ADG 106, Utomilumab, and Urelumab.

FIG. 2 shows treatment duration and response of patients treated with 0.03, 0.1, 0.3, 1, 3, 5, or 10 mg/kg ADG106. Green bars denote patients continuing on study treatment (n=12); gray bars denote patients discontinued study treatment due to disease progression (n=22); yellow bars denotes patients discontinued study treatment due to other reasons (such as AE, lack of clinical benefit) (n=6).

FIG. 3 shows percentage changes of target lesions in patients before and after treatment with ADG106. Each line represents an individual patient. Green lines denote patients with tumor shrinkage (n=7); red line denotes patient with slight tumor shrinkage after initial enlargement; yellow lines denote other patients.

FIG. 4 shows PET CT images of a patient with follicular lymphoma. The left panel shows a PET CT image before treatment; the right panel shows a PET CT image at week six after treatment.

FIG. 5 shows receptor occupancy of ADG106 at different dose levels.

FIG. 6A shows mean serum concentrations of ADG106 in treatment cycle 1 in patients dosed with 0.03, 0.1, 0.3, 1, 3, 5, or 10 mg/kg ADG106 in the U.S. clinical study. FIG. 6B shows mean serum concentrations of ADG106 in treatment cycle 1 in patients dosed with 0.1, 0.5, 1.5, 3, 5, or 10 mg/kg ADG106 in the Chinese clinical study.

FIG. 7 shows mean serum concentrations of ADG106 in male and female cynomolgus monkeys after weekly repeat doses of 50, 100, or 200 mg/kg ADG 106.

FIG. 8A-8B show kinetics of Ki-67 expression on peripheral CD4+ and CD8+ T cells of patient R011 in stable disease with total dose of 170 mg vs the similar curve by Pembrolizumab with a total dose of 200mg from literature report. FIG. 8A show kinetics of Ki-67 expression on peripheral CD4+ and CD8+ T cells from patient R011 dosed at 3mg/kg with a body weight of 57 kg, with a total dose of 170mg per cycle of ADG106. FIG. 8B show kinetics of Ki-67 expression on

EP 4 559 477 A2

peripheral CD4+ and CD8+ T cells from one patient treated with a total dose of 200mg per cycle of Pembrolizumab. Cell proliferating antigen Ki-67 was stained in peripheral T cell samples pre-dose (day 0) and post-dose (21, 42, 63, 84, 105 days) and analyzed by flow cytometry. Both curves match in trend to stimulate T cell proliferation by ADG106 as Pembrolizumab clinically following multiple cycles of the drug treatment of 170mg of ADG106 every three weeks although its change in magnitude for CD8+ T cell proliferation is much stronger by ADG106 vs. Pembrolizumab.

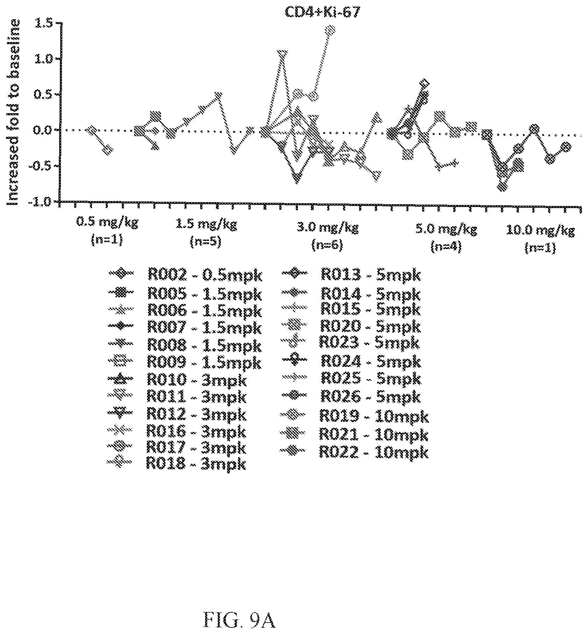

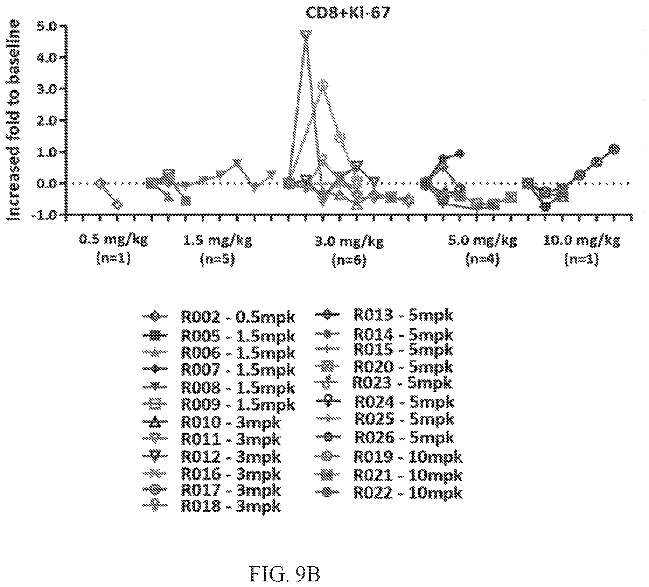

FIGS. 9A-9B show increased Ki-67 expression on peripheral CD4+ and CD8+ T cells of certain patients treated with high dose levels of ADG106 ranging from 0.5 mg/kg to 10mg/kg. FIG. 9A shows Ki-67 expression on peripheral CD4+ T cells from patients dosed with 0.5 mg/kg (n=1), 1.5 mg/kg (n=5), or 3mg/kg (n=3), 5 mg/kg (n=4), 10 mg/kg (n=2) of ADG106. FIG. 9B shows Ki-67 expression on peripheral CD8+ T cells from patients dosed at 0.5 mg/kg (n=1), 1.5 mg/kg (n=5), 3mg/kg (n=3), 5 mg/kg (n=4), 10 mg/kg (n=2) of ADG106 with 0.5 mg/kg (n=1), 1.5 mg/kg (n=5), or 3mg/kg (n=3) ADG106. It seems that the Ki67 expression on peripheral CD4+ and CD8+ T change as a function of dosage and specific patients with significant changes around 3 and 5 mg/kg with the patience samples dosed.

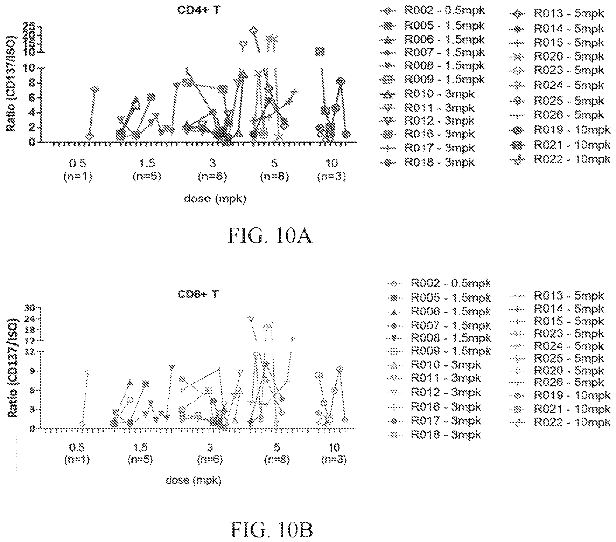

FIGS. 10A-10B show CD137 expression on CD4+ and CD8+ T cells of patients treated with ADG106 at different doses. FIG. 10A shows CD137 expression on peripheral CD4+ T cells from patients dosed with 0.5 mg/kg (n=1), 1.5 mg/kg (n=5), 3mg/kg (n=6), 5mg/kg (n=8) or 10mg/kg (n=3) ADG106. FIG. 10B shows CD137 expression on peripheral CD8+ T cells from patients dosed with 0.5 mg/kg (n=1), 1.5 mg/kg (n=5), 3mg/kg (n=3), 5mg/kg (n=8) or 10mg/kg (n=3) ADG106.

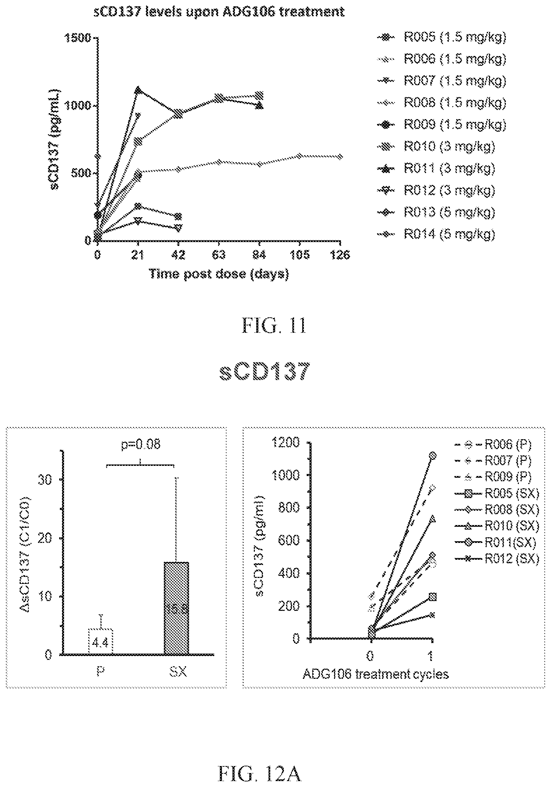

FIG. 11 shows soluble CD137 (sCD137) levels pre-dose (day 0) and post-dose (21, 42, 63, 84, 105, and 126 days) in patients treated with 1.5, 3, or 5 mg/kg ADG106 each cycle once every three weeks (Q3W). It is apparent that the soluble CD137 (sCD137) is induced and increased significantly and then level off as a function of dosage from 1.5 and 5 mg/kg with the patients.

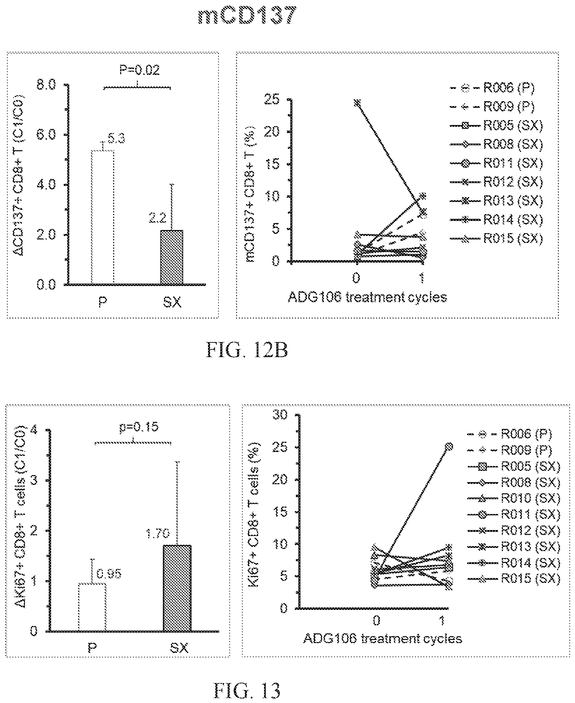

FIGS. 12A-12B show changes of CD137 levels in patients before and after receiving treatment with ADG106. FIG.12A shows the changes of soluble CD137 (sCD137) levels in patients pre-dose and post-dose. FIG.12B shows the changes of membrane-bound CD137 (mCD137) levels in patients pre-dose and post-dose. "P" denotes progression of diseases; "SX" denotes stable disease and followed by stable or progressive diseases, abbreviated as "SS" or "SP" respectively. The trend seems to indicate that mCD137 decreases for patients with stable disease vs patients with progressive disease up treatment of ADG 106.

FIG. 13 shows changes of Ki67+ CD8+ T cells levels in patients before and after receiving treatment with ADG106. "P" denotes progression of diseases; "SX" denotes stable disease, and followed by stable or progressive diseases, abbreviated as "SS" or "SP" respectively. The trend seems to indicate that Ki67+ CD8+ T cells increase for patients with stable disease vs patients with progressive disease up treatment of ADG106.

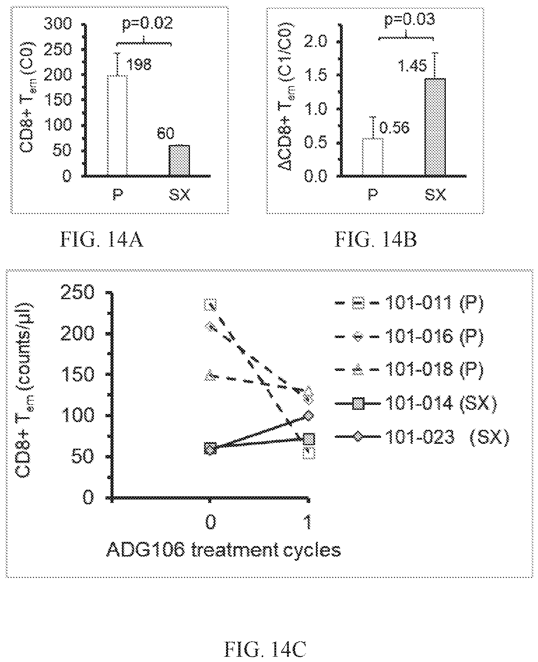

FIGS. 14A-14C show the levels of CD8+ effector memory T cells ($T_{em}$) pre-dose (C0) and changes of CD8+ $T_{em}$ levels post-dose (C1) in patients receiving ADG106 treatment. FIG. 14A shows the pre-dose (C0) levels of CD8+ $T_{em}$ in patients that showed progression of disease (P) and patients that showed stable disease (SX). FIG. 14B shows the ratio of pre-dose (C0) and post-dose (C1) levels of CD8+ $T_{em}$ in patients that showed progression of disease (P) and patients that showed stable disease (SX). FIG. 14C shows the pre-dose (C0) and post-dose (C1) levels of CD8+ $T_{em}$ in patients that showed progression of disease (P) and patients that showed stable disease (SX), and followed by stable or progressive diseases, abbreviated in SS or SP etc.

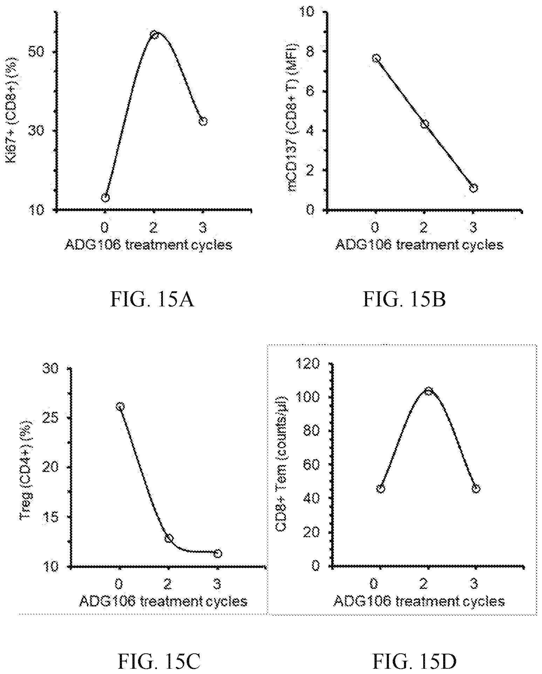

FIGS. 15A-15D show changes of Ki+CD8+ T cell percentages, membrane-bound CD137 levels on CD8+ T cells, CD4+ Treg percentages, CD8+ effector memory T cells $T_{em}$ in a patient (R017), who achieved >30% tumor size reduction ranging from 16% to 57% in 6 targeted lesions after receiving one cycle of ADG106 treatment. FIG. 15A shows changes of Ki+CD8+ T cell percentages through three treatment cycles. FIG. 15B shows changes of membrane-bound CD137 levels on CD8+ T cells through three treatment cycles. FIG. 15C shows changes of CD4+ $T_{reg}$ percentages through three treatment cycles. FIG. 15D shows changes of CD8+ effector memory T cells Tem through three treatment cycles.

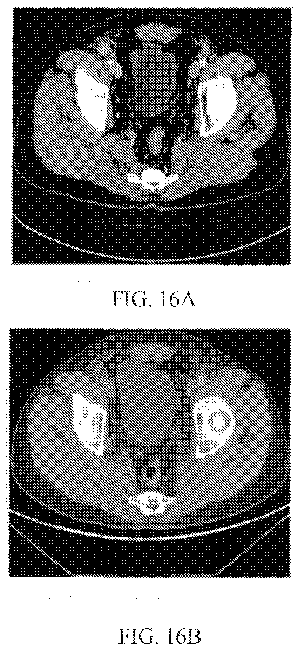

FIG. 16A-B show a target lesion by CT images before and after only one administration of ADG106 for patient with a stage III angioimmunoblastic T cell lymphoma. FIG. 16A show PET CT images before treatment (left panels). FIG. 16B show CT images at cycle 2 day 21 after only one administration of ADG106 (right panels). LDi denotes longest diameter; SDi denotes shortest diameter.

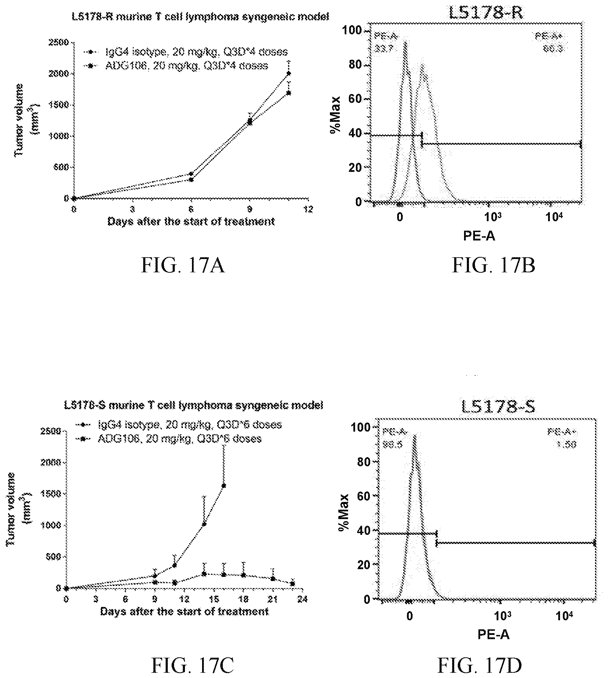

FIGS. 17A-17D show tumor volumes in L5178-R and L5178-S murine T cell lymphoma syngeneic models treated with isotype control antibody or ADG106. FIG. 17A shows tumor volumes up to 11 days after the start of treatment in L5178-R murine T cell lymphoma syngeneic model with IgG4 isotype control antibody or ADG106 at a dose of 20 mg/kg. FIG. 17B shows the staining patterns of the CD137 ligand expression in L5178-R cells. FIG. 17C shows tumor volume up to 23 days after the start of treatment in L5178-S murine T cell lymphoma syngeneic model with IgG4 isotype control antibody or ADG106 at dose of 20 mg/kg. FIG. 17D shows the staining patterns of the CD137 ligand expression in L5178-S cells.

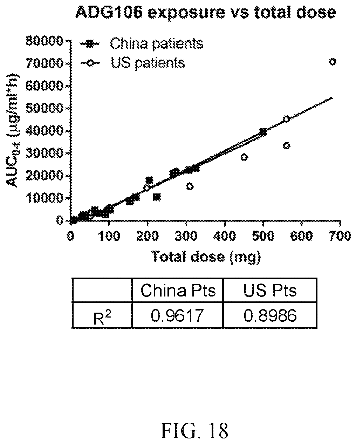

FIG. 18 shows ADG106 exposure in relation to total dose.

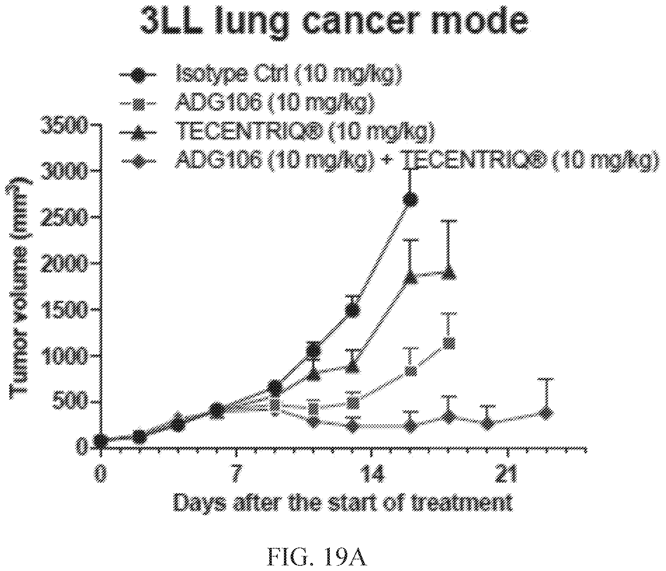

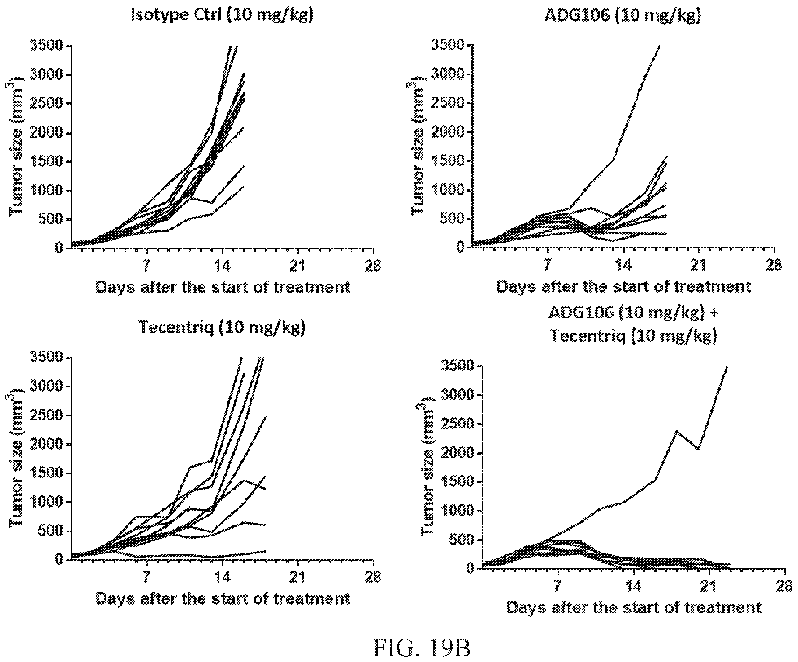

FIGs. 19A-19B show in vivo treatment effects of isotype control antibody, ADG106, TECENTRIQ® (atezolizumab), or a combination of ADG106 and TECENTRIQ® (atezolizumab) in mouse 3LL lung cancer model in C57BL/6 mice. The number of days post inoculation is shown on the x-axis, and the tumor volume in mm$^3$ is shown on the y-axis. FIG. 19A shows tumor growth curves of different treatment groups. Data points represent group mean, and error bars represent SEM. FIG. 19B shows tumor growth curves of individual mice in each tested group.

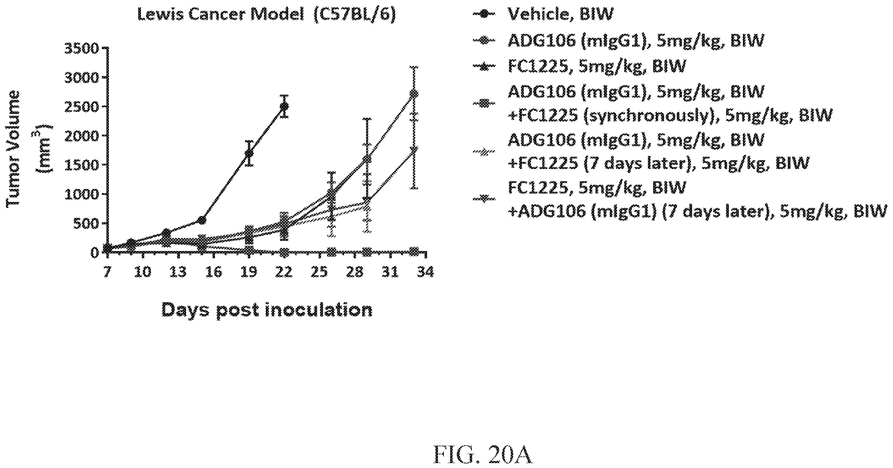

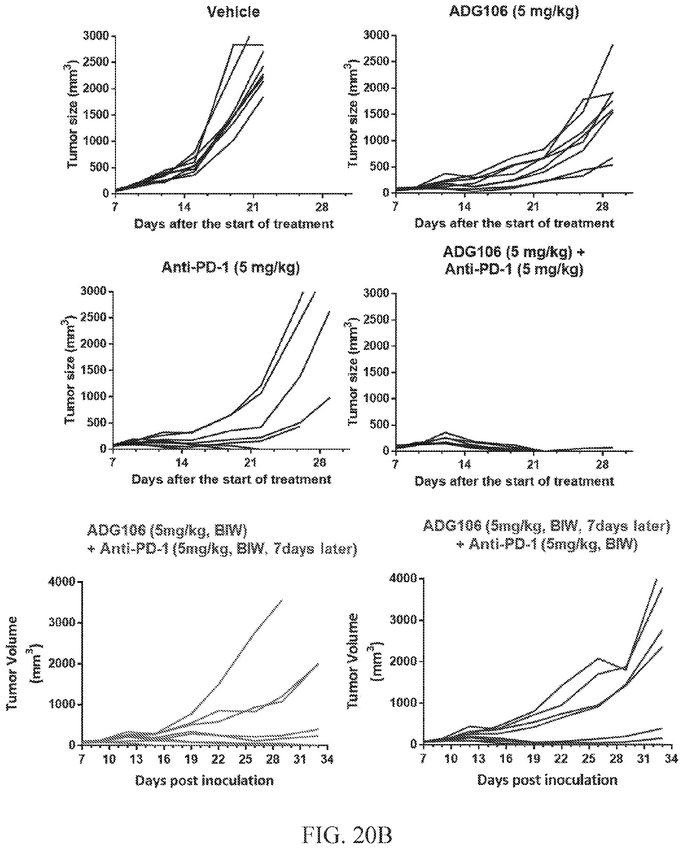

FIGs. 20A-20B show *in vivo* treatment effects of vehicle, ADG106, an anti-PD-1 antibody, synchronous administration of a combination of ADG106 and anti-PD-1 antibody, administrate ADG106 followed by anti-PD-1 antibody after 7 days, or administrate anti-PD-1 antibody followed by ADG106 after 7 days in mouse Lewis lung cancer model in C57BL/6 mice. FIG. 20A shows tumor growth curves of different treatment groups. Data points represent group mean, and error bars represent SEM. FIG. 20B shows tumor growth curves of individual mice in each tested group.

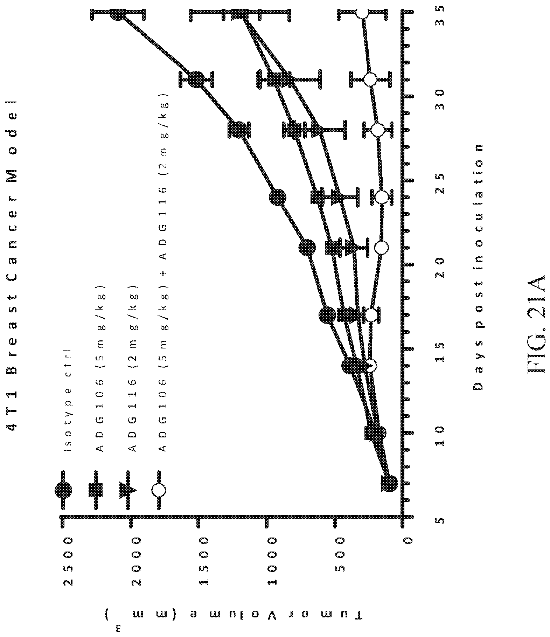

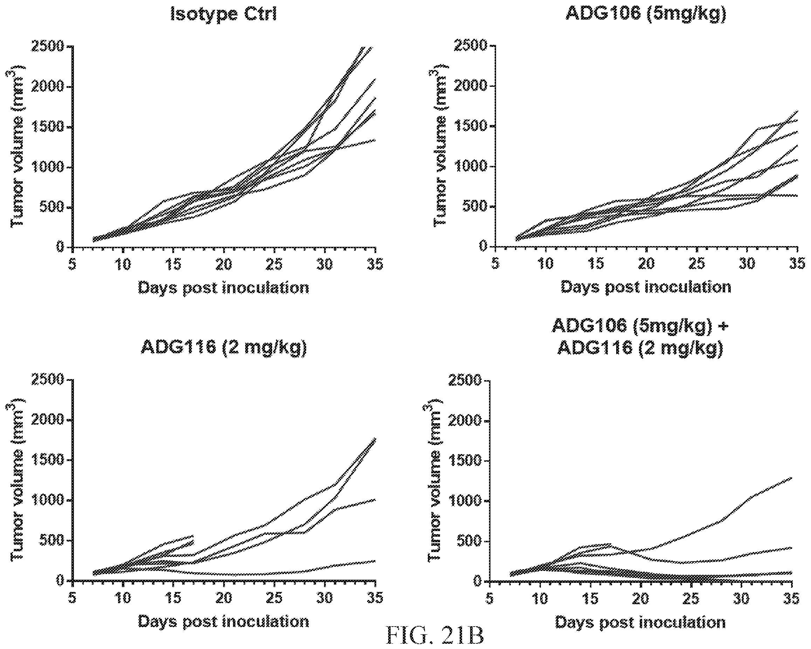

FIGs. 21A-21B show *in vivo* treatment effects of isotype control antibody, ADG106, ADG116 (anti-CTLA-4 antibody), or a combination of ADG106 and ADG116 in mouse 4T1 breast cancer model in C57BL/6 mice. FIG. 21A shows tumor growth curves of different treatment groups. Data points represent group mean, and error bars represent SEM. FIG. 21B shows tumor growth curves of individual mice in each tested group.

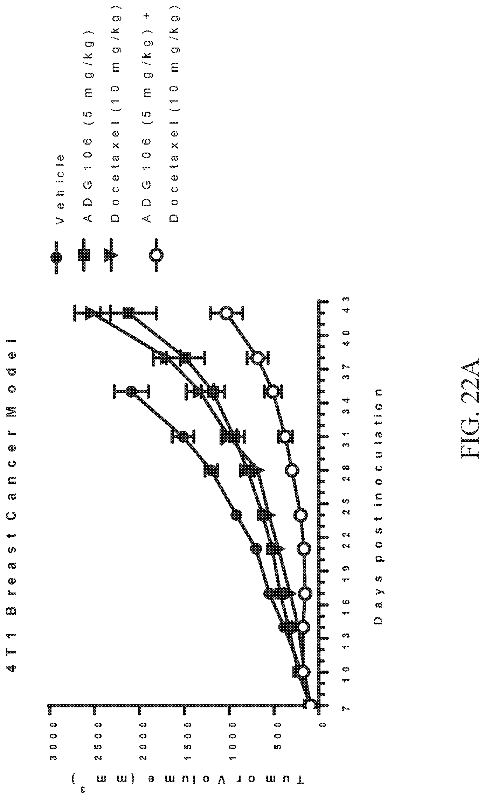

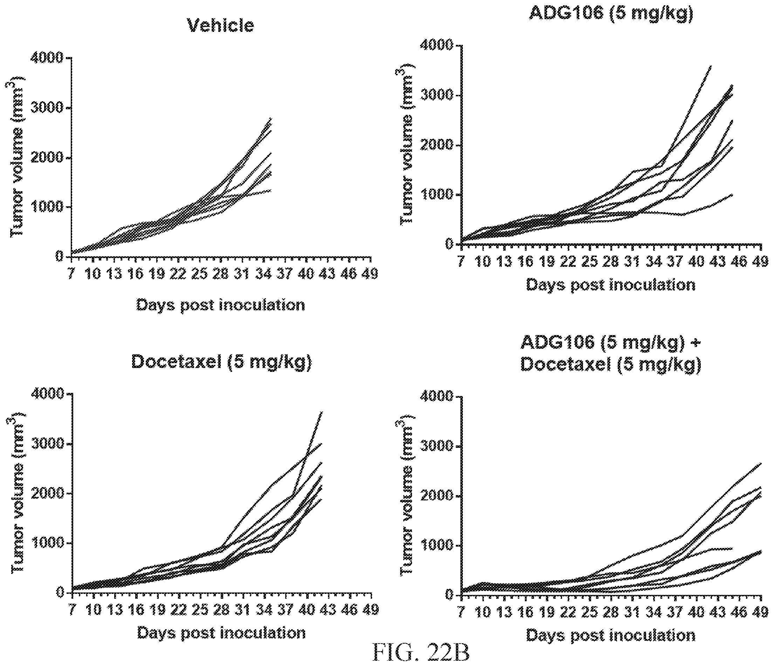

FIGs. 22A-22B show *in vivo* treatment effects of vehicle, ADG106, docetaxel, or a combination of ADG106 and docetaxel in mouse 4T1 breast cancer model in C57BL/6 mice. FIG. 22A shows tumor growth curves of different treatment groups. Data points represent group mean, and error bars represent SEM. FIG. 22B shows tumor growth curves of individual mice in each tested group.

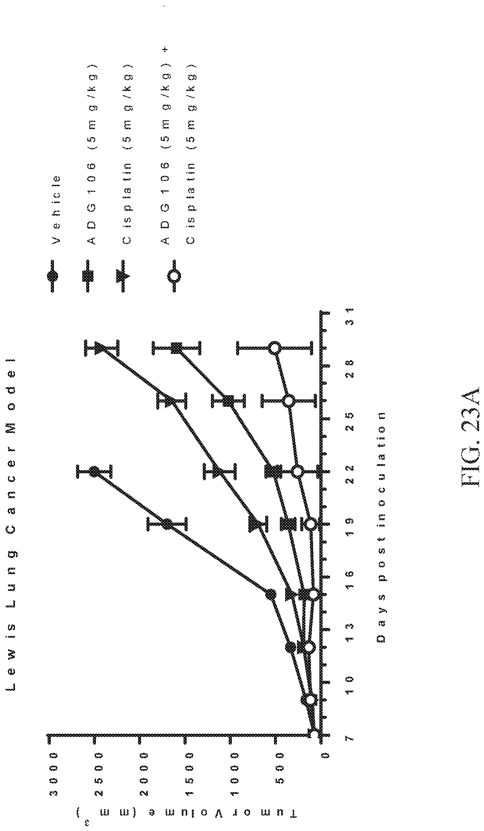

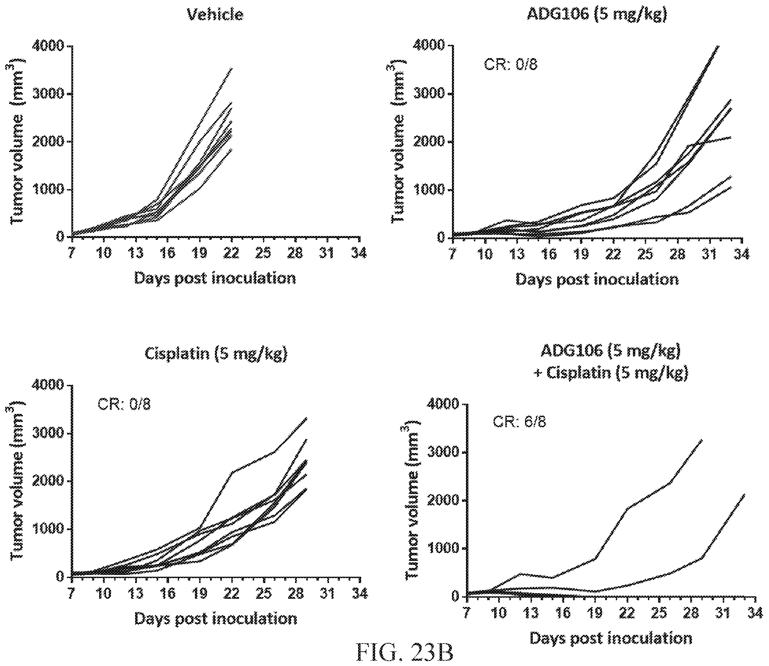

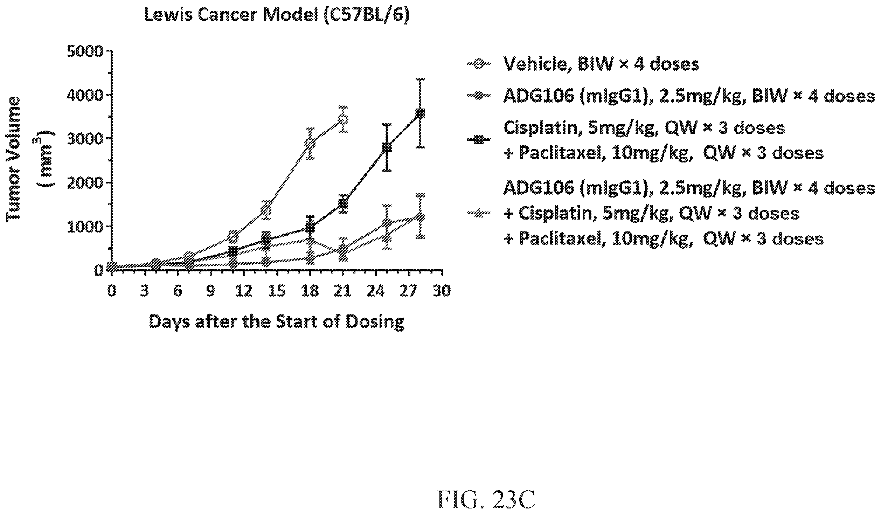

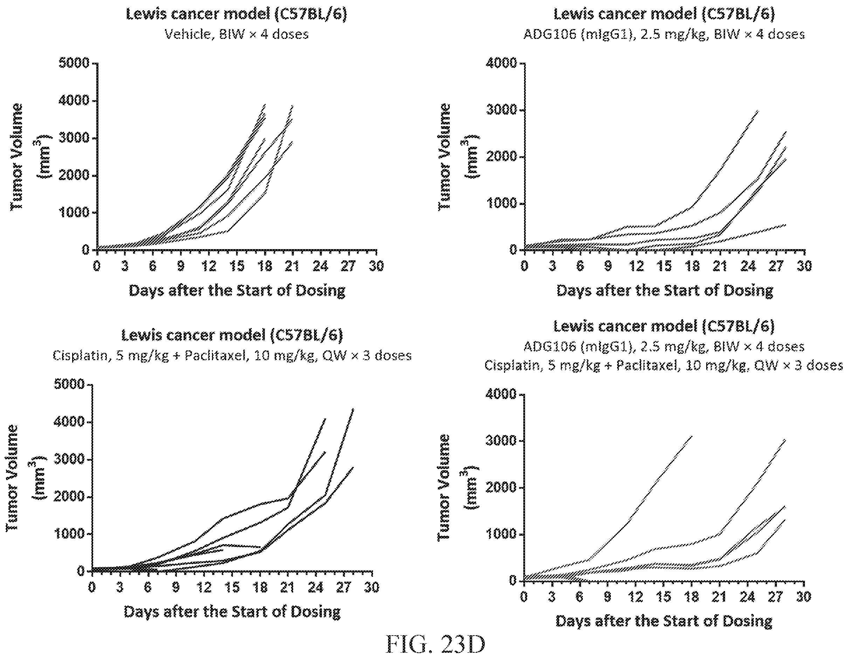

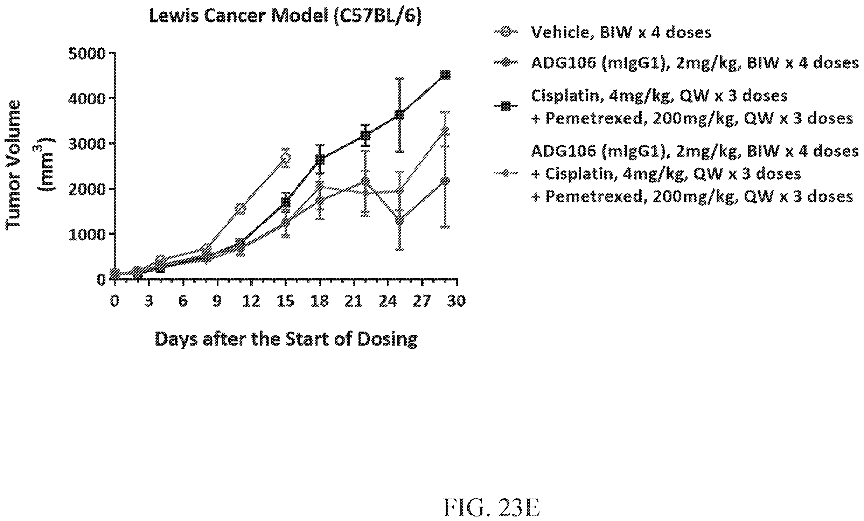

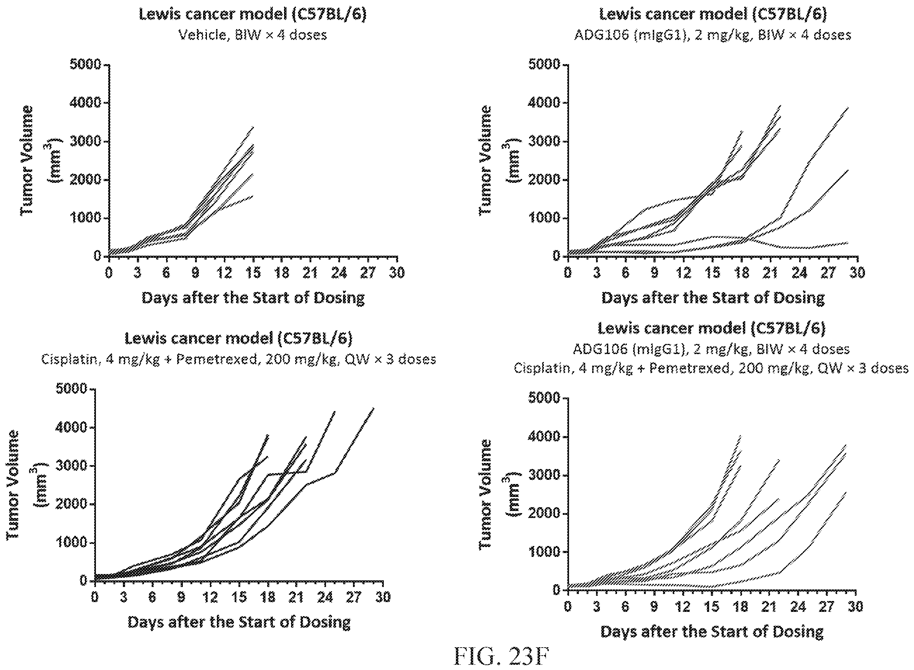

FIGs. 23A-23F show *in vivo* treatment effects of vehicle, ADG106, cisplatin, a combination of ADG106 and cisplatin, a combination of cisplatin and Paclitaxel, a combination of ADG106, cisplatin, and Paclitaxel, a combination of cisplatin and Pemetrexed, or a combination of ADG 106, cisplatin, and Pemetrexed in mouse Lewis lung cancer model in C57BL/6 mice. FIG. 23A shows tumor growth curves of vehicle, ADG106, cisplatin, or a combination of ADG106 and cisplatin treatment groups. Data points represent group mean, and error bars represent SEM. FIG. 23B shows tumor growth curves of individual mice in vehicle, ADG106, cisplatin, or a combination of ADG106 and cisplatin treatment group. FIG. 23C shows tumor growth curves of vehicle, ADG106, cisplatin and Paclitaxel, or a combination of ADG106, cisplatin, and Paclitaxel treatment groups. Data points represent group mean, and error bars represent SEM. FIG. 23D shows tumor growth curves of individual mice in vehicle, ADG106, cisplatin and Paclitaxel, or a combination of ADG106, cisplatin, and Paclitaxel treatment group. FIG. 23E shows tumor growth curves of vehicle, ADG106, cisplatin and Pemetrexed, or a combination of ADG106, cisplatin, and Pemetrexed treatment groups. Data points represent group mean, and error bars represent SEM. FIG. 23F shows tumor growth curves of individual mice in vehicle, ADG106, cisplatin and Pemetrexed, or a combination of ADG106, cisplatin, and Pemetrexed treatment group.

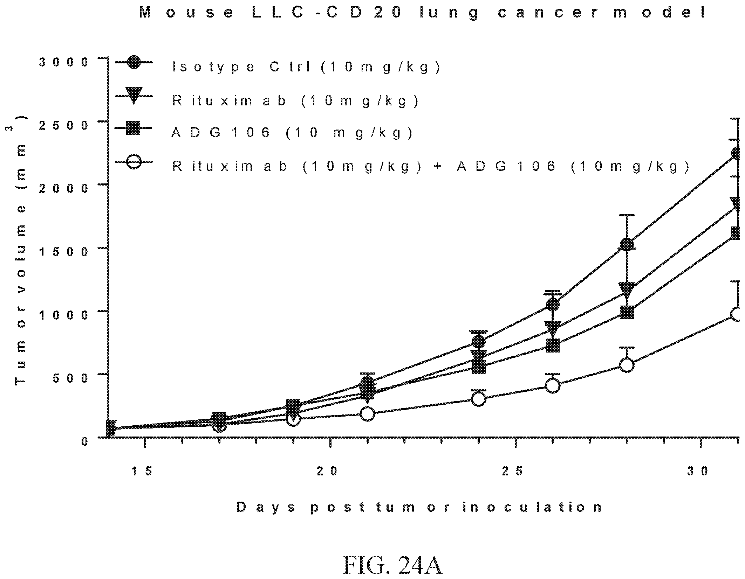

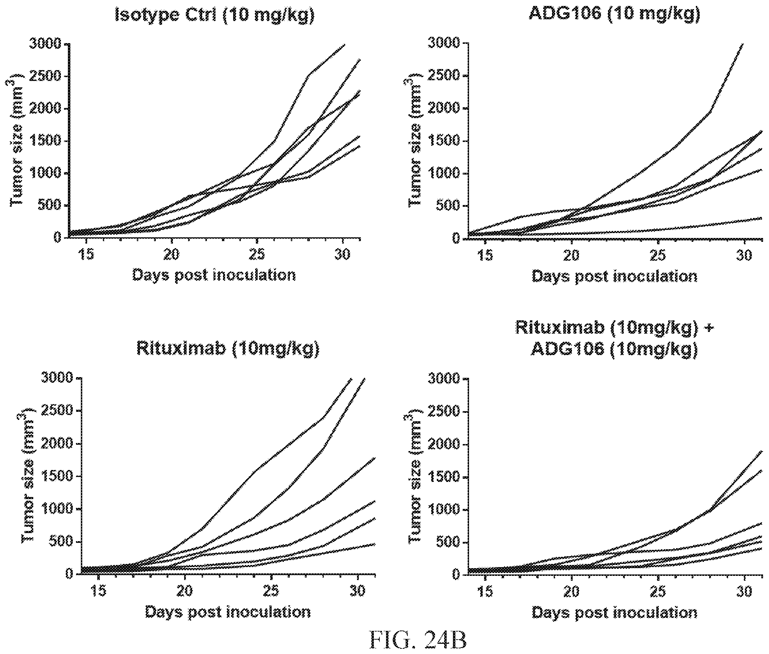

FIGs. 24A-24B show *in vivo* treatment effects of isotype control antibody, ADG106, anti-CD20 antibody (Rituximab), or a combination of ADG106 and Rituximab in a mouse Lewis lung cancer model stably transfected with human CD20 in C57BL/6 mice. The number of days post inoculation is shown on the x-axis, and the volume of the tumor in mm$^3$ is shown on the y-axis. FIG. 24A shows tumor growth curves of different treatment groups. Data points represent group mean, and error bars represent SEM. FIG. 24B shows tumor growth curves of individual mice in each tested group.

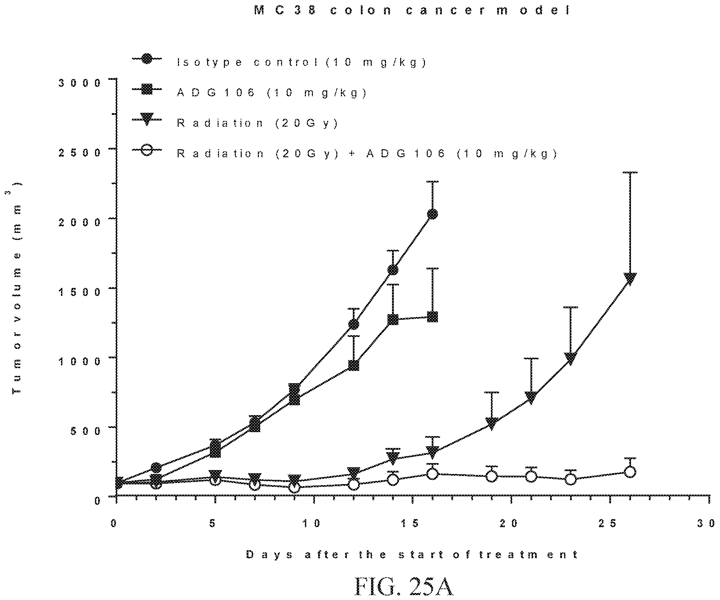

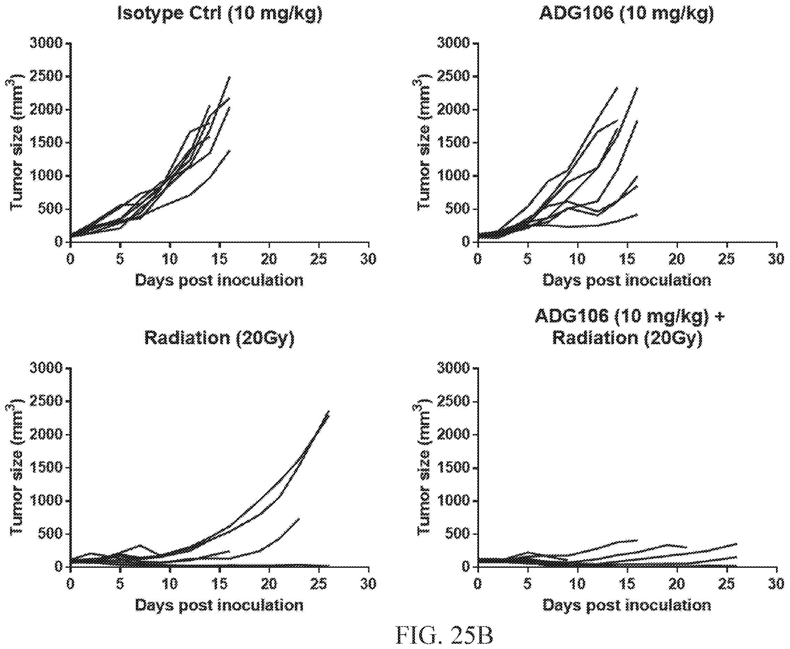

FIGs. 25A-25B show *in vivo* treatment effects of isotype control antibody, local radiation, ADG106, or a combination of local radiation and ADG106 in mouse MC38 colon cancer model in C57BL/6 mice. The number of days after the start of treatment is shown on the x-axis, and the volume of the tumor in mm$^3$ is shown on the y-axis. FIG. 25A shows tumor growth curves of different treatment groups. Data points represent group mean, and error bars represent SEM. FIG. 25B shows tumor growth curves of individual mice in each tested group.

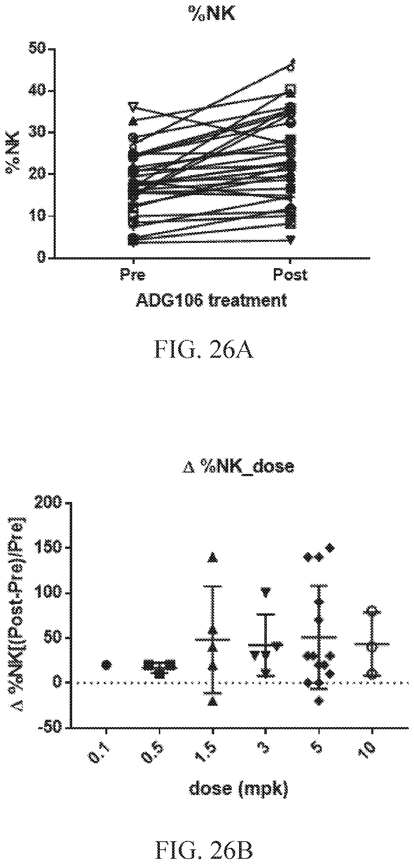

FIGs. 26A-26B show changes of NK cell percentages in patients treated with ADG106. FIG. 26A shows NK cell percentages before and after ADG106 treatment. FIG. 26B shows changes of NK cell percentages in patients dosed with 0.1, 0.5, 1.5, 3, 5, or 10 mg/kg ADG106.

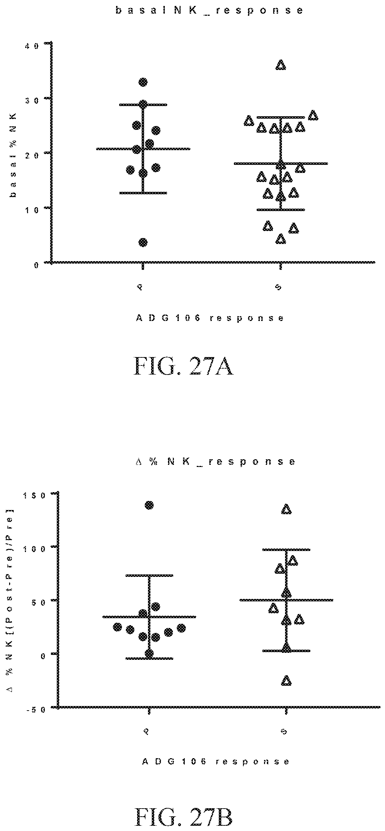

FIGs. 27A-27B show comparison of the base line NK cell percentages and changes in NK cell percentages in patients whose cancer progressed (P) versus patients with stable disease (S) after treatment of ADG106. FIG. 27A shows baseline base line NK cell percentages in patients whose cancer progressed versus patients with stable disease after treatment of ADG106. FIG.27B shows changes in NK cell percentages in patients whose cancer progressed versus patients with stable disease after treatment of ADG106.

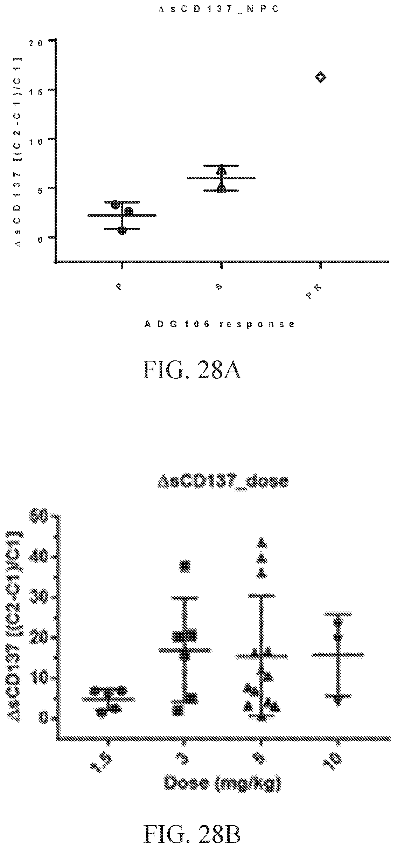

FIGs. 28A-28B show correlations of sCD137 serum level in nasopharyngeal cancer (NPC) patients who showed progression of disease (P), stable disease (S), or partial response (PR). FIG. 28A shows changes of sCD137 expression levels, which are expressed as ratios of induced changes in soluble CD137 expression levels after treatment (C2-C1) to baseline sCD137 expression levels before treatment (C1) in patients who showed progression of disease (P), stable disease (S), or partial response (PR) after treatment of ADG106. FIG. 28B shows changes in sCD137 expression levels, which are expressed as ratios of induced changes in soluble CD137 expression levels after treatment (C2-C1) to baseline sCD137 expression levels (C1) before treatment in patients dosed with 1.5, 3, 5, or 10 mg/kg ADG106.

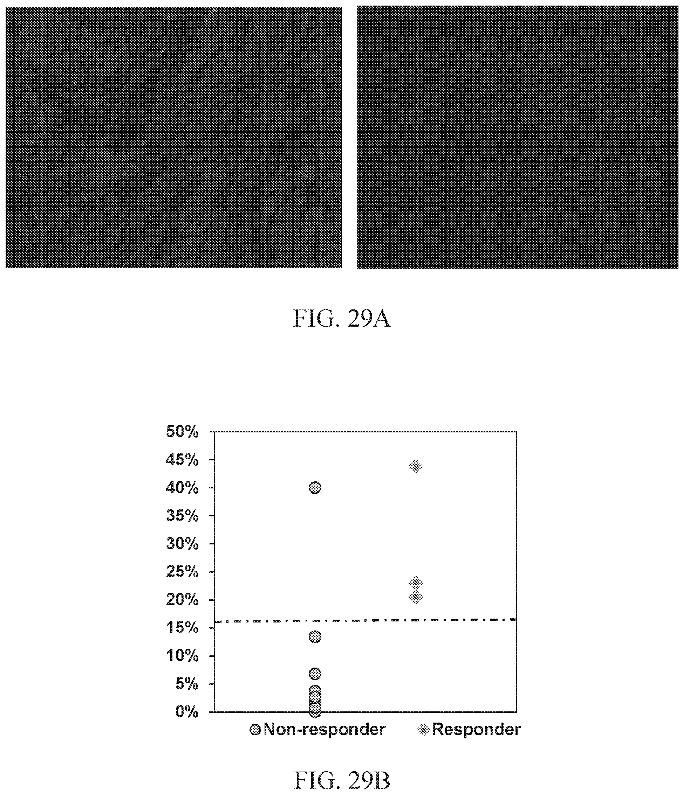

FIGs. 29A-29B show comparison of CD137L protein expression levels between patients that responded to ADG106 treatment (responders) and patients that did not respond to ADG106 treatment (non-responders). FIG. 29A shows formalin-fixed paraffin-embedded (FFPE) specimens staining of CD137L protein in a responder (left) versus a non-responder (right). FIG.29B shows analysis of CD137L protein expression in the responders (diamonds) and non-responders (circles). The dotted line shows the tentative cut-off value of 18%. The outlier in the non-responder group was a patient with non-small cell lung cancer (NSCLC) who achieved stable disease after 0.5 mg/kg ADG106 treatment.

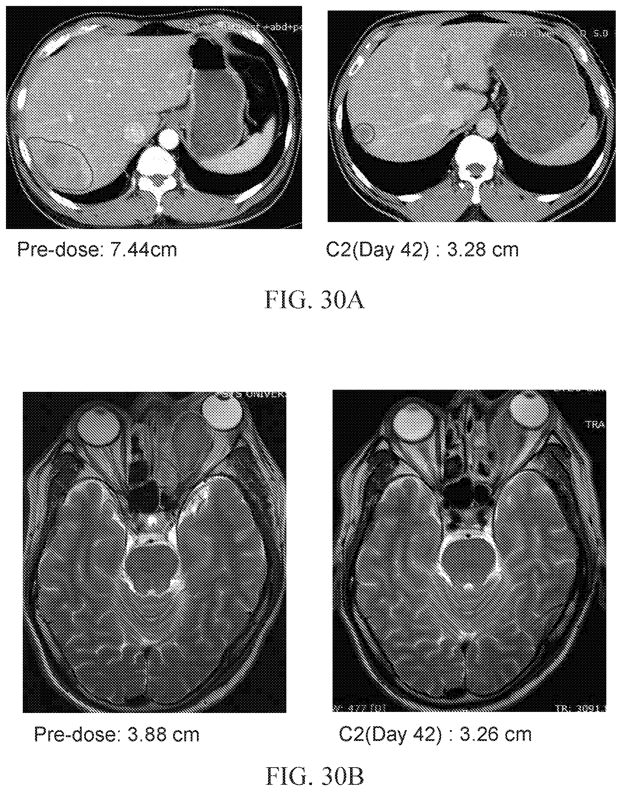

FIGs. 30A-30B show CT scans of liver and left ethmoid sinus lesions of a nasopharyngeal carcinoma (NPC) patient who had 40% tumor reduction after two cycles of ADG106 treatment. FIG. 30A shows liver lesion of the patient before treatment (left) and after two cycles of ADG106 treatment at day 42 (right). FIG. 30B shows left ethmoid sinus lesion of the patient before treatment (left) and after two cycles of ADG1 06 treatment at day 42 (right).

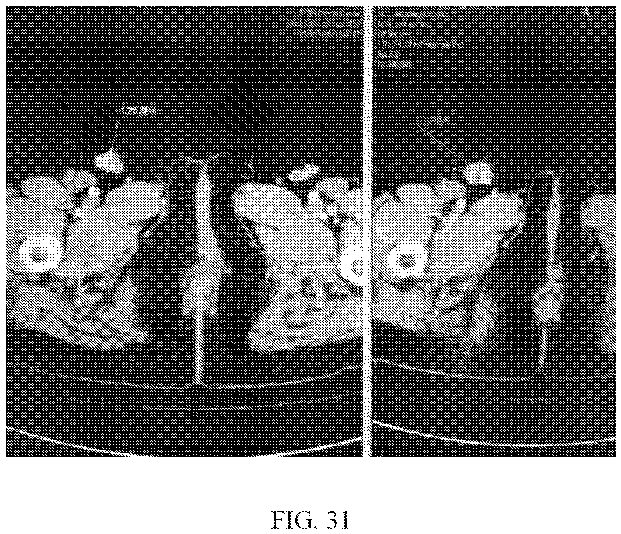

FIG. 31 shows CT scan of a lymphoma patient before treatment (left) and after two cycles of ADG106 treatment (right). The patient achieved 32% tumor reduction.

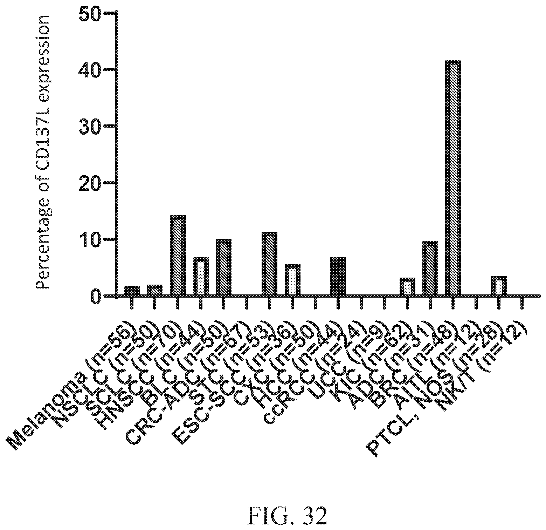

FIG. 32 shows CD137L expression in 746 Tumor microarray samples.

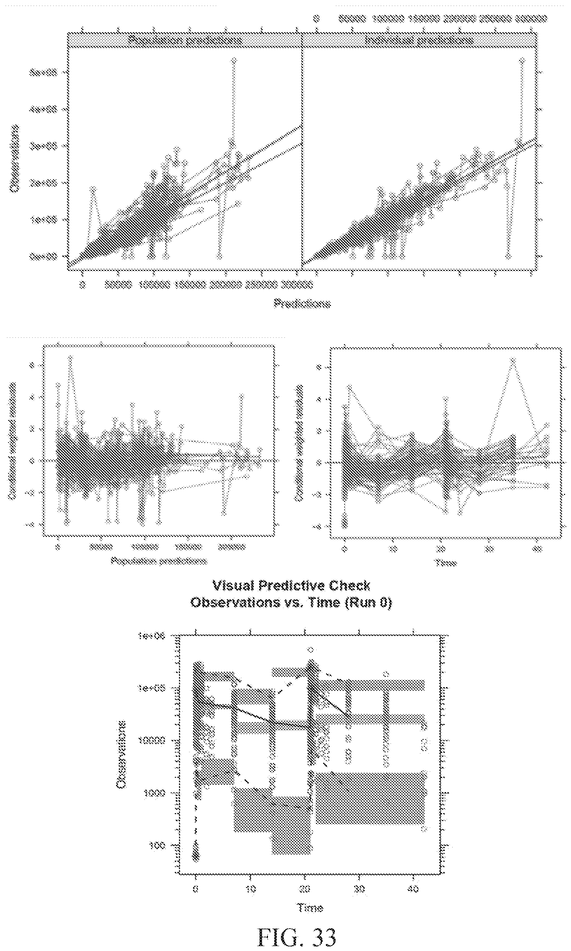

FIG. 33 shows Goodness-of-fit plots and Visual Predictive Check (VPC) of population pharmacokinetic modeling of ADG106 in ADG106-1001 and ADG106-1002 trials.

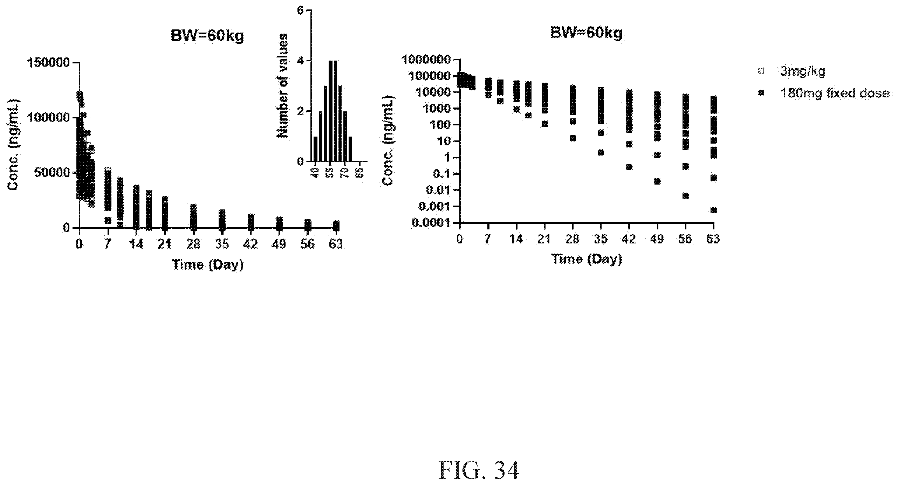

FIG. 34 shows simulated PK profiles of ADG106 for 20 virtual patients. The left and right panels show time vs. concentrations at each time point of the for the virtual patients dosed at 3mg/kg BW-based dosing vs. 180mg fixed IV dosing. The middle panel shows body weight (BW) distribution histogram of the 20 virtual patients, with mean body weight of 60 kg and body weight standard deviation (SD) of 10 %.

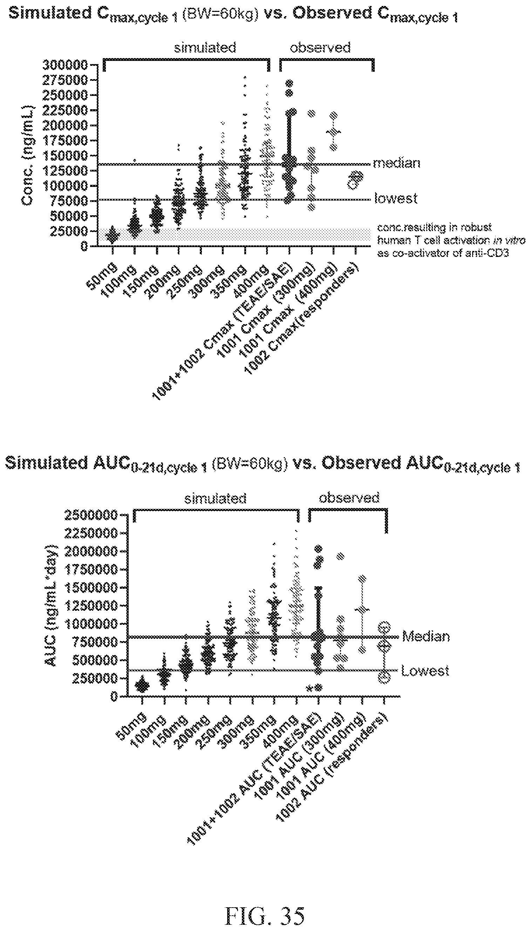

FIG. 35 shows simulated $C_{max}$ and $AUC_{0-21day}$ after the 1st dose (cycle 1) for a virtual patent population (BW=60kg, 100 subjects) vs. observed $C_{max}$ and $AUC_{0-21d}$ associated with $\geq$ G3 TEAEs and DLTs in the US and China ADG 106 Phase I clinical studies. Additional data from 1) the 300mg and 400mg fixed dose in the US ADG 106 Phase I clinical study, and 2) three responders in the China ADG 106 Phase I clinical study were plotted. Median +/- interquartile range for the simulated or observed data were shown. For the AUC plot, * represents one subject who only had concentration data till 24-hour post dosing and therefore the data was excluded as the lowest AUC value for benchmarking against simulated values.

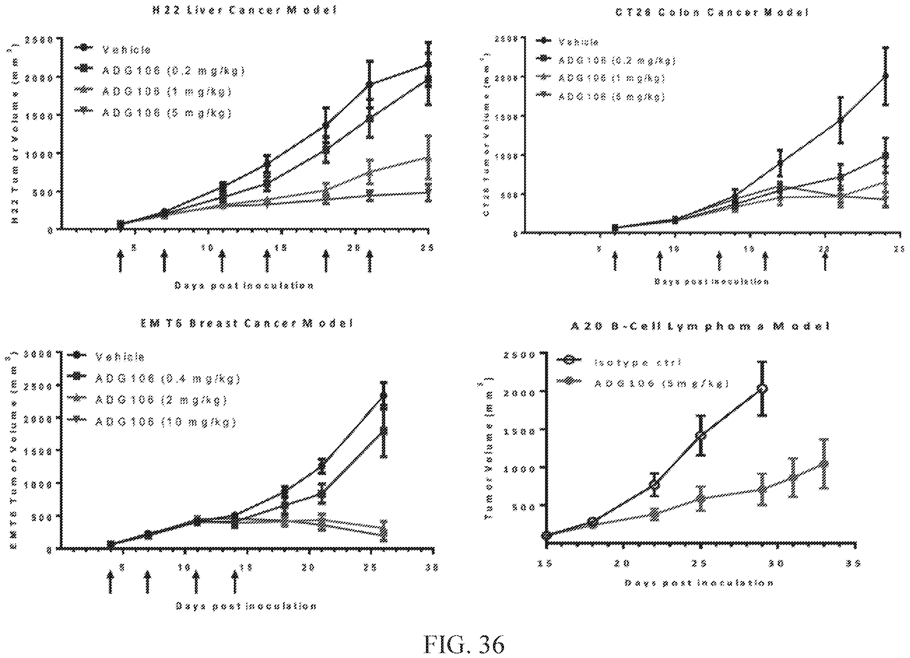

FIG. 36 shows *in vivo* anti-tumor efficacy of ADG106 single agent in different mouse tumor models.

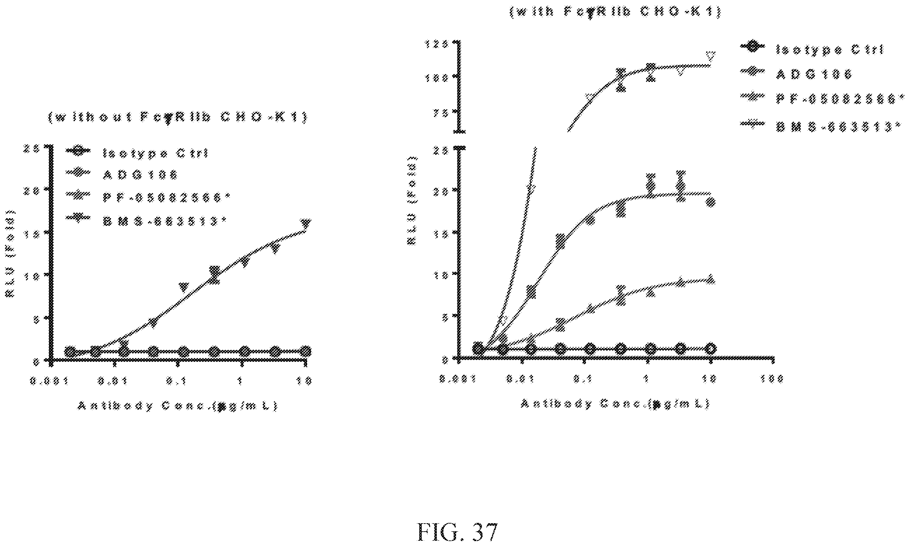

FIG. 37 shows activation of CD137 receptor signaling. The CD137-expressing NFκB-Luc Jurkat reporter cells were stimulated with the anti-CD137 antibodies in the absence (left panel) or presence (right panel) of co-cultured CHO-K1 cell expressing human FcyRIIb. Luciferase activity was measured by bioluminescence assay.

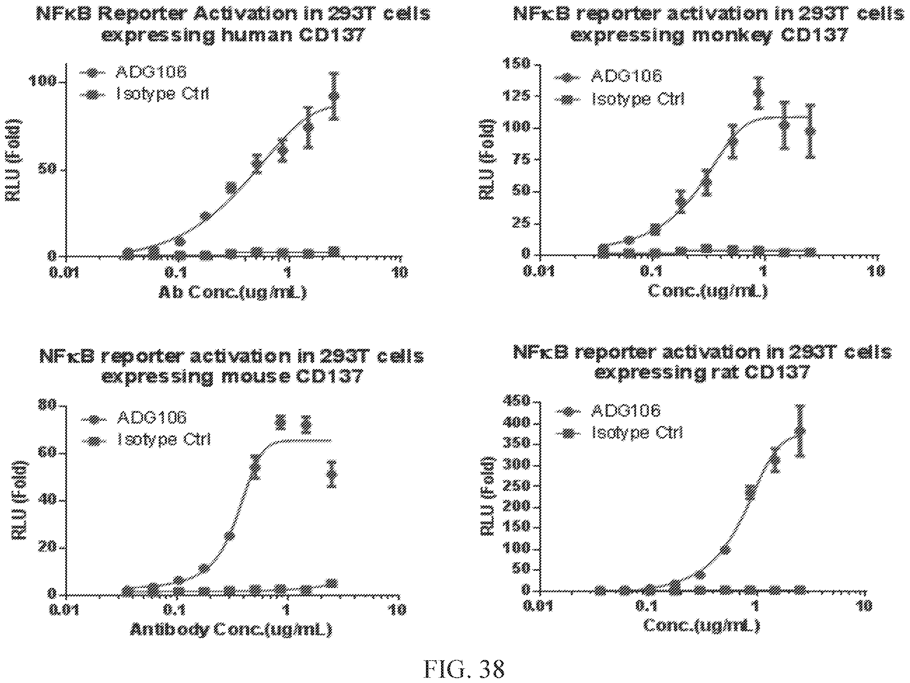

FIG. 38 shows ADG106 activated NFκB signaling stimulation through CD137 of different species.

## DETAILED DESCRIPTION

The present application provides a method of treating a cancer in a subject using an anti-CD137 antibody that specifically binds to an extracellular domain of human CD137. The anti-CD137 antibodies described herein specifically binds an epitope that mimics the binding site of CD137L. Administration of the anti-CD137 antibody lead to high receptor occupancy at a therapeutically effective dose. In some embodiments, the anti-CD137 antibody is administered at a dose of no more than 500 mg (*e.g.,* about 300-400 mg). The methods described herein can be used to treat a variety of cancers, including follicular lymphoma, T cell lymphoma, and adenoid cystic carcinoma (ACC). The methods described herein can be used to treat cancers that are advanced-stage cancer, metastatic cancer, and/or resistant or refractory to standard therapies. In some embodiments, the method comprises determining the levels of one or more biomarkers (e.g., prognostic biomarkers) selected from the group consisting of total CD137, membrane bound (mCD137), soluble CD137 (sCD137), CD137L, Ki67, NK cells, CD8+ effector memory T ($T_{em}$) cells, and regulatory T ($T_{reg}$) cells.

Accordingly, the present application in one aspect provides a method of treating a cancer in a subject, comprising administering to the subject an effective amount of an anti-CD137 antibody that specifically binds to an extracellular domain of human CD137, wherein the antibody binds to one or more amino acid residues selected from the group consisting of amino acid residues 51, 53, 62-73, 83, 89, 92, 95-104 and 112-116 of SEQ ID NO: 1, and wherein the anti-CD137 antibody is administered at a dose of no more than 500 mg.

In one aspect, there is provided a method of treating a cancer in a subject, comprising administering to the subject an effective amount of an anti-CD137 antibody that specifically binds to an extracellular domain of human CD137, wherein the antibody binds to one or more amino acid residues selected from the group consisting of amino acid residues 51, 53, 62-73, 83, 89, 92, 95-104 and 112-116 of SEQ ID NO: 1, and wherein the cancer is resistant or refractory to a prior therapy, *e.g.,* a prior immunotherapy such as an anti-CD20 antibody. In some embodiments, the prior therapy is rituximab.

In one aspect, there is provided a method of providing a prognosis for a subject who has been administered with an effective amount of an anti-CD137 antibody that specifically binds to an extracellular domain of human CD137, wherein the antibody binds to one or more amino acid residues selected from the group consisting of amino acid residues 51, 53, 62-73, 83, 89, 92, 95-104 and 112-116 of SEQ ID NO: 1; the method comprising determining a level of one or more biomarkers selected from the group consisting of total CD137, membrane bound (mCD137), soluble CD137 (sCD137), CD137L, Ki67, NK cells, CD8+ effector memory T ($T_{em}$) cells, regulatory T ($T_{reg}$) cells and NK cells in a sample of the subject, wherein an increased level of one or more biomarkers selected from the group consisting of total CD137, sCD137, Ki67, CD137L, NK cells, and CD8 $T_{em}$ cells, and/or a decreased level of one or more biomarkers selected from the group consisting of mCD137 and Treg cells after administration of the anti-CD137 antibody compared to the level of the one or more biomarkers before administration of the anti-CD137 antibody identifies the subject as having a high likelihood of responding to the anti-CD137 antibody treatment.

In one aspect, there is provided a method of treating a cancer in a subject, comprising administering to the subject an effective amount of an anti-CD137 antibody that specifically binds to an extracellular domain of human CD137, wherein the antibody binds to one or more amino acid residues selected from the group consisting of amino acid residues 51, 53, 62-73, 83, 89, 92, 95-104 and 112-116 of SEQ ID NO: 1, and wherein the cancer is selected from the group consisting of follicular lymphoma, T cell lymphoma, and ACC.

In one aspect, there is provided a method of treating a cancer (e.g., lung cancer or breast cancer such as triple-negative breast cancer) in a subject, comprising administering to the subject an effective amount of an anti-CD137 antibody that specifically binds to an extracellular domain of human CD137, wherein the antibody binds to one or more amino acid residues selected from the group consisting of amino acid residues 51, 53, 62-73, 83, 89, 92, 95-104 and 112-116 of SEQ ID NO: 1, and an immune checkpoint inhibitor (e.g., an anti-PD-1 antibody, an anti-PD-L1 antibody, or an anti-CTLA-4 antibody). In some embodiments, the subject is selected for the treatment based on a level of one or more biomarkers selected from the group consisting of total CD137, membrane bound (mCD137), soluble CD137 (sCD137), CD137L, Ki67, NK cells, CD8+ effector memory T ($T_{em}$) cells, regulatory T ($T_{reg}$) cells and NK cells in a sample.

In one aspect, there is provided a method of treating a cancer (e.g., lung cancer or breast cancer such as triple-negative breast cancer) in a subject, comprising administering to the subject an effective amount of an anti-CD137 antibody that specifically binds to an extracellular domain of human CD137, wherein the antibody binds to one or more amino acid residues selected from the group consisting of amino acid residues 51, 53, 62-73, 83, 89, 92, 95-104 and 112-116 of SEQ ID NO: 1, and a chemotherapeutic agent (e.g., docetaxel or cisplatin).

## I. Definitions

Unless otherwise defined herein, scientific and technical terms used in connection with the present disclosure shall have the meanings that are commonly understood by those of ordinary skill in the art. Further, unless otherwise required by context, singular terms shall include pluralities and plural terms shall include the singular. Generally, nomenclatures used in connection with, and techniques of, antibody engineering, immunotherapy, cell and tissue culture, molecular biology, immunology, microbiology, genetics and protein and nucleic acid chemistry described herein are those well-known and commonly used in the art.

The terms "CD137" and "CD137 receptor" are used interchangeably in the present application, and include the human CD137 receptor, as well as variants, isoforms, and species homologs thereof. Accordingly, a binding molecule, as defined and disclosed herein, may also bind CD137 from species other than human. In other cases, a binding molecule may be completely specific for the human CD137 and may not exhibit species or other types of cross-reactivity.

The term "CD137 antibody" refers to an antibody, as defined herein, capable of binding to human CD137 receptor.

The term "antibody" is used herein in the broadest sense and specifically covers monoclonal antibodies (including full length monoclonal antibodies), polyclonal antibodies, multispecific antibodies (e.g., bispecific antibodies), and antibody fragments (e.g., a single-chain variable fragment or scFv) so long as they exhibit the desired biological activity.

The term "antibody" is an art-recognized term and may refer to an antigen-binding protein (i.e., immunoglobulin) having a basic four-polypeptide chain structure consisting of two identical heavy (H) chains and two identical light (L) chains. Each L chain is linked to an H chain by one covalent disulfide bond, while the two H chains are linked to each other by one or more disulfide bonds depending on the H chain isotype. Each heavy chain has, at the N-terminus, a variable region (abbreviated herein as VH) followed by a constant region. The heavy chain constant region is comprised of three domains, CH1, CH2 and CH3. Each light chain has, at the N-terminus, a variable region (abbreviated herein as VL) followed by a constant region at its other end. The light chain constant region is comprised of one domain, CL. The VL, is aligned with the VH and the CL is aligned with the first constant domain of the heavy chain (CH1). The pairing of a VH and VL together forms a single antigen-binding site. An IgM antibody consists of 5 of the basic heterotetramer units along with an additional polypeptide called J chain, and therefore contains 10 antigen binding sites, while secreted IgA antibodies can polymerize to form polyvalent assemblages comprising 2-5 of the basic 4-chain units along with J chain.

The VH and VL regions can be further subdivided into regions of hypervariability, termed hyper-variable regions (HVR) based on the structural and sequence analysis. HVRs are interspersed with regions that are more conserved, termed framework regions (FW). For comparison, the Kabat CDR definition by Yvonne Chen, et al. (Selection and Analysis of an Optimized Anti-VEGF Antibody: Crystal Structure of an Affinity-matured Fab in Complex with Antigen, J. Mol. Biol. (1999) 293, 865-881) is listed below. Each VH and VL is composed of three HVRs and four FWs, arranged from amino-terminus to carboxy-terminus in the following order: FW1, HVR1, FW2, HVR2, FW3, HVR3, FW4. Throughout the present disclosure, the three HVRs of the heavy chain are referred to as HVR_H1, HVR_H2, and HVR_H3. Similarly, the three HVRs of the light chain are referred to as HVR_L1, HVR_L2, and HVR_L3.

As used herein, the term "CDR" or "complementarity determining region" is intended to mean the non-contiguous antigen combining sites found within the variable region of both heavy and light chain polypeptides. These particular regions have been described by Kabat et al., J. Biol. Chem. 252:6609-6616 (1977); Kabat et al., U.S. Dept. of Health and Human Services, "Sequences of proteins of immunological interest" (1991); Chothia et al., J. Mol. Biol. 196:901-917 (1987); Al-Lazikani B. et al., J. Mol. Biol., 273: 927-948 (1997); MacCallum et al., J. Mol. Biol. 262:732-745 (1996); Abhinandan and Martin, Mol. Immunol., 45: 3832-3839 (2008); Lefranc M.P. et al., Dev. Comp. Immunol., 27: 55-77 (2003); and Honegger and Plückthun, J. Mol. Biol., 309:657-670 (2001), where the definitions include overlapping or subsets of amino acid residues when compared against each other. Nevertheless, application of either definition to refer to a CDR of an antibody or grafted antibodies or variants thereof is intended to be within the scope of the term as defined and used herein. The amino acid residues, which encompass the CDRs as defined by each of the above-cited references, are set forth below in Table A as a comparison. CDR prediction algorithms and interfaces are known in the art, including, for example, Abhinandan and Martin, Mol. Immunol., 45: 3832-3839 (2008); Ehrenmann F. et al., Nucleic Acids Res., 38: D301-D307 (2010); and Adolf-Bryfogle J. et al., Nucleic Acids Res., 43: D432-D438 (2015). The contents of the references cited in this paragraph are incorporated herein by reference in their entireties for use in the present invention and for possible inclusion in one or more claims herein.

### TABLE A: CDR DEFINITIONS

|  | Kabat[1] | Chothia[2] | MacCallum[3] | IMGT[4] | AHo[5] |
|---|---|---|---|---|---|
| VH CDR1 | 31-35 | 26-32 | 30-35 | 27-38 | 25-40 |
| VH CDR2 | 50-65 | 53-55 | 47-58 | 56-65 | 58-77 |
| VH CDR3 | 95-102 | 96-101 | 93-101 | 105-117 | 109-137 |
| VL CDR1 | 24-34 | 26-32 | 30-36 | 27-38 | 25-40 |
| VL CDR2 | 50-56 | 50-52 | 46-55 | 56-65 | 58-77 |
| VL CDR3 | 89-97 | 91-96 | 89-96 | 105-117 | 109-137 |

[1]Residue numbering follows the nomenclature of Kabat *et al., supra*
[2]Residue numbering follows the nomenclature of Chothia *et al., supra*
[3]Residue numbering follows the nomenclature of MacCallum *et al., supra*
[4]Residue numbering follows the nomenclature of Lefranc *et al., supra*
[5]Residue numbering follows the nomenclature of Honegger and Plückthun, *supra*

The variable regions of the heavy and light chains contain a binding domain that interacts with an antigen. The constant regions of the antibodies may mediate the binding of the immunoglobulin to host tissues or factors, including various cells of the immune system (e.g., effector cells) and the first component (Clq) of the classical complement system. Within light and heavy chains, the variable and constant regions are joined by a "J" region of about 12 or more amino acids, with the heavy chain also *including* a "D" region of about 10 or more amino acids. See generally, Fundamental Immunology Ch. 7 (Paul, W., ed., 2nd ed. Raven Press, N.Y. (1989)).

*The* L chain from any vertebrate species can be assigned to one of two clearly distinct types, called kappa and lambda, based on the amino acid sequences of their constant domains. Depending on the amino acid sequence of the constant domain of their heavy chains (CH), antibodies can be assigned to different classes or isotypes. There are five classes of antibodies: IgA, IgD, IgE, IgG, and IgM, having heavy chains designated $\alpha$ (alpha), $\delta$ (delta), $\varepsilon$ (epsilon), $\gamma$ (gamma), and $\mu$ (mu), respectively. The IgG class of antibody can be further classified into four subclasses IgG1, IgG2, IgG3, and IgG4 by the gamma heavy chains, Y1-Y4, respectively.

"Fc region" as used herein refers to the polypeptide comprising the constant region of an antibody heavy chain excluding the first constant region immunoglobulin domain. For IgG, the Fc region may comprise immunoglobulin domains CH2 and CH3 and the hinge between CH1 and CH2.

*The* term "antibody derivative" or "derivative" of an antibody refers to a molecule that is capable of binding to the same antigen (e.g., CD137) that the antibody binds to and comprises an amino acid sequence of the antibody linked to an additional molecular entity. The amino acid sequence of the antibody that is contained in the antibody derivative may be a full-length heavy chain, a full-length light chain, any portion or portions of a full-length heavy chain, any portion or portions of the full-length light chain of the antibody, any other fragment(s) of an antibody, or the complete antibody. The additional molecular entity may be a chemical or biological molecule. Examples of additional molecular entities include chemical groups, amino acids, peptides, proteins (such as enzymes, antibodies), and chemical compounds. The additional molecular entity may have any utility, such as for use as a detection agent, label, marker, pharmaceutical or therapeutic agent. The amino acid sequence of an antibody may be attached or linked to the additional molecular entity by chemical coupling, genetic fusion, noncovalent association, or otherwise. The term "antibody derivative" also encompasses chimeric antibodies, humanized antibodies, and molecules that are derived from modifications of the amino acid sequences of an antibody (*e.g.,* a CD137 antibody), such as conservation amino acid substitutions, additions, and insertions.

As used herein, "sequence identity" between two polypeptide sequences indicates the percentage of amino acids that are *identical* between the sequences. The amino acid sequence identity of polypeptides can be determined conventionally using known computer programs such as Bestfit, FASTA, or BLAST (see, e.g. Pearson, Methods Enzymol. 183:63-98 (1990); Pearson, Methods Mol. Biol. 132:185-219 (2000); Altschul et al., J. Mol. Biol. 215:403-410 (1990); Altschul et al., Nucleic Acids Res. 25:3389-3402 (1997)). When using Bestfit or any other sequence alignment program to determine whether a particular sequence is, for instance, 95% identical to a reference amino acid sequence, the parameters are set such that the percentage of identity is calculated over the full length of the reference amino acid sequence and that gaps in homology of up to 5% of the total number of amino acid residues in the reference sequence are allowed. This aforementioned method in determining the percentage of identity between polypeptides is applicable to all proteins, fragments, or variants thereof disclosed herein.

The term "antigen-binding fragment" or "antigen binding portion" of an antibody refers to one or more portions of an antibody that retain the ability to bind to the antigen that the antibody bonds to (e.g., CD137). Examples of "antigen-binding fragment" of an antibody include (i) a Fab fragment, a monovalent fragment consisting of the VL, VH, CL and CH1 domains; (ii) a F(ab')2 fragment, a bivalent fragment comprising two Fab fragments linked by a disulfide bridge at the hinge region; (iii) a Fd fragment consisting of the VH and CH1 domains; (iv) a Fv fragment consisting of the VL and VH domains of a single arm of an antibody, (v) a dAb fragment (Ward et al., Nature 341:544-546 (1989)), which consists of a VH domain; and (vi) an isolated complementarity determining region (CDR).

The term "binding molecule" encompasses (1) antibody, (2) antigen-binding fragment of an antibody, and (3) derivative of an antibody, each as defined herein.

The term "binding CD137," "binds CD137," "binding to CD137," or "binds to CD137" refers to the binding of a binding molecule, as defined herein, to the human CD137 in an *in vitro* assay, such as a Biacore assay, with an affinity ($K_D$) of 100 nM or less.

The term "specifically binds" or "specifically binds to," in reference to the interaction of a binding molecule, as defined herein, (e.g., an antibody) with its binding partner (e.g., an antigen), refers to the ability of the binding molecule to discriminate between an antigen of interest from an animal species and the antigen orthologue from a different animal species under a given set of conditions. A CD137 binding molecule is said to specifically bind to human CD137 if it binds to human CD137 at an EC50 that is below 50 percent of the EC50 at which it binds CD137 of rat or mouse as determined in an *in vitro* assay. Binding specificity of an antibody can be determined using methods known in the art. Examples of such methods include FACS using PHA stimulated primary cells, Western blots, ELISA-, RIA-, ECL-, IRMA-tests and peptide scans.

The term "compete for binding" refers to the interaction of two antibodies in their binding to a binding target. A first antibody competes for binding with a second antibody if binding of the first antibody with its cognate epitope is detectably decreased in the presence of the second antibody compared to the binding of the first antibody in the absence of the second antibody. The alternative, where the binding of the second antibody to its epitope is also detectably decreased in the presence of the first antibody, can, but need not, be the case. That is, a first antibody can inhibit the binding of a second antibody to its epitope without that second antibody inhibiting the binding of the first antibody to its respective epitope. However, where each antibody detectably inhibits the binding of the other antibody with its cognate epitope, whether to the same, greater, or lesser extent, the antibodies are said to "cross-compete" with each other for binding of their respective epitope(s).

The term "epitope" refers to a part of an antigen to which an antibody (or antigen-binding fragment thereof) binds. Epitopes can be formed both from contiguous amino acids or noncontiguous amino acids juxtaposed by tertiary folding of a protein. Epitopes formed from contiguous amino acids are typically retained on exposure to denaturing solvents whereas epitopes formed by tertiary folding are typically lost on treatment with denaturing solvents. An epitope can include various numbers of amino acids in a unique spatial conformation. Methods of determining spatial conformation of epitopes include, for example, x-ray crystallography, 2-dimensional nuclear magnetic resonance, deuterium and hydrogen exchange in combination with mass spectrometry, or site-directed mutagenesis, or all methods used in combination with computational modeling of antigen and its complex structure with its binding antibody and its variants. See, e.g., Epitope Mapping Protocols in Methods in Molecular Biology, Vol. 66, G. E. Morris, Ed. (1996). Once a desired epitope of an antigen is determined, antibodies to that epitope can be generated, e.g., using the techniques described herein. The generation and characterization of antibodies may also elucidate information about desirable epitopes. From this information, it is then possible to competitively screen antibodies for binding to the same epitope. An approach to achieve this is to conduct cross-competition studies to find antibodies that competitively bind with one another, i.e., the antibodies compete for binding to the antigen. A high throughput process for "binning" antibodies based upon their cross-competition is described in PCT Publication No. WO 03/48731.

The term "human antibody" refers to an antibody in which the entire amino acid sequences of the light chains and heavy chains are from the human immunoglobulin genes. A human antibody may contain murine carbohydrate chains if produced in a mouse, in a mouse cell or in a hybridoma derived from a mouse cell. Human antibodies may be prepared in a variety of ways known in the art.

The term "humanized antibody" refers to a chimeric antibody that contains amino acid residues derived from human antibody sequences. A humanized antibody may contain some or all of the CDRs or HVRs from a non-human animal or synthetic antibody while the framework and constant regions of the antibody contain amino acid residues derived from human antibody sequences.

The term "chimeric antibody" refers to an antibody that comprises amino acid sequences derived from different animal species, such as those having a variable region derived from a human antibody and a murine immunoglobulin constant region.

The term "isolated antibody" or "isolated binding molecule" refers to an antibody or a binding molecule, as defined herein, that: (1) is not associated with naturally associated components that accompany it in its native state; (2) is free of other proteins from the same species; (3) is expressed by a cell from a different species; or (4) does not occur in nature. Examples of isolated antibodies include a CD137 antibody that has been affinity purified using CD137, a CD137 antibody that has been generated by hybridomas or other cell line *in vitro,* and a CD137 antibody derived from a transgenic animal.

The term "isolated nucleic acid" refers to a nucleic acid molecule of genomic, cDNA, or synthetic origin, or a combination thereof, which is separated from other nucleic acid molecules present in the natural source of the nucleic acid. For example, with regard to genomic DNA, the term "isolated" includes nucleic acid molecules, which are separated from the chromosome with which the genomic DNA is naturally associated. Preferably, an "isolated" nucleic acid is free of sequences, which naturally flank the nucleic acid *(i.e.,* sequences located at the 5' and 3' ends of the nucleic acid of interest.

An "individual" or a "subject" is a mammal, more preferably a human. Mammals also include, but are not limited to, farm animals, sport animals, pets (such as cats, dogs, and horses), primates, mice and rats.

The term "treat", "treating", or "treatment", with reference to a certain disease condition in a mammal, refers causing a desirable or beneficial effect in the mammal having the disease condition. The desirable or beneficial effect may include reduced frequency or severity of one or more symptoms of the disease *(i.e.,* tumor growth and/or metastasis, or other effect mediated by the numbers and/or activity of immune cells, and the like), or arrest or inhibition of further development of the disease, condition, or disorder. In the context of treating cancer in a mammal, the desirable or beneficial effect may include inhibition of further growth or spread of cancer cells, death of cancer cells, inhibition of reoccurrence of cancer, reduction of pain associated with the cancer, or improved survival of the mammal. The effect can be either subjective or objective. For example, if the mammal is human, the human may note improved vigor or vitality or decreased pain as subjective symptoms of improvement or response to therapy. Alternatively, the clinician may notice a decrease in tumor size or tumor burden based on physical exam, laboratory parameters, tumor markers or radiographic findings. Some laboratory signs that the clinician may observe for response to treatment include normalization of tests, such as white blood cell count, red blood cell count, platelet count, erythrocyte sedimentation rate, and various enzyme levels. Additionally, the clinician may observe a decrease in a detectable tumor marker. Alternatively, other tests can be used to evaluate objective improvement, such as sonograms, nuclear magnetic resonance testing and positron emissions testing.

The term "prevent" or "preventing," with reference to a certain disease condition in a mammal, refers to preventing or delaying the onset of the disease, or preventing the manifestation of clinical or subclinical symptoms thereof.

As used herein, an "effective amount" refers to an amount of an agent or drug effective to treat a disease or disorder in a subject. In the case of cancer, the effective amount of the agent may reduce the number of cancer cells; reduce the tumor size; inhibit *(i.e.,* slow to some extent and preferably stop) cancer cell infiltration into peripheral organs; inhibit *(i.e.,* slow to some extent and preferably stop) tumor metastasis; inhibit, to some extent, tumor growth; and/or relieve to some extent one or more of the symptoms associated with the cancer. As is understood in the clinical context, an effective amount of a drug, compound, or pharmaceutical composition may or may not be achieved in conjunction with another drug, compound, or pharmaceutical composition. Thus, an "effective amount" may be considered in the context of administering one or more therapeutic agents, and a single agent may be considered to be given in an effective amount if, in conjunction with one or more other agents, a desirable result may be or is achieved.

The terms "recurrence," "relapse" or "relapsed" refers to the return of a cancer or disease after clinical assessment of the disappearance of disease. A diagnosis of distant metastasis or local recurrence can be considered a relapse.

The term "refractory" or "resistant" refers to a cancer or disease that has not responded to treatment.

An "adverse event" or "AE" as used herein refers to any untoward medical occurrence in an individual receiving a marketed pharmaceutical product or in an individual who is participating on a clinical trial who is receiving an investigational or non-investigational pharmaceutical agent. The AE does not necessarily have a causal relationship with the individual's treatment. Therefore, an AE can be any unfavorable and unintended sign, symptom, or disease temporally associated with the use of a medicinal product, whether or not considered to be related to the medicinal product. An AE includes, but is not limited to: an exacerbation of a pre-existing illness; an increase in frequency or intensity of a pre-existing episodic event or condition; a condition detected or diagnosed after study drug administration even though it may have been present prior to the start of the study; and continuously persistent disease or symptoms that were present at baseline and worsen following the start of the study. An AE generally does not include: medical or surgical procedures (e.g., surgery, endoscopy, tooth extraction, or transfusion); however, the condition that leads to the procedure is an adverse event; pre-existing diseases, conditions, or laboratory abnormalities present or detected at the start of the study that do not worsen; hospitalizations or procedures that are done for elective purposes not related to an untoward medical occurrence (e.g., hospitalizations for cosmetic or elective surgery or social/convenience admissions); the disease being studied or signs/symptoms associated with the disease unless more severe than expected for the individual's condition; and overdose of study drug without any clinical signs or symptoms.

A "serious adverse event" or (SAE) as used herein refers to any untoward medical occurrence at any dose including, but not limited to, that: a) is fatal; b) is life-threatening (defined as an immediate risk of death from the event as it occurred); c) results in persistent or significant disability or incapacity; d) requires in-patient hospitalization or prolongs an existing hospitalization (exception: Hospitalization for elective treatment of a pre-existing condition that did not worsen during the study is not considered an adverse event. Complications that occur during hospitalization are AEs and if a complication prolongs hospitalization, then the event is serious); e) is a congenital anomaly/birth defect in the offspring of an individual who received medication; or f) conditions not included in the above definitions that may jeopardize the individual or may require intervention to prevent one of the outcomes listed above unless clearly related to the individual's underlying disease. "Lack of efficacy" (progressive disease) is not considered an AE or SAE. The signs and symptoms or clinical sequelae resulting from lack of efficacy should be reported if they fulfill the AE or SAE definitions.

The following definitions may be used to evaluate response based on target lesions: "complete response" or "CR" refers to disappearance of all target lesions; "partial response" or "PR" refers to at least a 30% decrease in the sum of the longest diameters (SLD) of target lesions, taking as reference the baseline SLD; "stable disease" or "SD" refers to neither sufficient shrinkage of target lesions to qualify for PR, nor sufficient increase to qualify for PD, taking as reference the nadir SLD since the treatment started; and "progressive disease" or "PD" refers to at least a 20% increase in the SLD of target lesions, taking as reference the nadir SLD recorded since the treatment started, or, the presence of one or more new lesions.

The following definitions of response assessments may be used to evaluate a non-target lesion: "complete response" or "CR" refers to disappearance of all non-target lesions; "stable disease" or "SD" refers to the persistence of one or more non-target lesions not qualifying for CR or PD; and "progressive disease" or "PD" refers to the "unequivocal progression" of existing non-target lesion(s) or appearance of one or more new lesion(s) is considered progressive disease (if PD for the individual is to be assessed for a time point based solely on the progression of non-target lesion(s), then additional criteria are required to be fulfilled.

"Progression free survival" (PFS) indicates the length of time during and after treatment that the cancer does not grow. Progression-free survival includes the amount of time individuals have experienced a complete response or a partial response, as well as the amount of time individuals have experienced stable disease.

The terms "polypeptide," "protein," and "peptide" are used interchangeably herein and may refer to polymers of two or more amino acids.

"Polynucleotide," or "nucleic acid," as used interchangeably herein, refer to polymers of nucleotides of any length, and include DNA and RNA. The nucleotides can be deoxyribonucleotides, ribonucleotides, modified nucleotides or bases, and/or their analogs, or any substrate that can be incorporated into a polymer by DNA or RNA polymerase or by a synthetic reaction. A polynucleotide may comprise modified nucleotides, such as methylated nucleotides and their analogs. If present, modification to the nucleotide structure may be imparted before or after assembly of the polymer. The sequence of nucleotides may be interrupted by non-nucleotide components. A polynucleotide may comprise modification(s) made after synthesis, such as conjugation to a label. Other types of modifications include, for example, "caps," substitution of one or more of the naturally occurring nucleotides with an analog, internucleotide modifications such as, for example, those with uncharged linkages (*e.g.,* methyl phosphonates, phosphotriesters, phosphoamidates, carbamates, *etc.*) and with charged linkages (*e.g.,* phosphorothioates, phosphorodithioates, *etc.),* those containing pendant moieties, such as, for example, proteins (*e.g.,* nucleases, toxins, antibodies, signal peptides, ply-L-lysine, *etc.*), those with intercalators (*e.g.,* acridine, psoralen, *etc.*), those containing chelators (*e.g.,* metals, radioactive metals, boron, oxidative metals, *etc.*), those containing alkylators, those with modified linkages (*e.g.,* alpha anomeric nucleic acids, *etc.),* as well as unmodified forms of the polynucleotides(s). Further, any of the hydroxyl groups ordinarily present in the sugars may be replaced, for example, by phosphonate groups, phosphate groups, protected by standard protecting groups, or activated to prepare additional linkages to additional nucleotides, or may be conjugated to solid or semi-solid supports. The 5' and 3' terminal OH can be phosphorylated or substituted with amines or organic capping group moieties of from 1 to 20 carbon atoms. Other hydroxyls may also be derivatized to standard protecting groups. Polynucleotides can also contain analogous forms of ribose or deoxyribose sugars that are generally known in the art, including, for example, 2'-O-methyl-, 2'-O-allyl-, 2'-fluoro- or 2'-azido-ribose, carbocyclic sugar analogs, $\alpha$-anomeric sugars, epimeric sugars such as arabinose, xyloses or lyxoses, pyranose sugars, furanose sugars, sedoheptuloses, acyclic analogs, and basic nucleoside analogs such as methyl riboside. One or more phosphodiester linkages may be replaced by alternative linking groups. These alternative linking groups include, but are not limited to, embodiments wherein phosphate is replaced by P(O)S ("thioate"), P(S)S ("dithioate"), (O)NR2 ("amidate"), P(O)R, P(O)OR', CO, or CH2 ("formacetal"), in which each R or R' is independently H or substituted or unsubstituted alkyl (1-20 C) optionally containing an ether (-O-) linkage, aryl, alkenyl, cycloalkyl, cycloalkenyl or araldyl. Not all linkages in a polynucleotide need be identical. The preceding description applies to all polynucleotides referred to herein, including RNA and DNA.

As used herein, the term "biomarker" or "marker" refers generally to a molecule (*e.g.*, pre-mRNA, mRNA, protein, *etc.)* or cell population (e.g., effector memory T cell or $T_{em}$ cell, or regulatory T cell or $T_{reg}$ cell), the level of which in or on a subject's tissue (e.g., tumor), or in case of a molecule, secreted by the subject's tissue or cell, can be detected by known methods (or methods disclosed herein) and is predictive or can be used to predict (or aid prediction) for a subject's sensitivity to, and in some embodiments, to predict (or aid prediction) a subject's responsiveness to, treatment regimens (e.g., treatments with an anti-CD137 antibody).

As used herein, the term "sample", refers to a composition that is obtained or derived from a subject of interest that contains a cellular and/or other molecular entity that is to be characterized and/or identified, for example based on physical, biochemical, chemical and/or physiological characteristics.

As used herein, the term "tissue or cell sample" refers to a collection of similar cells obtained from a tissue of a subject or patient. The source of the tissue or cell sample may be solid tissue as from a fresh, frozen and/or preserved organ or tissue sample or biopsy or aspirate; blood or any blood constituents; bodily fluids such as cerebral spinal fluid, amniotic fluid, peritoneal fluid, or interstitial fluid; cells from any time in gestation or development of the subject. The tissue sample may also be primary or cultured cells. Optionally, the tissue or cell sample is obtained from a disease tissue or organ. The tissue sample may contain compounds, which are not naturally intermixed with the tissue in nature such as preservatives, anticoagulants, buffers, fixatives, nutrients, antibiotics, or the like. As used herein, a "reference value" or "reference level" may be an absolute value; a relative value; a value that has an upper and/or lower limit; a range of values; an average value; a median value; a mean value; or a value as compared to a particular level or baseline level.

The methods and techniques of the present disclosure are generally performed according to methods well known in the art and as described in various general and more specific references that are cited and discussed throughout the present specification unless otherwise indicated. Such references include, *e.g.,* Sambrook and Russell, Molecular Cloning, A Laboratory Approach, Cold Spring Harbor Press, Cold Spring Harbor, N.Y. (2001), Ausubel et al., Current Protocols in Molecular Biology, John Wiley & Sons, NY (2002), and Harlow and Lane Antibodies: A Laboratory Manual, Cold Spring Harbor Laboratory Press, Cold Spring Harbor, N.Y. (1990). Enzymatic reactions and purification techniques are performed according to manufacturer's specifications, as commonly accomplished in the art or as described herein. The nomenclatures used in connection with, and the laboratory procedures and techniques of, analytical chemistry, synthetic organic chemistry, and medicinal and pharmaceutical chemistry described herein are those well-known and commonly used in the art. Standard techniques are used for chemical syntheses, chemical analyses, pharmaceutical preparation, formulation, and delivery, and treatment of patients.

As used herein, the twenty conventional amino acids and their abbreviations follow conventional usage. See Immunology-A Synthesis (2nd Edition, E. S. Golub and D. R. Gren, Eds., Sinauer Associates, Sunderland, Mass. (1991)).

It is understood that embodiments of the present application described herein include "comprising," "consisting," and "consisting essentially of" aspects and embodiments.

The term "about" as used herein refers to the usual error range for the respective value readily known to the skilled person in this technical field. Reference to "about" a value or parameter herein includes (and describes) variations that are directed to that value or parameter *per se.* For example, description referring to "about X" includes description of "X".

As used herein, reference to "not" a value or parameter generally means and describes "other than" a value or parameter. For example, the method is not used to treat cancer of type X means the method is used to treat cancer of types other than X.

The term "about X-Y" used herein has the same meaning as "about X to about Y."

As used herein and in the appended claims, the singular forms "a," "an," and "the" include plural referents unless the context clearly dictates otherwise.

The term "and/or" as used herein a phrase such as "A and/or B" is intended to include both A and B; A or B; A (alone); and B (alone). Likewise, the term "and/or" as used herein a phrase such as "A, B, and/or C" is intended to encompass each of the following embodiments: A, B, and C; A, B, or C; A or C; A or B; B or C; A and C; A and B; B and C; A (alone); B (alone); and C (alone).

## II. Methods of Treatment

The present application provides methods for treating cancers using a binding molecule (*e.g.,* an anti-CD137 antibody) that specifically binds to an extracellular domain of human CD137. Any one of the anti-CD137 antibodies (including full-length antibodies and antigen-binding fragments thereof) in Section III "Anti-CD137 Antibodies" may be used in the methods described herein.

In some embodiments, there is provided a method of treating a cancer in a subject, comprising administering to the subject an effective amount of an anti-CD137 antibody that specifically binds to an extracellular domain of human CD137, wherein the antibody binds to one or more amino acid residues selected from the group consisting of amino acid residues 51, 53, 62-73, 83, 89, 92, 95-104 and 112-116 of SEQ ID NO: 1, and wherein the anti-CD137 antibody is administered at a dose of no more than 500 mg (*e.g.,* about 50 mg to about 200 mg, about 100 mg to about 200 mg, about 150 mg to about 500 mg, about 150 mg to about 300 mg, or about 300 mg to about 400 mg; for example, about 50 mg, about 100 mg, about 200 mg, about 300 mg or about 400 mg). In some embodiments, the anti-CD137 antibody is administered at a dose of about 0.1 mg/kg to about 10 mg/kg, such as about 3 mg/kg to about 8 mg/kg, *e.g.,* about 3 mg/kg, about 5 mg/kg, or about 8 mg/kg. In some embodiments, the anti-CD137 antibody is administered intravenously. In some embodiments, the anti-CD137 antibody is administered about once every three weeks. In some embodiments, the cancer is selected from the group consisting of colon cancer (e.g., Sigmoid colon cancer), breast cancer, lung cancer (e.g., non-small cell lung cancer or NSCLC; or small cell lung cancer or SCLC), esophageal cancer, endometrial cancer, gastrointestinal cancer (e.g., gastrointestinal neuroectodermal tumor), cholangiocarcinoma, nasopharyngeal cancer (NPC), adenoid cystic carcinoma (ACC), melanoma, mesothelioma (*e.g.,* malignant pleural mesothelioma or MPM), mantle cell lymphoma, anal cancer, head and neck cancer (e.g., head and neck squamous cell carcinoma or HNSCC), appendiceal and sebaceous cancer, follicular lymphoma, non-Hodgkin's lymphoma (NHL), and T cell lymphoma (e.g., angioimmunoblastic T-cell lymphoma or AITL, or Peripheral T-cell lymphoma or PTCL). In some embodiments, the cancer is resistant or refractory to a prior therapy, such as an immunotherapy.

In some embodiments, there is provided a method of treating a cancer in a subject, comprising administering to the subject an effective amount of an anti-CD137 antibody that specifically binds to an extracellular domain of human CD137, wherein the antibody binds to amino acid residues 51, 63-67, 69-73, 83, 89, 92, 98-104 and 112-114 of SEQ ID NO: 1, and wherein the anti-CD137 antibody is administered at a dose of no more than 500 mg (*e.g.,* about 50 mg to about 200 mg, about 100 mg to about 200 mg, about 150 mg to about 500 mg, about 150 mg to about 300 mg, or about 300 mg to about 400 mg; for example, about 50 mg, about 100 mg, about 200 mg, about 300 mg or about 400 mg). In some embodiments, the anti-CD137 antibody is administered at a dose of about 0.1 mg/kg to about 10 mg/kg, such as about 3 mg/kg to about 8 mg/kg, *e.g.,* about 3 mg/kg, about 5 mg/kg, or about 8 mg/kg. In some embodiments, the anti-CD137 antibody is administered intravenously. In some embodiments, the anti-CD137 antibody is administered about once every three weeks. In some embodiments, the cancer is selected from the group consisting of colon cancer (*e.g.,* Sigmoid colon cancer), breast cancer, lung cancer (*e.g.,* NSCLC), esophageal cancer, endometrial cancer, gastrointestinal cancer (*e.g.,* gastrointestinal neuroectodermal tumor), cholangiocarcinoma, NPC, ACC, melanoma, mesothelioma (e.g., MPM), mantle cell lymphoma, anal cancer, head and neck cancer (*e.g.,* HNSCC), appendiceal and sebaceous cancer, follicular lymphoma, NHL, and T cell lymphoma (e.g., AITL, or PTCL). In some embodiments, the cancer is resistant or refractory to a prior therapy, such as an immunotherapy.

In some embodiments, there is provided a method of treating a cancer in a subject, comprising administering to the subject an effective amount of an anti-CD137 antibody that specifically binds to an extracellular domain of human CD137, wherein the anti-CD137 antibody comprises a heavy chain variable region (VH) and a light chain variable region (VL), wherein the VH comprises a HVR-H1 comprising the amino acid sequence of SEQ ID NO: 2, a HVR-H2 comprising the amino acid sequence of SEQ ID NO: 3, and a HVR-H3 comprising the amino acid sequence of SEQ ID NO: 4, and wherein the VL comprises a HVR-L1 comprising the amino acid sequence of SEQ ID NO: 5, a HVR-L2 comprising the amino acid sequence of SEQ ID NO: 6, and a HVR-L3 comprising the amino acid sequence of SEQ ID NO: 7; and wherein the anti-CD137 antibody is administered at a dose of no more than 500 mg (*e.g.,* about 50 mg to about 200 mg, about 100 mg to about 200 mg, about 150 mg to about 500 mg, about 150 mg to about 300 mg, or about 300 mg to about 400 mg; for example, about 50 mg, about 100 mg, about 200 mg, about 300 mg or about 400 mg). In some embodiments, the VH comprises the amino acid sequence of SEQ ID NO: 8, and/or the VL comprises the amino acid sequence of SEQ ID NO: 9. In some embodiments, the antibody comprises a heavy chain and a light chain, and wherein the heavy chain comprises the amino acid sequence of SEQ ID NO: 10, and/or the light chain comprises the amino acid sequence of SEQ ID NO: 11. In some embodiments, the anti-CD137 antibody is administered at a dose of about 0.1 mg/kg to about 10 mg/kg, such as about 3 mg/kg to about 8 mg/kg, *e.g.,* about 3 mg/kg, about 5 mg/kg, or about 8 mg/kg. In some embodiments, the anti-CD137 antibody is administered intravenously. In some embodiments, the anti-CD137 antibody is administered about once every three weeks. In some embodiments, the cancer is selected from the group consisting of colon cancer (*e.g.,* Sigmoid colon cancer), breast cancer, lung cancer (*e.g.,* NSCLC), esophageal cancer, endometrial cancer, gastrointestinal cancer (*e.g.,* gastrointestinal neuroectodermal tumor), cholangiocarcinoma, NPC, ACC, melanoma, mesothelioma

(*e.g.,* MPM), mantle cell lymphoma, anal cancer, head and neck cancer (*e.g.,* HNSCC), appendiceal and sebaceous cancer, follicular lymphoma, NHL, and T cell lymphoma (*e.g.,* AITL, or PTCL). In some embodiments, the cancer is resistant or refractory to a prior therapy, such as an immunotherapy.

In some embodiments, there is provided a method of treating a cancer in a subject, comprising administering to the subject an effective amount of an anti-CD137 antibody that specifically binds to an extracellular domain of human CD137, wherein the anti-CD137 antibody comprises a VH and a VL, wherein the VH comprises a HVR-H1 comprising the amino acid sequence of SEQ ID NO: 12, a HVR-H2 comprising the amino acid sequence of SEQ ID NO: 13, and a HVR-H3 comprising the amino acid sequence of SEQ ID NO: 14, and wherein the VL comprises a HVR-L1 comprising the amino acid sequence of SEQ ID NO: 15, a HVR-L2 comprising the amino acid sequence of SEQ ID NO: 16, and a HVR-L3 comprising the amino acid sequence of SEQ ID NO: 17; and wherein the anti-CD137 antibody is administered at a dose of no more than 500 mg (*e.g.,* about 50 mg to about 200 mg, about 100 mg to about 200 mg, about 150 mg to about 500 mg, about 150 mg to about 300 mg, or about 300 mg to about 400 mg; for example, about 50 mg, about 100 mg, about 200 mg, about 300 mg or about 400 mg). In some embodiments, the VH comprises the amino acid sequence of SEQ ID NO: 18, and/or the VL comprises the amino acid sequence of SEQ ID NO: 19. In some embodiments, the antibody comprises a heavy chain and a light chain, wherein the heavy chain comprises the amino acid sequence of SEQ ID NO: 20, and/or the light chain comprises the amino acid sequence of SEQ ID NO: 21. In some embodiments, the anti-CD137 antibody is administered at a dose of about 0.1 mg/kg to about 10 mg/kg, such as about 3 mg/kg to about 8 mg/kg, e.g., about 3 mg/kg, about 5 mg/kg, or about 8 mg/kg. In some embodiments, the anti-CD137 antibody is administered intravenously. In some embodiments, the anti-CD137 antibody is administered about once every three weeks. In some embodiments, the cancer is selected from the group consisting of colon cancer (e.g., Sigmoid colon cancer), breast cancer, lung cancer (*e.g.,* NSCLC), esophageal cancer, endometrial cancer, gastrointestinal cancer (*e.g.,* gastrointestinal neuroectodermal tumor), cholangiocarcinoma, NPC, ACC, melanoma, mesothelioma (*e.g.,* MPM), mantle cell lymphoma, anal cancer, head and neck cancer (*e.g.,* HNSCC), appendiceal and sebaceous cancer, follicular lymphoma, NHL, and T cell lymphoma (*e.g.,* AITL, or PTCL). In some embodiments, the cancer is resistant or refractory to a prior therapy, such as an immunotherapy.

In some embodiments, there is provided a method of treating a cancer in a subject, comprising administering to the subject an effective amount of an anti-CD137 antibody that specifically binds to an extracellular domain of human CD137, wherein the anti-CD137 antibody comprises a VH and a VL, wherein the VH comprises a HVR-H1 comprising the amino acid sequence of SEQ ID NO: 22, a HVR-H2 comprising the amino acid sequence of SEQ ID NO: 23, and a HVR-H3 comprising the amino acid sequence of SEQ ID NO: 24, and wherein the VL comprises a HVR-L1 comprising the amino acid sequence of SEQ ID NO: 25, a HVR-L2 comprising the amino acid sequence of SEQ ID NO: 26, and a HVR-L3 comprising the amino acid sequence of SEQ ID NO: 27; and wherein the anti-CD137 antibody is administered at a dose of no more than 500 mg (*e.g.,* about 50 mg to about 200 mg, about 100 mg to about 200 mg, about 150 mg to about 500 mg, about 150 mg to about 300 mg, or about 300 mg to about 400 mg; for example, about 50 mg, about 100 mg, about 200 mg, about 300 mg or about 400 mg). In some embodiments, the VH comprises the amino acid sequence of SEQ ID NO: 28, and/or the VL comprises the amino acid sequence of SEQ ID NO: 29. In some embodiments, the antibody comprises a heavy chain and a light chain, wherein the heavy chain comprises the amino acid sequence of SEQ ID NO: 30, and/or the light chain comprises the amino acid sequence of SEQ ID NO: 31. In some embodiments, the anti-CD137 antibody is administered at a dose of about 0.1 mg/kg to about 10 mg/kg, such as about 3 mg/kg to about 8 mg/kg, *e.g.,* about 3 mg/kg, about 5 mg/kg, or about 8 mg/kg. In some embodiments, the anti-CD137 antibody is administered intravenously. In some embodiments, the anti-CD137 antibody is administered about once every three weeks. In some embodiments, the cancer is selected from the group consisting of colon cancer (*e.g.,* Sigmoid colon cancer), breast cancer, lung cancer (*e.g.,* NSCLC), esophageal cancer, endometrial cancer, gastrointestinal cancer (*e.g.,* gastrointestinal neuroectodermal tumor), cholangiocarcinoma, NPC, ACC, melanoma, mesothelioma (*e.g.,* MPM), mantle cell lymphoma, anal cancer, head and neck cancer (*e.g.,* HNSCC), appendiceal and sebaceous cancer, follicular lymphoma, NHL, and T cell lymphoma (*e.g.,* AITL, or PTCL). In some embodiments, the cancer is resistant or refractory to a prior therapy, such as an immunotherapy.

In some embodiments, there is provided a method of treating a cancer in a subject, comprising administering to the subject an effective amount of an anti-CD137 antibody that specifically binds to an extracellular domain of human CD137, wherein the antibody binds to one or more amino acid residues selected from the group consisting of amino acid residues 51, 53, 62-73, 83, 89, 92, 95-104 and 112-116 of SEQ ID NO: 1; and wherein the cancer is resistant or refractory to a prior therapy (e.g., a prior immunotherapy such as an anti-CD20 antibody, for example, rituximab). In some embodiments, the anti-CD137 antibody is administered at a dose of no more than 500 mg (e.g., about 50 mg to about 200 mg, about 100 mg to about 200 mg, about 150 mg to about 500 mg, about 150 mg to about 300 mg, or about 300 mg to about 400 mg; for example, about 50 mg, about 100 mg, about 200 mg, about 300 mg or about 400 mg). In some embodiments, the anti-CD137 antibody is administered at a dose of about 0.1 mg/kg to about 10 mg/kg, such as about 3 mg/kg to about 8 mg/kg, *e.g.*, about 3 mg/kg, about 5 mg/kg, or about 8 mg/kg. In some embodiments, the anti-CD137 antibody is administered intravenously. In some embodiments, the anti-CD137 antibody is administered about once every three weeks. In some embodiments, the cancer is selected from the group consisting of colon cancer (*e.g.,* Sigmoid colon cancer), breast cancer, lung cancer (*e.g.,* NSCLC), esophageal cancer, endometrial cancer, gastrointestinal cancer (*e.g.*,

gastrointestinal neuroectodermal tumor), cholangiocarcinoma, NPC, ACC, melanoma, mesothelioma *(e.g.,* MPM), anal cancer, head and neck cancer *(e.g.,* HNSCC), mantle cell lymphoma, appendiceal and sebaceous cancer, follicular lymphoma, NHL, and T cell lymphoma *(e.g.,* AITL, or PTCL).

In some embodiments, there is provided a method of treating a cancer in a subject, comprising administering to the subject an effective amount of an anti-CD137 antibody that specifically binds to an extracellular domain of human CD137, wherein the antibody binds to amino acid residues 51, 63-67, 69-73, 83, 89, 92, 98-104 and 112-114 of SEQ ID NO: 1, and wherein the cancer is resistant or refractory to a prior therapy *(e.g.,* a prior immunotherapy such as an anti-CD20 antibody, for example, rituximab). In some embodiments, the anti-CD137 antibody is administered at a dose of no more than 500 mg *(e.g.,* about 50 mg to about 200 mg, about 100 mg to about 200 mg, about 150 mg to about 500 mg, about 150 mg to about 300 mg, or about 300 mg to about 400 mg; for example, about 50 mg, about 100 mg, about 200 mg, about 300 mg or about 400 mg). In some embodiments, the anti-CD137 antibody is administered at a dose of about 0.1 mg/kg to about 10 mg/kg, such as about 3 mg/kg to about 8 mg/kg, *e.g.,* about 3 mg/kg, about 5 mg/kg, or about 8 mg/kg. In some embodiments, the anti-CD137 antibody is administered intravenously. In some embodiments, the anti-CD137 antibody is administered about once every three weeks. In some embodiments, the cancer is selected from the group consisting of colon cancer *(e.g.,* Sigmoid colon cancer), breast cancer, lung cancer *(e.g.,* NSCLC), esophageal cancer, endometrial cancer, gastrointestinal cancer *(e.g.,* gastrointestinal neuroectodermal tumor), cholangiocarcinoma, NPC, ACC, melanoma, mesothelioma (e.g., MPM), anal cancer, head and neck cancer *(e.g.,* HNSCC), mantle cell lymphoma, appendiceal and sebaceous cancer, follicular lymphoma, NHL, and T cell lymphoma (e.g., AITL, or PTCL).

In some embodiments, there is provided a method of treating a cancer in a subject, comprising administering to the subject an effective amount of an anti-CD137 antibody that specifically binds to an extracellular domain of human CD137, wherein the anti-CD137 antibody comprises a heavy chain variable region (VH) and a light chain variable region (VL), wherein the VH comprises a HVR-H1 comprising the amino acid sequence of SEQ ID NO: 2, a HVR-H2 comprising the amino acid sequence of SEQ ID NO: 3, and a HVR-H3 comprising the amino acid sequence of SEQ ID NO: 4, and wherein the VL comprises a HVR-L1 comprising the amino acid sequence of SEQ ID NO: 5, a HVR-L2 comprising the amino acid sequence of SEQ ID NO: 6, and a HVR-L3 comprising the amino acid sequence of SEQ ID NO: 7; and wherein the cancer is resistant or refractory to a prior therapy (e.g., a prior immunotherapy such as an anti-CD20 antibody, for example, rituximab). In some embodiments, the VH comprises the amino acid sequence of SEQ ID NO: 8, and/or the VL comprises the amino acid sequence of SEQ ID NO: 9. In some embodiments, the antibody comprises a heavy chain and a light chain, and wherein the heavy chain comprises the amino acid sequence of SEQ ID NO: 10, and/or the light chain comprises the amino acid sequence of SEQ ID NO: 11. In some embodiments, the anti-CD137 antibody is administered at a dose of no more than 500 mg *(e.g.,* about 50 mg to about 200 mg, about 100 mg to about 200 mg, about 150 mg to about 500 mg, about 150 mg to about 300 mg, or about 300 mg to about 400 mg; for example, about 50 mg, about 100 mg, about 200 mg, about 300 mg or about 400 mg). In some embodiments, the anti-CD137 antibody is administered at a dose of about 0.1 mg/kg to about 10 mg/kg, such as about 3 mg/kg to about 8 mg/kg, *e.g.,* about 3 mg/kg, about 5 mg/kg, or about 8 mg/kg. In some embodiments, the anti-CD137 antibody is administered intravenously. In some embodiments, the anti-CD137 antibody is administered about once every three weeks. In some embodiments, the cancer is selected from the group consisting of colon cancer *(e.g.,* Sigmoid colon cancer), breast cancer, lung cancer *(e.g.,* NSCLC), esophageal cancer, endometrial cancer, gastrointestinal cancer *(e.g.,* gastrointestinal neuroectodermal tumor), cholangiocarcinoma, NPC, ACC, melanoma, mesothelioma *(e.g.,* MPM), anal cancer, head and neck cancer *(e.g.,* HNSCC), mantle cell lymphoma, appendiceal and sebaceous cancer, follicular lymphoma, NHL, and T cell lymphoma (e.g., AITL, or PTCL).

In some embodiments, there is provided a method of treating a cancer in a subject, comprising administering to the subject an effective amount of an anti-CD137 antibody that specifically binds to an extracellular domain of human CD137, wherein the anti-CD137 antibody comprises a VH and a VL, wherein the VH comprises a HVR-H1 comprising the amino acid sequence of SEQ ID NO: 12, a HVR-H2 comprising the amino acid sequence of SEQ ID NO: 13, and a HVR-H3 comprising the amino acid sequence of SEQ ID NO: 14, and wherein the VL comprises a HVR-L1 comprising the amino acid sequence of SEQ ID NO: 15, a HVR-L2 comprising the amino acid sequence of SEQ ID NO: 16, and a HVR-L3 comprising the amino acid sequence of SEQ ID NO: 17; and wherein the cancer is resistant or refractory to a prior therapy *(e.g.,* a prior immunotherapy such as an anti-CD20 antibody, for example, rituximab). In some embodiments, the VH comprises the amino acid sequence of SEQ ID NO: 18, and/or the VL comprises the amino acid sequence of SEQ ID NO: 19. In some embodiments, the antibody comprises a heavy chain and a light chain, wherein the heavy chain comprises the amino acid sequence of SEQ ID NO: 20, and/or the light chain comprises the amino acid sequence of SEQ ID NO: 21. In some embodiments, the anti-CD137 antibody is administered at a dose of no more than 500 mg *(e.g.,* about 50 mg to about 200 mg, about 100 mg to about 200 mg, about 150 mg to about 500 mg, about 150 mg to about 300 mg, or about 300 mg to about 400 mg; for example, about 50 mg, about 100 mg, about 200 mg, about 300 mg or about 400 mg). In some embodiments, the anti-CD137 antibody is administered at a dose of about 0.1 mg/kg to about 10 mg/kg, such as about 3 mg/kg to about 8 mg/kg, e.g., about 3 mg/kg, about 5 mg/kg, or about 8 mg/kg. In some embodiments, the anti-CD137 antibody is administered intravenously. In some embodiments, the anti-CD137 antibody is administered about once every three weeks. In some embodiments, the cancer is selected from the group consisting of colon cancer *(e.g.,* Sigmoid colon cancer), breast cancer, lung cancer (*e.g.,* NSCLC), esophageal cancer, endometrial cancer, gastrointestinal cancer (*e.g.,* gastrointestinal neuroectodermal tumor), cholangiocarcinoma, NPC, ACC, melanoma, mesothelioma (e.g., MPM), anal cancer, head and neck cancer (*e.g.,* HNSCC), mantle cell lymphoma, appendiceal and sebaceous cancer, follicular lymphoma, NHL, and T cell lymphoma (e.g., AITL, or PTCL).

In some embodiments, there is provided a method of treating a cancer in a subject, comprising administering to the subject an effective amount of an anti-CD137 antibody that specifically binds to an extracellular domain of human CD137, wherein the anti-CD137 antibody comprises a VH and a VL, wherein the VH comprises a HVR-H1 comprising the amino acid sequence of SEQ ID NO: 22, a HVR-H2 comprising the amino acid sequence of SEQ ID NO: 23, and a HVR-H3 comprising the amino acid sequence of SEQ ID NO: 24, and wherein the VL comprises a HVR-L1 comprising the amino acid sequence of SEQ ID NO: 25, a HVR-L2 comprising the amino acid sequence of SEQ ID NO: 26, and a HVR-L3 comprising the amino acid sequence of SEQ ID NO: 27; and wherein the cancer is resistant or refractory to a prior therapy (e.g., a prior immunotherapy such as an anti-CD20 antibody, for example, rituximab). In some embodiments, the VH comprises the amino acid sequence of SEQ ID NO: 28, and/or the VL comprises the amino acid sequence of SEQ ID NO: 29. In some embodiments, the antibody comprises a heavy chain and a light chain, wherein the heavy chain comprises the amino acid sequence of SEQ ID NO: 30, and/or the light chain comprises the amino acid sequence of SEQ ID NO: 31. In some embodiments, the anti-CD137 antibody is administered at a dose of no more than 500 mg (e.g., about 50 mg to about 200 mg, about 100 mg to about 200 mg, about 150 mg to about 500 mg, about 150 mg to about 300 mg, or about 300 mg to about 400 mg; for example, about 50 mg, about 100 mg, about 200 mg, about 300 mg or about 400 mg). In some embodiments, the anti-CD137 antibody is administered at a dose of about 0.1 mg/kg to about 10 mg/kg, such as about 3 mg/kg to about 8 mg/kg, *e.g.,* about 3 mg/kg, about 5 mg/kg, or about 8 mg/kg. In some embodiments, the anti-CD137 antibody is administered intravenously. In some embodiments, the anti-CD137 antibody is administered about once every three weeks. In some embodiments, the cancer is selected from the group consisting of colon cancer (*e.g.,* Sigmoid colon cancer), breast cancer, lung cancer (*e.g.,* NSCLC), esophageal cancer, endometrial cancer, gastrointestinal cancer (*e.g.,* gastrointestinal neuroectodermal tumor), cholangiocarcinoma, NPC, ACC, melanoma, mesothelioma (e.g., MPM), anal cancer, head and neck cancer (e.g., HNSCC), mantle cell lymphoma, appendiceal and sebaceous cancer, follicular lymphoma, NHL, and T cell lymphoma (e.g., AITL, or PTCL).

In some embodiments, there is provided a method of treating a follicular lymphoma in a subject, comprising administering to the subject an effective amount of an anti-CD137 antibody that specifically binds to an extracellular domain of human CD137, wherein the antibody binds to one or more amino acid residues selected from the group consisting of amino acid residues 51, 53, 62-73, 83, 89, 92, 95-104 and 112-116 of SEQ ID NO: 1. In some embodiments, the anti-CD137 antibody is administered at a dose of no more than 500 mg (*e.g.,* about 50 mg to about 200 mg, about 100 mg to about 200 mg, about 150 mg to about 500 mg, about 150 mg to about 300 mg, or about 300 mg to about 400 mg; for example, about 50 mg, about 100 mg, about 200 mg, about 300 mg or about 400 mg). In some embodiments, the anti-CD137 antibody is administered at a dose of about 0.1 mg/kg to about 10 mg/kg, such as about 3 mg/kg to about 8 mg/kg, e.g., about 3 mg/kg, about 5 mg/kg, or about 8 mg/kg. In some embodiments, the anti-CD137 antibody is administered intravenously. In some embodiments, the anti-CD137 antibody is administered about once every three weeks. In some embodiments, the cancer is resistant or refractory to a prior therapy, such as an immunotherapy.

In some embodiments, there is provided a method of treating a follicular lymphoma in a subject, comprising administering to the subject an effective amount of an anti-CD137 antibody that specifically binds to an extracellular domain of human CD137, wherein the antibody binds to amino acid residues 51, 63-67, 69-73, 83, 89, 92, 98-104 and 112-114 of SEQ ID NO: 1. In some embodiments, the anti-CD137 antibody is administered at a dose of no more than 500 mg (*e.g.,* about 50 mg to about 200 mg, about 100 mg to about 200 mg, about 150 mg to about 500 mg, about 150 mg to about 300 mg, or about 300 mg to about 400 mg; for example, about 50 mg, about 100 mg, about 200 mg, about 300 mg or about 400 mg). In some embodiments, the anti-CD137 antibody is administered at a dose of about 0.1 mg/kg to about 10 mg/kg, such as about 3 mg/kg to about 8 mg/kg, *e.g.,* about 3 mg/kg, about 5 mg/kg, or about 8 mg/kg. In some embodiments, the anti-CD137 antibody is administered intravenously. In some embodiments, the anti-CD137 antibody is administered about once every three weeks. In some embodiments, the cancer is resistant or refractory to a prior therapy, such as an immunotherapy.

In some embodiments, there is provided a method of treating a follicular lymphoma in a subject, comprising administering to the subject an effective amount of an anti-CD137 antibody that specifically binds to an extracellular domain of human CD137, wherein the anti-CD137 antibody comprises a heavy chain variable region (VH) and a light chain variable region (VL), wherein the VH comprises a HVR-H1 comprising the amino acid sequence of SEQ ID NO: 2, a HVR-H2 comprising the amino acid sequence of SEQ ID NO: 3, and a HVR-H3 comprising the amino acid sequence of SEQ ID NO: 4, and wherein the VL comprises a HVR-L1 comprising the amino acid sequence of SEQ ID NO: 5, a HVR-L2 comprising the amino acid sequence of SEQ ID NO: 6, and a HVR-L3 comprising the amino acid sequence of SEQ ID NO: 7. In some embodiments, the VH comprises the amino acid sequence of SEQ ID NO: 8, and/or the VL comprises the amino acid sequence of SEQ ID NO: 9. In some embodiments, the antibody comprises a heavy chain and a light chain, and wherein the heavy chain comprises the amino acid sequence of SEQ ID NO: 10, and/or the light chain comprises the amino acid sequence of SEQ ID NO: 11. In some embodiments, the anti-CD137 antibody is administered at a dose of no more than 500 mg (e.g., about 50 mg to about 200 mg, about 100 mg to about 200 mg, about 150 mg to about 500 mg, about 150 mg to about 300 mg, or about 300 mg to about 400 mg; for example, about 50 mg, about 100 mg, about 200 mg, about 300 mg or about 400 mg). In some embodiments, the anti-CD137 antibody is administered at a dose of about 0.1 mg/kg to about 10 mg/kg, such as about 3 mg/kg to about 8 mg/kg, e.g., about 3 mg/kg, about 5 mg/kg, or about 8 mg/kg. In some embodiments, the anti-CD137 antibody is administered intravenously. In some embodiments, the anti-CD137 antibody is administered about once every three weeks. In some embodiments, the cancer is resistant or refractory to a prior therapy, such as an immunotherapy.

In some embodiments, there is provided a method of treating a follicular lymphoma in a subject, comprising administering to the subject an effective amount of an anti-CD137 antibody that specifically binds to an extracellular domain of human CD137, wherein the anti-CD137 antibody comprises a VH and a VL, wherein the VH comprises a HVR-H1 comprising the amino acid sequence of SEQ ID NO: 12, a HVR-H2 comprising the amino acid sequence of SEQ ID NO: 13, and a HVR-H3 comprising the amino acid sequence of SEQ ID NO: 14, and wherein the VL comprises a HVR-L1 comprising the amino acid sequence of SEQ ID NO: 15, a HVR-L2 comprising the amino acid sequence of SEQ ID NO: 16, and a HVR-L3 comprising the amino acid sequence of SEQ ID NO: 17. In some embodiments, the VH comprises the amino acid sequence of SEQ ID NO: 18, and/or the VL comprises the amino acid sequence of SEQ ID NO: 19. In some embodiments, the antibody comprises a heavy chain and a light chain, wherein the heavy chain comprises the amino acid sequence of SEQ ID NO: 20, and/or the light chain comprises the amino acid sequence of SEQ ID NO: 21. In some embodiments, the anti-CD137 antibody is administered at a dose of no more than 500 mg (e.g., about 50 mg to about 200 mg, about 100 mg to about 200 mg, about 150 mg to about 500 mg, about 150 mg to about 300 mg, or about 300 mg to about 400 mg; for example, about 50 mg, about 100 mg, about 200 mg, about 300 mg or about 400 mg). In some embodiments, the anti-CD137 antibody is administered at a dose of about 0.1 mg/kg to about 10 mg/kg, such as about 3 mg/kg to about 8 mg/kg, e.g., about 3 mg/kg, about 5 mg/kg, or about 8 mg/kg. In some embodiments, the anti-CD137 antibody is administered intravenously. In some embodiments, the anti-CD137 antibody is administered about once every three weeks. In some embodiments, the cancer is resistant or refractory to a prior therapy, such as an immunotherapy.

In some embodiments, there is provided a method of treating a follicular lymphoma in a subject, comprising administering to the subject an effective amount of an anti-CD137 antibody that specifically binds to an extracellular domain of human CD137, wherein the anti-CD137 antibody comprises a VH and a VL, wherein the VH comprises a HVR-H1 comprising the amino acid sequence of SEQ ID NO: 22, a HVR-H2 comprising the amino acid sequence of SEQ ID NO: 23, and a HVR-H3 comprising the amino acid sequence of SEQ ID NO: 24, and wherein the VL comprises a HVR-L1 comprising the amino acid sequence of SEQ ID NO: 25, a HVR-L2 comprising the amino acid sequence of SEQ ID NO: 26, and a HVR-L3 comprising the amino acid sequence of SEQ ID NO: 27. In some embodiments, the VH comprises the amino acid sequence of SEQ ID NO: 28, and/or the VL comprises the amino acid sequence of SEQ ID NO: 29. In some embodiments, the antibody comprises a heavy chain and a light chain, wherein the heavy chain comprises the amino acid sequence of SEQ ID NO: 30, and/or the light chain comprises the amino acid sequence of SEQ ID NO: 31. In some embodiments, the anti-CD137 antibody is administered at a dose of no more than 500 mg (e.g., about 50 mg to about 200 mg, about 100 mg to about 200 mg, about 150 mg to about 500 mg, about 150 mg to about 300 mg, or about 300 mg to about 400 mg; for example, about 50 mg, about 100 mg, about 200 mg, about 300 mg or about 400 mg). In some embodiments, the anti-CD137 antibody is administered at a dose of about 0.1 mg/kg to about 10 mg/kg, such as about 3 mg/kg to about 8 mg/kg, e.g., about 3 mg/kg, about 5 mg/kg, or about 8 mg/kg. In some embodiments, the anti-CD137 antibody is administered intravenously. In some embodiments, the anti-CD137 antibody is administered about once every three weeks. In some embodiments, the cancer is resistant or refractory to a prior therapy, such as an immunotherapy.

In some embodiments, there is provided a method of treating a T cell lymphoma in a subject, comprising administering to the subject an effective amount of an anti-CD137 antibody that specifically binds to an extracellular domain of human CD137, wherein the antibody binds to one or more amino acid residues selected from the group consisting of amino acid residues 51, 53, 62-73, 83, 89, 92, 95-104 and 112-116 of SEQ ID NO: 1. In some embodiments, the T cell lymphoma is AITL or PTCL. In some embodiments, the anti-CD137 antibody is administered at a dose of no more than 500 mg (e.g., about 50 mg to about 200 mg, about 100 mg to about 200 mg, about 150 mg to about 500 mg, about 150 mg to about 300 mg, or about 300 mg to about 400 mg; for example, about 50 mg, about 100 mg, about 200 mg, about 300 mg or about 400 mg). In some embodiments, the anti-CD137 antibody is administered at a dose of about 0.1 mg/kg to about 10 mg/kg, such as about 3 mg/kg to about 8 mg/kg, e.g., about 3 mg/kg, about 5 mg/kg, or about 8 mg/kg. In some embodiments, the anti-CD137 antibody is administered intravenously. In some embodiments, the anti-CD137 antibody is administered about once every three weeks. In some embodiments, the cancer is resistant or refractory to a prior therapy, such as an immunotherapy.

In some embodiments, there is provided a method of treating a T cell lymphoma in a subject, comprising administering to the subject an effective amount of an anti-CD137 antibody that specifically binds to an extracellular domain of human CD137, wherein the antibody binds to amino acid residues 51, 63-67, 69-73, 83, 89, 92, 98-104 and 112-114 of SEQ ID NO: 1. In some embodiments, the T cell lymphoma is AITL or PTCL. In some embodiments, the anti-CD137 antibody is administered at a dose of no more than 500 mg (*e.g.,* about 50 mg to about 200 mg, about 100 mg to about 200 mg, about 150 mg to about 500 mg, about 150 mg to about 300 mg, or about 300 mg to about 400 mg; for example, about 50 mg, about 100 mg, about 200 mg, about 300 mg or about 400 mg). In some embodiments, the anti-CD137 antibody is administered at a dose of about 0.1 mg/kg to about 10 mg/kg, such as about 3 mg/kg to about 8 mg/kg, *e.g.,* about 3 mg/kg, about 5 mg/kg, or about 8 mg/kg. In some embodiments, the anti-CD137 antibody is administered intravenously. In some embodiments, the anti-CD137 antibody is administered about once every three weeks. In some embodiments, the cancer is resistant or refractory to a prior therapy, such as an immunotherapy.

In some embodiments, there is provided a method of treating a T cell lymphoma in a subject, comprising administering to the subject an effective amount of an anti-CD137 antibody that specifically binds to an extracellular domain of human CD137, wherein the anti-CD137 antibody comprises a heavy chain variable region (VH) and a light chain variable region (VL), wherein the VH comprises a HVR-H1 comprising the amino acid sequence of SEQ ID NO: 2, a HVR-H2 comprising the amino acid sequence of SEQ ID NO: 3, and a HVR-H3 comprising the amino acid sequence of SEQ ID NO: 4, and wherein the VL comprises a HVR-L1 comprising the amino acid sequence of SEQ ID NO: 5, a HVR-L2 comprising the amino acid sequence of SEQ ID NO: 6, and a HVR-L3 comprising the amino acid sequence of SEQ ID NO: 7. In some embodiments, the VH comprises the amino acid sequence of SEQ ID NO: 8, and/or the VL comprises the amino acid sequence of SEQ ID NO: 9. In some embodiments, the antibody comprises a heavy chain and a light chain, and wherein the heavy chain comprises the amino acid sequence of SEQ ID NO: 10, and/or the light chain comprises the amino acid sequence of SEQ ID NO: 11. In some embodiments, the T cell lymphoma is AITL or PTCL. In some embodiments, the anti-CD137 antibody is administered at a dose of no more than 500 mg (*e.g.,* about 50 mg to about 200 mg, about 100 mg to about 200 mg, about 150 mg to about 500 mg, about 150 mg to about 300 mg, or about 300 mg to about 400 mg; for example, about 50 mg, about 100 mg, about 200 mg, about 300 mg or about 400 mg). In some embodiments, the anti-CD137 antibody is administered at a dose of about 0.1 mg/kg to about 10 mg/kg, such as about 3 mg/kg to about 8 mg/kg, *e.g.,* about 3 mg/kg, about 5 mg/kg, or about 8 mg/kg. In some embodiments, the anti-CD137 antibody is administered intravenously. In some embodiments, the anti-CD137 antibody is administered about once every three weeks. In some embodiments, the cancer is resistant or refractory to a prior therapy, such as an immunotherapy.

In some embodiments, there is provided a method of treating a T cell lymphoma in a subject, comprising administering to the subject an effective amount of an anti-CD137 antibody that specifically binds to an extracellular domain of human CD137, wherein the anti-CD137 antibody comprises a VH and a VL, wherein the VH comprises a HVR-H1 comprising the amino acid sequence of SEQ ID NO: 12, a HVR-H2 comprising the amino acid sequence of SEQ ID NO: 13, and a HVR-H3 comprising the amino acid sequence of SEQ ID NO: 14, and wherein the VL comprises a HVR-L1 comprising the amino acid sequence of SEQ ID NO: 15, a HVR-L2 comprising the amino acid sequence of SEQ ID NO: 16, and a HVR-L3 comprising the amino acid sequence of SEQ ID NO: 17. In some embodiments, the VH comprises the amino acid sequence of SEQ ID NO: 18, and/or the VL comprises the amino acid sequence of SEQ ID NO: 19. In some embodiments, the antibody comprises a heavy chain and a light chain, wherein the heavy chain comprises the amino acid sequence of SEQ ID NO: 20, and/or the light chain comprises the amino acid sequence of SEQ ID NO: 21. In some embodiments, the T cell lymphoma is AITL or PTCL. In some embodiments, the anti-CD137 antibody is administered at a dose of no more than 500 mg (*e.g.,* about 50 mg to about 200 mg, about 100 mg to about 200 mg, about 150 mg to about 500 mg, about 150 mg to about 300 mg, or about 300 mg to about 400 mg; for example, about 50 mg, about 100 mg, about 200 mg, about 300 mg or about 400 mg). In some embodiments, the anti-CD137 antibody is administered at a dose of about 0.1 mg/kg to about 10 mg/kg, such as about 3 mg/kg to about 8 mg/kg, *e.g.,* about 3 mg/kg, about 5 mg/kg, or about 8 mg/kg. In some embodiments, the anti-CD137 antibody is administered intravenously. In some embodiments, the anti-CD137 antibody is administered about once every three weeks. In some embodiments, the cancer is resistant or refractory to a prior therapy, such as an immunotherapy.

In some embodiments, there is provided a method of treating a T cell lymphoma in a subject, comprising administering to the subject an effective amount of an anti-CD137 antibody that specifically binds to an extracellular domain of human CD137, wherein the anti-CD137 antibody comprises a VH and a VL, wherein the VH comprises a HVR-H1 comprising the amino acid sequence of SEQ ID NO: 22, a HVR-H2 comprising the amino acid sequence of SEQ ID NO: 23, and a HVR-H3 comprising the amino acid sequence of SEQ ID NO: 24, and wherein the VL comprises a HVR-L1 comprising the amino acid sequence of SEQ ID NO: 25, a HVR-L2 comprising the amino acid sequence of SEQ ID NO: 26, and a HVR-L3 comprising the amino acid sequence of SEQ ID NO: 27. In some embodiments, the VH comprises the amino acid sequence of SEQ ID NO: 28, and/or the VL comprises the amino acid sequence of SEQ ID NO: 29. In some embodiments, the antibody comprises a heavy chain and a light chain, wherein the heavy chain comprises the amino acid sequence of SEQ ID NO: 30, and/or the light chain comprises the amino acid sequence of SEQ ID NO: 31. In some embodiments, the T cell lymphoma is AITL or PTCL. In some embodiments, the anti-CD137 antibody is administered at a dose of no more than 500 mg (*e.g.,* about 50 mg to about 200 mg, about 100 mg to about 200 mg, about 150 mg to about 500 mg, about 150 mg to about 300 mg, or about 300 mg to about 400 mg; for example, about 50 mg, about 100 mg, about 200 mg, about 300 mg or about 400 mg). In some embodiments, the anti-CD137 antibody is administered at a dose of about 0.1 mg/kg to about 10 mg/kg, such as about 3 mg/kg to about 8 mg/kg, *e.g.,* about 3 mg/kg, about 5 mg/kg, or about 8 mg/kg. In some embodiments, the anti-CD137 antibody is administered intravenously. In some embodiments, the anti-CD137 antibody is administered about once every three weeks. In some embodiments, the cancer is resistant or refractory to a prior therapy, such as an immunotherapy.

In some embodiments, there is provided a method of treating an adenoid cystic carcinoma (ACC) in a subject, comprising administering to the subject an effective amount of an anti-CD137 antibody that specifically binds to an extracellular domain of human CD137, wherein the antibody binds to one or more amino acid residues selected from the group consisting of amino acid residues 51, 53, 62-73, 83, 89, 92, 95-104 and 112-116 of SEQ ID NO: 1. In some embodiments, the anti-CD137 antibody is administered at a dose of no more than 500 mg (*e.g.,* about 50 mg to about 200 mg, about 100 mg to about 200 mg, about 150 mg to about 500 mg, about 150 mg to about 300 mg, or about 300 mg to about 400 mg; for example, about 50 mg, about 100 mg, about 200 mg, about 300 mg or about 400 mg). In some embodiments, the anti-CD137 antibody is administered at a dose of about 0.1 mg/kg to about 10 mg/kg, such as about 3 mg/kg to about 8 mg/kg, *e.g.,* about 3 mg/kg, about 5 mg/kg, or about 8 mg/kg. In some embodiments, the anti-CD137 antibody is administered intravenously. In some embodiments, the anti-CD137 antibody is administered about once every three weeks. In some embodiments, the cancer is resistant or refractory to a prior therapy, such as an immunotherapy.

In some embodiments, there is provided a method of treating an adenoid cystic carcinoma (ACC) in a subject, comprising administering to the subject an effective amount of an anti-CD137 antibody that specifically binds to an extracellular domain of human CD137, wherein the antibody binds to amino acid residues 51, 63-67, 69-73, 83, 89, 92, 98-104 and 112-114 of SEQ ID NO: 1. In some embodiments, the anti-CD 137 antibody is administered at a dose of no more than 500 mg (*e.g.,* about 50 mg to about 200 mg, about 100 mg to about 200 mg, about 150 mg to about 500 mg, about 150 mg to about 300 mg, or about 300 mg to about 400 mg; for example, about 50 mg, about 100 mg, about 200 mg, about 300 mg or about 400 mg). In some embodiments, the anti-CD137 antibody is administered at a dose of about 0.1 mg/kg to about 10 mg/kg, such as about 3 mg/kg to about 8 mg/kg, *e.g.,* about 3 mg/kg, about 5 mg/kg, or about 8 mg/kg. In some embodiments, the anti-CD137 antibody is administered intravenously. In some embodiments, the anti-CD137 antibody is administered about once every three weeks. In some embodiments, the cancer is resistant or refractory to a prior therapy, such as an immunotherapy.

In some embodiments, there is provided a method of treating an adenoid cystic carcinoma (ACC) in a subject, comprising administering to the subject an effective amount of an anti-CD137 antibody that specifically binds to an extracellular domain of human CD137, wherein the anti-CD137 antibody comprises a heavy chain variable region (VH) and a light chain variable region (VL), wherein the VH comprises a HVR-H1 comprising the amino acid sequence of SEQ ID NO: 2, a HVR-H2 comprising the amino acid sequence of SEQ ID NO: 3, and a HVR-H3 comprising the amino acid sequence of SEQ ID NO: 4, and wherein the VL comprises a HVR-L1 comprising the amino acid sequence of SEQ ID NO: 5, a HVR-L2 comprising the amino acid sequence of SEQ ID NO: 6, and a HVR-L3 comprising the amino acid sequence of SEQ ID NO: 7. In some embodiments, the VH comprises the amino acid sequence of SEQ ID NO: 8, and/or the VL comprises the amino acid sequence of SEQ ID NO: 9. In some embodiments, the antibody comprises a heavy chain and a light chain, and wherein the heavy chain comprises the amino acid sequence of SEQ ID NO: 10, and/or the light chain comprises the amino acid sequence of SEQ ID NO: 11. In some embodiments, the anti-CD137 antibody is administered at a dose of no more than 500 mg (*e.g.,* about 50 mg to about 200 mg, about 100 mg to about 200 mg, about 150 mg to about 500 mg, about 150 mg to about 300 mg, or about 300 mg to about 400 mg; for example, about 50 mg, about 100 mg, about 200 mg, about 300 mg or about 400 mg). In some embodiments, the anti-CD137 antibody is administered at a dose of about 0.1 mg/kg to about 10 mg/kg, such as about 3 mg/kg to about 8 mg/kg, *e.g.,* about 3 mg/kg, about 5 mg/kg, or about 8 mg/kg. In some embodiments, the anti-CD137 antibody is administered intravenously. In some embodiments, the anti-CD137 antibody is administered about once every three weeks. In some embodiments, the cancer is resistant or refractory to a prior therapy, such as an immunotherapy.

In some embodiments, there is provided a method of treating an adenoid cystic carcinoma (ACC) in a subject, comprising administering to the subject an effective amount of an anti-CD137 antibody that specifically binds to an extracellular domain of human CD137, wherein the anti-CD137 antibody comprises a VH and a VL, wherein the VH comprises a HVR-H1 comprising the amino acid sequence of SEQ ID NO: 12, a HVR-H2 comprising the amino acid sequence of SEQ ID NO: 13, and a HVR-H3 comprising the amino acid sequence of SEQ ID NO: 14, and wherein the VL comprises a HVR-L1 comprising the amino acid sequence of SEQ ID NO: 15, a HVR-L2 comprising the amino acid sequence of SEQ ID NO: 16, and a HVR-L3 comprising the amino acid sequence of SEQ ID NO: 17. In some embodiments, the VH comprises the amino acid sequence of SEQ ID NO: 18, and/or the VL comprises the amino acid sequence of SEQ ID NO: 19. In some embodiments, the antibody comprises a heavy chain and a light chain, wherein the heavy chain comprises the amino acid sequence of SEQ ID NO: 20, and/or the light chain comprises the amino acid sequence of SEQ ID NO: 21. In some embodiments, the anti-CD137 antibody is administered at a dose of no more than 500 mg (*e.g.,* about 50 mg to about 200 mg, about 100 mg to about 200 mg, about 150 mg to about 500 mg, about 150 mg to about 300 mg, or about 300 mg to about 400 mg; for example, about 50 mg, about 100 mg, about 200 mg, about 300 mg or about 400 mg). In some embodiments, the anti-CD137 antibody is administered at a dose of about 0.1 mg/kg to about 10 mg/kg, such as about 3 mg/kg to about 8 mg/kg, *e.g.,* about 3 mg/kg, about 5 mg/kg, or about 8 mg/kg. In some embodiments, the anti-CD137 antibody is administered intravenously. In some embodiments, the anti-CD137 antibody is administered about once every three weeks. In some embodiments, the cancer is resistant or refractory to a prior therapy, such as an immunotherapy.

In some embodiments, there is provided a method of treating an adenoid cystic carcinoma (ACC) in a subject, comprising administering to the subject an effective amount of an anti-CD137 antibody that specifically binds to an extracellular domain of human CD137, wherein the anti-CD137 antibody comprises a VH and a VL, wherein the VH comprises a HVR-H1 comprising the amino acid sequence of SEQ ID NO: 22, a HVR-H2 comprising the amino acid sequence of SEQ ID NO: 23, and a HVR-H3 comprising the amino acid sequence of SEQ ID NO: 24, and wherein the VL comprises a HVR-L1 comprising the amino acid sequence of SEQ ID NO: 25, a HVR-L2 comprising the amino acid sequence of SEQ ID NO: 26, and a HVR-L3 comprising the amino acid sequence of SEQ ID NO: 27. In some embodiments, the VH comprises the amino acid sequence of SEQ ID NO: 28, and/or the VL comprises the amino acid sequence of SEQ ID NO: 29. In some embodiments, the antibody comprises a heavy chain and a light chain, wherein the heavy chain comprises the amino acid sequence of SEQ ID NO: 30, and/or the light chain comprises the amino acid sequence of SEQ ID NO: 31. In some embodiments, the anti-CD137 antibody is administered at a dose of no more than 500 mg (*e.g.,* about 50 mg to about 200 mg, about 100 mg to about 200 mg, about 150 mg to about 500 mg, about 150 mg to about 300 mg, or about 300 mg to about 400 mg; for example, about 50 mg, about 100 mg, about 200 mg, about 300 mg or about 400 mg). In some embodiments, the anti-CD137 antibody is administered at a dose of about 0.1 mg/kg to about 10 mg/kg, such as about 3 mg/kg to about 8 mg/kg, *e.g.,* about 3 mg/kg, about 5 mg/kg, or about 8 mg/kg. In some embodiments, the anti-CD137 antibody is administered intravenously. In some embodiments, the anti-CD137 antibody is administered about once every three weeks. In some embodiments, the cancer is resistant or refractory to a prior therapy, such as an immunotherapy.

Cancer treatments can be evaluated by, e.g., tumor regression, tumor weight or size shrinkage, time to progression, duration of survival, progression free survival, overall response rate, duration of response, quality of life, protein expression and/or activity. Approaches to determining efficacy of therapy can be employed, including for example, measurement of response through radiological imaging.

The anti-CD137 antibodies and compositions provided by the present disclosure can be administered via any suitable enteral route or parenteral route of administration. The term "enteral route" of administration refers to the administration via any part of the gastrointestinal tract. Examples of enteral routes include oral, mucosal, buccal, and rectal route, or intragastric route. "Parenteral route" of administration refers to a route of administration other than enteral route. Examples of parenteral routes of administration include intravenous, intramuscular, intradermal, intraperitoneal, intratumor, intravesical, intraarterial, intrathecal, intracapsular, intraorbital, intracardiac, transtracheal, intraarticular, subcapsular, subarachnoid, intraspinal, epidural and intrasternal, subcutaneous, or topical administration. The antibodies and compositions of the disclosure can be administered using any suitable method, such as by oral ingestion, nasogastric tube, gastrostomy tube, injection, infusion, implantable infusion pump, and osmotic pump. The suitable route and method of administration may vary depending on a number of factors such as the specific antibody being used, the rate of absorption desired, specific formulation or dosage form used, type or severity of the disorder being treated, the specific site of action, and conditions of the patient, and can be readily selected by a person skilled in the art. In some embodiments, the anti-CD137 antibody is administered intravenously.

In some embodiments, the anti-CD137 antibody is administered at a flat dose. In some embodiments, the anti-CD137 antibody is administered at a dose of no more than any one of 500 mg, 475 mg, 450 mg, 425 mg, 400 mg, 390 mg, 380 mg, 370 mg, 360 mg, 350 mg, 340 mg, 330 mg, 320 mg, 310 mg, 300 mg, 275 mg, 250 mg, 225 mg, 200 mg, 175 mg, 150 mg, 125 mg, 100 mg, or less. In some embodiments, the dose of the anti-CD137 antibody is within any one of the following ranges, wherein the ranges have an upper limit of any one of: 500 mg, 475 mg, 450 mg, 425 mg, 400 mg, 390 mg, 380 mg, 370 mg, 360 mg, 350 mg, 340 mg, 330 mg, 320 mg, 310 mg, 300 mg, 275 mg, 250 mg, 225 mg, 200 mg, 175 mg, 150 mg, or 125 mg, and an independently selected lower limit of any one of 475 mg, 450 mg, 425 mg, 400 mg, 390 mg, 380 mg, 370 mg, 360 mg, 350 mg, 340 mg, 330 mg, 320 mg, 310 mg, 300 mg, 275 mg, 250 mg, 225 mg, 200 mg, 175 mg, 150 mg, 125 mg, or 100 mg, and wherein the lower limit is less than the upper limit. In some embodiments, the anti-CD137 antibody is administered at a dose of any one of about 50 mg to about 100 mg, about 50 mg to about 150 mg, about 50 mg to about 200 mg, about 50 mg to about 250 mg, about 50 mg to about 300 mg, about 50 mg to about 400 mg, about 50 mg to about 500 mg, about 150 mg to about 200 mg, about 150 mg to about 300 mg, about 150 mg to about 400 mg, about 150 mg to about 500 mg, about 100 mg to about 200 mg, about 200 mg to about 300 mg, about 300 mg to about 400 mg, about 400 mg to about 500 mg, about 100 mg to about 300 mg, about 300 mg to about 500 mg, about 200 mg to about 400 mg, about 100 mg to about 250 mg, about 250 mg to about 500 mg, about 250 mg to about 400 mg, about 100 mg to about 400 mg, about 200 mg to about 500 mg, or about 100 mg to about 500 mg. The doses described herein may refer to a suitable dose for a human, or an equivalent dose for the specific species of the subject. In some embodiments, the anti-CD137 antibody is administered at a dose equivalent to about 300 mg to about 500 mg (such as about 300 mg to about 400 mg) for a human subject. In some embodiments, the anti-CD137 antibody is administered at a dose equivalent to about 50 mg to about 200 mg (such as about 50 mg, about 100 mg, or about 400 mg) for a human subject. In some embodiments, the anti-CD137 antibody is administered at a dose equivalent to no more than 500 mg (such as no more than 400 mg, 300 mg, 200 mg, or 100 mg) for a human subject. In some embodiments, the anti-CD137 antibody is administered at a dose of about 100 mg to about 500 mg, such as about any one of 100, 150, 200, 250, 300, 350, 400, 450 or 500 mg. In some embodiments, the anti-CD137 antibody is administered at a dose of about 50 mg to about 200 mg, such as about any one of 50, 100, 150, or 200 mg. In some embodiments, the subject has a body weight of no more than about any one of 200 kg, 175 kg, 150 kg, 140 kg, 130 kg, 120 kg, 110 kg, or 100 kg. In some embodiments, the subject has a body weight of about about 40 kg to about 100 kg, 50 kg to about 100 kg, or about 60 kg to about 100 kg. In some embodiments, the anti-CD137 antibody is administered as a monotherapy. An exemplary monotherapy dose of the anti-CD1 37 antibody can be about 100 mg, about 200 mg, about 300 mg, or about 400 mg. In some embodiments, the anti-CD137 antibody is administered in combination with one or more additional therapeutic agents, such as an immune checkpoint inhibitor, e.g., an anti-PD-1 antibody. An exemplary dose of the anti-CD137 antibody in a combination therapy can be about 50 mg, about 100 mg, or about 200 mg.

In some embodiments, the anti-CD137 antibody is administered at a dose of no more than any one of 10 mg/kg, 9 mg/kg, 8 mg/kg, 7 mg/kg, 6 mg/kg, 5 mg/kg, 4 mg/kg, 3 mg/kg, 2 mg/kg, 1 mg/kg, 0.8 mg/kg, 0.6 mg/kg, 0.5 mg/kg, 0.4 mg/kg, 0.3 mg/kg, 0.2 mg/kg, 0.1 mg/kg, 0.08 mg/kg, 0.05 mg/kg, 0.04 mg/kg, or 0.03 mg/kg. In some embodiments, the dose of the anti-CD137 antibody is within any one of the following ranges, wherein the ranges have an upper limit of any one of: 10 mg/kg, 9 mg/kg, 8 mg/kg, 7 mg/kg, 6 mg/kg, 5 mg/kg, 4 mg/kg, 3 mg/kg, 2 mg/kg, 1 mg/kg, 0.8 mg/kg, 0.6 mg/kg, 0.5 mg/kg, 0.4 mg/kg, 0.3 mg/kg, 0.2 mg/kg, 0.1 mg/kg, 0.08 mg/kg, 0.05 mg/kg, or 0.04 mg/kg, and an independently selected lower limit of any one of 9 mg/kg, 8 mg/kg, 7 mg/kg, 6 mg/kg, 5 mg/kg, 4 mg/kg, 3 mg/kg, 2 mg/kg, 1 mg/kg, 0.8 mg/kg, 0.6 mg/kg, 0.5 mg/kg, 0.4 mg/kg, 0.3 mg/kg, 0.2 mg/kg, 0.1 mg/kg, 0.08 mg/kg, 0.05 mg/kg, 0.04 mg/kg, or 0.03 mg/kg, and wherein the lower limit is less than the upper limit. In some embodiments, the anti-CD1 37 antibody is administered at a dose of any one of about 0.03 mg/kg to about 10 mg/kg, about 0.1 mg/kg to about 10 mg/kg, about 0.3 mg/kg to about 10 mg/kg, about 1 mg/kg to about 10 mg/kg, about 3 mg/kg to about 10 mg/kg, about 5 mg/kg to about 10 mg/kg, about 0.03 mg/kg to about 0.1 mg/kg, about 0.1 mg/kg to about 0.3 mg/kg, about 0.3 mg/kg to about 1 mg/kg, about 1 mg/kg to about 3 mg/kg, about 3 mg/kg to about 5 mg/kg, about 0.1 mg/kg to about 3 mg/kg, or about 1 mg/kg to about 5 mg/kg. The doses described herein may refer to a suitable dose for a human, or an equivalent dose for the specific species of the subject. In some embodiments, the anti-CD137 antibody is administered at a dose equivalent to about 0.1 mg/kg to about 10 mg/kg (such as about 3 mg/kg to about 8 mg/kg, or about 5 mg/kg to about 10 mg/kg) for a human subject. In some embodiments, the anti-CD137 antibody is administered at a dose equivalent to no more than 10 mg/kg (such as no more than 8 mg/kg, or no more than 5 mg/kg) for a human subject. In some embodiments, the anti-CD137 antibody is administered at a dose of about 0.03 mg/kg to about 10 mg/kg, such as about any one of 0.03, 0.1, 0.3, 1, 3, 5, 8 or 10 mg/kg.

The effective amount of the anti-CD137 antibody may be administered in a single dose or in multiple doses. For methods that comprises administration of the anti-CD137 antibody in multiple doses, exemplary dosing frequencies include, but are not limited to, weekly, weekly without break, weekly for two out of three weeks, weekly for three out of four weeks, once every three weeks, once every two weeks, monthly, every six months, yearly, *etc.* In some embodiments, the anti-CD137 antibody is administered about weekly, once every 2 weeks, or once every 3 weeks. In some embodiments, the intervals between each administration are less than about any of 3 years, 2 years, 12 months, 11 months, 10 months, 9 months, 8 months, 7 months, 6 months, 5 months, 4 months, 3 months, 2 months, 1 month, 4 weeks, 3 weeks, 2 weeks, or 1 week. In some embodiments, the intervals between each administration are more than about any of 1 week, 2 weeks, 3 weeks, 4 weeks, 1 month, 2 months, 3 months, 4 months, 5 months, 6 months, 7 months, 8 months, 9 months, 10 months, 11 months, 12 months, 2 years, or 3 years. In some embodiments, there is no break in the dosing schedule.

In some embodiments, the anti-CD137 antibody is administered at a low frequency, for example, any one of no more frequent than once per week, once every other week, once per three weeks, once per month, once per 2 months, once per 3 months, once per 4 months, once per 5 months, once per 6 months, once per 7 months, once per 8 months, once per 9 months, once per 10 months, once per 11 months, once per year, or less. In some embodiments, the anti-CD137 antibody is administered in a single dose. In some embodiments, the anti-CD137 antibody is administered about once every three weeks.

In some embodiments, the anti-CD137 antibody is administered at a dose of no more than 500 mg, such as no more than any one of 400 mg, 350 mg, 300 mg, 250 mg, 200 mg, 150 mg, 100 mg or 50 mg once every three weeks. In some embodiments, the anti-CD137 antibody is administered at a dose of about 150 mg to about 500 mg, such as about any one of 150 mg, 200 mg, 250 mg, 300 mg, 350 mg, or 400 mg, once every three weeks. In some embodiments, the anti-CD137 antibody is administered at a dose of about 50 mg to about 200 mg, such as about any one of 50 mg, 100 mg, 150 mg, or 200 mg, once every three weeks.

In some embodiments, the anti-CD137 antibody is administered at a dose of no more than 10 mg/kg, such as no more than any one of 8 mg/kg, 5 mg/kg, 3 mg/kg, 2 mg/kg, or 1 mg/kg once every three weeks. In some embodiments, the anti-CD137 antibody is administered at a dose of about 0.03 mg/kg to about 10 mg/kg, such as about any one of 0.03, 0.1, 0.3, 1, 3, 5, 8, or 10mg/kg, once every three weeks.

In some embodiments, the anti-CD137 antibody is administered for 2 or more cycles, such as about any one of 2, 3, 4, 5, 6, 7, 8, 9, 10, 11, 12, or more cycles. The administration of the anti-CD137 antibody can be extended over an extended period of time, such as from about a week to about a month, from about a month to about a year, from about a year to about several years. In some embodiments, the anti-CD137 antibody is administered over a period of at least any of about 1 week, 2 weeks, 3 weeks, 4 weeks, 5 weeks, 1 month, 2 months, 3 months, 4 months, 5 months, 6 months, 7 months, 8 months, 9 months, 10 months, 11 months, 12 months, 1 year, 2 years, 3 years, 4 years, or more.

The methods described herein are useful for treating a variety of cancers. In some embodiments, the cancer is a solid cancer. In some embodiments, the cancer is a liquid cancer. A variety of cancers where CD137 is implicated, whether malignant or benign and whether primary or secondary, may be treated or prevented with a method provided by the disclosure. Examples of such cancers include lung cancers such as bronchogenic carcinoma (e.g., squamous cell carcinoma, small cell carcinoma, large cell carcinoma, and adenocarcinoma), alveolar cell carcinoma, bronchial adenoma, chondromatous hamartoma (noncancerous), and sarcoma (cancerous); heart cancer such as myxoma, fibromas, and rhabdomyomas; bone cancers such as osteochondromas, condromas, chondroblastomas, chondromyxoid fibromas, osteoid osteomas, giant cell tumors, chondrosarcoma, multiple myeloma, osteosarcoma, fibrosarcomas, malignant fibrous histiocytomas, Ewing's tumor (Ewing's sarcoma), and reticulum cell sarcoma; brain cancer such as gliomas (e.g., glioblastoma multiforme), anaplastic astrocytomas, astrocytomas, oligodendrogliomas, medulloblastomas, chordoma, Schwannomas, ependymomas, meningiomas, pituitary adenoma, pinealoma, osteomas, hemangioblastomas, craniopharyngiomas, chordomas, germinomas, teratomas, dermoid cysts, and angiomas; cancers in digestive system such as leiomyoma, epidermoid carcinoma, adenocarcinoma, leiomyosarcoma, stomach adenocarcinomas, intestinal lipomas, intestinal neurofibromas, intestinal fibromas, polyps in large intestine, and colorectal cancers; liver cancers such as hepatocellular adenomas, hemangioma, hepatocellular carcinoma, fibrolamellar carcinoma, cholangiocarcinoma, hepatoblastoma, and angiosarcoma; kidney cancers such as kidney adenocarcinoma, renal cell carcinoma, hypernephroma, and transitional cell carcinoma of the renal pelvis; bladder cancers; hematological cancers such as acute lymphocytic (lymphoblastic) leukemia, acute myeloid (myelocytic, myelogenous, myeloblastic, myelomonocytic) leukemia, chronic lymphocytic leukemia (e.g., Sezary syndrome and hairy cell leukemia), chronic myelocytic (myeloid, myelogenous, granulocytic) leukemia, Hodgkin's lymphoma, non-Hodgkin's lymphoma, B cell lymphoma, mycosis fungoides, and myeloproliferative disorders (including myeloproliferative disorders such as polycythemia vera, myelofibrosis, thrombocythemia, and chronic myelocytic leukemia); skin cancers such as basal cell carcinoma, squamous cell carcinoma, melanoma, Kaposi's sarcoma, and Paget's disease; head and neck cancers; eye-related cancers such as retinoblastoma and intraoccular melanocarcinoma; male reproductive system cancers such as benign prostatic hyperplasia, prostate cancer, and testicular cancers (e.g., seminoma, teratoma, embryonal carcinoma, and choriocarcinoma); breast cancer; female reproductive system cancers such as uterine cancer (endometrial carcinoma), cervical cancer (cervical carcinoma), cancer of the ovaries (ovarian carcinoma), vulvar carcinoma, vaginal carcinoma, fallopian tube cancer, and hydatidiform mole; thyroid cancer (including papillary, follicular, anaplastic, or medullary cancer); pheochromocytomas (adrenal gland); noncancerous growths of the parathyroid glands; pancreatic cancers; and hematological cancers such as leukemias, myelomas, non-Hodgkin's lymphomas, and Hodgkin's lymphomas.

In some embodiments, the subject has been previously treated with a prior therapy. In some embodiments, the subject has previously received any one of 1, 2, 3, 4, or more prior therapies. In some embodiments, the subject has exhausted all other available therapies. In some embodiments, the subject is unresponsive or resistant to a prior therapy. In some embodiments, the subject has disease reoccurrence subsequent to a prior therapy. In some embodiments, the subject is refractory to a prior therapy. In some embodiments, the subject has failed a prior therapy within about 1 year, 6 months, 3 months or less. In some embodiments, the subject has not previously received a prior therapy.

In some embodiments, the subject has been previously treated with a standard therapy for the cancer. In some embodiments, the subject is unresponsive or resistant to a standard therapy. In some embodiments, the subject has disease reoccurrence subsequent to a standard therapy. In some embodiments, the subject is refractory to a standard therapy. In some embodiments, the subject has failed a standard therapy within about 1 year, 6 months, 3 months or less. In some embodiments, the subject has not previously received a standard therapy. In some embodiments, the subject has refused or is ineligible for a standard therapy.

In some embodiments, the prior therapy (e.g., standard therapy) is selected from the group consisting of viral gene therapy, immunotherapy, targeted therapy, radiation therapy, and chemotherapy. In some embodiments, the prior therapy is selected from the group consisting of pomalyst, revlimid, lenalidomide, pomalidomide, thalidomide, a DNA-alkylating platinum-containing derivative, cisplatin, 5-fluorouracil, cyclophosphamide, an anti-CTLA-4 antibody, an anti-PD-1 antibody, an anti-PD-L1 antibody, an anti-CD20 antibody, an anti-CD40 antibody, an anti-DR5 antibody, an anti-CD1d antibody, an anti-TIM3 antibody, an anti-SLAMF7 antibody, an anti-KIR receptor antibody, an anti-OX40 antibody, an anti-HER2 antibody, an anti-ErbB-2 antibody, an anti-EGFR antibody, cetuximab, rituximab, trastuzumab, pembrolizumab, radiotherapy, single dose radiation, fractionated radiation, focal radiation, whole organ radiation, IL-12, IFNα, GM-CSF, a chimeric antigen receptor, adoptively transferred T cells, an anti-cancer vaccine, and an oncolytic virus.

One of ordinary skill in the art will recognize the presence and development of various therapies for different types of cancers, which are not restricted to those forms of therapy set forth herein. Examples of categories of prior therapies (including standard therapies) for treating cancer include (1) chemotherapeutic agents, (2) immunotherapeutic agents, and (3) hormone therapeutic agents.

The term "chemotherapeutic agent" refers to a chemical or biological substance that can cause death of cancer cells, or interfere with growth, division, repair, and/or function of cancer cells. Examples of chemotherapeutic agents include those that are disclosed in WO 2006/129163, and US 20060153808, the disclosures of which are incorporated herein by reference. Examples of particular chemotherapeutic agents include: (1) alkylating agents, such as chlorambucil (LEUKERAN®), mcyclophosphamide (CYTOXAN®), ifosfamide (IFEX®), mechlorethamine hydrochloride (MUSTARGEN®), thiotepa (THIOPLEX®), streptozotocin (ZANOSAR®), carmustine (BICNU®, GLIADEL WAFER®), lomustine (CEENU®), and dacarbazine (DTIC-DOME®); (2) alkaloids or plant vinca alkaloids, including cytotoxic antibiotics, such as doxorubicin (ADRIAMYCIN®), epirubicin (ELLENCE®, PHARMORUBICIN®), daunorubicin (CERUBIDINE®, DAUNOXOME®), nemorubicin, idarubicin (IDAMYCIN® PFS, ZAVEDOS®), mitoxantrone (DHAD®, NOVANTRONE®). dactinomycin (actinomycin D, COSMEGEN®), plicamycin (MITHRACIN®), mitomycin (MUTAMYCIN®), and bleomycin (BLENOXANE®), vinorelbine tartrate (NAVELBINE®), vinblastine (VELBAN®), vincristine (ONCOVIN®), and vindesine (ELDISINE®); (3) antimetabolites, such as capecitabine (XELODA®), cytarabine (CYTOSAR-U®), fludarabine (FLUDARA®), gemcitabine (GEMZAR®), hydroxyurea (HYDRA®), methotrexate (FOLEX®, MEXATE®, TREXALL®), nelarabine (ARRANON®), trimetrexate (NEUTREXIN®), and pemetrexed (ALIMTA®); (4) Pyrimidine antagonists, such as 5-fluorouracil (5-FU); capecitabine (XELODA®), raltitrexed (TOMUDEX®), tegafur-uracil (UFTORAL®), and gemcitabine (GEMZAR®); (5) taxanes, such as docetaxel (TAXOTERE®), paclitaxel (TAXOL®); (6) platinum drugs, such as cisplatin (PLATINOL®) and carboplatin (PARAPLATIN®), and oxaliplatin (ELOXATIN®); (7) topoisomerase inhibitors, such as irinotecan (CAMPTOSAR®), topotecan (HYCAMTIN®), etoposide (ETOPOPHOS®, VEPESSID®, TOPOSAR®), and teniposide (VUMON®); (8) epipodophyllotoxins (podophyllotoxin derivatives), such as etoposide (ETOPOPHOS®, VEPESSID®, TOPOSAR®); (9) folic acid derivatives, such as leucovorin (WELLCOVORIN®); (10) nitrosoureas, such as carmustine (BiCNU), lomustine (CeeNU); (11) inhibitors of receptor tyrosine kinase, including epidermal growth factor receptor (EGFR), vascular endothelial growth factor (VEGF), insulin receptor, insulin-like growth factor receptor (IGFR), hepatocyte growth factor receptor (HGFR), and platelet-derived growth factor receptor (PDGFR), such as gefitinib (IRESSA®), erlotinib (TARCEVA®), bortezomib (VELCADE®), imatinib mesylate (GLEEVEC®), genefitinib, lapatinib, sorafenib, thalidomide, sunitinib (SUTENT®), axitinib, rituximab (RITUXAN®, MABTHERA®), trastuzumab (HERCEPTIN®), cetuximab (ERBITUX®), bevacizumab (AVASTIN®), and ranibizumab (LUCENTIS®), lym-1 (ONCOLYM®), antibodies to insulin-like growth factor-1 receptor (IGF-1R) that are disclosed in WO2002/053596); (12) angiogenesis inhibitors, such as bevacizumab (AVASTIN®), suramin (GERMANIN®), angiostatin, SU5416, thalidomide, and matrix metalloproteinase inhibitors (such as batimastat and marimastat), and those that are disclosed in WO2002055106; and (13) proteasome inhibitors, such as bortezomib (VELCADE®).

The term "immunotherapeutic agents" refers to a chemical or biological substance that can enhance an immune response of a mammal. Examples of immunotherapeutic agents include: *bacillus* Calmette-Guerin (BCG); cytokines such as interferons; vaccines such as MyVax personalized immunotherapy, Onyvax-P, Oncophage, GRNVAC1, Favld, Provenge, GVAX®, Lovaxin C, BiovaxID, GMXX, and NeuVax; and antibodies such as alemtuzumab (CAMPATH®), bevacizumab (AVASTIN®), cetuximab (ERBITUX®), gemtuzunab ozogamicin (MYLOTARG®), ibritumomab tiuxetan (ZEVALIN®), panitumumab (VECTIBIX®), rituximab (RITUXAN®, MABTHERA®), trastuzumab (HERCEPTIN®), tositumomab (BEXXAR®), ipilimumab (YERVOY®) tremelimumab, CAT-3888, agonist antibodies to OX40 receptor (such as those disclosed in WO2009/079335), agonist antibodies to CD40 receptor (such as those disclosed in WO2003/040170, and TLR-9 agonists (such as those disclosed in WO2003/015711, WO2004/016805, and WO2009/022215).

The term "hormone therapeutic agent" refers to a chemical or biological substance that inhibits or eliminates the production of a hormone, or inhibits or counteracts the effect of a hormone on the growth and/or survival of cancerous cells. Examples of such agents suitable for the methods herein include those that are disclosed in US20070117809. Examples of particular hormone therapeutic agents include tamoxifen (NOLVADEX®), toremifene (FARESTON®), fulvestrant (FASLODEX®), anastrozole (ARIMIDEX®), exemestane (AROMASIN®), letrozole (FEMARA®), megestrol acetate (MEGACE®), goserelin (ZOLADEX®), and leuprolide (LUPRON®). The binding molecules of this disclosure may also be used in combination with non-drug hormone therapies such as (1) surgical methods that remove all or part of the organs or glands which participate in the production of the hormone, such as the ovaries, the testicles, the adrenal gland, and the pituitary gland, and (2) radiation treatment, in which the organs or glands of the patient are subjected to radiation in an amount sufficient to inhibit or eliminate the production of the targeted hormone.

Prior therapies (e.g., standard therapies) also encompass surgery to remove a tumor and radiation therapy. Exemplary radiation therapies include, but are not limited to, ionizing (electromagnetic) radiotherapy (e.g., X-rays or gamma rays) and particle beam radiation therapy (e.g., high linear energy radiation). The source of radiation can be external or internal to the subject.

In some embodiments, the subject has previously received an immunotherapy. In some embodiments, the subject is resistant or refractory to the immunotherapy. In some embodiments, the immunotherapy is an anti-CD20 antibody. In some embodiments, the immunotherapy is rituximab or a biosimilar thereof.

In some embodiments, the cancer is selected from the group consisting of colon cancer *(e.g.,* Sigmoid colon

26

cancer), breast cancer, lung cancer (e.g., non-small cell lung cancer or NSCLC), esophageal cancer, endometrial cancer, gastrointestinal cancer (e.g., gastrointestinal neuroectodermal tumor), cholangiocarcinoma, nasopharyngeal cancer (NPC), adenoid cystic carcinoma (ACC), melanoma, mesothelioma (e.g., malignant pleural mesothelioma or MPM), anal cancer, head and neck cancer (e.g., head and neck squamous cell carcinoma or HNSCC), mantle cell lymphoma, appendiceal and sebaceous cancer, follicular lymphoma, non-Hodgkin's lymphoma (NHL), and T cell lymphoma (e.g., angioimmunoblastic T-cell lymphoma or AITL, or Peripheral T-cell lymphoma or PTCL).

In some embodiments, the cancer is a non-Hodgkin's lymphoma (NHL). In some embodiments, the NHL arises from B-lymphocytes. In some embodiments, the cancer is a B cell lymphoma. In some embodiments, the cancer is a diffuse large B-cell lymphoma (DLBCL).

In some embodiments, the cancer is follicular lymphoma (FL). In some embodiments, the cancer is a low-grade FL. In some embodiments, the cancer is a high-grade FL. In some embodiments, the cancer is early stage FL, non-metastatic FL, primary FL, advanced FL, locally advanced FL, metastatic FL, FL in remission, or recurrent FL. In some embodiments, the FL has metastasized to other organs (e.g., lung, lymph nodes, or bone). In some embodiments, the FL is Grade 1-2, wherein the tumor has no more than 15 centroblasts per high-power field. In some embodiments, the FL is Grade 3A, wherein the tumor has more than 15 centroblasts per high-power field, and the tumor is a mix of centrocytes and centroblasts. In some embodiments, the FL is Grade 3B, wherein the tumor has more than 15 centroblasts per high-power field, and the tumor has sheets of large centroblasts and no centrocytes. In some embodiments, the subject having FL has previously received one or more prior therapies. In some embodiments, the subject is resistant to or refractory to the one or more prior therapies. In some embodiments, the subject has exhausted all available therapies for FL. Exemplary therapies for FL include, but are not limited to, antibody treatment (e.g., rituximab and obinutuzumab), chemotherapies (e.g., vinca alkaloids such as etoposide and vincristine, anthracyclines such as doxorubicin, alkylating agents such as bendamustine, cyclophosphamide and ifosfamide, or antimetabolites such as methotrexate and cytarabine), corticosteroids (e.g., prednisone and dexamethasone), kinase inhibitors (e.g., PI3K inhibitors such as idelalisib, duvelisib, and copalisib), immunomodulators (e.g., lenalidomide), radioimmunotherapy (e.g., ibritumomab tiuxetan), CAR T-cell immunotherapy (e.g., tisagenlecleucel and axicabtagene ciloleucel), radiation therapy (e.g., high-energy x-rays, involved-site radiation therapy or ISRT), stem cell transplant, and combinations thereof. FL can be diagnosed and monitored using known methods in the art, for example, by imaging tests, biopsy, hematopathology review, immunohistochemistry (IHC) panel or genetic tests, as described in the National Comprehensive Cancer Network (NCCN) Guidelines for Follicular Lymphoma.

In some embodiments, the cancer is T cell lymphoma (TCL). In some embodiments, the cancer is T-lymphoblastic lymphoma or leukemia. In some embodiments, the cancer is peripheral T-cell lymphoma. In some embodiments, the cancer is angioimmunoblastic T-cell lymphoma (AITL). In some embodiments, the cancer is extranodal natural killer/T-cell lymphoma, e.g., nasal type. In some embodiments, the cancer is enteropathy-associated intestinal T-cell lymphoma (EATL). In some embodiments, the cancer is lymph node/tonsil type of TCL. In some embodiments, the cancer is anaplastic large cell lymphoma (ALCL). In some embodiments, the cancer is peripheral T-cell lymphoma (PTCL). In some embodiments, the cancer is early stage TCL, non-metastatic TCL, primary TCL, advanced TCL, locally advanced TCL, metastatic TCL, TCL in remission, or recurrent TCL. In some embodiments, the TCL has metastasized to other organs (e.g., lung, lymph nodes, or bone). In some embodiments, the TCL is Stage I, wherein the cancer is in only one cluster of lymph nodes. In some embodiments, the TCL is Stage II, wherein the cancer is in 2 or more clusters either above or below the diaphragm of the subject. In some embodiments, the TCL is Stage III, wherein the cancer is in lymph tissue on both sides of the diaphragm of the subject. In some embodiments, the cancer is Stage IV, wherein the cancer has widely spread outside the lymphatic system. In some embodiments, the subject having TCL has previously received one or more prior therapies. In some embodiments, the subject is resistant to or refractory to the one or more prior therapies, such as targeted therapy, chemotherapy, steroids, immunotherapy, radiation therapy, stem cell transplant, and combinations thereof. In some embodiments, the subject has exhausted all available therapies for TCL. Exemplary therapies for TCL include, but are not limited to, alemtuzumab, belinostat, bendamustine hydrochloride, bortezomib, brentuximab vedotin, carboplatin, cisplatin, cyclophosphamide, cyclosporine, cytarabine, dexamethasone, doxorubicin, epirubicin, etoposide, gemcitabine, ifosfamide, lenalidomide, leucovorin calcium, mesna, methotrexate, methylprednisolone, oxaliplatin, pralatrexate, prednisone, romidepsin, vincristine sulfate, and combinations thereof. TCL can be diagnosed and monitored using known methods in the art, for example, by imaging tests (e.g., CT scan or PET scan), biopsy, hematopathology review, immunohistochemistry (IHC) panel or genetic tests, as described in the NCCN Guidelines for Peripheral T cell Lymphoma.

In some embodiments, the cancer is adenoid cystic carcinoma (ACC). In some embodiments, the ACC is a head and neck ACC. In some embodiments, the ACC is in breast, skin, respiratory system, and/or a reproductive organ. In some embodiments, the cancer is early stage ACC, non-metastatic ACC, primary ACC, advanced ACC, locally advanced ACC, metastatic ACC, ACC in remission, or recurrent ACC. In some embodiments, the ACC has metastasized to other organs (e.g., lung, lymph nodes, or bone). In some embodiments, the ACC is Grade I, wherein the subject has tumors having tubular and cribriform areas but without solid components. In some embodiments, the ACC is Grade II, wherein the subject has cribriform tumors that are either pure or mixed with less than 30% of solid areas. In some embodiments, the ACC is Grade III, wherein the subject has tumors with predominantly solid pattern. In some embodiments, the subject having ACC has previously received one or more prior therapies. In some embodiments, the subject is resistant to or refractory to the one or more prior therapies. In some embodiments, the subject has exhausted all available therapies for ACC. Exemplary therapies for ACC include, but are not limited to, surgery, chemotherapies (e.g., cisplatin, doxorubicin, cyclophosphamide, 5-FU, mitoxantrone, epirubicin, vinorelbine, paclitaxel, gemcitabine), targeted therapies (e.g., imatinib, dasatinib, cetuximab, gefitinib, lapatinib), kinase inhibitors, immunomodulators, and radiation therapy, and combinations thereof. ACC can be diagnosed and monitored using known methods in the art, for example, by imaging tests, biopsy, immunohistochemistry (IHC) panel or genetic tests.

The methods described herein are useful for various aspects of cancer treatment. In some embodiments, there is provided a method of inhibiting cell proliferation (such as tumor growth) in an individual, comprising administering to the individual an effective amount of any one of the anti-CD137 antibodies described herein. In some embodiments, at least about 10% (including for example at least about any of 20%, 30%, 40%, 60%, 70%, 80%, 90%, 95% or more) cell proliferation is inhibited.

In some embodiments, there is provided a method of inhibiting tumor metastasis in an individual, comprising administering to the individual an effective amount of any one of the anti-CD137 antibodies described herein. In some embodiments, at least about 10% (including for example at least about any of 20%, 30%, 40%, 60%, 70%, 80%, 90%, 95% or more) metastasis is inhibited.

In some embodiments, there is provided a method of reducing (such as eradicating) pre-existing tumor metastasis (such as metastasis to the lymph node) in an individual, comprising administering to the individual an effective amount of any one of the anti-CD137 antibodies described herein. In some embodiments, at least about 10% (including for example at least about any of 20%, 30%, 40%, 60%, 70%, 80%, 90%, 95% or more) metastasis is reduced.

In some embodiments, there is provided a method of reducing incidence or burden of preexisting tumor metastasis (such as metastasis to the lymph node) in an individual, comprising administering to the individual an effective amount of any one of the anti-CD137 antibodies described herein.

In some embodiments, there is provided a method of reducing tumor size in an individual, comprising administering to the individual an effective amount of any one of the anti-CD137 antibodies described herein. In some embodiments, the method reduces tumor size by at least about 10% (including for example at least about any of 20%, 30%, 40%, 60%, 70%, 80%, 90%, 95% or more).

In some embodiments, there is provided a method of prolonging time to disease progression of cancer in an individual, comprising administering to the individual an effective amount of any one of the anti-CD137 antibodies described herein. In some embodiments, the method prolongs the time to disease progression by at least any of 1, 2, 3, 4, 5, 6, 7, 8, 9, 10, 11, 12, 16, 20, 24, 28, 32, 36, or more weeks.

In some embodiments, there is provided a method of prolonging survival (e.g., overall survival or progression-free survival) of an individual having cancer, comprising administering to the individual an effective amount of any one of the anti-CD137 antibodies described herein. In some embodiments, the method prolongs the survival of the individual by at least any of 1, 2, 3, 4, 5, 6, 7, 8, 9, 10, 11, 12, 18, or 24 months.

In some embodiments, there is provided a method of alleviating one or more symptoms in an individual having cancer, comprising administering to the individual an effective amount of any one of the anti-CD137 antibodies described herein.

In some embodiments, there is provided a method of improving the quality of life in an individual having cancer, comprising administering to the individual an effective amount of any one of the anti-CD137 antibodies described herein.

The anti-CD137 antibody may be administered alone as monotherapy, or administered in combination with one or more additional therapeutic agents or therapies. In some embodiment, anti-CD137 antibody is administered in combination with one or more additional therapeutic agents for separate, sequential or simultaneous administration. The term "additional therapeutic agent" refers to any therapeutic agent other than an anti-CD137 antibody provided by the disclosure. In some embodiments, there is provided a combination therapy for treating cancer in a subject, which comprises administering to the subject a therapeutically effective amount of an anti-CD137 antibody described herein in combination with one or more additional therapeutic agents. In some embodiments, anti-CD137 antibody is administered in combination with one or more additional therapeutic agents comprising chemotherapeutic agents, immunotherapeutic agents, and/or hormone therapeutic agents. In some embodiments, the one or more additional therapeutic agents are selected from the group consisting of selected from the group consisting of viral gene therapy, immune checkpoint inhibitors, targeted therapies, radiation therapies, and chemotherapies.

Also provided are compositions of any one of the anti-CD137 antibodies described herein for use in the methods described in this section, and use of the anti-CD137 antibodies in the manufacture of a medicament for treating cancer (e.g., follicular lymphoma, T cell lymphoma, or ACC).

## Combination Therapy

The present application also provides combination therapies for treating cancer in a subject, which comprises administering to the subject a therapeutically effective amount of any one of the anti-CD137 antibodies described herein in combination with an one or more immune checkpoint inhibitors, and/or one or more chemotherapeutic agents.

In some embodiments, there is provided a method of treating a lung cancer in a subject, comprising administering to the subject: (a) an effective amount of an anti-CD137 antibody that specifically binds to an extracellular domain of human CD137, wherein the antibody binds to one or more amino acid residues selected from the group consisting of amino acid residues 51, 53, 62-73, 83, 89, 92, 95-104 and 112-116 of SEQ ID NO: 1; and (b) an effective amount of an immune checkpoint inhibitor.

In some embodiments, there is provided a method of treating a breast cancer (e.g., triple negative breast cancer) in a subject, comprising administering to the subject: (a) an effective amount of an anti-CD137 antibody that specifically binds to an extracellular domain of human CD137, wherein the antibody binds to one or more amino acid residues selected from the group consisting of amino acid residues 51, 53, 62-73, 83, 89, 92, 95-104 and 112-116 of SEQ ID NO: 1; and (b) an effective amount of an immune checkpoint inhibitor.

Immune checkpoint inhibitors are compounds that inhibit the activity of control mechanisms of the immune system. Immune system checkpoints, or immune checkpoints, are inhibitory pathways in the immune system that generally act to maintain self-tolerance or modulate the duration and amplitude of physiological immune responses to minimize collateral tissue damage. Checkpoint inhibitors can inhibit an immune system checkpoint by stimulating the activity of a stimulatory checkpoint molecule, or inhibiting the activity of an inhibitory checkpoint molecule in the pathway. Immune system checkpoint molecules include, but are not limited to, cytotoxic T-lymphocyte antigen 4 (CTLA-4), programmed cell death 1 protein (PD-1), programmed cell death 1 ligand 1 (PD-L1), programmed cell death 1 ligand 2 (PD-L2), lymphocyte activation gene 3 (LAG3), B7-1, B7-H3, B7-H4, T cell membrane protein 3 (TIM3), B- and T-lymphocyte attenuator (BTLA), V-domain immunoglobulin (Ig)-containing suppressor of T-cell activation (VISTA), Killer-cell immunoglobulin-like receptor (KIR), and A2A adenosine receptor (A2aR). As such, checkpoint inhibitors include antagonists of CTLA-4, PD-1, PD-L1, PD-L2, LAG3, B7-1, B7-H3, B7-H4, BTLA, VISTA, KIR, A2aR, or TIM3. For example, antibodies that bind to CTLA-4, PD-1, PD-L1, PD-L2, LAG3, B7-1, B7-H3, B7-H4, BTLA, VISTA, KIR, A2aR, or TIM3 and antagonize their function are checkpoint inhibitors. Moreover, any molecule (e.g., peptide, nucleic acid, small molecule, etc.) that inhibits the inhibitory function of an immune system checkpoint is a checkpoint inhibitor.

In some embodiments, the immune checkpoint inhibitor is an antibody that specifically binds to an immune checkpoint molecule. In some embodiments, the immune checkpoint inhibitor is selected from the group consisting of an anti-PD-1 antibody, an anti-PD-L1 antibody, and an anti-CTLA-4 antibody.

In some embodiments, the immune checkpoint inhibitor is an anti-PD-1 antibody. Exemplary anti-PD-1 antibodies include, but are not limited to, 2E5 (Cstone Pharmaceuticals), tislelizumab (BGB-A317), BGB-108, STI-A1110, AM0001, BI 754091, sintilimab (IBI308), cetrelimab (JNJ-63723283), toripalimab (JS-001), camrelizumab (SHR-1210, INCSHR-1210, HR-301210), MEDI-0680 (AMP-514), MGA-012 (INCMGA 0012), nivolumab (BMS-936558, MDX1106, ONO-4538), spartalizumab (PDR001), pembrolizumab (MK-3475, SCH 900475), PF-06801591, cemiplimab (REGN-2810, REGEN2810), dostarlimab (TSR-042, ANB011), pidilizumab (CT-011), FITC-YT-16 (PD-1 binding peptide), APL-501 or CBT-501 or genolimzumab (GB-226), AB-122, AK105, AMG 404, BCD-100, F520, HLX10, HX008, JTX-4014, LZM009, Sym021, PSB205, AMP-224 (fusion protein targeting PD-1), CX-188 (PD-1 probody), AGEN-2034, GLS-010, budigalimab (ABBV-181), AK-103, BAT-1306, CS-1003, AM-0001, TILT-123, BH-2922, BH-2941, BH-2950, ENUM-244C8, ENUM-388D4, HAB-21, H EISCOI 11-003, IKT-202, MCLA-134, MT-17000, PEGMP-7, PRS-332, RXI-762, STI-1110, VXM-10, XmAb-23104, AK-112, HLX-20, SSI-361, AT-16201, SNA-01, AB122, PD1-PIK, PF-06936308, RG-7769, CAB PD-1 Abs, AK-123, MEDI-3387, MEDI-5771, 4H1128Z-E27, REMD-288, SG-001, BY-24.3, CB-201, IBI-319, ONCR-177, Max-1, CS-4100, JBI-426, CCC-0701, CCX- 4503, biosimilars thereof, and derivatives thereof. In some embodiments, the antibodies that compete with any of these art-recognized antibodies for binding to PD-1 also can be used. In some embodiments, the immune checkpoint inhibitor is 2E5. 2E5 and related anti-PD-1 antibodies have been described, for example, in CN107840887A, which is incorporated herein by reference in its entirety. In some embodiments, the immune checkpoint inhibitor is toripalimab. Toripalimab and related anti-PD-1 antibodies have been described, for example, in US10066013B2, which is incorporated herein by reference in its entirety.

In some embodiments, the immune checkpoint inhibitor is an anti-PD-L1 antibody. Exemplary anti-PD-L1 antibodies include, but are not limited to, atezolizumab, avelumab, durvalumab (imfinzi), BGB-A333, SHR-1316 (HTI-1088), CK-301, BMS-936559, envafolimab (KN035, ASC22), CS1001, MDX-1105 (BMS-936559), LY3300054, STI-A1014, FAZ053, CX-072, INCB086550, GNS-1480, CA-170, CK-301, M-7824, HTI-1088 (HTI-131 , SHR-1316), MSB-2311, AK- 106, AVA-004, BBI-801, CA-327, CBA-0710, CBT-502, FPT-155, IKT-201, IKT-703, 10-103, JS-003, KD-033, KY-1003, MCLA-145, MT-5050, SNA-02, BCD-135, APL-502 (CBT-402 or TQB2450), IMC-001, KD-045, INBRX-105, KN-046, IMC-2102, IMC-2101, KD-005, IMM-2502, 89Zr-CX-072, 89Zr-DFO-6E11, KY-1055, MEDI-1109, MT-5594, SL-279252, DSP-106, Gensci-047, REMD-290, N-809, PRS-344, FS-222, GEN-1046, BH-29xx, FS-118, biosimilars thereof, and derivatives thereof. In some embodiments, the antibodies that compete with any of these art-recognized antibodies for binding to PD-L1 also can be used. In some embodiments, the immune checkpoint inhibitor is atezolizumab.

In some embodiments, the immune checkpoint inhibitor is an anti-CTLA-4 antibody. Exemplary anti-CTLA-4 antibodies include, but are not limited to, ipilimumab (IBI310, BMS-734016, MDX010, MDX-CTLA4, MEDI4736), tremelimumab (CP-675, CP-675,206), APL-509, AGEN1884, and CS1002, AGEN1181, Abatacept (Orencia, BMS-188667, RG2077), BCD-145, ONC-392, ADU-1604, REGN4659, ADG116, KN044, KN046, biosimilars thereof and derivatives thereof. In some embodiments, art recognized anti-CTLA-4 antibodies can be used. For example, the anti-CTLA-4 antibodies disclosed in: WO2019/149281, U.S. Patent No. 8,119,129, WO 01/14424, WO 98/42752; WO 00/37504 (CP675,206, also known as tremelimumab; formerly ticilimumab), U.S. Patent No. 6,207,156; WO2001014424, WO2000037504, and U.S. Patent No. 8,017,114; Hurwitz et al. (1998) Proc Natl Acad Sci USA 95(17): 10067-10071; Camacho et al. (2004) J Clin Oncology 22(145): Abstract No. 2505 (antibody CP-675206); and Mokyr et al. (1998) Cancer Res 58:5301-5304 can be used in the methods disclosed herein. The teachings of each of the aforementioned publications are hereby incorporated by reference. In some embodiments, the antibodies that compete with any of these art-recognized antibodies for binding to CTLA-4 also can be used. In some embodiments, the anti-CTLA-4 antibody is ADCi116. ADG116 (also known as TY21580) and related anti-CTLA-4 antibodies have been described, for example, in WO2019/149281, which is incorporated herein by reference in its entirety.

In some embodiments, there is provided a method of treating a lung cancer in a subject, comprising administering to the subject: (a) an effective amount of any one of the anti-CD137 antibodies described herein, and (b) an effective amount of an anti-PD-L1 antibody. In some embodiments, the anti-PD-L1 antibody is atezolizumab, a biosimilar thereof, or a derivative thereof. In some embodiments, the anti-CD137 antibody comprises a VH and a VL, wherein the VH comprises a HVR-H1 comprising the amino acid sequence of SEQ ID NO: 2, a HVR-H2 comprising the amino acid sequence of SEQ ID NO: 3, and a HVR-H3 comprising the amino acid sequence of SEQ ID NO: 4, and wherein the VL comprises a HVR-L1 comprising the amino acid sequence of SEQ ID NO: 5, a HVR-L2 comprising the amino acid sequence of SEQ ID NO: 6, and a HVR-L3 comprising the amino acid sequence of SEQ ID NO: 7. In some embodiments, the anti-CD137 antibody comprises a VH comprising the amino acid sequence of SEQ ID NO: 8, and/or a VL comprises the amino acid sequence of SEQ ID NO: 9. In some embodiments, the anti-CD137 antibody comprises a heavy chain and a light chain, and wherein the heavy chain comprises the amino acid sequence of SEQ ID NO: 10, and/or the light chain comprises the amino acid sequence of SEQ ID NO: 11. In some embodiments, the anti-PD-L1 antibody comprises a VH and a VL, wherein the VH comprises a HVR-H1 comprising the amino acid sequence of SEQ ID NO: 56, a HVR-H2 comprising the amino acid sequence of SEQ ID NO: 57, and a HVR-H3 comprising the amino acid sequence of SEQ ID NO: 58, and wherein the VL comprises a HVR-L1 comprising the amino acid sequence of SEQ ID NO: 59, a HVR-L2 comprising the amino acid sequence of SEQ ID NO: 60, and a HVR-L3 comprising the amino acid sequence of SEQ ID NO: 61. In some embodiments, the anti-PD-L1 antibody comprises a VH comprising the amino acid sequence of SEQ ID NO: 62, and/or a VL comprising the amino acid sequence of SEQ ID NO: 63.

In some embodiments, there is provided a method of treating a lung cancer in a subject, comprising administering to the subject: (a) an effective amount of any one of the anti-CD137 antibodies described herein, and (b) an effective amount of an anti-PD-1 antibody. In some embodiments, the anti-PD-1 antibody is 2E5, a biosimilar thereof, or a derivative thereof. In some embodiments, the anti-PD-1 antibody is toripalimab, a biosimilar thereof, or a derivative thereof. In some embodiments, the anti-CD137 antibody comprises a VH and a VL, wherein the VH comprises a HVR-H1 comprising the amino acid sequence of SEQ ID NO: 2, a HVR-H2 comprising the amino acid sequence of SEQ ID NO: 3, and a HVR-H3 comprising the amino acid sequence of SEQ ID NO: 4, and wherein the VL comprises a HVR-L1 comprising the amino acid sequence of SEQ ID NO: 5, a HVR-L2 comprising the amino acid sequence of SEQ ID NO: 6, and a HVR-L3 comprising the amino acid sequence of SEQ ID NO: 7. In some embodiments, the anti-CD137 antibody comprises a VH comprising the amino acid sequence of SEQ ID NO: 8, and/or a VL comprises the amino acid sequence of SEQ ID NO: 9. In some embodiments, the anti-CD137 antibody comprises a heavy chain and a light chain, and wherein the heavy chain comprises the amino acid sequence of SEQ ID NO: 10, and/or the light chain comprises the amino acid sequence of SEQ ID NO: 11. In some embodiments, the anti-PD-1 antibody comprises a VH and a VL, wherein the VH comprises a HVR-H1 comprising the amino acid sequence of SEQ ID NO: 64, a HVR-H2 comprising the amino acid sequence of SEQ ID NO: 65, and a HVR-H3 comprising the amino acid sequence of SEQ ID NO: 66, and wherein the VL comprises a HVR-L1 comprising the amino acid sequence of SEQ ID NO: 67, a HVR-L2 comprising the amino acid sequence of SEQ ID NO: 68, and a HVR-L3 comprising the amino acid sequence of SEQ ID NO: 69. In some embodiments, the anti-PD-1 antibody comprises a VH comprising the amino acid sequence of SEQ ID NO: 70, and/or a VL comprising the amino acid sequence of SEQ ID NO: 71. In some embodiments, the anti-PD-1 antibody comprises a VH and a VL, wherein the VH comprises a HVR-H1 comprising the amino acid sequence of SEQ ID NO: 82, a HVR-H2 comprising the amino acid sequence of SEQ ID NO: 83, and a HVR-H3 comprising the amino acid sequence of SEQ ID NO: 84, and wherein the VL comprises a HVR-L1 comprising the amino acid sequence of SEQ ID NO: 85, a HVR-L2 comprising the amino acid sequence of SEQ ID NO: 86, and a HVR-L3 comprising the amino acid sequence of SEQ ID NO: 87. In some embodiments, the anti-PD-1 antibody comprises a VH comprising the amino acid sequence of SEQ ID NO: 88, and/or a VL comprising the amino acid sequence of SEQ ID NO: 89. In some embodiments, the anti-PD-1 antibody is administered at a dose of about 240 mg. In some embodiments, the anti-PD-1 antibody is administered once every three weeks. In some embodiments, the anti-CD137 antibody is administered at a dose of about 50 mg, about 100 mg, or about 200 mg. In some embodiments, the anti-CD137 antibody is adminsitered once every three weeks.

In some embodiments, there is provided a method of treating a breast cancer (e.g., triple negative breast cancer) in a subject, comprising administering to the subject: (a) an effective amount of any one of the anti-CD137 antibodies described herein, and (b) an effective amount of an anti-CTLA-4 antibody. In some embodiments, the anti-CTLA-4 antibody is ADG116, a biosimilar thereof, or a derivative thereof. In some embodiments, the anti-CD137 antibody comprises a VH and a VL, wherein the VH comprises a HVR-H1 comprising the amino acid sequence of SEQ ID NO: 2, a HVR-H2 comprising the amino acid sequence of SEQ ID NO: 3, and a HVR-H3 comprising the amino acid sequence of SEQ ID NO: 4, and wherein the VL comprises a HVR-L1 comprising the amino acid sequence of SEQ ID NO: 5, a HVR-L2 comprising the amino acid sequence of SEQ ID NO: 6, and a HVR-L3 comprising the amino acid sequence of SEQ ID NO: 7. In some embodiments, the anti-CD137 antibody comprises a VH comprising the amino acid sequence of SEQ ID NO: 8, and/or a VL comprises the amino acid sequence of SEQ ID NO: 9. In some embodiments, the anti-CD137 antibody comprises a heavy chain and a light chain, and wherein the heavy chain comprises the amino acid sequence of SEQ ID NO: 10, and/or the light chain comprises the amino acid sequence of SEQ ID NO: 11. In some embodiments, the anti-CTLA-4 antibody comprises a VH and a VL, wherein the VH comprises a HVR-H1 comprising the amino acid sequence of SEQ ID NO: 48, a HVR-H2 comprising the amino acid sequence of SEQ ID NO: 49, and a HVR-H3 comprising the amino acid sequence of SEQ ID NO: 50, and wherein the VL comprises a HVR-L1 comprising the amino acid sequence of SEQ ID NO: 51, a HVR-L2 comprising the amino acid sequence of SEQ ID NO: 52, and a HVR-L3 comprising the amino acid sequence of SEQ ID NO: 53. In some embodiments, the anti-CTLA-4 antibody comprises a VH comprising the amino acid sequence of SEQ ID NO: 54, and/or a VL comprising the amino acid sequence of SEQ ID NO: 55.

In some embodiments, there is provided a method of treating a lung cancer in a subject, comprising administering to the subject: (a) an effective amount of an anti-CD137 antibody that specifically binds to an extracellular domain of human CD137, wherein the antibody binds to one or more amino acid residues selected from the group consisting of amino acid residues 51, 53, 62-73, 83, 89, 92, 95-104 and 112-116 of SEQ ID NO: 1; and (b) an effective amount of a chemotherapeutic agent.

In some embodiments, there is provided a method of treating a breast cancer (e.g., triple negative breast cancer) in a subject, comprising administering to the subject: (a) an effective amount of an anti-CD137 antibody that specifically binds to an extracellular domain of human CD137, wherein the antibody binds to one or more amino acid residues selected from the group consisting of amino acid residues 51, 53, 62-73, 83, 89, 92, 95-104 and 112-116 of SEQ ID NO: 1; and (b) an effective amount of a chemotherapeutic agent.

Exemplary chemotherapeutic agents include, but are not limited to, taxoids, e.g., paclitaxel, docetaxel; and platinum coordination complexes, such as cisplatin, oxaliplatin, and carboplatin. In some embodiments, the chemotherapeutic agent is docetaxel *(e.g.,* TAXOTERE®). In some embodiments, the chemotherapeutic agent is cisplatin *(e.g.,* PLATINOL® or PLATINOL-AQ®).

In some embodiments, there is provided a method of treating a breast cancer *(e.g.,* triple negative breast cancer) in a subject, comprising administering to the subject: (a) an effective amount of any one of the anti-CD137 antibodies described herein, and (b) an effective amount of a taxoid (e.g., docetaxel). In some embodiments, the anti-CD137 antibody comprises a VH and a VL, wherein the VH comprises a HVR-H1 comprising the amino acid sequence of SEQ ID NO: 2, a HVR-H2 comprising the amino acid sequence of SEQ ID NO: 3, and a HVR-H3 comprising the amino acid sequence of SEQ ID NO: 4, and wherein the VL, comprises a HVR-L1 comprising the amino acid sequence of SEQ ID NO: 5, a HVR-L2 comprising the amino acid sequence of SEQ ID NO: 6, and a HVR-L3 comprising the amino acid sequence of SEQ ID NO: 7. In some embodiments, the anti-CD137 antibody comprises a VH comprising the amino acid sequence of SEQ ID NO: 8, and/or a VL comprises the amino acid sequence of SEQ ID NO: 9. In some embodiments, the anti-CD137 antibody comprises a heavy chain and a light chain, and wherein the heavy chain comprises the amino acid sequence of SEQ ID NO: 10, and/or the light chain comprises the amino acid sequence of SEQ ID NO: 11.

In some embodiments, there is provided a method of treating a lung cancer in a subject, comprising administering to the subject: (a) an effective amount of any one of the anti-CD137 antibodies described herein, and (b) an effective amount of a platinum coordination complex (e.g., cisplatin). In some embodiments, the anti-CD137 antibody comprises a VH and a VL, wherein the VH comprises a HVR-H1 comprising the amino acid sequence of SEQ ID NO: 2, a HVR-H2 comprising the amino acid sequence of SEQ ID NO: 3, and a HVR-H3 comprising the amino acid sequence of SEQ ID NO: 4, and wherein the VL comprises a HVR-L1 comprising the amino acid sequence of SEQ ID NO: 5, a HVR-L2 comprising the amino acid sequence of SEQ ID NO: 6, and a HVR-L3 comprising the amino acid sequence of SEQ ID NO: 7. In some embodiments, the anti-CD137 antibody comprises a VH comprising the amino acid sequence of SEQ ID NO: 8, and/or a VL comprises the amino acid sequence of SEQ ID NO: 9. In some embodiments, the anti-CD137 antibody comprises a heavy chain and a light chain, and wherein the heavy chain comprises the amino acid sequence of SEQ ID NO: 10, and/or the light chain comprises the amino acid sequence of SEQ ID NO: 11. In some embodiments, the method does not comprise administering to the subject paclitaxel. In some embodiments, the method does not comprise administering to the subject pemetrexed.

In some embodiments, there is provided a method of treating a lung cancer in a subject, comprising administering to the subject: (a) an effective amount of an anti-CD137 antibody that specifically binds to an extracellular domain of human CD137, wherein the antibody binds to one or more amino acid residues selected from the group consisting of amino acid residues 51, 53, 62-73, 83, 89, 92, 95-104 and 112-116 of SEQ ID NO: 1; and (b) an effective amount of an anti-CD20 antibody.

Exemplary anti-CD20 antibodies include, but are not limited to, rituximab, obinutuzumab, B-Lyl, 11B8, AT80, HI47, 2C6, 2F2, 2H7 and GA101, biosimilars thereof, and derivatives thereof. In some embodiments, the anti-CD20 antibody is a type I anti-CD20 antibody. In some embodiments, the anti-CD20 antibody is a type II anti-CD20 antibody. In some embodiments, art recognized anti-CD20 antibodies can be used. For example, the anti-CD-20 antibodies disclosed in U.S. Pat. No. 7,879,984, WO2005/044859, WO2004/035607, WO2005/103081, WO2004/056312, WO2007/031875, and WO2015/095410 can be used in the methods disclosed herein. The teachings of each of the aforementioned publications are hereby incorporated by reference. In some embodiments, the antibodies that compete with any of these art-recognized antibodies for binding to CD-20 also can be used. In some embodiments, the anti-CD20 antibody is rituximab.

In some embodiments, there is provided a method of treating a lung cancer in a subject, comprising administering to the subject: (a) an effective amount of any one of the anti-CD137 antibodies described herein, and (b) an effective amount of an anti-CD20 antibody (e.g., rituximab). In some embodiments, the anti-CD137 antibody comprises a VH and a VL, wherein the VH comprises a HVR-H1 comprising the amino acid sequence of SEQ ID NO: 2, a HVR-H2 comprising the amino acid sequence of SEQ ID NO: 3, and a HVR-H3 comprising the amino acid sequence of SEQ ID NO: 4, and wherein the VL comprises a HVR-L1 comprising the amino acid sequence of SEQ ID NO: 5, a HVR-L2 comprising the amino acid sequence of SEQ ID NO: 6, and a HVR-L3 comprising the amino acid sequence of SEQ ID NO: 7. In some embodiments, the anti-CD137 antibody comprises a VH comprising the amino acid sequence of SEQ ID NO: 8, and/or a VL comprises the amino acid sequence of SEQ ID NO: 9. In some embodiments, the anti-CD137 antibody comprises a heavy chain and a light chain, and wherein the heavy chain comprises the amino acid sequence of SEQ ID NO: 10, and/or the light chain comprises the amino acid sequence of SEQ ID NO: 11.

In some embodiments, there is provided a method of treating a colorectal (*e.g.*, colon) cancer in a subject, comprising administering to the subject: (a) an effective amount of an anti-CD137 antibody that specifically binds to an extracellular domain of human CD137, wherein the antibody binds to one or more amino acid residues selected from the group consisting of amino acid residues 51, 53, 62-73, 83, 89, 92, 95-104 and 112-116 of SEQ ID NO: 1; and (b) an effective amount of a radiation therapy.

Exemplary radiation therapies include, but are not limited to, ionizing (electromagnetic) radiotherapy (e.g., X-rays or gamma rays) and particle beam radiation therapy (e.g., high linear energy radiation). The source of radiation can be external or internal to the subject. In some embodiments, the radiation therapy is local radiation. In some embodiments, the radiation therapy is single dose radiation. In some embodiments, the radiation therapy is high-dose radiation, e.g., at least about any one of 10, 20, 30, 40, 50, or more Gy.

In some embodiments, there is provided a method of treating a colon cancer in a subject, comprising administering to the subject: (a) an effective amount of any one of the anti-CD137 antibodies described herein, and (b) an effective amount of a radiation therapy (e.g., high-energy x-rays, involved-site radiation therapy or ISRT). In some embodiments, the anti-CD137 antibody comprises a VH and a VL, wherein the VH comprises a HVR-H1 comprising the amino acid sequence of SEQ ID NO: 2, a HVR-H2 comprising the amino acid sequence of SEQ ID NO: 3, and a HVR-H3 comprising the amino acid sequence of SEQ ID NO: 4, and wherein the VL comprises a HVR-L1 comprising the amino acid sequence of SEQ ID NO: 5, a HVR-L2 comprising the amino acid sequence of SEQ ID NO: 6, and a HVR-L3 comprising the amino acid sequence of SEQ ID NO: 7. In some embodiments, the anti-CD137 antibody comprises a VH comprising the amino acid sequence of SEQ ID NO: 8, and/or a VL comprises the amino acid sequence of SEQ ID NO: 9. In some embodiments, the anti-CD137 antibody comprises a heavy chain and a light chain, and wherein the heavy chain comprises the amino acid sequence of SEQ ID NO: 10, and/or the light chain comprises the amino acid sequence of SEQ ID NO: 11. In some embodiments, the radiation therapy is local radiation.

III. **Biomarkers**

The present application also provides biomarkers, which can be used in conjunction with any one of the methods of treatment described herein. Suitable biomarkers include total CD137, membrane bound CD137 (mCD137), soluble CD137 (sCD137), CD137L, Ki67, PD-L1, FcγRIIb, tumor mutational burden (TMB), CD20, Microsatellite instability (MSI), inflammatory cytokines (e.g., TNFα, IFNγ, IL-2, IL-6, IL-10), peripheral blood immune cell profiles, such as absolute cell counts for circulating T cells, natural killer (NK) cells, B cells, effector T cell subpopulations (e.g., CD4+ and CD8+ T cells), and memory T cell subpopulations (e.g.,effector memory T ($T_{em}$) cells), and regulatory T ($T_{reg}$) cells.

In some embodiments, there is provided a method of treating or delaying progression of cancer in a subject by administering an effective amount of an anti-CD137 antibody based on a level of one or more biomarkers selected from the group consisting of mCD137, sCD137, CD137L, Ki67, PD-L1, Fc$\gamma$RIIb, TMB, CD20, MSI, TNF$\alpha$, IFN$\gamma$, IL-2, IL-6, IL-10, NK cells, $T_{em}$ cells and $T_{reg}$ cells in one or more samples obtained from the subject

In some embodiments, there is provided a method of determining whether a subject is likely to respond to a therapy comprising an anti-CD137 antibody by determining a level of one or more biomarkers selected from the group consisting of total CD137, mCD137, sCD137, CD137L, Ki67, PD-L1, Fc$\gamma$RIIb, TMB, CD20, MSI, TNF$\alpha$, IFN$\gamma$, IL-2, IL-6, IL-10, NK cells, $T_{em}$ cells and $T_{reg}$ cells in one or more samples obtained from the subject. In some embodiments, there is provided a method of treating or delaying progression of cancer in a subject by administering an effective amount of an anti-CD137 antibody after it has been determined that the subject is likely to respond to the anti-CD137 antibody. In some embodiments, the therapy further comprises an immune checkpoint inhibitor, such as an anti-PD-1 antibody.

In some embodiments, there is provided a method of selecting a subject to receive or not to receive a therapy comprising an anti-CD137 antibody based on a level of one or more biomarkers selected from the group consisting of total CD137, mCD137, sCD137, CD137L, Ki67, PD-L1, Fc$\gamma$RIIb, TMB, CD20, MSI, TNF$\alpha$, IFNy, IL-2, IL-6, IL-10, NK cells, $T_{em}$ cells and $T_{reg}$ cells in one or more samples obtained from the subject. In some embodiments, the therapy further comprises an immune checkpoint inhibitor, such as an anti-PD-1 antibody.

In some embodiments, there is provided a method of predicting responsiveness and/or monitoring treatment and/or responsiveness of a subject to a therapy comprising an anti-CD137 antibody by determining a level of one or more biomarkers selected from the group consisting of total CD137, mCD137, sCD137, CD137L, Ki67, PD-L1, Fc$\gamma$RIIb, TMB, CD20, MSI, TNF$\alpha$, IFN$\gamma$, IL-2, IL-6, IL-10, NK cells, $T_{em}$ cells and $T_{reg}$ cells in one or more samples obtained from the subject. In some embodiments, the therapy further comprises an immune checkpoint inhibitor, such as an anti-PD-1 antibody.

In some embodiments, there is provided a method of positively and/or negatively stratifying patients into particular treatment regimen groups based upon a level of one or more biomarkers selected from the group consisting of total CD137, mCD137, sCD137, CD137L, Ki67, PD-L1, Fc$\gamma$RIIb, TMB, CD20, MSI, TNF$\alpha$, IFN$\gamma$, IL-2, IL-6, IL-10, NK cells, $T_{em}$ cells and $T_{reg}$ cells in one or more samples obtained from the patients. In some embodiments, the treatment comprises administration of an anti-CD137 antibody. In some embodiments, the treatment further comprises administration of an immune checkpoint inhibitor, such as an anti-PD-1 antibody.

In some embodiments, there is provided an assay for determining a level of one or more biomarkers selected from the group consisting of total CD137, mCD137, sCD137, CD137L, Ki67, PD-L1, Fc$\gamma$RIIb, TMB, CD20, MSI, TNF$\alpha$, IFNy, IL-2, IL-6, TL-10, NK cells, $T_{em}$ cells and $T_{reg}$ cells in one or more samples obtained from the subject. In some embodiments, the assay is an immunohistochemistry (IHC) assay. In some embodiments, the assay is a multiplex IHC assay capable of detecting two or more biomarkers. In some embodiments, the assay is an immunoassay, such as a Meso Scale Discovery (MSD) assay.

In some embodiments, there is provided a method of treating a cancer in a subject, comprising administering to the subject an effective amount of an anti-CD137 antibody that specifically binds to an extracellular domain of human CD137, wherein the antibody binds to one or more amino acid residues selected from the group consisting of amino acid residues 51, 53, 62-73, 83, 89, 92, 95-104 and 112-116 of SEQ ID NO: 1; and wherein the subject has a high level in one or more biomarkers selected from the group consisting of total CD137, membrane bound CD137 (mCD137), CD137L and PD-L1 and/or a low level of CD8+ effector memory T ($T_{em}$) cells or natural killer (NK) cells compared to a reference level. In some embodiments, the level of one or more biomarkers comprises a level of CD137 (e.g., in a tumor biopsy sample). In some embodiments, the level of one or more biomarkers comprises a level of sCD137 in a blood sample (e.g., a plasma sample). In some embodiments, the level of one or more biomarkers comprises a level of mCD137 on CD8$^+$ T cells. In some embodiments, the level of one or more biomarkers comprises a level of Ki67 on CD8$^+$ T cells. In some embodiments, the CD8$^+$ T cells are tumor infiltrating T cells. In some embodiments, the level of one or more biomarkers comprises a level of CD137L (e.g., in a tumor biopsy sample). In some embodiments, the level of one or more biomarkers comprises a level of NK cells in a blood sample. In some embodiments, the sample is a tumor biopsy sample. In some embodiments, the sample is a FFPE sample. In some embodiments, the level of the one or more biomarkers is detected by IHC. In some embodiments, the anti-CD137 antibody is administered at a dose of no more than 500 mg (e.g., about 50 mg to about 200 mg, about 100 mg to about 200 mg, about 150 mg to about 500 mg, about 150 mg to about 300 mg, or about 300 mg to about 400 mg; for example, about 50 mg, about 100 mg, about 200 mg, about 300 mg or about 400 mg). In some embodiments, the anti-CD137 antibody is administered at a dose of about 0.1 mg/kg to about 10 mg/kg, such as about 3 mg/kg to about 8 mg/kg, e.g., about 3 mg/kg, about 5 mg/kg, or about 8 mg/kg. In some embodiments, the anti-CD137 antibody is administered intravenously. In some embodiments, the anti-CD137 antibody is administered about once every three weeks. In some embodiments, the cancer is selected from the group consisting of colon cancer (e.g., Sigmoid colon cancer), breast cancer, lung cancer (e.g., non-small cell lung cancer or NSCLC), esophageal cancer, endometrial cancer, gastrointestinal cancer (e.g., gastrointestinal neuroectodermal tumor), cholangiocarcinoma, nasopharyngeal cancer (NPC), adenoid cystic carcinoma (ACC), melanoma, mesothelioma (e.g., malignant pleural mesothelioma or MPM), anal cancer, head and neck cancer (e.g., head and neck squamous cell carcinoma or HNSCC), appendiceal and sebaceous cancer, mantle cell lymphoma, follicular lymphoma, non-Hodgkin's lymphoma (NHL), and T cell lymphoma (e.g., angioimmunoblastic T-cell lymphoma or AITL, or Peripheral T-cell lymphoma or PTCL). In some embodiments, the cancer is resistant or refractory to a prior therapy, such as an immunotherapy. In some embodiments, the method further comprises administering to the subject an effective amount of an anti-PD-1 antibody (e.g., toripalimab).

In some embodiments, there is provided a method of treating a cancer in a subject, comprising: (a) administering to the subject an effective amount of an anti-CD137 antibody that specifically binds to an extracellular domain of human CD137, wherein the antibody binds to one or more amino acid residues selected from the group consisting of amino acid residues 51, 53, 62-73, 83, 89, 92, 95-104 and 112-116 of SEQ ID NO: 1; and (b) subsequently determining a level of one or more biomarkers selected from the group consisting of total CD137, membrane bound (mCD137), soluble CD137 (sCD137), CD137L, Ki67, NK cells, CD8+ effector memory T ($T_{em}$) cells, regulatory T ($T_{reg}$) cells and NK cells in a sample of the subject. In some embodiments, the level of one or more biomarkers comprises a level of CD137 (e.g., in a tumor biopsy sample). In some embodiments, the level of one or more biomarkers comprises a level of sCD137 in a blood sample (e.g., a plasma sample). In some embodiments, the level of one or more biomarkers comprises a level of mCD137 on CD8+ T cells. In some embodiments, the level of one or more biomarkers comprises a level of Ki67 on CD8+ T cells. In some embodiments, the CD8+ T cells are tumor infiltrating T cells. In some embodiments, the level of one or more biomarkers comprises a level of CD137L (e.g., in a tumor biopsy sample). In some embodiments, the level of one or more biomarkers comprises a level of NK cells in a blood sample. In some embodiments, the sample is a tumor biopsy sample. In some embodiments, the sample is an FFPE sample. In some embodiments, the level of the one or more biomarkers is detected by IHC. In some embodiments, the anti-CD137 antibody is administered at a dose of no more than 500 mg (e.g., about 50 mg to about 200 mg, about 100 mg to about 200 mg, about 150 mg to about 500 mg, about 150 mg to about 300 mg, or about 300 mg to about 400 mg; for example, about 50 mg, about 100 mg, about 200 mg, about 300 mg or about 400 mg). In some embodiments, the anti-CD137 antibody is administered at a dose of about 0.1 mg/kg to about 10 mg/kg, such as about 3 mg/kg to about 8 mg/kg, e.g., about 3 mg/kg, about 5 mg/kg, or about 8 mg/kg. In some embodiments, the anti-CD137 antibody is administered intravenously. In some embodiments, the anti-CD137 antibody is administered about once every three weeks. In some embodiments, the cancer is selected from the group consisting of colon cancer (e.g., Sigmoid colon cancer), breast cancer, lung cancer (e.g., non-small cell lung cancer or NSCLC), esophageal cancer, endometrial cancer, gastrointestinal cancer (e.g., gastrointestinal neuroectodermal tumor), cholangiocarcinoma, nasopharyngeal cancer (NPC), adenoid cystic carcinoma (ACC), melanoma, mesothelioma (e.g., malignant pleural mesothelioma or MPM), anal cancer, head and neck cancer (e.g., head and neck squamous cell carcinoma or HNSCC), appendiceal and sebaceous cancer, mantle cell lymphoma, follicular lymphoma, non-Hodgkin's lymphoma (NHL), and T cell lymphoma (e.g., angioimmunoblastic T-cell lymphoma or AITL, or Peripheral T-cell lymphoma or PTCL). In some embodiments, the cancer is resistant or refractory to a prior therapy, such as an immunotherapy. In some embodiments, an increased level of one or more biomarkers selected from the group consisting of total CD137, sCD137, Ki67, CD137L, NK cells, and CD8 $T_{em}$ cells, and/or a decreased level of one or more biomarkers selected from the group consisting of mCD137 and $T_{reg}$ cells after administration of the anti-CD137 antibody compared to the level of the one or more biomarkers before administration of the anti-CD137 antibody indicates that the subject may benefit from the administration of the ant-CD137 antibody. In some embodiments, wherein the sample has an increased level of one or more biomarkers selected from the group consisting of total CD137, sCD137, Ki67, CD137L, NK cells, and CD8 $T_{em}$ cells, and/or a decreased level of one or more biomarkers selected from the group consisting of mCD137 and $T_{reg}$ cells after administration of the anti-CD137 antibody compared to the level of the one or more biomarkers before administration of the anti-CD137 antibody, the method further comprises administering to the subject an effective amount of the anti-CD137 antibody. In some embodiments, the method further comprises administering to the subject an effective amount of an anti-PD-1 antibody (e.g., toripalimab).

In some embodiments, there is provided a method of providing a prognosis for a subject who has been administered with an effective amount of an anti-CD137 antibody that specifically binds to an extracellular domain of human CD137, wherein the antibody binds to one or more amino acid residues selected from the group consisting of amino acid residues 51, 53, 62-73, 83, 89, 92, 95-104 and 112-116 of SEQ ID NO: 1; the method comprising determining a level of one or more biomarkers selected from the group consisting of total CD137, membrane bound (mCD137), soluble CD137 (sCD137), CD137L, Ki67, NK cells, CD8+ effector memory T ($T_{em}$) cells, regulatory T ($T_{reg}$) cells and NK cells in a sample of the subject, wherein an increased level of one or more biomarkers selected from the group consisting of total CD137, sCD137, Ki67, CD137L, NK cells, and CD8 $T_{em}$ cells, and/or a decreased level of one or more biomarkers selected from the group consisting of mCD137 and $T_{reg}$ cells after administration of the anti-CD137 antibody compared to the level of the one or more biomarkers before administration of the anti-CD137 antibody identifies the subject as having a high likelihood of responding to the anti-CD137 antibody treatment. In some embodiments, the level of one or more biomarkers comprises a level of CD137 (e.g., in a tumor biopsy sample). In some embodiments, the level of one or more biomarkers comprises a level of sCD137 in a blood sample (e.g., a plasma sample). In some embodiments, the level of one or more biomarkers comprises a level of mCD137 on CD8+ T cells. In some embodiments, the level of one or more biomarkers comprises a level of Ki67 on CD8+ T cells. In some embodiments, the CD8+ T cells are tumor infiltrating T cells. In some embodiments, the level of one or more biomarkers comprises a level of CD137L (e.g., in a tumor biopsy sample). In some embodiments, the level of one or more biomarkers comprises a level of NK cells in a blood sample. In some embodiments, the sample is a tumor biopsy sample. In some embodiments, the sample is an FFPE sample. In some embodiments, the level of the one or more biomarkers is detected by IHC. In some embodiments, the anti-CD137 antibody is administered at a dose of no more than 500 mg (e.g., about 50 mg to about 200 mg, about 100 mg to about 200 mg, about 150 mg to about 500 mg, about 150 mg to about 300 mg, or about 300 mg to about 400 mg; for example, about 50 mg, about 100 mg, about 200 mg, about 300 mg or about 400 mg). In some embodiments, the anti-CD137 antibody is administered at a dose of about 0.1 mg/kg to about 10 mg/kg, such as about 3 mg/kg to about 8 mg/kg, e.g., about 3 mg/kg, about 5 mg/kg, or about 8 mg/kg. In some embodiments, the anti-CD137 antibody is administered intravenously. In some embodiments, the anti-CD137 antibody is administered about once every three weeks. In some embodiments, the cancer is selected from the group consisting of colon cancer (e.g., Sigmoid colon cancer), breast cancer, lung cancer (e.g., non-small cell lung cancer or NSCLC), esophageal cancer, endometrial cancer, gastrointestinal cancer (e.g., gastrointestinal neuroectodermal tumor), cholangiocarcinoma, nasopharyngeal cancer (NPC), adenoid cystic carcinoma (ACC), melanoma, mesothelioma (e.g., malignant pleural mesothelioma or MPM), anal cancer, head and neck cancer (e.g., head and neck squamous cell carcinoma or HNSCC), appendiceal and sebaceous cancer, mantle cell lymphoma, follicular lymphoma, non-Hodgkin's lymphoma (NHL), and T cell lymphoma (e.g., angioimmunoblastic T-cell lymphoma or AITL, or Peripheral T-cell lymphoma or PTCL). In some embodiments, the cancer is resistant or refractory to a prior therapy, such as an immunotherapy. In some embodiments, the method further comprises administering to the subject an effective amount of an anti-PD-1 antibody (e.g., toripalimab).

In some embodiments, the method comprises determining the level of total CD137 in a sample of the subject. In some embodiments, determining the level of CD137 in a sample comprises measuring the level of CD137 in a tumor biopsy sample, such as an FFPE sample. In some embodiments, determining the level of CD137 in a sample comprises measuring the level of protein expression of CD137. In some embodiments, an increase in the level of CD137 after receiving an anti-CD137 antibody in a subject indicates that the subject is likely to respond to the anti-CD137 antibody treatment, e.g., the subject is likely to have stable disease. In some embodiments, the level of CD137 increases by at least about 10%, 20%, 30%, 50%, 60%, 70%, 80%, 90%, 100%, 150%, 2 fold, 3 fold, 4 fold, 5 fold or more in a subject likely to respond to the anti-CD137 antibody treatment after the subject receives an anti-CD137 antibody than before the subject receives the anti-CD137 antibody. In some embodiments, the level of total CD137 is percentage of nucleated cells expressing CD137 as determined by an IHC assay. In some embodiments, an increase in the CD137 level of at least about 5% after the subject receives an anti-CD137 antibody than before the subject receives the anti-CD137 antibody indicates that the subject is likely to benefit from the anti-CD137 antibody treatment.

In some embodiments, the method comprises determining the level of mCD137 in a sample of the subject. In some embodiments, determining the level of mCD137 in a sample comprises measuring the level of mCD137 on CD8$^+$ T cells, such as tumor infiltrating T cells. In some embodiments, determining the level of mCD137 in a sample comprises measuring the level of protein expression of mCD137. In some embodiments, the amino acid sequence of human mCD137 is SEQ ID NO: 1. In some embodiments, a decrease in the level of mCD137 after receiving an anti-CD137 antibody in a subject indicates that the subject is likely to respond to the anti-CD137 antibody treatment, e.g., the subject is likely to have stable disease. In some embodiments, the level of mCD137 decreases by at least about 10%, 20%, 30%, 50%, 60%, 70%, 80%, 90% or more in a subject likely to respond to the anti-CD137 antibody treatment after the subject receives an anti-CD137 antibody than before the subject receives the anti-CD137 antibody. In some embodiments, a high level of mCD137 prior to receiving an anti-CD137 antibody in a subject compared to a reference level (e.g., a mCD137 level of a healthy subject) indicates that the subject is likely to respond to the anti-CD137 antibody treatment. In some embodiments, the high level is at least about any one of 50%, 100%, 150%, 2 fold, 2.5 fold, 3 fold, 4 fold, 5 fold or more than the reference level.

In some embodiments, the method comprises determining the level of sCD137 in a sample of the subject In some embodiments, determining the level of sCD137 in a sample comprises measuring the level of sCD137 in a blood sample such as a blood sample (e.g., a plasma sample). In some embodiments, determining the level of sCD137 in a sample comprises measuring the level of protein expression of sCD137. In some embodiments, the amino acid sequence of human sCD137 is SEQ ID NO: 43. In some embodiments, an increase in the level of sCD137 after receiving an anti-CD137 antibody in a subject indicates that the subject is likely to respond to the anti-CD137 antibody treatment, e.g., the subject is likely to have stable disease. In some embodiments, the level of sCD137 increases by at least about 10%, 20%, 30%, 50%, 60%, 70%, 80%, 90%, 100%, 150%, 2 fold, 3 fold, 4 fold, 5 fold, 6 fold, 7 fold, 8 fold, 9 fold, 10 fold, 12 fold, 15 fold, 20 fold or more in a subject likely to respond to the anti-CD137 antibody treatment after the subject receives an anti-CD137 antibody than before the subject receives the anti-CD137 antibody. In some embodiments, the method comprises determining a ratio ("induction ratio") between the difference in the sCD137 level after administration of the anti-CD137 antibody (C2) and the sCD137 level before administration of the anti-CD137 antibody (C1) and the sCD137 level before administration of the anti-CD137 antibody (C1): (C2-C1)/C1. In some embodiments, an induction ratio of at least about any one of 5, 6, 7, 8, 9, 10 or more indicates a high likelihood of responding to the anti-CD137 antibody treatment, i.e., having stable disease (SD) or partial response (PR) after the treatment.

In some embodiments, the method comprises determining the level of CD137L in a sample. In some embodiments, determining the level of CD137L in a sample comprises measuring the level of CD137L in a tumor tissue. In some embodiments, determining the level of CD137L in a sample comprises measuring the level of CD137L in a tumor biopsy sample, such as an FFPE sample. In some embodiments, determining the level of CD137L in a sample comprises measuring the level of expression of a nucleic acid molecule encoding CD137L (*e.g.*, measuring the level of RNA (such as pre-mRNA or mRNA) transcript expression from a gene encoding CD137L) and/or measuring the level of protein expression of CD137L. In some embodiments, the nucleic acid sequence encoding human CD137L is SEQ ID NO: 44. In some embodiments, the amino acid sequence of human CD137L is SEQ ID NO: 45. Methods for measuring the level of CD137L have been described, for example, in WO2019105468A1, which is incorporated herein by reference in its entirety. In some embodiments, an increase in the level of CD137L after receiving an anti-CD137 antibody in a subject indicates that the subject is likely to respond to the anti-CD137 antibody treatment, e.g., the subject is likely to have stable disease. In some embodiments, the level of CD137L increases by at least about 10%, 20%, 30%, 50%, 60%, 70%, 80%, 90% or more in a subject likely to respond to the anti-CD137 antibody treatment after the subject receives an anti-CD137 antibody than before the subject receives the anti-CD137 antibody. In some embodiments, a high level of CD137L prior to receiving an anti-CD137 antibody in a subject compared to a reference level *(e.g.,* a CD137L level of a healthy subject) indicates that the subject is likely to respond to the anti-CD137 antibody treatment. In some embodiments, the high level is at least about any one of 50%, 100%, 150%, 2 fold, 2.5 fold, 3 fold, 4 fold, 5 fold or more than the reference level.

In some embodiments, the level of CD137L is percentage of nucleated cells expressing CD137 as determined by an IHC assay. In some embodiments, a CD137L level of at least about 15%, such as at least about any one of 15%, 20%, 25%, 30%, 35%, 40%, 45%, or more, indicates that the subject is likely to respond to the anti-CD137 antibody treatment.

In some embodiments, there is provided a method of selecting a subject for a therapy comprising administration of an anti-CD137 antibody, comprising: a) determining a level of CD137L in a sample of the subject, and b) selecting the subject for the therapy if the level of CD137L is higher than a reference level. In some embodiments, the reference level is expression of CD137L in at least about 15% of nucleated cells in the sample. In some embodiments, the sample is a tumor biopsy sample (e.g., an FFPE sample). In some embodiments, the level of CD137L is determined by IHC. In some embodiments, the therapy further comprises administering to the subject an effective amount of an anti-PD-1 antibody *(e.g.,* toripalimab).

In some embodiments, there is provided a method of treating cancer in a subject, comprising administering an effective amount of an anti-CD137 antibody to the subject, if the level of CD137L in a sample of the subject is higher than a reference level. In some embodiments, the reference level is expression of CD137L in at least about 15% of nucleated cells in the sample. In some embodiments, the sample is a tumor biopsy sample *(e.g.,* an FFPE sample). In some embodiments, the level of CD137L is determined by IHC. In some embodiments, the method further comprises administering to the subject an effective amount of an anti-PD-1 antibody *(e.g.,* toripalimab). In some embodiments, the method further comprises the steps of: a) obtaining the sample from the subject; and b) measuring the level of expression of CD137L in the sample prior to administration of the anti-CD137 antibody to the subject

In some embodiments, there is provided a method of treating cancer in a subject, comprising administering an effective amount of an anti-CD137 antibody to the subject, wherein it has been determined that the subject is likely to respond to the anti-CD137 antibody when the level of CD137L in a sample of the subject is higher than a reference level. In some embodiments, the reference level is expression of CD137L in at least about 15% of nucleated cells in the sample. In some embodiments, the sample is a tumor biopsy sample *(e.g.,* an FFPE sample). In some embodiments, the level of CD137L is determined by IHC. In some embodiments, the method further comprises administering to the subject an effective amount of an anti-PD-1 antibody *(e.g.,* toripalimab).

In some embodiments, there is provided a method of determining if a subject is likely to respond to a therapy comprising an anti-CD137 antibody, the method comprising: a) obtaining a sample from the subject; b) measuring the level of expression of CD137L in the sample; and c) determining that the subject is likely to respond to the therapy when the level of expression of CD137L in the sample is higher than a reference level. In some embodiments, the reference level is expression of CD137L in at least about 15% of nucleated cells in the sample. In some embodiments, the sample is a tumor biopsy sample *(e.g.,* an FFPE sample). In some embodiments, the level of CD137L is determined by IHC. In some embodiments, the therapy further comprises administering to the subject an effective amount of an anti-PD-1 antibody (e.g., toripalimab).

In some embodiments, the CD137L expression level is determined using an anti-CD137L antibody. Exemplary anti-CD137L antibodies suitable for determination of the CD137L expression level have been described, for example, in PCT/CN2020/094371, which is incorporated herein by reference in its entirety. In some embodiments, the anti-CD137L antibody binds to an intracellular or transmembrane region of human CD137 ligand (CD137L). In some embodiments, the anti-CD137L antibody specifically binds to a peptide comprising the amino acid sequence of MEYASDASLDPEAPWPPAPRARACRVLP (SEQ ID NO:72) and/or binds to a peptide comprising the amino acid sequence of MEYASDASLDPEAPWPPAPRARA (SEQ ID NO:73). In some embodiments, the anti-CD137L antibody comprises a heavy chain variable region comprises an HVR-H1 comprising the amino acid sequence of SEQ ID NO:74, an HVR-H2 comprising the amino acid sequence of SEQ ID NO:75, and an HVR-H3 comprising the amino acid sequence of SEQ ID NO:76, and a light chain variable region comprises an HVR-L1 comprising the amino acid sequence of SEQ ID NO:77, an HVR-L2 comprising the amino acid sequence of SEQ ID NO:78, and an HVR-L3 comprising the amino acid sequence of SEQ ID NO:79. In some embodiments, the anti-CD137L antibody comprises a heavy chain variable region comprises the amino acid sequence of SEQ ID NO:80, and a light chain variable region comprises the amino acid sequence of SEQ ID NO:81. In some embodiments, the anti-CD137L antibody is TY23561. In some embodiments, the CD137L expression level is determined by contacting a human tissue sample *(e.g.,* a tumor biopsy sample) with the anti-CD137L antibody and detecting binding of the antibody or antigen-binding fragment to the sample by immunohistochemistry (IHC), binding of the anti-CD137L antibody to the sample indicates the level of expression of human CD137L in the sample.

In some embodiments, the method comprises determining the level of PD-L1 in a sample. In some embodiments, determining the level of PD-L1 in a sample comprises measuring the level of PD-L1 on tumor cells. In some embodiments, determining the level of PD-L1 in a sample comprises measuring the level of expression of a nucleic acid molecule encoding PD-L1 *(e.g.,* measuring the level of RNA (such as pre-mRNA or mRNA) transcript expression from a gene encoding PD-L1) and/or measuring the level of protein expression of PD-L1. In some embodiments, the nucleic acid sequence encoding human PD-L1 has GenBank Accession No. NM_001267706.1. In some embodiments, the amino acid sequence of human PD-L1 is SEQ ID NO: 46. In some embodiments, a decrease in the level of PD-L1 after receiving an anti-CD137 antibody in a subject indicates that the subject is likely to respond to the anti-CD137 antibody treatment, *e.g.,* the subject is likely to have stable disease. In some embodiments, the level of PD-L1 decreases by at least about 10%, 20%, 30%, 50%, 60%, 70%, 80%, 90% or more in a subject likely to respond to the anti-CD137 antibody treatment after the subject receives an anti-CD137 antibody than before the subject receives the anti-CD137 antibody. In some embodiments, a high level of PD-L1 prior to receiving an anti-CD137 antibody in a subject compared to a reference level *(e.g.,* a PD-L1 level of a healthy subject) indicates that the subject is likely to respond to the anti-CD137 antibody treatment. In some embodiments, the high level is at least about any one of 50%, 100%, 150%, 2 fold, 2.5 fold, 3 fold, 4 fold, 5 fold or more than the reference level.

In some embodiments, the method comprises determining the level of Ki67 in a sample. In some embodiments, determining the level of Ki67 in a sample comprises measuring the level of Ki67 on CD8$^+$ T cells, such as tumor infiltrating T cells. In some embodiments, determining the level of Ki67 in a sample comprises measuring the level of expression of a nucleic acid molecule encoding Ki67 (e.g., measuring the level of RNA (such as pre-mRNA or mRNA) transcript expression from a gene encoding Ki67) and/or measuring the level of protein expression of Ki67. In some embodiments, the nucleic acid sequence encoding human Ki67 has GenBank Accession No. NM_001145966.2. In some embodiments, the amino acid sequence of human Ki67 is SEQ ID NO: 47. In some embodiments, an increase in the level of Ki67 after receiving an anti-CD137 antibody in a subject indicates that the subject is likely to respond to the anti-CD137 antibody treatment, *e.g.,* the subject is likely to have stable disease. In some embodiments, the level of Ki67 increases by at least about 10%, 20%, 30%, 50%, 60%, 70%, 80%, 90%, 100%, 150%, 2 fold, 3 fold, 4 fold, 5 fold or more in a subject likely to respond to the anti-CD137 antibody treatment after the subject receives an anti-CD137 antibody than before the subject receives the anti-CD137 antibody.

In some embodiments, the level of a biomarker in a sample is measured by determining the level of RNA transcript expression of the biomarker. Suitable methods of measuring RNA transcript levels in a sample are known in the art, including, for example, by Northern blot analysis, nuclease protection assays, *in situ* hybridization, PCR analysis *(e.g.,* qPCR, RT-PCR, RT-qPCR, *etc.*)*,* and next generation sequencing *(e.g.,* RNAseq). In some embodiments, the level of transcript expression of the biomarker is measured by RT-PCR, *in situ* hybridization, and/or RNAseq.

In some embodiments, the level of a biomarker in a sample is measured by determining the level of protein expression of the biomarker. Suitable methods of measuring protein expression in a sample are known in the art, including, for example, immunoassays (e.g., Meso Scale Discovery or MSD assay), immunohistochemistry (IHC), PET imaging, Western blotting, enzyme-linked immunosorbent assays (ELISAs), flow cytometry, and mass spectrometry. In some embodiments, the level of protein expression of the biomarker is measured by immunoassay, Western blotting, ELISA, IHC, and/or flow cytometry.

In some embodiments, the biomarker is tumor mutational burden (TMD). Suitable methods for determining levels of TMD in a sample are known in the art, including, for example, by DNAseq.

In some embodiments, the biomarker is MSI. Suitable methods for determining levels of microsatellite instability in a sample are known in the art, including, for example, by DNAseq. *See,* for example, Forbes, S.A. et al. Nucleic Acids Res. 2011 39. In some embodiments, MSI is determined from a plurality of loci that are associated with microsatellite instability. In some embodiments, the MSI is determined from at least about 50 loci, at least about 60 loci, at least about 70 loci, at least about 80 loci, at least about 90 loci, at least about 100 loci, or more.

In some embodiments, the biomarker is a cell population. Suitable methods for determining levels of cell populations in a sample are known in the art, including, for example, fluorescence-activated cell sorting (FACS).

In some embodiments, the method comprises determining the level of NK cells in a sample, such as a blood sample, *e.g.,* peripheral blood sample. In some embodiments, an increase in the level of NK cells after receiving an anti-CD137 antibody in a subject indicates that the subject is likely to respond to the anti-CD137 antibody treatment, *e.g.,* the subject is likely to have stable disease. In some embodiments, the level of NK cells increases by at least about 10%, 20%, 30%, 50%, 60%, 70%, 80%, 90% or more in a subject likely to respond to the anti-CD137 antibody treatment after the subject receives an anti-CD137 antibody than before the subject receives the anti-CD137 antibody. In some embodiments, the method comprises determining a ratio ("induction ratio") between the difference in the NK cell level after administration of the anti-CD137 antibody (C2) and the NK cell level before administration of the anti-CD137 antibody (C1) and the NK cell level before administration of the anti-CD137 antibody (C1): (C2-C1)/C1. In some embodiments, an induction ratio of at least about any one of 40%, 50%, 60%, 70%, 80%, 90%, 100%, or more indicates a high likelihood of responding to the anti-CD137 antibody treatment, *i.e.,* having stable disease (SD) or partial response (PR) after the treatment. In some embodiments, a low level of NK cells prior to receiving an anti-CD137 antibody in a subject compared to a reference level *(e.g.,* a level of NK cells of a healthy subject) indicates that the subject is likely to respond to the anti-CD137 antibody treatment. In some embodiments, the low level is no more than any one of 90%, 80%, 70%, 60%, 50%, 40%, 30%, 20%, 10% or less than the reference level.

In some embodiments, the method comprises determining the level of $T_{em}$ cells in a sample. In some embodiments, the $T_{em}$ cells are CD8+ $T_{em}$ cells. In some embodiments, the $T_{em}$ cells are CD45RO+ CCR7- L-selectin- T cells. In some embodiments, the $T_{em}$ cells have intermediate to high expression of CD44. In some embodiments, the level of $T_{em}$ cells increases by at least about 10%, 20%, 30%, 50%, 60%, 70%, 80%, 90%, 100%, 150%, 2 fold, 3 fold, 4 fold, 5 fold or more in a subject likely to respond to the anti-CD137 antibody treatment after the subject receives an anti-CD137 antibody than before the subject receives the anti-CD137 antibody. In some embodiments, a low level of $T_{em}$ cells prior to receiving an anti-CD137 antibody in a subject compared to a reference level *(e.g.,* a level of $T_{em}$ cells of a healthy subject) indicates that the subject is likely to respond to the anti-CD137 antibody treatment. In some embodiments, the low level is no more than any one of 90%, 80%, 70%, 60%, 50%, 40%, 30%, 20%, 10% or less than the reference level.

In some embodiments, the method comprises determining the level of $T_{reg}$ cells in a sample. In some embodiments, the $T_{reg}$ cells are CD4+ CD25+ FOXP3+ T cells. In some embodiments, a decrease in the level of $T_{reg}$ cells after receiving an anti-CD137 antibody in a subject indicates that the subject is likely to respond to the anti-CD137 antibody treatment, *e.g.,* the subject is likely to have stable disease. In some embodiments, the level of $T_{reg}$ cells decreases by at least about 10%, 20%, 30%, 50%, 60%, 70%, 80%, 90% or more in a subject likely to respond to the anti-CD137 antibody treatment after the subject receives an anti-CD137 antibody than before the subject receives the anti-CD137 antibody.

In some embodiments, the level of one or more biomarkers is measured in one or more (e.g., one or more, two or more, three or more, four or more, etc.) samples obtained from a subject. Any suitable sample in the form of tissues and/or fluids that are known or believed to contain diseased cells and/or the target of interest may be used in the methods described herein, including, for example, sputum, pleural fluid, lymph fluid, bone marrow, blood, plasma, serum, urine, tissue samples (samples known or expected to contain cancer cells), tumor samples, tumor biopsies, etc. In some embodiments, the sample is a serum sample. In some embodiments, the sample is a tumor sample. In some embodiments, the sample is a tumor biopsy. In some embodiments, the sample comprises one or more cancer cells.

Methods of obtaining suitable tissue and/or fluid samples *(e.g.,* methods that are appropriate for obtaining a representative sample from a particular type, location, disease tissue, *etc.)* are well known to one of ordinary skill in the art, including, for example, by resection, bone marrow biopsy or bone marrow aspiration, endoscopic biopsy or endoscopic aspiration *(e.g.,* cystoscopy, bronchoscopy, colonoscopy, *etc.),* needle biopsy or needle aspiration *(e.g.,* fine needle aspiration, core needle biopsy, vacuum-assisted biopsy, image-guided biopsy, *etc.)* skin biopsy *(e.g.,* shave biopsy, punch biopsy, incisional biopsy, excisional biopsy, *etc.),* various other surgical tissue *(e.g.,* tumor tissue) biopsy and/or excision strategies, and fluid collections *(e.g.,* collecting urine, blood, serum, plasma, sputum, *etc.).*

In some embodiments, the one or more samples obtained from the subject are enriched for diseased *(e.g.,* cancerous) cells. Methods of enriching a tissue or fluid preparation for diseased *(e.g.,* cancerous) cells are known in the art, including, for example, by separating diseased *(e.g.,* cancerous) cells from normal cells by flow cytometry. In some embodiments, the level of one or more biomarkers is measured in the enriched samples. In some embodiments, the level of one or more biomarkers is measured in samples that have not been enriched or otherwise altered after isolation.

In some embodiments, the one or more samples are fixed *(i.e.* preserved) by conventional methodology (*See e.g.,* "Manual of Histological Staining Method of the Armed Forces Institute of Pathology," 3rd edition (1960) Lee G. Luna, HT (ASCP) Editor, The Blakston Division McGraw-Hill Book Company, New York; The Armed Forces Institute of Pathology Advanced Laboratory Methods in Histology and Pathology (1994) Ulreka V. Mikel, Editor, Armed Forces Institute of Pathology, American Registry of Pathology, Washington, D.C.). The choice of a fixative may be determined by one of ordinary skill in the art for the purpose for which the sample is to be analyzed. The length of fixation will depend upon the size and type of the tissue sample and the fixative used *(e.g.,* neutral buffered formalin, paraformaldehyde, *etc.),* as will be appreciated by one of ordinary skill in the art. In some embodiments, the level of one or more biomarkers is measured in a sample that is fixed. In some embodiments, the level of one or more biomarkers is measured in samples that have not been fixed or otherwise altered after isolation.

In some embodiments, one or more samples are obtained from the subject prior to administration with an anti-CD137 antibody. In some embodiments, one or more samples are obtained from the subject after administration of a first and/or subsequent dose of an anti-CD137 antibody. In some embodiments, one or more samples are obtained from the subject after completion of an anti-CD137 antibody therapy. In some embodiments, one or more samples are obtained from the subject, prior to, during, and after completion of an anti-CD137 antibody therapy.

In some embodiments, the method comprises comparing the level of a biomarker in a sample obtained from a subject to a reference level of the biomarker. In some embodiments, the reference level is the level of the biomarker in a reference sample *(e.g.,* a reference cell (such as a cell line, including but not limited to Raji (ATCC, CC-86) or Daudi (ATCC, CCL-213) cell lines), a corresponding sample taken from one or more patients determined to be responsive to anti-CD137 antibody therapy, a corresponding sample taken from one or more patients determined to be non-responsive to anti-CD137 antibody therapy, a corresponding adjacent normal tissue, *etc.).* In some embodiments, the reference level is measured in the reference sample using the same method as was used to measure the level of the biomarker in the subject's sample. In some embodiments, the reference level is measured in the reference sample using a different method than was used to measure the level of the biomarker in the subject's sample.

In some embodiments, the reference level is a pre-determined level of a biomarker *(e.g.,* the average level of the biomarker in a database of diseased samples (such as tissue biopsies or serum samples) isolated from multiple reference patients; the average level of the biomarker in a database of samples (such as tissue biopsies or serum samples) isolated from multiple healthy reference patients; *etc.).*

In some embodiments, the reference level of a biomarker refers to a detectable level of expression. That is to say, in some embodiments, the level of a biomarker measured in the sample obtained from the subject is considered to be lower than a reference level when the level of the biomarker in the sample is undetectable, e.g., below the limit of detection.

In some embodiments, the level of a biomarker measured in the sample obtained from the subject is considered to be lower than the reference level when the level of the biomarker in the sample is at least about 25% lower than the reference level. For example, the level of a biomarker measured in the sample obtained from the subject is considered to be lower than the reference level when the level of the biomarker in the sample is at least about 25%, at least about 30%, at least about 35%, at least about 40%, at least about 45%, at least about 50%, at least about 55%, at least about 60%, at least about 65%, at least about 70%, at least about 75%, at least about 80%, at least about 85%, at least about 90%, at least about 95%, or at least about 99% lower than the reference level. In some embodiments, the level of a biomarker measured in the sample obtained from the subject is considered to be lower than the reference level when the level of the biomarker in the sample is at least about 1-fold lower than the reference level. For example, the level of a biomarker measured in the sample obtained from the subject is considered to be lower than the reference level when the level of the biomarker in the sample is at least about 1-fold, at least about 1.5-fold, at least about 2-fold, at least about 2.5-fold, at least about 3-fold, at least about 3.5-fold, at least about 4-fold, at least about 4.5-fold, at least about 5-fold, at least about 5.5-fold, at least about 6-fold, at least about 6.5-fold, at least about 7-fold, at least about 7.5 fold, at least about 8-fold, at least about 8.5-fold, at least about 9-fold, at least about 9.5-fold, at least about 10-fold, at least about 100-fold, or at least about 1000-fold lower than the reference level. In some embodiments, the level of a biomarker in the sample obtained from the subject is below the limit of detection. In some embodiments, the level of a biomarker measured in the sample obtained from the subject is considered to be lower than the reference level when the level of the biomarker in the sample is below the limit of detection while the reference level is above the limit of detection, is detectable, and/or is not zero. In some embodiments, a level is considered to be below the limit of detection when the level does not give an appreciable signal, a detectable signal, and/or is not significantly different than an appropriate negative control when performing an assay for measuring the level of a biomarker *(e.g.,* below the limit of detection of an assay measuring RNA transcript expression of the biomarker (such as RT-PCR, in situ hybridization, and/or next generation sequencing), below the limit of detection of an assay measuring protein expression of a biomarker (such as an immunoassay, PET imaging, Western blotting, ELISA, immunohistochemistry, and/or flow cytometry), *etc.).*

In some embodiments, a subject is administered an effective amount of an anti-CD137 antibody when the level of a biomarker in a sample obtained from the subject is lower than the reference level. In some embodiments, a subject is determined to be likely to respond to an anti-CD137 antibody when the level of the biomarker in a sample obtained from the subject is lower than the reference level. In some embodiments, a subject is administered an effective amount of an anti-CD137 antibody after the subject has been determined to be likely to respond to the anti-CD137 antibody. In some embodiments, a subject having cancer is selected for treatment with an anti-CD137 antibody when the level of the biomarker in a sample obtained from the subject is lower than the reference level. In some embodiments, a subject is positively stratified for enrollment into an anti-CD137 antibody therapy when the level of a biomarker in a sample obtained from the subject is lower than the reference level. In some embodiments, the anti-CD137 antibody is administered to the subject in combination with an anti-PD-1 antibody.

In some embodiments, the level of a biomarker measured in the sample obtained from the subject is considered to be higher than the reference level when the level of the biomarker in the sample is at least about 5% higher than the reference level. For example, the level of a biomarker measured in the sample obtained from the subject is considered to be higher than the reference level when the level of the biomarker in the sample is at least about 5%, at least about 10%, at least about 15%, at least about 20%, at least about 25%, at least about 30%, at least about 35%, at least about 40%, at least about 45%, at least about 50%, at least about 55%, at least about 60%, at least about 65%, at least about 70%, at least about 75%, at least about 80%, at least about 85%, at least about 90%, at least about 95%, or at least about 99% higher than the reference level. In some embodiments, the level of a biomarker measured in the sample obtained from the subject is considered to be higher than the reference level when the level of the biomarker in the sample is at least about 1-fold higher than the reference level. For example, the level of a biomarker measured in the sample obtained from the subject is considered to be higher than the reference level when the level of the biomarker in the sample is at least about 1-fold, at least about 1.5-fold, at least about 2-fold, at least about 2.5-fold, at least about 3-fold, at least about 3.5-fold, at least about 4-fold, at least about 4.5-fold, at least about 5-fold, at least about 5.5-fold, at least about 6-fold, at least about 6.5-fold, at least about 7-fold, at least about 7.5 fold, at least about 8-fold, at least about 8.5-fold, at least about 9-fold, at least about 9.5-fold, at least about 10-fold, at least about 100-fold, or at least about 1000-fold higher than the reference level. In some embodiments, the level of a biomarker in the reference sample is below the limit of detection. In some embodiments, the level of a biomarker measured in the sample obtained from the subject is considered to be higher than the reference level when the level of the biomarker in the sample is above the limit of detection, is detectable, and/or is not zero while the level of the biomarker in the reference sample is below the limit of detection. In some embodiments, a level is considered to be below the limit of detection when the level does not give an appreciable signal, a detectable signal, and/or is not significantly different than an appropriate negative control when performing an assay for measuring the level of a biomarker (e.g., below the limit of detection of an assay measuring RNA transcript expression of the biomarker (such as RT-PCR, in situ hybridization, and/or next generation sequencing), below the limit of detection of an assay measuring protein expression of the biomarker (such as an immunoassay, PET imaging, Western blotting, ELISA, immunohistochemistry, and/or flow cytometry), etc.).

In some embodiments, a subject is administered an effective amount of an anti-CD137 antibody when the level of a biomarker in a sample obtained from the subject is higher than the reference level. In some embodiments, a subject is determined to be likely to respond to an anti-CD137 antibody when the level of a biomarker in a sample obtained from the subject is higher than the reference level. In some embodiments, a subject is administered an effective amount of an anti-CD137 antibody after the subject has been determined to be likely to respond to the anti-CD137 antibody. In some embodiments, a subject having cancer is selected for treatment with an anti-CD137 antibody when the level of expression of a biomarker in a sample obtained from the subject is higher than the reference level. In some embodiments, a subject is positively stratified for enrollment into an anti-CD137 antibody therapy when the level of a biomarker in a sample obtained from the subject is higher than the reference level. In some embodiments, the anti-CD137 antibody is administered to the subject in combination with an anti-PD-1 antibody.

In some embodiments, the method comprises determining the level of a biomarker at two or more time points during the course of the anti-CD137 antibody treatment. In some embodiments, the method comprises determining the level of a biomarker (e.g., sCD137, CD137L, NK cell level) in a sample obtained from the subject prior to the administration of the anti-CD137 antibody. In some embodiments, the method comprises determining the level of a biomarker in a sample obtained from the subject after the administration of the anti-CD137 antibody. In some embodiments, the method comprises determining the level of a biomarker in a sample obtained from the subject after each cycle of anti-CD137 antibody treatment. In some embodiments, the method comprises determining a ratio ("induction ratio") between the difference in the level of the biomarker after administration of the anti-CD137 antibody (C2) and the level of the biomarker before administration of the anti-CD137 antibody (C1) and the level of the biomarker before administration of the anti-CD137 antibody (C1): (C2-C1)/C1. In some embodiments, an induction ratio of at least about any one of 50%, 1, 2, 3, 4, 5, 6, 7, 8, 9, 10 or more indicates a high likelihood of responding to the anti-CD137 antibody treatment, i.e., having stable disease (SD) or partial response (PR) after the treatment.

### IV. Anti-CD137 Antibodies

The method described herein comprise administration of an anti-CD137 antibody that specifically binds to an extracellular domain of human CD137. The anti-CD137 antibodies described herein include full-length anti-CD137 antibodies, antigen-binding fragments of the CD137 antibodies, and derivatives of the CD137 antibodies. In some embodiments, the anti-CD137 antibody is any one of the antibodies described herein, including antibodies described with reference to epitope binding and antibodies described with reference to specific amino acid sequences of CDRs, variable regions (VL, VH), and IgG (e.g., IgG4) light and heavy chains. In some embodiments, the anti-CD137 antibody has at least one (e.g., at least one, at least two, at least three, at least four, at least five, at least six, at least seven, eight, or all nine) of the following functional properties: (a) bind to human CD137 with a KD of 500 nM or less; (b) have agonist activity on human CD137; (c) do not bind to human OX40, CD40, GITR and/or CD27 receptor at concentration up to 1000 nM; (d) is cross-reactive with monkey, mouse, rat, or dog CD137; (e) do not induce ADCC effects; (f) are capable of inhibiting tumor cell growth; (g) have therapeutic effect on a cancer; (h) blocks binding between CD137 and CD137L; and (i) blocks CD137 signaling stimulated by CD137L *(e.g.,* CD137L-stimulated NF-κB-dependent transcription) in a cell that expresses CD137. In some embodiments, the antibodies disclosed herein can also block, e.g., completely block, the binding between CD137 and its ligand CD137L. In some embodiments, the anti-CD137 antibody is an antibody (or an antigen-binding fragment thereof) that cross-competes for binding to human CD137 with one or more of the antibodies or antigen-binding fragments as described herein. Exemplary anti-CD137 antibodies that are suitable for the methods described herein have been described, for example, in US20190055314A1, WO2019036855A1, and WO2019037711A1, which are incorporated herein by reference in their entirety.

Human CD137 is a 255 amino acid protein *(e.g.,* GenBank Accession No. NM_001561; NP_001552; SEQ ID NO.: 1). The protein comprises a signal sequence (amino acid residues 1-17), followed by an extracellular domain (169 amino acids), a transmembrane region (27 amino acids), and an intracellular domain (42 amino acids) (Cheuk ATC et al. 2004 Cancer Gene Therapy 11: 215-226). The receptor is expressed on the cell surface in monomer and dimer forms and likely trimerizes with CD137 ligand to signal.

In some embodiments, the anti-CD137 antibody specifically binds to one or more amino acid residues within amino acid residues 34-108 of SEQ ID NO: 1. In some embodiments, the anti-CD137 antibody specifically binds to one or more amino acid residues within amino acid residues 34-93 of SEQ ID NO: 1. In some embodiments, the anti-CD137 antibody specifically binds to one or more amino acid residues selected from the group consisting of amino acid residues 34-36, 53-55, and 92-93 of SEQ ID NO:1. In some embodiments, the anti-CD137 antibody specifically binds to one or more of amino acid residues 34-36, one or more of amino acid residues 53-55, and one or more or amino acid residues 92-93 of SEQ ID NO: 1. In some embodiments, the anti-CD137 antibody does not bind to one or more of amino acid residues selected from the group consisting of amino acid residues 109-112, 125, 126, 135-138, 150 and 151 of SEQ ID NO: 1. In some embodiments, the anti-CD137 antibody specifically does not bind to amino acid residues 109-112, 125, 126, 135-138, 150 and 151 of SEQ ID NO: 1. Methods of measuring an antibody or antigen-binding fragment's ability to bind a target antigen may be carried out using any method known in the art, including for example, by surface plasmon resonance, an ELISA, isothermal titration calorimetry, a filter binding assay, an EMSA, *etc.,* or based on the crystal structure of the anti-CD137 antibody with CD137.

In some embodiments, the anti-CD1137 antibody specifically binds to one or more amino acid residues selected from the group consisting of amino acid residues 51, 53, 62-73, 83, 89, 92, 95-104 and 112-116 of SEQ ID NO: 1. In some embodiments, the anti-CD137 antibody specifically binds to one or more amino acid residues selected from the group consisting of amino acid residues 51, 53, 63-67, 69-73, 83, 89, 92, 98-104, and 112-116 of SEQ ID NO: 1. In some embodiments, the anti-CD137 antibody specifically binds to one or more amino acid residues selected from the group consisting of amino acid residues 51, 63-67, 69-73, 83, 89, 92, 98-104 and 112-114 of SEQ ID NO: 1.

In some embodiments, the anti-CD137 antibody specifically binds to amino acid residues 51, 53, 62-73, 83, 89, 92, 95-104 and 112-116 of SEQ ID NO: 1. In some embodiments, the anti-CD137 antibody specifically binds to amino acid residues 51, 53, 63-67, 69-73, 83, 89, 92, 98-104, and 112-116 of SEQ ID NO: 1. In some embodiments, the anti-CD137 antibody specifically binds to amino acid residues 51, 63-67, 69-73, 83, 89, 92, 98-104 and 112-114 of SEQ ID NO: 1.

In some embodiments, the anti-CD137 antibody specifically binds to human CD137 with a KD of about 500 nM or less (e.g., about 500 nM or less, about 400 nM or less, about 300 nM or less, about 200 nM or less, about 150 nM or less, about 100 nM or less, about 90 nM or less, about 80 nM or less, about 75 nM or less, about 70 nM or less, about 60 nM or less, about 50 nM or less, about 40 nM or less, about 30 nM or less, about 25 nM or less, about 20 nM or less, about 10 nM or less, about 1 nM or less, about 0.1 nM or less, *etc.)* In some embodiments, the anti-CD137 antibody specifically binds to human CD137 with a KD of about 100 nM or less. In some embodiments, the anti-CD137 antibody specifically binds to human CD137 with a KD of about 50 nM or less. Methods of measuring the KD of an antibody or antigen-binding fragment may be carried out using any method known in the art, including for example, by surface plasmon resonance, an ELISA, isothermal titration calorimetry, a filter binding assay, an EMSA, *etc.*

Anti-CD137 antibodies need to be cross-linked to become agonistic. For example, cross-linking is achieved *in vivo* through Fcgamma receptors, while typically polyclonal anti-Fe antibodies are used in cell-based experiments *in vitro.* In some embodiments, the anti-CD137 antibodies described herein have agonist activity on human CD137. In some embodiments, the anti-CD137 antibody induces one or more *(e.g.,* one or more, two or more, three or more, *etc.)* activities of human CD137 when a cell *(e.g.,* a human cell) expressing human CD137 is contacted by the anti-CD137 antibody. Various CD137 activities are known in the art and may include, without limitation, induction of NF-κB-dependent transcription, induction of T cell proliferation, prolonging T cell survival, co-stimulation of activated T cells, induction of cytokine secretion (such as IL-2), and induction of monocyte activation. In some embodiments, the one or more CD137 activities is not CD137 binding to its ligand. Methods of measuring CD137 activity *(e.g.,* the induction of NF-κB-dependent transcription and/or T cell proliferation, *etc.)* are known in the art. In some embodiments, the anti-CD137 antibody increases NF-κB dependent transcription in cells *(e.g.,* human cells) expressing human CD137. In some embodiments, NF-κB dependent transcription is increased by about 10% or more, about 20% or more, about 30% or more, about 40% or more, about 50% or more, about 60% or more, about 70% or more, about 80% or more, about 90% or more, or about 99% or more in cells (e.g., human cells) expressing CD137 contacted with the anti-CD137 antibody, relative to a corresponding cell not contacted with the anti-CD137 antibody (e.g., a corresponding cell not contacted with an antibody, or contacted with an isotype control antibody). In some embodiments, NF-κB dependent transcription is increased by about 2-fold, 3-fold, 4-folr, 5-fold, 6-fold, 7-fold, 8-fold, 9-fold, 10-fold, 100-fold, 1000-fold or more in cells (e.g., human cells) expressing CD137 contacted with the anti-CD137 antibody, relative to a corresponding cell not contacted with the anti-CD137 antibody (e.g., a corresponding cell not contacted with an antibody, or contacted with an isotype control antibody).

In some embodiments, the anti-CD137 antibody is cross-reactive with monkey (e.g., cynomolgus monkey), mouse, rat, and/or dog CD137. In some embodiments, the anti-CD137 antibody is cross-reactive with monkey CD137. In some embodiments, the anti-CD137 antibody is cross-reactive with mouse CD137. In some embodiments, the anti-CD137 antibody is cross-reactive with rat CD137. In some embodiments, the anti-CD137 antibody is cross-reactive with dog CD137. In some embodiments, the anti-CD137 antibody is cross-reactive with monkey and mouse CD137; monkey and rat CD137; monkey and dog CD137; mouse and rat CD137; mouse and dog CD137; rat and dog CD137; monkey, mouse, and rat CD137; monkey, mouse, and dog CD137; monkey, rat, and dog CD137; mouse, rat, and dog CD137; or monkey, mouse, rat, and dog CD137. In some embodiments, the anti-CD137 antibody is cross-reactive at about 100 nM (e.g., at about 1nM, at about 10nM, at about 25nM, at about 50nM, at about 75nM, at about 100nM). Methods of measuring antibody cross-reactivity are known in the art, including, without limitation, surface plasmon resonance, an ELISA, isothermal titration calorimetry, a filter binding assay, an EMSA, etc.

In some embodiments, the anti-CD137 antibody does not induce ADCC effects. Methods of measuring ADCC effects (e.g., in vivo methods) are known in the art. In some embodiments, the anti-CD137 antibody does not ADCC effects by more than about 10% (do not induce ADCC by more than about 10%, more than about 5%, more than about 1%, more than about 0.1%, more than about 0.01%) relative to a control.

In some embodiments, the anti-CD137 antibody is capable of inhibiting tumor cell growth/proliferation. In some embodiments, the tumor cell growth/proliferation is inhibited by at least about 5% (e.g., at least about 5%, at least about 10%, at least about 20%, at least about 30%, at least about 40%, at least about 50%, at least about 60%, at least about 70%, at least about 80%, at least about 90%, or at least about 99%) when contacted with the anti-CD137 antibody relative to corresponding tumor cells not contacted with the anti-CD137 antibody. In some embodiments, the anti-CD137 antibody is capable of reducing tumor volume in a subject when the subject is administered the anti-CD137 antibody. In some embodiments, the anti-CD137 antibody is capable of reducing tumor volume in a subject by at least about 5% (e.g., at least about 5%, at least about 10%, at least about 20%, at least about 30%, at least about 40%, at least about 50%, at least about 60%, at least about 70%, at least about 80%, at least about 90%, or at least about 99%) relative to the initial tumor volume in the subject (e.g., prior to administration of the anti-CD137 antibody). Methods of monitoring tumor cell growth/proliferation, tumor volume, and/or tumor inhibition are known in the art.

In some embodiments, the anti-CD137 antibody has therapeutic effect on a cancer. In some embodiments, the anti-CD137 antibody reduces one or more signs or symptoms of a cancer. In some embodiments, a subject suffering from a cancer goes into partial or complete remission when administered the anti-CD137 antibody.

In some embodiments, the anti-CD137 antibody is selected from the group consisting of AG10058, AG10059 and ADG106. In some embodiments, the anti-CD137 antibody competes or cross-competes for binding to human CD137 with any of the illustrative antibodies of the present application, such as AG10058, AG10059 and ADG106. In some embodiments, the anti-CD137 antibody is an antibody that competes or cross-competes for binding to the same epitope on human CD137 as AG10058, AG10059 or ADG106. The ability of an antibody to compete or cross-compete for binding with another antibody can be determined using standard binding assays known in the art, such as BIAcore analysis, ELISA assays, or flow cytometry. For example, one can allow an illustrative antibody of the disclosure to bind to human CD137 under saturating conditions and then measure the ability of the test antibody to bind to the CD137. If the test antibody is able to bind to the CD137 at the same time as the illustrative antibody, then the test antibody binds to a different epitope as the illustrative antibody. However, if the test antibody is not able to bind to the CD137 at the same time, then the test antibody binds to the same epitope, an overlapping epitope, or an epitope that is in close proximity to the epitope bound by the illustrative antibody. This experiment can be performed using various methods, such as ELISA, RIA, FACS or surface plasmon resonance.

In some embodiments, the anti-CD137 antibody blocks the binding between CD137 and its ligand (e.g., human CD137 and human CD137L). In some embodiments, the anti-CD137 antibody blocks the binding between CD137 and its ligand in vitro. In some embodiments, the anti-CD137 antibody has a half maximal inhibitory concentration (IC50) of about 500 nM or less (e.g., about 500 nM or less, about 400nM or less, about 300nM or less, about 200nM or less, about 100nM or less, about 50nM or less, about 25nM or less, about 10nM or less, about 1nM or less, etc.) for blocking binding of CD137 its ligand. In some embodiments, the anti-CD137 antibody has a half-maximal inhibitory concentration (IC50) of about 100 nM or less for blocking binding of CD137 its ligand. In some embodiments, the anti-CD137 antibody completely blocks binding of human CD137 to its ligand when provided at a concentration of about 100 nM or greater (e.g., about 100nM or greater, about 500nM or greater, about 1μM or greater, about 10μM or greater, etc.). As used herein, the term "complete blocking" or "completely blocks" refers to the antibody or antigen-binding fragment's ability to reduce binding between a first protein and a second protein by at least about 80% *(e.g.,* at least about 80%, at least about 85%, at least about 90%, at least about 95%, at least about 99%, *etc.).* Methods of measuring the ability of an antibody or antigen-binding fragment to block binding of a first protein *(e.g.,* a CD137) and a second protein *(e.g.,* CD137L) are known in the art, including, without limitation, via BIAcore analysis, ELISA assays, and flow cytometry.

In some embodiments, the anti-CD137 antibody comprises a heavy chain variable region (VH) and a light chain variable region (VL), a) wherein the VH comprises an HVR-H1, an HVR-H2, and an HVR-H3, wherein the HVR-H1 comprises an amino acid sequence according to a formula selected from the group consisting of: Formula (I): X1TFX2X3YX4IHWV (SEQ ID NO: 32), wherein X1 is For Y, X2 is S or T, X3 is G, N, or S, and X4 is A, G, or W; Formula (II): YSIX1SGX2X3WX4WI (SEQ ID NO: 33), wherein X1 is S or T, X2 is H or Y, X3 is H or Y, and X4 is A, D, G, N, S, or T; and Formula (III): FSLSTX1GVX2VX3WI (SEQ ID NO: 34), wherein X1 is G or S, X2 is A or G, and X3 is A, G, S, or T; wherein the HVR-H2 comprises an amino acid sequence according to a formula selected from the group consisting of: Formula (IV): LALIDWX1X2DKX3YSX4SLKSRL (SEQ ID NO: 35), wherein X1 is A, D, or Y, X2 is D or G, X3 is R, S, or Y, and X4 is P or T; Formula (V): IGX1IYHSGX2TYYX3PSLKSRV (SEQ ID NO: 36), wherein X1 is D or E, X2 is N or S, and X3 is N or S; and Formula (VI): VSX1ISGX2GX3X4TYYADSVKGRF (SEQ ID NO: 37), wherein X1 is A, G, S, V, or Y, X2 is A, D, S, or Y, X3 is D, G, or S, and X4 is S or T; and wherein the HVR-H3 comprises an amino acid sequence according to Formula (VII): ARX1GX2X3X4VX5GOWFX6Y (SEQ ID NO: 38), wherein X1 is E or G, X2 is E or S, X3 is D or T, X4 is A, T, or V, X5 is A, I, L, T, or V, and X6 is A, D, or G; and/or b) wherein the VL comprises an HVR-L1, an HVR-L2, and an HVR-L3, wherein the HVR-L1 comprises an amino acid sequence according to Formula (VIII): X1ASQX2X3X4X5X6X7X8 (SEQ ID NO: 39), wherein X1 is Q or R, X2 is D, G, or S, X3 is I or V, X4 is G, R, S, or T, X5 is P, R, S, or T, X6 is A, D, F, S, V, or Y, X7 is L or V, and X8 is A, G, or N; wherein the HVR-L2 comprises an amino acid sequence according to Formula (IX): X1ASX2X3X4X5GX6 (SEQ ID NO: 40), wherein X1 is A or D, X2 is N, S, or T, X3 is L or R, X4 is A, E, or Q, X5 is S or T, and X6 is I or V; and wherein the HVR-L3 comprises an amino acid sequence according to a formula selected from the group consisting of: Formula (X): YCQQX1YX2X3X4T (SEQ ID NO: 41), wherein X1 is A, G, S, or Y, X2 is Q, S, or Y, X3 is I, L, T, or Y, and X4 is I, S, V, or W; and Formula (XI): YCX1QX2X3X4X5PX6T (SEQ ID NO: 42), wherein X1 is E or Q, X2 is P, S, or Y, X3 is D, L, S, T, or Y, X4 is D, E, H, S, or T, X5 is D, L T, or W, and X6 is L, P, R, or V.

In some embodiments, the anti-CD137 antibody comprises a VH and a VL, wherein the VH comprises a HVR-H1 comprising the amino acid sequence of SEQ ID NO: 34, a HVR-H2 comprising the amino acid sequence of SEQ ID NO:35, and a HVR-H3 comprising the amino acid sequence of SEQ ID NO: 38; and/or wherein the VL comprises a HVR-L1 comprising the amino acid sequence of SEQ ID NO: 39, a HVR-L2 comprising the amino acid sequence of SEQ ID NO: 40, and a HVR-L3 comprising the amino acid sequence of SEQ ID NO: 41.

Sequences of exemplary anti-CD137 antibodies are shown in Table B below.

Table B. Exemplary anti-CD137 antibodies.

| Antibody | HVR-H1 | HVR-H2 | HVR-H3 | HVR-L1 | HVR-L2 | HVR-L3 |
|---|---|---|---|---|---|---|
| ADG106 | FSLSTGG VGVGWI (SEQ ID NO: 2) | LALIDWA DDKYYSP SLKSRL (SEQ ID NO: 3) | ARGGSDT VIGDWFA Y (SEQ ID NO: 4) | RASQSIGS YLA (SEQ ID NO: 5) | DASNLET GV (SEQ ID NO: 6) | YCQQGY YLWT (SEQ ID NO: 7) |
| AG10059 | YSITSGHY WAWI (SEQ ID NO: 12) | VSSISGYG STTYYAD SVKGRF (SEQ ID NO: 13) | ARGGSDA VLGDWF AY (SEQ ID NO: 14) | RASQGIG SFLA (SEQ ID NO: 15) | DASNLET GV (SEQ ID NO: 16) | YCQQGY YLWT (SEQ ID NO: 17) |
| AG10058 | FSLSTSGV GVGWI (SEQ ID NO: 22) | LALIDWD DDKYYSP SLKSRL (SEQ ID NO: 23) | ARGGSDT VLGDWF AY (SEQ ID NO: 24) | RASQSVS PYLA (SEQ ID NO: 25) | DASSLES GV (SEQ ID NO: 26) | YCQQGYS LWT (SEQ ID NO: 27) |

In some embodiments, the anti-CD137 antibody comprises a VH and a VL, wherein the VH comprises a HVR-H1 comprising the amino acid sequence of SEQ ID NO: 2, a HVR-H2 comprising the amino acid sequence of SEQ ID NO: 3, and a HVR-H3 comprising the amino acid sequence of SEQ ID NO: 4; and/or wherein the VL comprises a HVR-L1 comprising the amino acid sequence of SEQ ID NO:5, a HVR-L2 comprising the amino acid sequence of SEQ ID NO: 6, and a HVR-L3 comprising the amino acid sequence of SEQ ID NO: 7.

In some embodiments, the anti-CD137 antibody comprises a VH comprising a heavy chain complementarity determining region (HC-CDR) 1, a HC-CDR2, and a HC-CDR3 of the amino acid sequence of SEQ ID NO: 8; and/or a VL comprising a light chain complementarity determining region (LC-CDR) 1, a LC-CDR2, and a LC-CDR3 of the amino acid sequence of SEQ ID NO: 9. In certain embodiments, the anti-CD137 antibody comprises a heavy chain variable region comprises the amino acid sequence of SEQ ID NO: 8, and/or a light chain variable region comprising the amino acid sequence of SEQ ID NO: 9. In certain embodiments, the anti-CD137 antibody comprises a heavy chain comprising the amino acid sequence of SEQ ID NO: 10, and/or a light chain comprising the amino acid sequence of SEQ ID NO: 11.

In some embodiments, the anti-CD137 antibody comprises a VH and a VL, wherein the VH comprises a HVR-H1 comprising the amino acid sequence of SEQ ID NO: 12, a HVR-H2 comprising the amino acid sequence of SEQ ID NO: 13, and a HVR-H3 comprising the amino acid sequence of SEQ ID NO: 14; and/or wherein the VL comprises a HVR-L1 comprising the amino acid sequence of SEQ ID NO: 15, a HVR-L2 comprising the amino acid sequence of SEQ ID NO: 16, and a HVR-L3 comprising the amino acid sequence of SEQ ID NO: 17.

In some embodiments, the anti-CD137 antibody comprises a VH comprising a HC-CDR1, a HC-CDR2, and a HC-CDR3 of the amino acid sequence of SEQ ID NO: 18; and/or a VL comprising a LC-CDR1, a LC-CDR2, and a LC-CDR3 of the amino acid sequence of SEQ ID NO: 19. In certain embodiments, the anti-CD137 antibody comprises a heavy chain variable region comprises the amino acid sequence of SEQ ID NO: 18, and/or a light chain variable region comprising the amino acid sequence of SEQ ID NO: 19. In certain embodiments, the anti-CD137 antibody comprises a heavy chain comprising the amino acid sequence of SEQ ID NO: 20, and/or a light chain comprising the amino acid sequence of SEQ ID NO: 21.

In some embodiments, the anti-CD137 antibody comprises a VH and a VL, wherein the VH comprises a HVR-H1 comprising the amino acid sequence of SEQ ID NO: 22, a HVR-H2 comprising the amino acid sequence of SEQ ID NO: 23, and a HVR-H3 comprising the amino acid sequence of SEQ ID NO: 24; and/or wherein the VL comprises a HVR-L1 comprising the amino acid sequence of SEQ ID NO: 25, a HVR-L2 comprising the amino acid sequence of SEQ ID NO: 26, and a HVR-L3 comprising the amino acid sequence of SEQ ID NO: 27.

In some embodiments, the anti-CD137 antibody comprises a VH comprising a HC-CDR1, a HC-CDR2, and a HC-CDR3 of the amino acid sequence of SEQ ID NO: 28; and/or a VL comprising a LC-CDR1, a LC-CDR2, and a LC-CDR3 of the amino acid sequence of SEQ ID NO: 29. In certain embodiments, the anti-CD137 antibody comprises heavy chain variable region comprises the amino acid sequence of SEQ ID NO: 28, and/or a light chain variable region comprising the amino acid sequence of SEQ ID NO: 29. In certain embodiments, the anti-CD137 antibody comprises a heavy chain comprising the amino acid sequence of SEQ ID NO: 30, and/or a light chain comprising the amino acid sequence of SEQ ID NO: 31.

The CD137 antibodies described herein can be in any class, such as IgG, IgM, IgE, IgA, or IgD. It is preferred that the CD137 antibodies are in the IgG class, such as IgG1, IgG2, IgG3, or IgG4 subclass. A CD137 antibody can be converted from one class or subclass to another class or subclass using methods known in the art. An exemplary method for producing an antibody in a desired class or subclass comprises the steps of isolating a nucleic acid encoding a heavy chain of an CD137 antibody and a nucleic acid encoding a light chain of a CD137 antibody, isolating the sequence encoding the VH region, ligating the VH sequence to a sequence encoding a heavy chain constant region of the desired class or subclass, expressing the light chain gene and the heavy chain construct in a cell, and collecting the CD137 antibody. In some embodiments, the anti-CD137 antibody comprises a human IgG4 Fc region. In some embodiments, the human IgG4 Fc region comprises an S241P mutation, wherein numbering is according to Kabat.

## Antigen-binding Fragments and Antibody Derivatives

In some embodiments, the anti-CD137 antibody is an antigen-binding fragment of any one of the anti-CD137 antibodies described herein.

In some embodiments, the antigen-binding fragments of an CD137 antibody include: (i) a Fab fragment, which is a monovalent fragment consisting of the $V_L$, $V_H$, $C_L$ and $C_H1$ domains; (ii) a F(ab')$_2$ fragment, which is a bivalent fragment comprising two Fab fragments linked by a disulfide bridge at the hinge region; (iii) a Fd fragment consisting of the $V_H$ and $C_H1$ domains; (iv) a Fv fragment consisting of the $V_L$ and $V_H$ domains of a single arm of an antibody; (v) a dAb fragment (Ward et al., (1989) Nature 341:544-546), which consists of a $V_H$ domain; (vi) an isolated CDR, and (vii) single chain antibody (scFv), which is a polypeptide comprising a $V_L$ region of an antibody linked to a $V_H$ region of an antibody. Bird et al., (1988) Science 242:423-426 and Huston et al., (1988) Proc. Natl. Acad. Sci. USA 85:5879-5883.

In some embodiments, the anti-CD137 antibody is a derivative of any one of the anti-CD137 antibodies described herein.

In some embodiments, the antibody derivative is derived from modifications of the amino acid sequences of an illustrative antibody ("parent antibody") of the disclosure while conserving the overall molecular structure of the parent antibody amino acid sequence. Amino acid sequences of any regions of the parent antibody chains may be modified, such as framework regions, CDR regions, or constant regions. Types of modifications include substitutions, insertions, deletions, or combinations thereof, of one or more amino acids of the parent antibody.

In some embodiments, the antibody derivative comprises a VH comprising an amino acid sequence that is at least 80%, at least 85%, at least 90%, at least 95%, at least 96%, at least 97%, at least 98%, or at least 99% identical to the amino acid sequence of SEQ ID NO: 8; and/or a VL comprising an amino acid sequence that is at least 80%, at least 85%, at least 90%, at least 95%, at least 96%, at least 97%, at least 98%, or at least 99% identical to the amino acid sequence of SEQ ID NO: 9. In some embodiments, the antibody derivative comprises a HVR-H1 amino acid sequence region that is at least 80%, at least 85%, at least 90%, at least 95%, at least 96%, at least 97%, at least 98%, or at least 99% identical to an amino acid sequence as set forth in SEQ ID NO: 2. In some embodiments, the antibody derivative comprises a HVR- H2 amino acid sequence region that is at least 80%, at least 90%, at least 95%, at least 96%, at least 97%, at least 98%, or at least 99% identical to an amino acid sequence as set forth in SEQ ID NO: 3. In some embodiments, the antibody derivative comprises a HVR-H3 amino acid sequence region that is at least 80%, at least 85%, at least 90%, at least 95%, at least 96%, at least 97%, at least 98%, or at least 99% identical to an amino acid sequence as set forth in SEQ ID NO: 4. In some embodiments, the antibody derivative comprises a HVR-L1 amino acid sequence region that is at least 80%, at least 85%, at least 90%, at least 95%, at least 96%, at least 97%, at least 98%, or at least 99% identical to an amino acid sequence as set forth in SEQ ID NO: 5. In some embodiments, the antibody derivative comprises a HVR-L2 amino acid sequence region that is at least 80%, at least 90%, at least 95%, at least 96%, at least 97%, at least 98%, or at least 99% identical to an amino acid sequence as set forth in SEQ ID NO: 6. In some embodiments, the antibody derivative comprises a HVR-L3 amino acid sequence region that is at least 80%, at least 85%, at least 90%, at least 95%, at least 96%, at least 97%, at least 98%, or at least 99% identical to an amino acid sequence as set forth in SEQ ID NO: 7. In some particular embodiments, the antibody derivative comprises 1, 2, 3, 4, 5, 6, 7, 8, 9, 10, 11, 12, 13, 14, or 15 conservative or non-conservative substitutions, and/or 1, 2, 3, 4, 5, 6, 7, 8, 9, 10, 11, 12, 13, 14, or 15 additions and/or deletions to an amino acid sequence as set forth in any of SEQ ID NOs: 8, 9, 10, and 11.

In some embodiments, the antibody derivative comprises a VH comprising an amino acid sequence that is at least 80%, at least 85%, at least 90%, at least 95%, at least 96%, at least 97%, at least 98%, or at least 99% identical to the amino acid sequence of SEQ ID NO: 18; and/or a VL comprising an amino acid sequence that is at least 80%, at least 85%, at least 90%, at least 95%, at least 96%, at least 97%, at least 98%, or at least 99% identical to the amino acid sequence of SEQ ID NO: 19. In some embodiments, the antibody derivative comprises a HVR-H1 amino acid sequence region that is at least 80%, at least 85%, at least 90%, at least 95%, at least 96%, at least 97%, at least 98%, or at least 99% identical to an amino acid sequence as set forth in SEQ ID NO: 12. In some embodiments, the antibody derivative comprises a HVR- H2 amino acid sequence region that is at least 80%, at least 90%, at least 95%, at least 96%, at least 97%, at least 98%, or at least 99% identical to an amino acid sequence as set forth in SEQ ID NO: 13. In some embodiments, the antibody derivative comprises a HVR-H3 amino acid sequence region that is at least 80%, at least 85%, at least 90%, at least 95%, at least 96%, at least 97%, at least 98%, or at least 99% identical to an amino acid sequence as set forth in SEQ ID NO: 14. In some embodiments, the antibody derivative comprises a HVR-L1 amino acid sequence region that is at least 80%, at least 85%, at least 90%, at least 95%, at least 96%, at least 97%, at least 98%, or at least 99% identical to an amino acid sequence as set forth in SEQ ID NO: 15. In some embodiments, the antibody derivative comprises a HVR-L2 amino acid sequence region that is at least 80%, at least 90%, at least 95%, at least 96%, at least 97%, at least 98%, or at least 99% identical to an amino acid sequence as set forth in SEQ ID NO: 16. In some embodiments, the antibody derivative comprises a HVR-L3 amino acid sequence region that is at least 80%, at least 85%, at least 90%, at least 95%, at least 96%, at least 97%, at least 98%, or at least 99% identical to an amino acid sequence as set forth in SEQ ID NO: 17. In some particular embodiments, the antibody derivative comprises 1, 2, 3, 4, 5, 6, 7, 8, 9, 10, 11, 12, 13, 14, or 15 conservative or non-conservative substitutions, and/or 1, 2, 3, 4, 5, 6, 7, 8, 9, 10, 11, 12, 13, 14, or 15 additions and/or deletions to an amino acid sequence as set forth in any of SEQ ID NOs: 18, 19, 20, and 21.

In some embodiments, the antibody derivative comprises a VH comprising an amino acid sequence that is at least 80%, at least 85%, at least 90%, at least 95%, at least 96%, at least 97%, at least 98%, or at least 99% identical to the amino acid sequence of SEQ ID NO: 28; and/or a VL comprising an amino acid sequence that is at least 80%, at least 85%, at least 90%, at least 95%, at least 96%, at least 97%, at least 98%, or at least 99% identical to the amino acid sequence of SEQ ID NO: 29. In some embodiments, the antibody derivative comprises a HVR-H1 amino acid sequence region that is at least 80%, at least 85%, at least 90%, at least 95%, at least 96%, at least 97%, at least 98%, or at least 99% identical to an amino acid sequence as set forth in SEQ ID NO: 22. In some embodiments, the antibody derivative comprises a HVR- H2 amino acid sequence region that is at least 80%, at least 90%, at least 95%, at least 96%, at least 97%, at least 98%, or at least 99% identical to an amino acid sequence as set forth in SEQ ID NO: 23. In some embodiments, the antibody derivative comprises a HVR-H3 amino acid sequence region that is at least 80%, at least 85%, at least 90%, at least 95%, at least 96%, at least 97%, at least 98%, or at least 99% identical to an amino acid sequence as set forth in SEQ ID NO: 24. In some embodiments, the antibody derivative comprises a HVR-L1 amino acid sequence region that is at least 80%, at least 85%, at least 90%, at least 95%, at least 96%, at least 97%, at least 98%, or at least 99% identical to an amino acid sequence as set forth in SEQ ID NO: 25. In some embodiments, the antibody derivative comprises a HVR-L2 amino acid sequence region that is at least 80%, at least 90%, at least 95%, at least 96%, at least 97%, at least 98%, or at least 99% identical to an amino acid sequence as set forth in SEQ ID NO: 26. In some embodiments, the antibody derivative comprises a HVR-L3 amino acid sequence region that is at least 80%, at least 85%, at least 90%, at least 95%, at least 96%, at least 97%, at least 98%, or at least 99% identical to an amino acid sequence as set forth in SEQ ID NO: 27. In some particular embodiments, the antibody derivative comprises 1, 2, 3, 4, 5, 6, 7, 8, 9, 10, 11, 12, 13, 14, or 15 conservative or non-conservative substitutions, and/or 1, 2, 3, 4, 5, 6, 7, 8, 9, 10, 11, 12, 13, 14, or 15 additions and/or deletions to an amino acid sequence as set forth in any of SEQ ID NOs: 28, 29, 30, and 31.

Amino acid substitutions encompass both conservative substitutions and non-conservative substitutions. The term "conservative amino acid substitution" means a replacement of one amino acid with another amino acid where the two amino acids have similarity in certain physico-chemical properties such as polarity, charge, solubility, hydrophobicity, hydrophilicity, and/or the amphipathic nature of the residues involved. For example, substitutions typically may be made within each of the following groups: (a) nonpolar (hydrophobic) amino acids, such as alanine, leucine, isoleucine, valine, proline, phenylalanine, tryptophan, and methionine; (b) polar neutral amino acids, such as glycine, serine, threonine, cysteine, tyrosine, asparagine, and glutamine; (c) positively charged (basic) amino acids, such as arginine, lysine, and histidine; and (d) negatively charged (acidic) amino acids, such as aspartic acid and glutamic acid.

The modifications may be made in any positions of the amino acid sequences of the antibody, including the CDRs, framework regions, or constant regions. In some embodiments, the present disclosure provides an antibody derivative that contains the VH and VL CDR sequences of an illustrative antibody of this disclosure, yet contains framework sequences different from those of the illustrative antibody. Such framework sequences can be obtained from public DNA databases or published references that include germline antibody gene sequences. For example, germline DNA sequences for human heavy and light chain variable region genes can be found in the Genbank database or in the "VBase" human germline sequence database (Kabat, E. A., et al., Sequences of Proteins of Immunological Interest, Fifth Edition, U.S. Department of Health and Human Services, NIH Publication No. 91-3242 (1991); Tomlinson, I. M., et al., J. Mol. Biol. 227:776-798 (1992); and Cox, J. P. L. et al., Eur. J. Immunol. 24:827-836 (1994)). Framework sequences that may be used in constructing an antibody derivative include those that are structurally similar to the framework sequences used by illustrative antibodies of the disclosure, *e.g.,* similar to the VH 3-23 framework sequences and/or the VL λ3 or λ1-13 framework sequences used by illustrative antibodies of the disclosure. For example, the HVR-H1, HVR-H2, and HVR-H3 sequences, and the HVR-L1, HVR-L2, and HVR-L3 sequences of an illustrative antibody can be grafted onto framework regions that have the identical sequence as that found in the germline immunoglobulin gene from which the framework sequence derive, or the CDR sequences can be grafted onto framework regions that contain one or more mutations as compared to the germline sequences.

In some embodiments, the antibody derivative is a chimeric antibody, which comprises an amino acid sequence of an illustrative antibody of the disclosure. In one example, one or more CDRs from one or more illustrative human antibodies are combined with CDRs from an antibody from a non-human animal, such as mouse or rat. In another example, all of the CDRs of the chimeric antibody are derived from one or more illustrative antibodies. In some particular embodiments, the chimeric antibody comprises one, two, or three CDRs from the heavy chain variable region or from the light chain variable region of an illustrative antibody. Chimeric antibodies can be generated using conventional methods known in the art.

Another type of modification is to mutate amino acid residues within the CDR regions of the VH and/or VL chain. Site-directed mutagenesis or PCR-mediated mutagenesis can be performed to introduce the mutation(s) and the effect on antibody binding, or other functional property of interest, can be evaluated in *in vitro* or *in vivo* assays known in the art. Typically, conservative substitutions are introduced. The mutations may be amino acid additions and/or deletions. Moreover, typically no more than one, two, three, four or five residues within a CDR region are altered. In some embodiments, the antibody derivative comprises 1, 2, 3, or 4 amino acid substitutions in the heavy chain CDRs and/or in the light chain CDRs. In some embodiments, the amino acid substitution is to change one or more cysteines in an antibody to another residue, such as, without limitation, alanine or serine. The cysteine may be a canonical or non-canonical cysteine. In some embodiments, the antibody derivative has 1, 2, 3, or 4 conservative amino acid substitutions in the heavy chain CDR regions relative to the amino acid sequences of an illustrative antibody.

Modifications may also be made to the framework residues within the VH and/or VL regions. Typically, such framework variants are made to decrease the immunogenicity of the antibody. One approach is to "back mutate" one or more framework residues to the corresponding germline sequence. An antibody that has undergone somatic mutation may contain framework residues that differ from the germline sequence from which the antibody is derived. Such residues can be identified by comparing the antibody framework sequences to the germline sequences from which the antibody is derived. To return the framework region sequences to their germline configuration, the somatic mutations can be "back mutated" to the germline sequence by, for example, site-directed mutagenesis or PCR-mediated mutagenesis.

In addition, modifications may also be made within the Fc region of an illustrative antibody, typically to alter one or more functional properties of the antibody, such as serum half-life, complement fixation, Fc receptor binding, and/or antigen-dependent cellular cytotoxicity. In one example, the hinge region of CH1 is modified such that the number of cysteine residues in the hinge region is altered, e.g., increased or decreased. This approach is described further in U.S. Pat. No. 5,677,425. The number of cysteine residues in the hinge region of CH1 is altered to, for example, facilitate assembly of the light and heavy chains or to increase or decrease the stability of the antibody. In another case, the Fc hinge region of an antibody is mutated to decrease the biological half-life of the antibody.

Furthermore, an antibody of the disclosure may be modified to alter its potential glycosylation site or pattern in accordance with routine experimentation known in the art. In some embodiments, the anti-CD137 antibody derivative contains at least one mutation in a variable region of a light chain or heavy chain that changes the pattern of glycosylation in the variable region. Such an antibody derivative may have an increased affinity and/or a modified specificity for binding an antigen. The mutations may add a novel glycosylation site in the V region, change the location of one or more V region glycosylation site(s), or remove a pre-existing V region glycosylation site. In some embodiments, the anti-CD137 antibody derivative has a potential N-linked glycosylation site at asparagine in the heavy chain variable region, wherein the potential N-linked glycosylation site in one heavy chain variable region is removed. In some embodiments, the anti-CD137 antibody derivative has having a potential N-linked glycosylation site at asparagine in the heavy chain variable region, wherein the potential N-linked glycosylation site in both heavy chain variable regions is removed. Method of altering the glycosylation pattern of an antibody is known in the art, such as those described in U.S. Pat. No. 6,933,368, the disclosure of which incorporated herein by reference.

In some embodiments, the antibody derivative is a CD137 antibody multimer, which is a multimeric form of a CD137 antibody, such as antibody dimers, trimers, or higher-order multimers of monomeric antibodies. Individual monomers within an antibody multimer may be identical or different. In addition, individual antibodies within a multimer may have the same or different binding specificities. Multimerization of antibodies may be accomplished through natural aggregation of antibodies. For example, some percentage of purified antibody preparations (*e.g.,* purified IgG4 molecules) spontaneously form protein aggregates containing antibody homodimers, and other higher-order antibody multimers. Alternatively, antibody homodimers may be formed through chemical linkage techniques known in the art, such as through using crosslinking agents. Suitable crosslinkers include those that are heterobifunctional, having two distinctly reactive groups separated by an appropriate spacer (such as m-maleimidobenzoyl-N-hydroxysuccinimide ester, succinimidyl 4-(maleimidomethyl)cyclohexane-1-carboxylate, and N-succinimidyl S-acethylthio-acetate) or homobifunctional (such as disuccinimidyl suberate). Such linkers are commercially available from, for example, Pierce Chemical Company, Rockford, IL. Antibodies can also be made to multimerize through recombinant DNA techniques known in the art.

In some embodiments, the anti-CD137 antibody is a multimeric antibody (*e.g.,* a bispecific antibody). In some embodiments, the anti-CD137 antibody is an IgM antibody, *e.g.,* comprises an IgM Fc region (*e.g.,* a human IgM Fc region).

Examples of other antibody derivatives provided by the present disclosure include single chain antibodies, diabodies, domain antibodies, and unibodies. A "single-chain antibody" (scFv) consists of a single polypeptide chain comprising a VL domain linked to a VH domain wherein VL domain and VH domain are paired to form a monovalent molecule. Single chain antibody can be prepared according to method known in the art (see, for example, Bird et al., (1988) Science 242:423-426 and Huston et al., (1988) Proc. Natl. Acad. Sci. USA 85:5879-5883). A "diabody" consists of two chains, each chain comprising a heavy chain variable region connected to a light chain variable region on the same polypeptide chain connected by a short peptide linker, wherein the two regions on the same chain do not pair with each other but with complementary domains on the other chain to form a bispecific molecule. Methods of preparing diabodies are known in the art (See, e.g., Holliger P. et al., (1993) Proc. Natl. Acad. Sci. USA 90:6444-6448, and Poljak R. J. et al., (1994) Structure 2:1121-1123). Domain antibodies (dAbs) are small functional binding units of antibodies, corresponding to the variable regions of either the heavy or light chains of antibodies. Domain antibodies are well expressed in bacterial, yeast, and mammalian cell systems. Further details of domain antibodies and methods of production thereof are known in the art (see, for example, U.S. Pat. Nos. 6,291,158; 6,582,915; 6,593,081; 6,172,197; 6,696,245; European Patents 0368684 & 0616640; WO05/035572, WO04/101790, WO04/081026, WO04/058821, WO04/003019 and WO03/002609). Unibodies consist of one light chain and one heavy chain of an IgG4 antibody. Unibodies may be made by the removal of the hinge region of IgG4 antibodies. Further details of unibodies and methods of preparing them may be found in WO2007/059782.

## Methods of Making

Antibodies of the present disclosure can be produced by techniques known in the art, including conventional monoclonal antibody methodology e.g., the standard somatic cell hybridization technique (See *e.g.,* Kohler and Milstein, Nature 256:495 (1975), viral or oncogenic transformation of B lymphocytes, or recombinant antibody technologies as described in detail herein below.

Hybridoma production is a very well established procedure. The common animal system for preparing hybridomas is the murine system. Immunization protocols and techniques for isolation of immunized splenocytes for fusion are known in the art. Fusion partners (e.g., murine myeloma cells) and fusion procedures are also known. One well-known method that may be used for making human CD137 antibodies provided by the present disclosure involves the use of a XENOMOUSE™ animal system. XENOMOUSE™ mice are engineered mouse strains that comprise large fragments of human immunoglobulin heavy chain and light chain loci and are deficient in mouse antibody production. See, *e.g.,* Green et al., Nature Genetics 7:13-21 (1994) and WO2003/040170. The animal is immunized with a CD137 antigen. The CD137 antigen is isolated and/or purified CD137, preferably CD137. It may be a fragment of CD137, such as the extracellular domain of CD137, particularly a CD137 extracellular domain fragment comprising amino acid resides 34-108 or 34-93 of SEQ ID NO: 1. Immunization of animals can be carried out by any method known in the art. See, *e.g.,* Harlow and Lane, Antibodies: A Laboratory Manual, New York: Cold Spring Harbor Press, 1990. Methods for immunizing non-human animals such as mice, rats, sheep, goats, pigs, cattle and horses are well known in the art. See, *e.g.,* Harlow and Lane, supra, and U.S. Pat. No. 5,994,619. The CD137 antigen may be administered with an adjuvant to stimulate the immune response. Exemplary adjuvants include complete or incomplete Freund's adjuvant, RIBI (muramyl dipeptides) or ISCOM (immunostimulating complexes). After immunization of an animal with a CD137 antigen, antibody-producing immortalized cell lines are prepared from cells isolated from the immunized animal. After immunization, the animal is sacrificed and lymph node and/or splenic B cells are immortalized. Methods of immortalizing cells include, but are not limited to, transferring them with oncogenes, inflecting them with the oncogenic virus cultivating them under conditions that select for immortalized cells, subjecting them to carcinogenic or mutating compounds, fusing them with an immortalized cell, *e.g.,* a myeloma cell, and inactivating a tumor suppressor gene. See, *e.g.,* Harlow and Lane, supra. If fusion with myeloma cells is used, the myeloma cells preferably do not secrete immunoglobulin polypeptides (a non-secretory cell line). Immortalized cells are screened using CD137, a portion thereof, or a cell expressing CD137. CD137 antibody-producing cells, *e.g.,* hybridomas, are selected, cloned and further screened for desirable characteristics, including robust growth, high antibody production and desirable antibody characteristics, as discussed further below. Hybridomas can be expanded *in vivo* in syngeneic animals, in animals that lack an immune system, *e.g.,* nude mice, or in cell culture *in vitro.* Methods of selecting, cloning and expanding hybridomas are well known to those of ordinary skill in the art.

Antibodies of the disclosure can also be prepared using phage display or yeast display methods. Such display methods for isolating human antibodies are established in the art, such as Achim Knappik, et al., "Fully Synthetic Human Combinatorial Antibody Libraries (HuCAL) Based on Modular Consensus Frameworks and CDRs Randomized with Trinucleotides." J. Mol. Biol. (2000) 296, 57-86; and Michael J. Feldhaus, et al., "Flow-cytometric isolation of human antibodies from a non-immune Saccharomyces cerevisiae surface display library" Nat Biotechnol (2003) 21:163-170.

In some embodiments, the anti-CD137 antibody is prepared by expressing one or more nucleic acids encoding the anti-CD137 antibody or polypeptide chains thereof in a host cell. In some embodiments, the one or more nucleic acids is a DNA or RNA, and may or may not contain intronic sequences. Typically, the nucleic acid is a cDNA molecule.

Nucleic acids of the disclosure can be obtained using any suitable molecular biology techniques. For antibodies expressed by hybridomas, cDNAs encoding the light and heavy chains of the antibody made by the hybridoma can be obtained by PCR amplification or cDNA cloning techniques. For antibodies obtained from an immunoglobulin gene library (e.g., using phage display techniques), the nucleic acid encoding the antibody can be recovered from the library.

The isolated DNA encoding the VHregion can be converted to a full-length heavy chain gene by operatively linking the VH-encoding DNA to another DNA molecule encoding heavy chain constant regions (CH1, CH2 and CH3). The sequences of human heavy chain constant region genes are known in the art (see *e.g.,* Kabat et al. (1991) Sequences of Proteins of Immunological Interest, Fifth Edition, U.S. Department of Health and Human Services, NIH Publication No. 91-3242) and DNA fragments encompassing these regions can be obtained by standard PCR amplification. The heavy chain constant region can be an IgG1, IgG2, IgG3, IgG4, IgA, IgE, IgM or IgD constant region, but most preferably is an IgG4 or IgG2 constant region without ADCC effect. The IgG4 constant region sequence can be any of the various alleles or allotypes known to occur among different individuals. These allotypes represent naturally occurring amino acid substitution in the IgG4 constant regions. For a Fab fragment heavy chain gene, the VH-encoding DNA can be operatively linked to another DNA molecule encoding only the heavy chain CH1 constant region.

The isolated DNA encoding the VL region can be converted to a full-length light chain gene (as well as a Fab light chain gene) by operatively linking the VL-encoding DNA to another DNA molecule encoding the light chain constant region, CL. The sequences of human light chain constant region genes are known in the art (see *e.g.*, Kabat et al. (1991) Sequences of Proteins of Immunological Interest, Fifth Edition, U.S. Department of Health and Human Services, NIH Publication No. 91-3242) and DNA fragments encompassing these regions can be obtained by standard PCR amplification. The light chain constant region can be a kappa or lambda constant region.

To create a scFv gene, the VH- and VL-encoding DNA fragments are operatively linked to another fragment encoding a flexible linker, *e.g.,* encoding the amino acid sequence $(Gly_4\text{-}Ser)_3$, such that the VH and VL sequences can be expressed as a contiguous single-chain protein, with the VL and VH regions joined by the flexible linker (see *e.g.,* Bird et al., Science 242:423-426 (1988); Huston et al., Proc. Natl. Acad. Sci. USA 85:5879-5883 (1988); and McCafferty et al., Nature 348:552-554 (1990)).

In some embodiments, there is provided a vector that comprises one or more nucleic acid molecules encoding an anti-CD137 antibody described herein. In some embodiments, the vector is an expression vector useful for the expression of the anti-CD137 antibody. In some embodiments, provided herein are vectors, wherein a first vector comprises a polynucleotide sequence encoding a heavy chain variable region as described herein, and a second vector comprises a polynucleotide sequence encoding a light chain variable region as described herein. In some embodiments, a single vector comprises polynucleotides encoding a heavy chain variable region as described herein and a light chain variable region as described herein.

To express an anti-CD137 antibody described herein, DNAs encoding partial or full-length light and heavy chains are inserted into expression vectors such that the DNA molecules are operatively linked to transcriptional and translational control sequences. In this context, the term "operatively linked" means that an antibody gene is ligated into a vector such that transcriptional and translational control sequences within the vector serve their intended function of regulating the transcription and translation of the DNA molecule. The expression vector and expression control sequences are chosen to be compatible with the expression host cell used. The antibody light chain gene and the antibody heavy chain gene can be inserted into separate vector or, more typically, both genes are inserted into the same expression vector. The antibody genes are inserted into the expression vector by any suitable methods (*e.g.,* ligation of complementary restriction sites on the antibody gene fragment and vector, or homologous recombination-based DNA ligation). The light and heavy chain variable regions of the antibodies described herein can be used to create full-length antibody genes of any antibody isotype and subclass by inserting them into expression vectors already encoding heavy chain constant and light chain constant regions of the desired isotype and subclass such that the VH segment is operatively linked to the CH segment(s) within the vector and the VL segment is operatively linked to the CL segment within the vector. Additionally or alternatively, the recombinant expression vector can encode a signal peptide that facilitates secretion of the antibody chain from a host cell. The antibody chain gene can be cloned into the vector such that the signal peptide is linked in-frame to the amino terminus of the antibody chain gene. The signal peptide can be an immunoglobulin signal peptide or a heterologous signal peptide (*i.e.,* a signal peptide from a non-immunoglobulin protein).

In addition to the antibody chain genes, the expression vectors of the disclosure typically carry regulatory sequences that control the expression of the antibody chain genes in a host cell. The term "regulatory sequence" is intended to include promoters, enhancers and other expression control elements (*e.g.,* polyadenylation signals) that control the transcription or translation of the antibody chain genes. Such regulatory sequences are described, for example, in Goeddel (Gene Expression Technology. Methods in Enzymology 185, Academic Press, San Diego, Calif. (1990)). It will be appreciated by those skilled in the art that the design of the expression vector, including the selection of regulatory sequences, may depend on such factors as the choice of the host cell to be transformed, the level of expression of protein desired, *etc.* Examples of regulatory sequences for mammalian host cell expression include viral elements that direct high levels of protein expression in mammalian cells, such as promoters and/or enhancers derived from cytomegalovirus (CMV), Simian Virus 40 (SV40), adenovirus, (e.g., the adenovirus major late promoter (AdMLP) and polyoma. Alternatively, nonviral regulatory sequences may be used, such as the ubiquitin promoter or β-globin promoter. Still further, regulatory elements composed of sequences from different sources, such as the SR promoter system, which contains sequences from the SV40 early promoter and the long terminal repeat of human T cell leukemia virus type 1 (Takebe, Y. et al. (1988) Mol. Cell. Biol. 8:466-472).

In addition to the antibody chain genes and regulatory sequences, the expression vectors may carry additional sequences, such as sequences that regulate replication of the vector in host cells (*e.g.,* origins of replication) and selectable marker genes. The selectable marker gene facilitates selection of host cells into which the vector has been introduced (see, *e.g.,* U.S. Pat. Nos. 4,399,216, 4,634,665 and 5,179,017, all by Axel *et al.*). For example, typically the selectable marker gene confers resistance to drugs, such as G418, hygromycin or methotrexate, on a host cell into which the vector has been introduced. Selectable marker genes include the dihydrofolate reductase (DHFR) gene (for use in dhfr-host cells with methotrexate selection/amplification) and the neo gene (for G418 selection).

For expression of the light and heavy chains, the expression vector(s) encoding the heavy and light chains is transfected into a host cell by any suitable techniques. The various forms of the term "transfection" are intended to encompass a wide variety of techniques commonly used for the introduction of exogenous DNA into a prokaryotic or eukaryotic host cell, *e.g.,* electroporation, calcium-phosphate precipitation, DEAE-dextran transfection and the like. Although it is possible to express the antibodies of the disclosure in either prokaryotic or eukaryotic host cells, expression of antibodies in eukaryotic cells, and typically mammalian host cells, is most typical.

In some embodiments, there is provided a host cell containing a nucleic acid molecule provided by the present disclosure. The host cell can be virtually any cell for which expression vectors are available. It may be, for example, a higher eukaryotic host cell, such as a mammalian cell, a lower eukaryotic host cell, such as a yeast cell, and may be a prokaryotic cell, such as a bacterial cell. Introduction of the recombinant nucleic acid construct into the host cell can be effected by calcium phosphate transfection, DEAE, dextran mediated transfection, electroporation or phage infection.

Suitable prokaryotic hosts for transformation include *E. coli, Bacillus subtilis, Salmonella typhimurium* and various species within the genera *Pseudomonas, Streptomyces,* and *Staphylococcus.*

Mammalian host cells for expressing a binding molecule of the disclosure include, for example, Chinese Hamster Ovary (CHO) cells (including dhfr-CHO cells, described in Urlaub and Chasin, Proc. Natl. Acad. Sci. USA 77:4216-4220 (1980), used with a DHFR selectable marker, *e.g.,* as described in Kaufman and Sharp, J. Mol. Biol. 159:601-621 (1982), NS0 myeloma cells, COS cells and Sp2 cells. In particular, for use with NS0 myeloma or CHO cells, another expression system is the GS (glutamine synthetase) gene expression system disclosed in WO 87/04462, WO 89/01036 and EP 338,841. When expression vectors encoding antibody genes are introduced into mammalian host cells, the antibodies are produced by culturing the host cells for a period of time sufficient to allow for expression of the antibody in the host cells or secretion of the antibody into the culture medium in which the host cells are grown. Antibodies can be recovered from the culture medium using any suitable protein purification methods.

## V. Pharmaceutical Compositions, Kits, and Articles of Manufacture

One aspect of the present application provides a composition comprising any one of the anti-CD137 antibodies described herein. In some embodiments, the composition is a pharmaceutical composition comprising the anti-CD 137 antibody and a pharmaceutically acceptable carrier. The compositions can be prepared by conventional methods known in the art.

The term "pharmaceutically acceptable carrier" refers to any inactive substance that is suitable for use in a formulation for the delivery of an active agent (*e.g.,* the anti-CD137 antibody). A carrier may be an antiadherent, binder, coating, disintegrant, filler or diluent, preservative (such as antioxidant, antibacterial, or antifungal agent), sweetener, absorption delaying agent, wetting agent, emulsifying agent, buffer, and the like. Examples of suitable pharmaceutically acceptable carriers include water, ethanol, polyols (such as glycerol, propylene glycol, polyethylene glycol, and the like) dextrose, vegetable oils (such as olive oil), saline, buffer, buffered saline, and isotonic agents such as sugars, polyalcohols, sorbitol, and sodium chloride.

The compositions may be in any suitable forms, such as liquid, semi-solid, and solid dosage forms. Examples of liquid dosage forms include solution (e.g., injectable and infusible solutions), microemulsion, liposome, dispersion, or suspension. Examples of solid dosage forms include tablet, pill, capsule, microcapsule, and powder. A particular form of the composition suitable for delivering an anti-CD137 antibody is a sterile liquid, such as a solution, suspension, or dispersion, for injection or infusion. Sterile solutions can be prepared by incorporating the antibody in the required amount in an appropriate carrier, followed by sterilization microfiltration. Generally, dispersions are prepared by incorporating the antibody into a sterile vehicle that contains a basic dispersion medium and other carriers. In the case of sterile powders for the preparation of sterile liquid, methods of preparation include vacuum drying and freeze-drying (lyophilization) to yield a powder of the active ingredient plus any additional desired ingredient from a previously sterile-filtered solution thereof. The various dosage forms of the compositions can be prepared by conventional techniques known in the art.

The relative amount of an anti-CD137 antibody included in the composition will vary depending upon a number of factors, such as the specific anti-CD137 antibody and carriers used, dosage form, and desired release and pharmacodynamic characteristics. The amount of an anti-CD137 antibody in a single dosage form will generally be that amount which produces a therapeutic effect, but may also be a lesser amount. Generally, this amount will range from about 0.01 percent to about 99 percent, from about 0.1 percent to about 70 percent, or from about 1 percent to about 30 percent relative to the total weight of the dosage form.

In addition to the anti-CD137 antibody, one or more additional therapeutic agents may be included in the composition. Examples of additional therapeutic agents are described herein in the "Methods of Treatment" section, including the "Combination Therapy" subsection. The suitable amount of the additional therapeutic agent to be included in the composition can be readily selected by a person skilled in the art, and will vary depending on a number of factors, such as the particular agent and carriers used, dosage form, and desired release and pharmacodynamic characteristics. The amount of the additional therapeutic agent included in a single dosage form will generally be that amount of the agent, which produces a therapeutic effect, but may be a lesser amount as well.

In some embodiments, there is provided an article of manufacture comprising materials useful for the treatment of a cancer. The article of manufacture can comprise a container and a label or package insert on or associated with the container. Suitable containers include, for example, bottles, vials, syringes, etc. The containers may be formed from a variety of materials such as glass or plastic. Generally, the container holds a composition, which is effective for treating a cancer, described herein, and may have a sterile access port (for example, the container may be an intravenous solution bag or a vial having a stopper pierceable by a hypodermic injection needle). Package insert refers to instructions customarily included in commercial packages of therapeutic products that contain information about the indications, usage, dosage, administration, contraindications and/or warnings concerning the use of such therapeutic products. In some embodiments, the package insert indicates that the composition is used for treating a cancer. The label or package insert may further comprise instructions for administering the composition to a patient.

Additionally, the article of manufacture may further comprise a second container comprising a pharmaceutically acceptable buffer, such as bacteriostatic water for injection (BWFI), phosphate-buffered saline, Ringer's solution and dextrose solution. It may further include other materials desirable from a commercial and user standpoint, including other buffers, diluents, filters, needles, and syringes.

Kits are also provided that are useful for various purposes, *e.g.,* for treatment of a cancer described herein, optionally in combination with the articles of manufacture. Kits of the present application include one or more containers comprising any one of the compositions described herein (or unit dosage form and/or article of manufacture). In some embodiments, the kit further comprises other agents *(e.g.,* one or more additional therapeutic agents) and/or instructions for use in accordance with any of the methods described herein. The kit may further comprise a description of selection of individuals suitable for treatment. Instructions supplied in the kits of the present application are typically written instructions on a label or package insert (*e.g.,* a paper sheet included in the kit), but machine-readable instructions (*e.g.,* instructions carried on a magnetic or optical storage disk) are also acceptable.

For example, in some embodiments, there is provided a kit comprising a pharmaceutical composition comprising any one of the anti-CD137 antibodies described herein and a pharmaceutically acceptable carrier; and instructions for administering the pharmaceutical composition to a subject having a cancer. In some embodiments, the kit further comprises a pharmaceutical composition comprising an additional therapeutic agent, such as an immune checkpoint inhibitor or a chemotherapeutic agent. In some embodiments, the kit comprises one or more assays or reagents thereof for determining a level of one or more biomarkers described herein (*e.g.,* total CD137, mCD137, sCD137, CD137L, Ki67, NK cells, $T_{em}$ and/or $T_{reg}$). In some embodiments, the kit comprises an IHC assay for determining a level of CD137L in a sample. In some embodiments, the kit comprises an anti-CD137L antibody (*e.g.*, TY23561). In some embodiments, the IHC assay is a multiplex IHC assay for determining levels of two or more (*e.g.,* 3, 4, 5, 6, or more) biomarkers, such as total CD137, CD137L, and PD-L1. In some embodiments, the kit comprises an immunoassay (*e.g.,* MSD assay) for determining a level of sCD137.

The kits of the present application are in suitable packaging. Suitable packaging includes, but is not limited to, vials, bottles, jars, flexible packaging (*e.g.,* sealed Mylar or plastic bags), and the like. Kits may optionally provide additional components such as buffers and interpretative information. The present application thus also provides articles of manufacture, which include vials (such as sealed vials), bottles, jars, flexible packaging, and the like.

The containers may be unit doses, bulk packages (*e.g.,* multi-dose packages) or sub-unit doses. Kits may also include multiple unit doses of the pharmaceutical compositions and instructions for use and packaged in quantities sufficient for storage and use in pharmacies, for example, hospital pharmacies and compounding pharmacies.

### VI. Exemplary Embodiments

Among the provided embodiments are:

Embodiment 1. A method of treating a cancer in a subject, comprising administering to the subject an effective amount of an anti-CD137 antibody that specifically binds to an extracellular domain of human CD137, wherein the antibody binds to one or more amino acid residues selected from the group consisting of amino acid residues 51, 53, 62-73, 83, 89, 92, 95-104 and 112-116 of SEQ ID NO: 1, and wherein the anti-CD137 antibody is administered at a dose of no more than 10mg/kg.

Embodiment 2. A method of treating a cancer in a subject, comprising administering to the subject an effective amount of an anti-CD137 antibody that specifically binds to an extracellular domain of human CD137, wherein the antibody binds to one or more amino acid residues selected from the group consisting of amino acid residues 51, 53, 62-73, 83, 89, 92, 95-104 and 112-116 of SEQ ID NO: 1, and wherein the anti-CD137 antibody is administered at a dose of no more than 500 mg.

Embodiment 3. A method of treating a cancer in a subject, comprising administering to the subject an effective amount of an anti-CD137 antibody that specifically binds to an extracellular domain of human CD137, wherein the antibody binds to one or more amino acid residues selected from the group consisting of amino acid residues 51, 53, 62-73, 83, 89, 92, 95-104 and 112-116 of SEQ ID NO: 1, and wherein the cancer is resistant or refractory to a prior therapy.

Embodiment 4. The method of embodiment 3, wherein the prior therapy is treatment with an anti-CD20 antibody.

Embodiment 5. The method of embodiment 4, wherein the anti-CD20 antibody is rituximab.

Embodiment 6. A method of treating a cancer in a subject, comprising administering to the subject an effective amount of an anti-CD137 antibody that specifically binds to an extracellular domain of human CD137, wherein the antibody binds to one or more amino acid residues selected from the group consisting of amino acid residues 51, 53, 62-73, 83, 89, 92, 95-104 and 112-116 of SEQ ID NO: 1; and wherein the subject has a high level in one or more biomarkers selected from the group consisting of total CD137, membrane bound CD137 (mCD137), CD137 ligand (CD137L), and PD-L1 and/or a low level of CD8+ effector memory T ($T_{em}$) cells or natural killer (NK) cells compared to a reference level.

Embodiment 7. A method of treating a cancer in a subject, comprising: (a) administering to the subject an effective amount of an anti-CD137 antibody that specifically binds to an extracellular domain of human CD137, wherein the antibody binds to one or more amino acid residues selected from the group consisting of amino acid residues 51, 53, 62-73, 83, 89, 92, 95-104 and 112-116 of SEQ ID NO: 1; and (b) subsequently determining a level of one or more biomarkers selected from the group consisting of total CD137, membrane bound (mCD137), soluble CD137 (sCD137), CD137L, Ki67, CD8+ effector memory T ($T_{em}$) cells, regulatory T ($T_{reg}$) cells, and NK cells in a sample of the subject.

Embodiment 8. The method of embodiment 7, wherein an increased level of one or more biomarkers selected from the group consisting of total CD137, sCD137, Ki67, CD137L, NK cells and CD8 $T_{em}$ cells, and/or a decreased level of one or more biomarkers selected from the group consisting of mCD137 and $T_{reg}$ cells after administration of the anti-CD137 antibody compared to the level of the one or more biomarkers before administration of the anti-CD137 antibody indicates that the subject may benefit from the administration of the anti-CD137 antibody.

Embodiment 9. The method of embodiment 7 or 8, wherein the sample has an increased level of one or more biomarkers selected from the group consisting of total CD137, sCD137, Ki67, CD137L, NK cells and CD8 $T_{em}$ cells, and/or a decreased level of one or more biomarkers selected from the group consisting of mCD137 and $T_{reg}$ cells after administration of the anti-CD137 antibody compared to the level of the one or more biomarkers before administration of the anti-CD137 antibody, the method further comprises administering to the subject an effective amount of the anti-CD137 antibody.

Embodiment 10. A method of providing a prognosis for a subject who has been administered with an effective amount of an anti-CD137 antibody that specifically binds to an extracellular domain of human CD137, wherein the antibody binds to one or more amino acid residues selected from the group consisting of amino acid residues 51, 53, 62-73, 83, 89, 92, 95-104 and 112-116 of SEQ ID NO: 1; the method comprising determining a level of one or more biomarkers selected from the group consisting of total CD137, membrane bound (mCD137), soluble CD137 (sCD137), Ki67, CD137L, NK cells, CD8+ effector memory T ($T_{em}$) cells, and regulatory T ($T_{reg}$) cells in a sample of the subject, wherein an increased level of one or more biomarkers selected from the group consisting of total CD137, sCD137, Ki67, CD137L, NK cells and CD8 $T_{em}$ cells, and/or a decreased level of one or more biomarkers selected from the group consisting of mCD137 and $T_{reg}$ cells after administration of the anti-CD137 antibody compared to the level of the one or more biomarkers before administration of the anti-CD137 antibody identifies the subject as having a high likelihood of responding to the anti-CD137 antibody treatment.

Embodiment 11. The method of any one of embodiments 6-10, wherein the level of one or more biomarkers comprise a level of total CD137.

Embodiment 12. The method of any one of embodiments 6-11, wherein the level of one or more biomarkers comprises a level of sCD137 in a plasma sample.

Embodiment 13. The method of any one of embodiments 6-12, wherein the level of one or more biomarkers comprises a level of mCD137 on CD8$^+$ T cells.

Embodiment 14. The method of any one of embodiments 6-13, wherein the level of one or more biomarkers comprises a level of Ki67 on CD8$^+$ T cells.

Embodiment 15. The method of embodiment 13 or 14, wherein the CD8$^+$ T cells are tumor infiltrating T cells.

Embodiment 16. The method of any one of embodiments 6-15, wherein the level of one or more biomarkers comprises a level of CD137L.

Embodiment 17. The method of any one of embodiments 5-16, wherein the level of one or more biomarkers comprises a level of NK cells in a blood sample.

Embodiment 18. The method of any one of embodiments 11 and 13-16, wherein the sample is a tumor biopsy sample.

Embodiment 19. The method of embodiment 18, wherein the tumor biopsy sample is a formalin-fixed paraffin-embedded (FFPE) sample.

Embodiment 20. The method of embodiment 18 or 19, wherein level of the one or more biomarkers is detected by immunohistochemistry (IHC).

Embodiment 21. The method of any one of embodiments 1-20, wherein the cancer is solid cancer.

Embodiment 22. The method of any one of embodiments 1-20, wherein the cancer is selected from the group consisting of colon cancer, breast cancer, lung cancer, esophageal cancer, endometrial cancer, gastrointestinal cancer, cholangiocarcinoma, nasopharyngeal cancer (NPC), adenoid cystic carcinoma (ACC), melanoma, mesothelioma, mantle cell lymphoma, T cell lymphoma, anal cancer, head and neck cancer, and appendiceal and sebaceous cancer.

Embodiment 23. The method of any one of embodiments 1-20, wherein the cancer is a liquid cancer.

Embodiment 24. The method of embodiment 23, wherein the cancer is non-Hodgkin's lymphoma.

Embodiment 25. The method of embodiment 21, wherein the cancer is a breast cancer.

Embodiment 26. The method of embodiment 25, wherein the cancer is triple-negative breast cancer (TNBC).

Embodiment 27. The method of embodiment 21, wherein the cancer is a lung cancer.

Embodiment 28. The method of embodiment 27, wherein the cancer is a small cell lung cancer (SCLC).

Embodiment 29. The method of embodiment 27, wherein the cancer is a non-small cell lung cancer (NSCLC).

Embodiment 30. A method of treating a cancer in a subject, comprising administering to the subject an effective amount of an anti-CD137 antibody that specifically binds to an extracellular domain of human CD137, wherein the antibody binds to one or more amino acid residues selected from the group consisting of amino acid residues 51, 53, 62-73, 83, 89, 92, 95-104 and 112-116 of SEQ ID NO: 1, and wherein the cancer is selected from the group consisting of follicular lymphoma, T cell lymphoma and ACC.

Embodiment 31. The method of embodiment 30, wherein the cancer is follicular lymphoma.

Embodiment 32. The method of embodiment 30 or 31, wherein the cancer is T cell lymphoma.

Embodiment 33. The method of embodiment 32, wherein the cancer is angioimmunoblastic T-cell lymphoma (AITL) or Peripheral T-cell lymphoma (PTCL).

Embodiment 34. The method of embodiment 30, wherein the cancer is ACC.

Embodiment 35. A method of treating a lung cancer in a subject, comprising administering to the subject: (a) an effective amount of an anti-CD137 antibody that specifically binds to an extracellular domain of human CD137, wherein the antibody binds to one or more amino acid residues selected from the group consisting of amino acid residues 51, 53, 62-73, 83, 89, 92, 95-104 and 112-116 of SEQ ID NO: 1; and (b) an effective amount of an immune checkpoint inhibitor.

Embodiment 36. A method of treating a breast cancer in a subject, comprising administering to the subject: (a) an effective amount of an anti-CD137 antibody that specifically binds to an extracellular domain of human CD137, wherein the antibody binds to one or more amino acid residues selected from the group consisting of amino acid residues 51, 53, 62-73, 83, 89, 92, 95-104 and 112-116 of SEQ ID NO: 1; and (b) an effective amount of an immune checkpoint inhibitor.

Embodiment 37. The method of embodiment 36, wherein the breast cancer is triple-negative breast cancer.

Embodiment 38. The method of any one of embodiments 35-37, wherein the immune checkpoint inhibitor is an anti-PD-L1 antibody.

Embodiment 39. The method of any one of embodiments 35-37, wherein the immune checkpoint inhibitor is an anti-PD-1 antibody.

Embodiment 40. The method of embodiment 39, wherein the anti-PD-1 antibody is toripalimab.

Embodiment 41. The method of embodiment 40, wherein the anti-CD137 antibody is administered at a dose of about 50 mg, about 100 mg, or about 200 mg.

Embodiment 42. The method of embodiment 40, wherein the anti-PD-1 antibody is administered at a dose of about 240 mg (*e.g.,* once every three weeks).

Embodiment 43. The method of any one of embodiments 35-37, wherein the immune checkpoint inhibitor is an anti-CTLA-4 antibody.

Embodiment 44. A method of treating a lung cancer in a subject, comprising administering to the subject: (a) an effective amount of an anti-CD137 antibody that specifically binds to an extracellular domain of human CD137, wherein the antibody binds to one or more amino acid residues selected from the group consisting of amino acid residues 51, 53, 62-73, 83, 89, 92, 95-104 and 112-116 of SEQ ID NO: 1; and (b) an effective amount of a chemotherapeutic agent.

Embodiment 45. A method of treating a breast cancer in a subject, comprising administering to the subject: (a) an effective amount of an anti-CD137 antibody that specifically binds to an extracellular domain of human CD137, wherein the antibody binds to one or more amino acid residues selected from the group consisting of amino acid residues 51, 53, 62-73, 83, 89, 92, 95-104 and 112-116 of SEQ ID NO: 1; and (b) an effective amount of a chemotherapeutic agent.

Embodiment 46. The method of embodiment 45, wherein the breast cancer is triple-negative breast cancer.

Embodiment 47. The method of any one of embodiments 44-46, wherein the chemotherapeutic agent is docetaxel.

Embodiment 48. The method of any one of embodiments 44-46, wherein the chemotherapeutic agent is cisplatin.

Embodiment 49. The method of embodiment 48, wherein the method does not comprise administering to the subject paclitaxel or pemetrexed.

Embodiment 50. A method of treating a lung cancer in a subject, comprising administering to the subject: (a) an effective amount of an anti-CD137 antibody that specifically binds to an extracellular domain of human CD137, wherein the antibody binds to one or more amino acid residues selected from the group consisting of amino acid residues 51, 53, 62-73, 83, 89, 92, 95-104 and 112-116 of SEQ ID NO: 1; and (b) an effective amount of an anti-CD20 antibody.

Embodiment 51. The method of embodiment 50, wherein the anti-CD20 antibody is rituximab.

Embodiment 52. A method of treating a colon cancer in a subject, comprising administering to the subject: (a) an effective amount of an anti-CD137 antibody that specifically binds to an extracellular domain of human CD137, wherein the antibody binds to one or more amino acid residues selected from the group consisting of amino acid residues 51, 53, 62-73, 83, 89, 92, 95-104 and 112-116 of SEQ ID NO: 1; and (b) an effective amount of a radiation therapy.

Embodiment 53. The method of any one of embodiments 2-52, wherein the anti-CD137 antibody is administered at a dose of about 50 mg to about 400 mg.

Embodiment 54. The method of embodiment 53, wherein the anti-CD137 antibody is administered at a dose of 50 mg, 100 mg, 200 mg, 300 mg or 400 mg.

Embodiment 55. The method of any one of embodiments 1-54, wherein the anti-CD137 antibody is administered at a dose of about 0.1 mg/kg to about 10 mg/kg.

Embodiment 56. The method of embodiment 55, wherein the anti-CD137 antibody is administered at a dose of about 3 mg/kg to about 8 mg/kg.

Embodiment 57. The method of embodiment 56, wherein the anti-CD137 antibody is administered at a dose of about 3mg/kg or about 5 mg/kg.

Embodiment 58. The method of any one of embodiments 1-57, wherein the anti-CD137 antibody is administered intravenously.

Embodiment 59. The method of any one of embodiments 1-58, wherein the anti-CD137 antibody is administered about once every three weeks.

Embodiment 60. The method of any one of embodiments 1-59, wherein the subject receives at least 2 cycles of treatment with the anti-CD137 antibody.

Embodiment 61. The method of any one of embodiments 1-60, wherein the cancer is advanced-stage cancer.

Embodiment 62. The method of any one of embodiments 1-61, wherein the cancer is metastatic cancer.

Embodiment 63. The method of any one of embodiments 1-62, wherein the cancer is resistant or refractory to a prior therapy.

Embodiment 64. The method of embodiment 63, wherein the prior therapy is selected from the group consisting of viral gene therapy, immunotherapy, targeted therapy, radiation therapy, and chemotherapy.

Embodiment 65. The method of any one of embodiments 1-64, wherein the anti-CD137 antibody is cross-reactive with a CD137 polypeptide from at least one non-human species selected from the group consisting of cynomolgus monkey, mouse, rat and dog.

Embodiment 66. The method of any one of embodiments 1-65, wherein the anti-CD137 antibody binds to amino acid residues 51, 63-67, 69-73, 83, 89, 92, 98-104 and 112-114 of SEQ ID NO: 1.

Embodiment 67. The method of any one of embodiments 1-66, wherein the anti-CD137 antibody comprises a heavy chain variable region (VH) and a light chain variable region (VL), wherein the VH comprises a HVR-H1 comprising the amino acid sequence of SEQ ID NO: 2, a HVR-H2 comprising the amino acid sequence of SEQ ID NO: 3, and a HVR-H3 comprising the amino acid sequence of SEQ ID NO: 4; and wherein the VL comprises a HVR-L1 comprising the amino acid sequence of SEQ ID NO: 5, a HVR-L2 comprising the amino acid sequence of SEQ ID NO: 6, and a HVR-L3 comprising the amino acid sequence of SEQ ID NO: 7.

Embodiment 68. The method of embodiment 67, wherein the VH comprises the amino acid sequence of SEQ ID NO: 8, and/or the VL comprises the amino acid sequence of SEQ ID NO: 9.

Embodiment 69. The method of embodiment 68, wherein the antibody comprises a heavy chain and a light chain, and wherein the heavy chain comprises the amino acid sequence of SEQ ID NO: 10, and/or the light chain comprises the amino acid sequence of SEQ ID NO: 11.

Embodiment 70. The method of any one of embodiments 1-66, wherein the anti-CD137 antibody comprises a VH and a VL, wherein the VH comprises a HVR-H1 comprising the amino acid sequence of SEQ ID NO: 12, a HVR-H2 comprising the amino acid sequence of SEQ ID NO: 13, and a HVR-H3 comprising the amino acid sequence of SEQ ID NO: 14; and wherein the VL comprises a HVR-L1 comprising the amino acid sequence of SEQ ID NO: 15, a HVR-L2 comprising the amino acid sequence of SEQ ID NO: 16, and a HVR-L3 comprising the amino acid sequence of SEQ ID NO: 17.

Embodiment 71. The method of embodiment 70, wherein the VH comprises the amino acid sequence of SEQ ID NO: 18, and/or the VL comprises the amino acid sequence of SEQ ID NO: 19.

Embodiment 72. The method of embodiment 71, wherein the antibody comprises a heavy chain and a light chain, wherein the heavy chain comprises the amino acid sequence of SEQ ID NO: 20, and/or the light chain comprises the amino acid sequence of SEQ ID NO: 21.

Embodiment 73. The method of any one of embodiments 1-66, wherein the anti-CD137 antibody comprises a VH and a VL, wherein the VH comprises a HVR-H1 comprising the amino acid sequence of SEQ ID NO: 22, a HVR-H2 comprising the amino acid sequence of SEQ ID NO: 23, and a HVR-H3 comprising the amino acid sequence of SEQ ID NO: 24; and wherein the VL comprises a HVR-L1 comprising the amino acid sequence of SEQ ID NO: 25, a HVR-L2 comprising the amino acid sequence of SEQ ID NO: 26, and a HVR-L3 comprising the amino acid sequence of SEQ ID NO: 27.

Embodiment 74. The method of embodiment 73, wherein the VH comprises the amino acid sequence of SEQ ID NO: 28, and/or the VL comprises the amino acid sequence of SEQ ID NO: 29.

Embodiment 75. The method of embodiment 74, wherein the antibody comprises a heavy chain and a light chain, wherein the heavy chain comprises the amino acid sequence of SEQ ID NO: 30, and/or the light chain comprises the amino acid sequence of SEQ ID NO: 31.

Embodiment 76. The method of any one of embodiments 1-75, wherein the anti-CD137 antibody comprises a human IgG4 Fc region.

Embodiment 77. The method of embodiment 76, wherein the human IgG4 Fc region comprises an S241P mutation, wherein numbering is according to Kabat.

Embodiment 78. The method of any one of embodiments 1-77, wherein the subject is a human subject.

Embodiment 79. The method of any one of embodiments 1-78, further comprising administering to the subject a therapeutically effective amount of at least one additional therapeutic agent.

## EXAMPLES

The invention can be further understood by reference to the following examples, which are provided by way of illustration and are not meant to be limiting.

## Example 1. Epitope Mapping

The CD137L binding site on CD137 as well as the epitopes of Utomilumab and Urelumab are determined based on the crystal structures with PDB IDs 6BWV, 6A3W and 6MHR respectively. The epitope of ADG106 is determined based on the crystal structure of CD137-ADG106 complex.

Crystals of the ADG106 fragment in complex with CD137 were obtained using sitting-drop vapor diffusion set-ups. Well diffracting crystals appeared within 4 days and grew to full size over 14 days. Crystals were cryo-protected by the addition of glycerol to a final concentration of 10% (v/v) to the crystallization drop before mounting. A complete 2.7 Å data set of an ADG106 fragment/CD137 crystal was collected and the data were integrated, analyzed and scaled. Complex formation results in a buried accessible surface area of approximately 2200 Å2 between the ADG106 fragment and CD137, with the area being approximately equally distributed between the variable regions of the light and heavy chains of the ADG106 fragment. The complex is stabilized through the formation of the prominent direct hydrogen bonds listed in Table 1, in addition to a number of van der Waal's interactions and water-mediated hydrogen bonds.

Table 1. Direct hydrogen bonds

| Light chain | CD137 | Distance (Å) |
|---|---|---|
| Gln27 (OE1) | Ile64 (N) | 2.8 |
| Tyr32 (OH) | Gly98 (O) | 3.6 |
| Asp50 (OD2) | Ser100 (OG) | 2.4 |
| Gly91 (O) | Arg66 (NH1) | 2.8 |
| Tyr92 (OH) | Ile64 (O) | 2.2 |
| Tyr92 (OH) | Asn83 (ND2) | 2.8 |
| Leu94 (N) | Gln67 (OE1) | 3.6 |
| **Heavy chain** | **CD137** | **Distance (Å)** |
| Asp 58 (OD2) | Arg73 (NH1) | 3.3 |
| Tyr60 (OH) | City70 (N) | 3.2 |
| Thr104 (O) | Gln104 (N) | 3.0 |
| Ile106 (O) | Cys102 (N) | 2.2 |
| Ile106 (N) | Cys102 (O) | 2.4 |
| Asp108 (OD2) | Arg66 (NH1) | 2.8 |
| Asp108 (OD1) | Lys69 (NZ) | 2.8 |
| Asp108 (OD2) | Arg66 (NH2) | 3.0 |

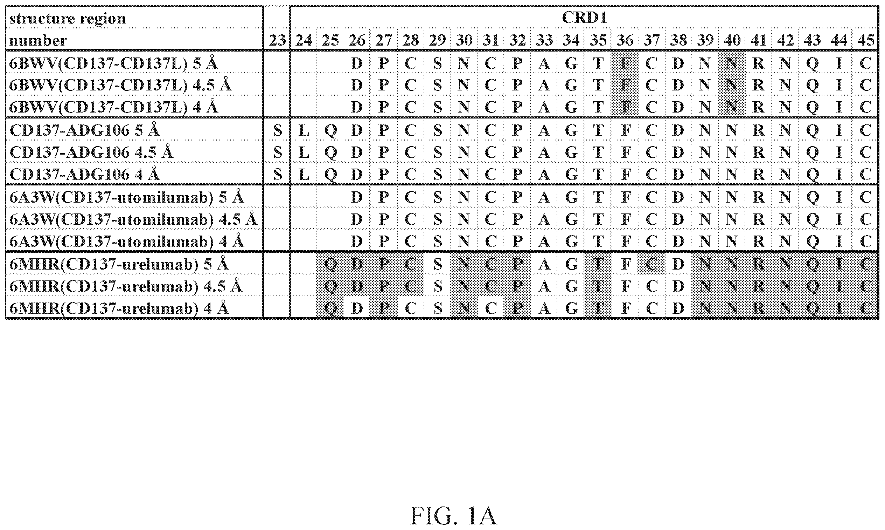

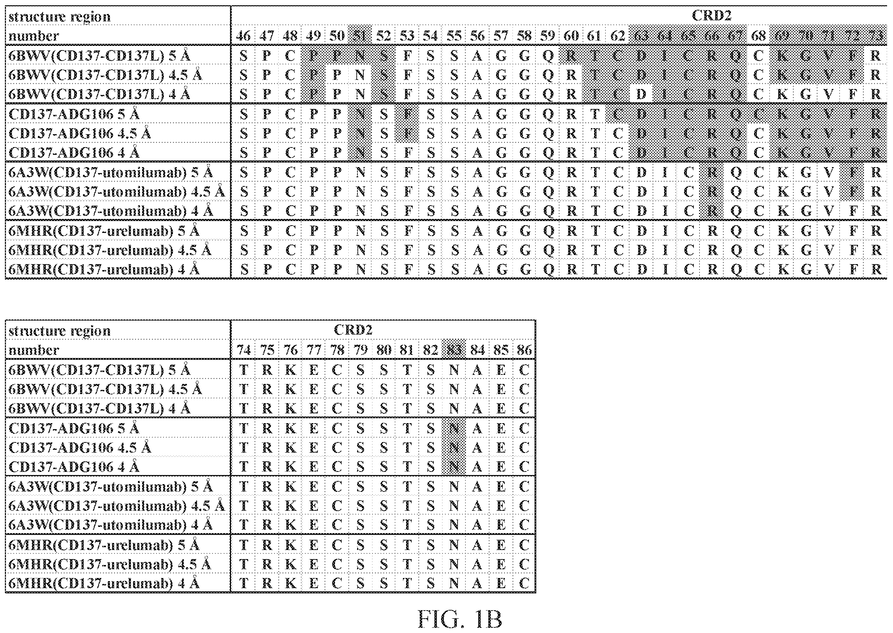

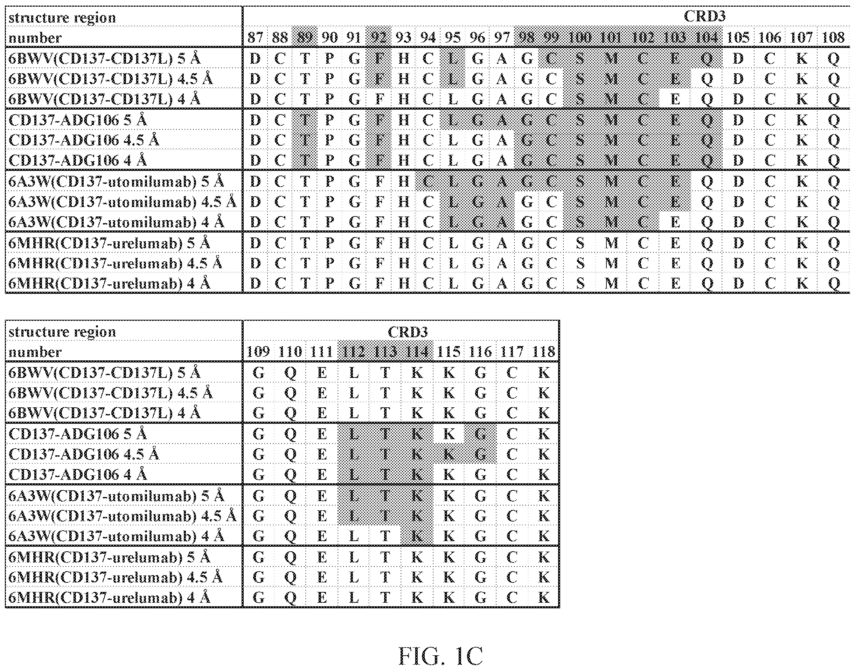

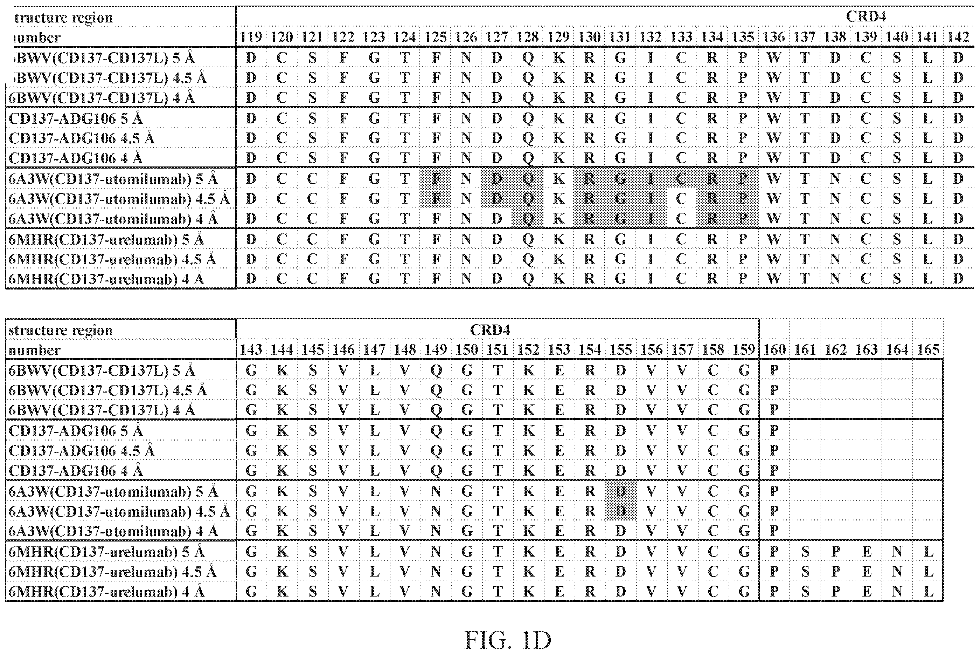

FIGS. 1A-1D show in grey amino acid residues in CD137 that are within 5Å, 4.5Å, or 4Å from one or more amino acid residues from CD137L, ADG106, Utomilumab or Urelumab. The epitope of ADG106 include amino acid residues 51, 63-67, 69-73, 83, 89, 92, 98-104 and 112-114 on CD137, the majority of which are located in the CDR2 domain of CD137.

ADG106 has a distinct epitope compared to the epitopes of known anti-CD137 antibodies Utomilumab and Urelumab. The epitope of ADG106 resembles the binding site of CD137L.

The epitope mapped by domain swapping/deletion plus site-directed mutagenesis has shown the binding epitope of CD137 across human, monkey and mouse species, this epitope spans domain some c-terminal part of domain 1 and most of the domains 2 and 3 (see WO2019/037711); the X-ray crystal structure complex between human CD137 and ADG106 shows that the epitope contact of CD137 with ADG106 locates mostly in domain 2 and 3. It is known the interaction between CD137 and its antibody in solution is dynamic in nature and would interchange between different conformations between CD137 and Adg106, although most of the contact sites in complex structure is consistent with epitope mapping by domain swapping/deletion plus site-directed mutagenesis, except for the C-terminal of domain one of CD137 which is not observed in the X-ray structure; however, using CD137 structure from its complex with its ligand in an alternative conformation, the C-terminal of domain of CRD1 of CD137 would interact with ADG106 at the previously mapped site by directed mutagenesis at 34 to 36 of CD137; therefore 34 to 36 residues would be considered to encompass CRD1 if CD137 dynamic fraying of its conformation is considered as shown by mutagenesis although the X-ray structure observed did not include them. The information provide here offer a more comprehensive understanding in the dynamic nature of the CD137 and ADG106 interaction, which would be important for their functional interpretation

### Example 2. Clinical studies of ADG106 treatment in patients with solid tumors and non-Hodgkin lymphoma

This example describes a phase 1, multicenter, open-label, dose-escalation and dose-expansion study to the safety and efficacy of ADG106 in patients with solid tumors and/or non-Hodgkin lymphoma. The primary objective of the study is assessment of the safety and tolerability of ADG106. The secondary objectives of the study are determination of the pharmacokinetic (PK) profile of ADG106, determination of the immunogenicity of ADG106, and evaluation of the antitumor activity of ADG106.

ADG106 is a fully human agonistic anti-CD137 monoclonal IgG4 antibody. ADG106 targets the evolutionally conserved epitope of CD137 with cross-species reactivity across mouse, rats, money and human CD137 and exhibits novel mechanism of action for CD137 agonism, CD137 antagonism and potent cross-linking via FcgRIIb.

### Objectives for Studies in United States (US)

The primary objective of the study is to assess safety and tolerability at increasing dose levels of single agent ADG106 in subjects with advanced or metastatic solid tumors and/or non-Hodgkin lymphoma. The secondary objectives of the study are to characterize the pharmacokinetic (PK) profiles of ADG106, to evaluate the immunogenicity of ADG106, and to evaluate the potential anti-tumor effect of ADG106. The exploratory objective of the study is to identify the potential biomarkers of ADG106.

### Outcome Measures for Studies in United States (US)

The primary outcome measures are:

1. Number of participants experiencing dose-limiting toxicities in the time frame of 2 Cycles (42 days).
2. Number of participants experiencing clinical and laboratory adverse events (AEs) in the time frame of first dose to 28 days post last dose.

The secondary outcome measures are:

1. The area under the curve (AUC) of plasma concentration of drug in the time frame from first dose (Cycle 1 Day 1, each cycle is 21 days) until the last dose (up to 2 years).
2. Maximum concentration (Cmax) in the time frame from first dose (Cycle 1 Day 1, each cycle is 21 days) until the last dose (up to 2 years).
3. Time at which maximum concentration (Tmax) in the time frame from first dose (Cycle 1 Day 1, each cycle is 21 days) until the last dose (up to 2 years).
4. Lowest plasma concentration (C[trough]) in the time frame from first dose (Cycle 1 Day 1, each cycle is 21 days) until the last dose (up to 2 years).

### Inclusion and Exclusion Criteria for Studies in United States (US)

Subjects were eligible to be included in the study in US only if all the following criteria apply:

1. Male or female, 18 years of age or older at the time of consent.
2. Provide written informed consent.
3. Subjects with advanced and/or metastatic histologically or cytologically confirmed solid tumor and/or non-Hodgkin lymphoma who were refractory or relapsed from standard therapy and who had exhausted all available therapies.
4. Life expectancy of 12 weeks or greater.
5. Eastern Cooperative Oncology Group (ECOG) performance status ≤ 2.
6. At least one measurable lesion per RECIST 1.1 for solid tumors and per Lugano Classification for non-Hodgkin lymphoma.
7. Adequate organ and bone marrow function
8. Women of childbearing potential (WOCBP) must have a negative serum pregnancy test within the 7 days prior to study drug administration.

Subjects who met any of the following criteria were not eligible to participate in the study in US:

1. Active central nervous system primary or secondary malignancies, active seizure disorder, spinal cord compression, or carcinomatous meningitis.
2. Any active autoimmune disease or documented history of autoimmune disease.
3. Infection of human immunodeficiency virus (HIV), hepatitis B virus (HBV), or hepatitis C virus (HCV), except for the following:
4. History of any non-infectious hepatitis (*e.g.*, alcohol or non-alcoholic steatohepatitis, drug-related or autoimmune hepatitis).
5. History of clinically significant cardiac disease.
6. Uncontrolled current illness.
8. WOCBP and sexually active fertile men with WOCBP partners who are unwilling or unable to use acceptable contraception method to avoid pregnancy.
9. Women who were pregnant at Screening or prior to study drug administration.
10. Women who were breastfeeding.
11. History of significant immune-mediated adverse event (AE).
13. Systemic use of the following therapies within 28 days prior to the first dose of study drug, or longer.
14. Subjects who received either of the below treatments:

- Any previous anti-CD137 mAb (*e.g.*, Utomilumab, or Urelumab) treatment.
- Subject who received allogenic hematopoietic stem cell transplant or autologous stem cell transplant.

## Objectives for Studies in China (CN)

The primary objectives of this study are to assess safety and tolerability at increasing dose levels of single agent ADG106 in subjects with advanced or metastatic solid tumors and/or non-Hodgkin lymphoma and to determine the recommended dosage and dosage regimen for further study. The secondary objectives of this study are to characterize the pharmacokinetic (PK) profiles of ADG106, to evaluate the immunogenicity of ADG106, to evaluate the potential anti-tumor effect of ADG106, and to investigate serum biomarkers related to immune regulation and cytokine releasing. The exploratory objective of this study is to identify the potential biomarkers of ADG106.

## Outcome Measures for Studies in China (CN)

The primary outcome measure is DLTs in the first 2 cycles of single drug administration.
The secondary outcome measures are:

1. Number of clinical and laboratory adverse events (AEs) in the time frame of first dose to 30 days post last dose.
2. Objective response rate (ORR) as assessed by RECIST version 1.1 and immune-related RECIST (irRECIST) for solid tumor and the Lugano Classification for non-Hodgkin Lymphoma in the time frame of 2 Cycles (42 days).
3. Duration of response (DOR) as assessed by RECIST version 1.1 and immune-related RECIST (irRECIST) for solid tumor and the Lugano Classification for non-Hodgkin Lymphoma in the time frame of 2 Cycles (42 days).
4. Time to progression (TTP) as assessed by RECIST version 1.1 and immune-related RECIST (irRECIST) for solid tumor and the Lugano Classification for non-Hodgkin Lymphoma in the time frame of 2 Cycles (42 days).
5. Disease control rate (DCR) as assessed by RECIST version 1.1 and immune-related RECIST (irRECIST) for solid tumor and the Lugano Classification for non-Hodgkin Lymphoma in the time frame of 2 Cycles (42 days).
6. Progression-free survival (PFS) as assessed by RECIST version 1.1 and immune-related RECIST (irRECIST) for solid tumor and the Lugano Classification for non-Hodgkin Lymphoma in the time frame of 2 Cycles (42 days).

7. Peak plasma concentration (Cmax) in the time frame of 2 Cycles (42 days).

8. Plasma concentration at the end of a dosing interval (Ctrough) in the time frame of 2 Cycles (42 days).

9. Time to reach Cmax (Tmax) in the time frame of 2 Cycles (42 days).

10. Area under the curve from time zero to the last timepoint (AUC0-last) in the time frame of 2 Cycles (42 days).

11. AUC from time zero to infinity (AUC0-∞) in the time frame of 2 Cycles (42 days).

12. AUC during a dosing interval (AUCtau) in the time frame of 2 Cycles (42 days).

13. Clearance (CL) in the time frame of 2 Cycles (42 days).

14. Volume of distribution at steady state (Vss) in the time frame of 2 Cycles (42 days).

15. ADA levels for ADG106 in the time frame of 2 Cycles (42 days).

16. Serum biomarkers linked to immunomodulation and cytokine release: such as TNFα, IFN-γ, IL 10, IL-6, IL-4, IL-2 in the time frame of 2 Cycles (42 days).

17. Cell counts for circulating T, natural killer (NK), and B cells in the time frame of 2 Cycles (42 days).

**Inclusion and Exclusion Criteria for Studies in China (CN)**

Subjects were eligible to be included in the study in China only if all the following criteria apply:

1. Male or female, 18 years to 75 years of age at the time of consent.

2. Provide written informed consent.

3. Subjects with advanced and/or metastatic histologically or cytologically confirmed solid tumor and/or non-Hodgkin lymphoma who were refractory or relapsed from standard therapy and who have exhausted all available therapies.

4. Provide tumor pathological section to the third party lab for PD-L1, CD137, CD137-L, Microsatellite instability (MSI) testing during screening period.

5. At least one measurable lesion per RECIST 1.1 for solid tumors and per Lugano Classification for non-Hodgkin lymphoma.

6. ECOG performance: 0-1.

7. Adequate organ and bone marrow function.

8. After receiving the last treatment (chemotherapy, radiotherapy, biotherapy, or other research drugs), the patient had a washout period of at least 4 weeks or more than 5 half-lives and had recovered from any toxic reaction of the previous treatment to less than 1 degree.

9. No other concomitant antineoplastic therapy (including cell therapy).

10. Women of childbearing potential (WOCBP) must have a negative serum pregnancy test within the 7 days prior to study drug administration.

11. Coagulation function was basically normal, INR≤1.5.

12. Cooperative in observation of adverse events and efficacy.

Subjects who met any of the following criteria were not eligible to participate in the study in China:

1. Subjects with positive HCV antibody, active hepatitis B (HBV DNA ≥ 10000 copies/mL or 2000 IU/mL), or positive hepatitis virus and taking antiviral drugs.

2. Subjects with meningeal metastasis, untreated brain metastasis lesions ≥ 1 cm, or brain metastasis requiring mannitol or other dehydration therapy.

3. Infection of human immunodeficiency virus (HIV), suffering from other acquired or congenital immunodeficiency disorders, or organ transplantation history.

4. Any active autoimmune disease, evidence-based autoimmune disease, or systemic syndrome requiring systemic steroids or immunosuppressive drugs (except for inactive vitiligo, psoriasis, asthma/specific reactivity in children after treatment within two years, or thyroid diseases controlled by alternative therapy/non-immunosuppressive therapy).

5. The residual toxicity of the patient's previous treatment was more than grade 1.

6. Fever body temperature above 38°C or there were clinically obvious active infections that can affect clinical trials.

7. Overdose of glucocorticoid (>10mg/d prednisone or equivalent dose) or other immunosuppressive agents was used within one month.

8. According to the investigator, any uncontrollable serious clinical problems include but not limited to: evidence of severe or uncontrollable systemic diseases (such as unstable or uncompensated respiratory, cardiac, liver, or kidney diseases); any unstable systemic diseases (including active infections, refractory high or drug failure Controlled hypertension (>150/100 mmHg), unstable angina pectoris, congestive heart failure, liver and kidney or metabolic diseases).

9. A clear history of neurological or psychiatric disorders, including epilepsy or dementia.

10. Non-research-related surgical procedures performed prior to the use of research drugs in patients within 28 days.
11. Investigator did not consider he/she appropriate to participate in this study.
12. Pregnant or lactating women.

**Phase 1a: Accelerated Titration and Conventional Dose Escalation Studies**

The phase 1a dose escalation study in US (ClinicalTrials.gov Identifier: NCT03707093) included accelerated titration (0.03, 0.1 and 0.3 mg/kg) and conventional dose escalation (1, 3, and 10 mg/kg). The phase 1a dose escalation study in China (ClinicalTrials.gov Identifier: NCT03802955) included accelerated titration (0.1 mg/kg) and conventional dose escalation (0.5, 1.5, 3, 5, and 10 mg/kg). For both studies, ADG106 was administered by intravenous infusion over 60 minutes on Day 1 of each cycle once every three weeks (Q3W). Patients with advanced or metastatic solid tumors and/or non-Hodgkin lymphoma who were refractory or relapsed and exhausted all available therapies were enrolled and received treatment until disease progression, intolerable toxicity, withdrawal with consent, or for a maximum of 24 months.
For phase 1a: accelerated titration and conventional dose escalation studies in US and China, 33 patients in 9 cohorts were enrolled. There were 5 patients with adenoid cystic carcinoma, 5 patients with colon cancer, 5 patients with non-small cell lung cancer (NSCLC), 2 patients with follicular lymphoma, 3 patients with nasopharyngeal carcinoma (NPC), and one each with anal, fibro-lung, fusiform cell, malignant pleural mesothelioma, mantle cell lymphoma, ovarian, breast, esophageal, endometrial, gastrointestinal (GI), cholangiocarcinoma, appendiceal, and sebaceous cancer.

**Phase 1b: Dose Expansion Studies**

Dose expansion studies were conducted for dose levels that were proven tolerable in Phase 1a and with evidence of clinical or biological activity. The dose levels studied in Phase 1b were 3 mg/kg and 5 mg/kg. ADG106 was administered by intravenous infusion over 60 minutes on Day 1 of each cycle once every three weeks (Q3 W).
For phase 1b: dose expansion study in US and China, 7 patients in 2 cohorts were enrolled (Table 2). There were two patients with nasopharyngeal caner (NPC), and one each with head and neck squamous cell carcinoma (HNSCC), mesothelioma, Sigmoid colon carcinoma, angioimmunoblastic T cell lymphoma, and melanoma.

Table 2. Dose expansion study enrollment

| Subject ID | Dose Level | Age | Gender | Indication | Treatment Cycle |
|---|---|---|---|---|---|
| 105-002 | 3.0 mg/kg | 64 | Male | HNSCC | 1 |
| 105-003 | 3.0 mg/kg | 59 | Female | Mesothelioma | 3 |
| 105-005 | 3.0 mg/kg | 69 | Male | Sigmoid Ca | 1 |
| R016 | 3.0 mg/kg | 45 | Male | NPC | 4 |
| R017 | 3.0 mg/kg | 46 | Male | Angioimmunoblastic T cell lymphoma | 4 |
| R018 | 3.0 mg/kg | 69 | Female | Melanoma | 4 |
| R020 | 5.0mg/kg | 63 | Male | NPC | 2 |

A total of 40 patient in 9 cohorts were enrolled for treatment in US and China phase 1a and 1b studies (Table 3A). Among the 40 patients, medium treatment duration was 2 cycles, the range of treatment duration was 1-10 cycles.

Table 3A. Study cohorts.

| | Accelerated Titration Phase | | | Conventional Dose Escalation Phase | | | | | | Dose Expansion | | Overall |
|---|---|---|---|---|---|---|---|---|---|---|---|---|
| | 0.03 mg/ kg | 0.1 mg/ kg | 0.3 mg/ kg | 0.5 mg/ kg | 1.0 mg/ kg | 1.5 mg/ kg | 3.0 mg/ kg | 5.0 mg/ kg | 10.0 mg/ kg | 3.0 mg/ kg | 5.0 mg /kg | |
| | US n=1 | US n=1, CN n=1 | US n=1 | CN n=3 | US n=4 | CN n=5 | US n=3, CN n=3 | US n=3, CN n=3 | US n=3, CN n=2 | US n=3, CN n=3 | CN n=1 | N=40 (%) |
| **Number of patients treated** | 1 | 2 | 1 | 3 | 4 | 5 | 6 | 6 | 5 | 6 | 1 | 40 |
| Number of patient discontinued the study treatment | 1 | 2 | 1 | 3 | 4 | 5 | 4 | 4 | 3 | 1 | 0 | 28 (70) |
| Number of patients still on study treatment | 0 | 0 | 0 | 0 | 0 | 0 | 2 | 2 | 2 | 5 | 1 | 12 (30) |
| **Primary reason of end of treatment** | | | | | | | | | | | | |
| Progressive disease | 1 | 2 | 0 | 3 | 3 | 4 | 4 | 3 | 2 | | | 22 (55) |
| Adverse event | | | | | 1 | | | 1 | 1 | 1 | | 4 (10) |
| Investigator decision | | | 1 | | | | | | | | | 1 (2.5) |
| lack of clinical benefit | | | | 1 | | | | | | | | 1 (2.5) |

Table 3B shows demographics and major cancer types of patients enrolled in the Chinese studies. Among the 23 Chinese patients tested so far, median treatment duration was 18.35 weeks (with minimum of 12.1 weeks and maximum of 33.1 weeks).

Table 3B. Demographics of Chinese Phase 1a and Phase 1b studies.

| Age, years | |
|---|---|
| Median[range] | 49 [21,72] |
| >65 years, n(%) | 3 (13.0) |
| Gender [n (%)] | |
| Male | 16 (69.6) |
| ECOG PS, n(%) | |
| 0 | 20 (87.0) |
| 1 | 3 (13.0) |
| Weight(kg) | |
| Median [range] | 60.8[41.1, 77.8] |

(continued)

| Primary Cancer n (%) | |
|---|---|
| Non-small cell lung cancer | 6(26.1) |
| Nasopharyngeal carcinoma | 6(26.1) |
| Adenoid cystic carcinoma | 5(21.7) |
| Malignant pleural mesothelioma | 1(4.3) |
| Melanoma | 1(4.3) |
| Gastrointestinal Neuroectodermal Tumor | 1(4.3) |
| Follicular lymphoma | 1(4.3) |
| Angioimmunoblastic T-cell lymphoma | 1(4.3) |
| Mantle cell lymphoma | 1(4.3) |

**Efficacy**

Efficacy of ADG106 was measured by the percentage of stable disease achieved and decline in standardized uptake values (SUV) on PET CT images. Among 40 patients in 7 cohorts, 17 (42.5%) patients achieved stable disease. Among the 17 patients who achieved stable diseases, 7 patients were observed to have tumor size reduction (Table 4). Table 4. Over all ADG106 efficacy data of accelerated titration phase, dose escalation phase, and dose expansion phase studies.

| Tumor Assessment | Accelerated Titration (mg/kg) | | | | Conventional Dose Escalation (mg/kg) | | | Dose Expansion (mg/kg) | | | Overall | |
|---|---|---|---|---|---|---|---|---|---|---|---|---|
| | 0.03 | 0.1 | 0.3 | 0.5 | 1.0 | 1.5 | 3.0 | 5.0 | 10.0 | 3.0 | 5.0 | |
| | US n=1 | US n=1, CN n=1 | US n=1 | CN n=3 | US n=4 | CN n=5 | US n=3, CN n=3 | US n=3, CN n=3 | US n=3, CN n=2 | US n=3, CN n=3 | CN n=1 | N=40 (%) |
| Stable Disease | **1** | | **1** | **1** | 1 | 2 | 4 | 4 | | 3 | | 17 (42.5) |
| Progressive disease | | 2 | | 2 | 2 | 3 | 2 | | 2 | | | 13 (32.5) |
| Not Evaluable | | | | | 1 | | | 2 | 1 | 1 | | 5 ( 12.5) |
| In plan | | | | | | | | | 2 | 2 | 1 | 5 (12.5) |

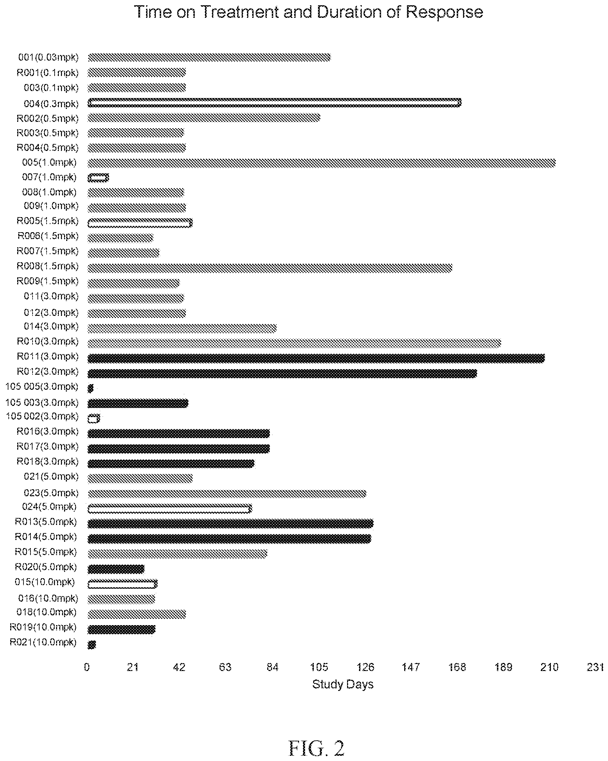

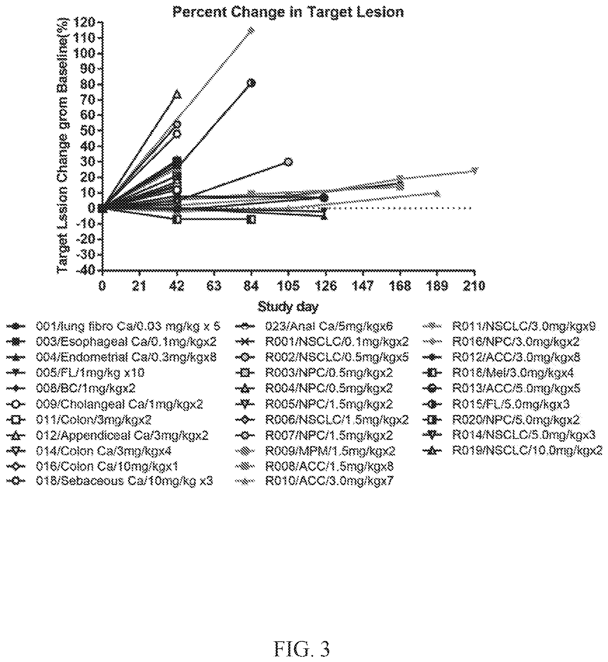

FIG. 2 and FIG. 3 show combined efficacy data of patients from both Chinese and U.S. clinical studies. FIG. 2 demonstrates a general trend of prolonged time on treatment and duration of response in patients who received higher dose levels of ADG106. FIG. 3 shows tumor shrinkage in several patients. One patient who experienced initial tumor enlargement had slight tumor shrinkage after receiving ADG106 treatment.

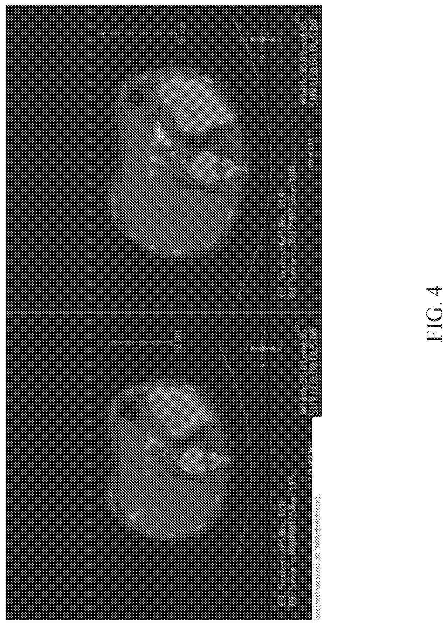

In particular, tumor shrinkage and decline in SUV scores on PET CT images were observed in one patient with stage IV Follicular Lymphoma (FL), who was refractory to multiple prior therapies including Rituximab (FIG. 4). Biopsy of liver metastasis was confirmed to be follicular lymphoma.

FIGS. 16A-16B show PET CT images of a 46 years old male patient with stage III angioimmunoblastic T cell lymphoma, who was treated with ADG106. Prior therapies included chemotherapy, folate analog metabolic inhibitor, and autologous hematopoietic stem cell transplantation. The patient achieved stable disease (SD) while receiving ADG106, an overall 33% tumor shrinkage of the targeted lesions after receiving one dose ADG106 and are supported by the biomarker studies described in Figures 15A-D. Shrinkage of two tumors were observed with 52% and 16% decrease in the volume of each tumor after only one administration of ADG106.

**Safety**

As shown in Table 5, Grade 3/4 adverse events (AEs) occurred in 15 patients (38%). serious adverse events (SAE) occurred in 10 patients (25%), and 4 SAE were considered drug-related. 1 patient in the U.S. study experienced dose limiting toxicity (DLT) at 5 mg/kg (with a body weight of about 110 kg); two patients had DLT at 10 mg/kg: one patient in the US study has suffered G3 adrenal insufficiency at 10mg/kg, and one patient in the Chinese study (with a body weight of about 50 kg) experienced DLT (grade 4 neutrophil count decreased) at 10 mg/kg. These results suggest that the DLT is around 10mg/kg by body weight and/or around a total dose of 500 mg. No difference was observed for the U.S. patient population versus the Chinese patient population.

As shown in Table 6, most of treatment emergent adverse events (TEAEs) were Grade 1 or 2, and there was no ≥Grade 4 TEAEs. The most common TEAEs (≥10%, regardless of causality) were decreased appetite (10%), anemia (10%), arthralgia (10%), lymphopenia (15%), dyspnea (10%), and respiratory failure (10%). G3 anemia at 10mg/kg cohort was drug related and rest G3 TEAEs were not related to the study treatment. There was no drug related death up to 10 mg/kg.

Table 5. Overall safety data of accelerated titration phase, dose escalation phase, and dose expansion phase studies

| Category | Accelerated Titration Phase | | | 3+3 Dose Escalation Phase | | | | | | |
| | 0.03mg/kg N=1 N (%) | 0.1 mg/kg N=2 n (%) | 0.3mg/kg N=1 N (%) | 0.5 mg/kg N=3 n (%) | 1mg/kg N=4 N (%) | 1.5 mg/kg N=5 n (%) | 3 mg/kg N=12 n (%) | 5 mg/kg N=7 n (%) | 10 mg/kg N=5 n (%) | Overall N=40 n (%) |
|---|---|---|---|---|---|---|---|---|---|---|
| Any Drug Related TEAEs | 1(100) | 2 (100) | 1(100) | 2(67) | 4(100) | 2(40) | 6(50) | 4(57) | 4(80) | 26( 65) |
| >= G3 | 1(100) | 0 | 1(100) | 0 | 3(75) | 0 | 4(33) | 2(39) | 4(80) | 15(38) |
| Any TEAEs leading to Study Drug Dose Reduction or Interruption | 1(100) | 0 | 0 | 0 | 0 | 0 | 0 | 0 | 0 | 1(3) |
| Any TEAEs Leading to Study Drug Discontinuation | 0 | 0 | 0 | 0 | 1(25) | 0 | 1(8) | 1(14) | 2(40) | 5(13) |
| Any TEAEs with Outcome of Death | 0 | 0 | 0 | 0 | 1(25) | 0 | 1(8) | 0 | 0 | 2(5) |
| Any SAE | 0 | 0 | 0 | 0 | 1(25) | 1(20) | 3(25) | 3(43) | 2(40) | 10(25) |
| Drug Related SAE | 0 | 0 | 0 | 0 | 0 | 0 | 0 | 2(29) | 2(40) | 4(10) |

(continued)

| | Accelerated Titration Phase | | | 3+3 Dose Escalation Phase | | | | | | |
|---|---|---|---|---|---|---|---|---|---|---|
| Category | 0.03mg/kg N=1 N (%) | 0.1 mg/kg N=2 n (%) | 0.3mg/kg N=1 N (%) | 0.5 mg/kg N=3 n (%) | 1mg/kg N=4 N (%) | 1.5 mg/kg N=5 n (%) | 3 mg/kg N=12 n (%) | 5 mg/kg N=7 n (%) | 10 mg/kg N=5 n (%) | Overall N=40 n (%) |
| SAE leading to Drug discontinuation | 0 | 0 | 0 | 0 | 0 | 0 | 0 | 1(14) | 2(40) | 3(7) |
| Any DLTs | 0 | 0 | 0 | 0 | 0 | 0 | 0 | 1(14) | 2(40) | 3(7) |
| *=DLT evaluated in the 1st 2 cycles (a cycle=21 days) | | | | | | | | | | |

Table 6. The most common TEAEs (>10%, regardless of causality).

| | Accelerated Titration Phase | | | | | | Dose Escalation Phase | | | | | | | | | |
|---|---|---|---|---|---|---|---|---|---|---|---|---|---|---|---|---|
| | 0.03 mg/kg | | 0.1 mg/kg | | 0.3 mg/kg | | 1 mg/kg | | 3 mg/kg | | 5 mg/kg | | 10 mg/kg | | Overall | |
| **Prefer Term** | n=1 | | n=1 | | n=1 | | n=4 | | n=7 | | n=3 | | n=3 | | N=20 (%) | |
| | G 3 | G 4 | G 3 | G 4 | G 3 | G 4 | G 3 | G 4 | G 3 | G 4 | G 3 | G 4 | G 3 | G 4 | G3 | G 4 |
| | 1 | 0 | 0 | 0 | 0 | 0 | 0 | 0 | 1 | 0 | 1 | 0 | 1 | 0 | 4 (20） | 0 |
| Appetite ↓ | 1 | 0 | 0 | 0 | 1 | 0 | 0 | 0 | 0 | 0 | 0 | 0 | 0 | 0 | 2 (10） | 0 |
| Anemia | 0 | 0 | 0 | 0 | 0 | 0 | 0 | 0 | 1 | 0 | 0 | 0 | 1 | 0 | 2 (10） | 0 |
| Tumor pain | 0 | 0 | 0 | 0 | 0 | 0 | 0 | 0 | 1 | 0 | 0 | 0 | 0 | 0 | 1 (5) | 0 |
| Arthralgia | 0 | 0 | 0 | 0 | 0 | 0 | 0 | 0 | 0 | 0 | 0 | 0 | 0 | 0 | 0 | 0 |
| Peripheral edema | 0 | 0 | 0 | 0 | 0 | 0 | 0 | 0 | 0 | 0 | 0 | 0 | 0 | 0 | 0 | 0 |
| Lymphopenia | 0 | 0 | 0 | 0 | 0 | 0 | 0 | 0 | 1 | 0 | 1 | 0 | 1 | 0 | 3 (15） | 0 |
| Diarrhea | 0 | 0 | 0 | 0 | 0 | 0 | 1 | 0 | 0 | 0 | 0 | 0 | 0 | 0 | 1 (5) | 0 |
| Dyspnea | 1 | 0 | 0 | 0 | 0 | 0 | 0 | 0 | 1 | 0 | 0 | 0 | 0 | 0 | 2 (10） | 0 |
| Respiratory failure | 0 | 0 | 0 | 0 | 0 | 0 | 0 | 0 | 2 | 0 | 0 | 0 | 0 | 0 | 2 (10） | 0 |
| AST increase | 0 | 0 | 0 | 0 | 0 | 0 | 0 | 0 | 0 | 0 | 0 | 0 | 1 | 0 | 1(5） | 0 |
| Vomiting | 0 | 0 | 0 | 0 | 0 | 0 | 1 | 0 | 0 | 0 | 0 | 0 | 0 | 0 | 1 (5) | 0 |
| Hypothyroidism | 0 | 0 | 0 | 0 | 0 | 0 | 0 | 0 | 0 | 0 | 0 | 0 | 0 | 0 | 0 | 0 |
| Cough | 0 | 0 | 0 | 0 | 0 | 0 | 0 | 0 | 0 | 0 | 0 | 0 | 0 | 0 | 0 | 0 |
| Dehydration | 0 | 0 | 0 | 0 | 0 | 0 | 0 | 0 | 0 | 0 | 0 | 0 | 0 | 0 | 0 | 0 |
| Weight ↓ | 0 | 0 | 0 | 0 | 0 | 0 | 0 | 0 | 0 | 0 | 0 | 0 | 0 | 0 | 0 | 0 |
| Myalgia | 0 | 0 | 0 | 0 | 0 | 0 | 0 | 0 | 0 | 0 | 0 | 0 | 0 | 0 | 0 | 0 |
| Fever | 0 | 0 | 0 | 0 | 0 | 0 | 0 | 0 | 0 | 0 | 0 | 0 | 0 | 0 | 0 | 0 |
| Constipation | 0 | 0 | 0 | 0 | 0 | 0 | 0 | 0 | 0 | 0 | 0 | 0 | 0 | 0 | 0 | 0 |

As shown in Table 7, across all dose cohorts of hematology laboratory abnormalities, one patient experienced both G3 hemoglobin decrease and G3 lymphocytes decrease, one patient experienced G3 hemoglobin decrease and one patient with G3 lymphocyte decrease.

Table 7. Hematologic Laboratory Abnormality

| Lab abnormality | Accelerated Titration | | | | | | Dose Escalation and expansion | | | | | | | | Total | |
|---|---|---|---|---|---|---|---|---|---|---|---|---|---|---|---|---|
| | 0.03 mg/kg | | 0.1 mg/kg | | 0.3 mg/kg | | 1 mg/kg | | 3 mg/kg | | 5 mg/kg | | 10 mg/kg | | Overall | |
| | n=1 | | n=1 | | n=1 | | n=4 | | n=7 | | n=3 | | n=3 | | N=20 (%) | |
| | G 3 | G 4 | G 3 | G 4 | G 3 | G 4 | G 3 | G 4 | G 3 | G 4 | G 3 | G 4 | G 3 | G 4 | G3 | G 4 |
| Hemoglobin ↓ | 0 | 0 | 0 | 0 | 0 | 0 | 0 | 0 | 1 | 0 | 0 | 0 | 1 | 0 | 2 (10) | 0 |
| Lymphocyte ↓ | 0 | 0 | 0 | 0 | 0 | 0 | 0 | 0 | 1 | 1* | 1 | 0 | 2 | 0 | 4 (20) | 1 (5) |
| Leukocyte | 0 | 0 | 0 | 0 | 0 | 0 | 0 | 0 | 0 | 0 | 0 | 0 | 0 | 0 | 0 | 0 |
| Platelet ↓ | 0 | 0 | 0 | 0 | 0 | 0 | 0 | 0 | 0 | 0 | 0 | 0 | 0 | 0 | 0 | 0 |
| Neutrophil ↓ | 0 | 0 | 0 | 0 | 0 | 0 | 0 | 0 | 0 | 0 | 0 | 0 | 0 | 0 | 0 | 0 |
| * represents two laboratory abnormalities/G3 occurred in one same patient. G4 lymphocyte decrease in patient occurred pre-dose and was not clinical significant. | | | | | | | | | | | | | | | | |

## Receptor Occupancy

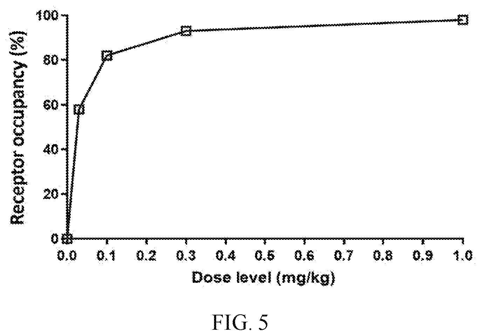

Receptor occupancy was estimated based on the binding affinities (KDs) of ADG106 (3.7nM), Urelumab (9.6nM) and Utomilumab (20.9nM), which were determined by surface plasmon resonance (SPR), with the following formula: Receptor occupancy % = [Drug plasma concentration] / ([Drug plasma concentration] + KD) x 100. Based on the calculation, receptor occupancy values of ADG106 at different dose levels were obtained and plotted. As shown in FIG.5, the receptor occupancy of ADG106 is estimated to be 58% at 0.03mg/kg, 82% at 0.1mg/kg, 93% at 0.3mg/kg and 98% at 1mg/kg dose levels, respectively. As shown in Table 8, the receptor occupancy of Urelumab at its maximal tolerated clinical dose (MTD, 0.1mg/kg) is estimated to be 64%, whereas Utomilumab is 98% at its maximal administered clinical dose (MAD, 10mg/kg). Therefore, ADG106 can achieve high receptor occupancy at a dose 10 times below its DLT dose.

Table 8. Receptor occupancy of ADG106 in comparison to Urelumab and Utomilumab.

| Compound | Dose (mg/kg) | Calculated Receptor Occupancy (%) |
|---|---|---|
| Urelumab | 0.1* | 64 |
| Utomilumab | 10* | 98 |
| ADG106 | 1 | 98 |
| * MTD for Urelumab, MAD for Utomilumab. | | |

## Pharmacokinetics (PK)

Pharmacokinetic profiles of ADG106 in subjects at dose levels of 0.03 mg/kg, 0.1 mg/kg, 0.3 mg/kg, 0.5 mg/kg, 1 mg/kg, 1.5mg/kg, 3 mg/kg, 5 mg/kg, and 10 mg/kg were determined. Blood samples for PK analysis were collected at Cycle 1 (Day 1: pre-dose and 2, 6, 12, and 24 hours after the end of infusion; once on Days 8, 15, and 22), Day 1 pre-dose and 2 hours after the end of infusion of subsequent cycles, and at the End of Treatment. The concentrations of ADG106 were determined from the serum samples using a validated ELISA method, in which an anti-ADG106 idiotype mouse monoclonal antibody was used to coated ELISA microplates for capture, and an HRP-labeled goat anti-hIgG4-Fc polyclonal antibody was used for detection. Mean serum concentrations of ADG106 in each dose group versus time were plotted. PK parameters were estimated using a non-compartmental method with WinNonlin.

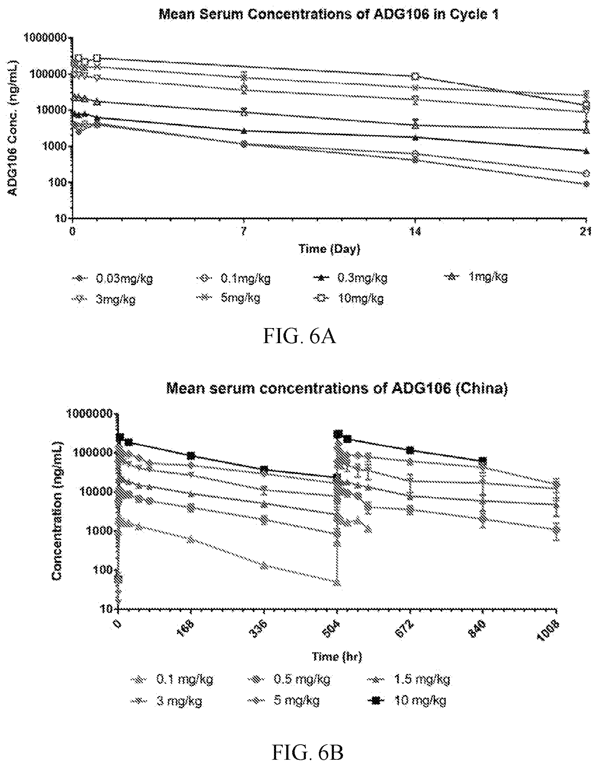

The pharmacokinetic analysis of ADG106 in FIGs. 6A-6B show dose-dependent proportional increase of serum ADG106 levels in response to systemic exposure. The mean half-life of ADG106 at doses ≥ 0.5mg/kg is around 7 days.

Table 9. Pharmacokinetics Parameters of ADG106 in Cycle 1 of the US clinical study.

| Parameter | Accelerated Titration Phase | | | Dose Escalation Phase | |
|---|---|---|---|---|---|
| | 0.03 mg/kg n=1 | 0.1 mg/kg n=1 | 0.3 mg/kg n=1 | 1 mg/kg n=3 | 3 mg/kg n=3 |
| $C_{max}$ ($\mu$g/L) | 4.25 | 4.18 | 7.95 | 23.3 | 95.03 |
| $AUC_{0-t}$ ($\mu$g/L·h) | 626 | 668 | 1387 | 3876.7 | 18420 |
| $T_{1/2}$ (h) | 87 | 108.5 | 159 | 146.3 | 149 |

In the U.S. study, following the first dose of ADG106, ADG106 achieved peak concentrations (Cmax) of 4.25, 4.18, 7.95, 23.3, and 95.03 $\mu$g/L for 0.03, 0.1, 0.3, 1, and 3 mg/kg dose levels (Table 10, FIG. 6), respectively. The AUC0-t values were 626, 668, 1387, 3876.7, and 18420 $\mu$g/L·h for 0.03, 0.1, 0.3, 1, and 3 mg/kg dose levels (Table 9), respectively. The ADG106 terminal phase half-life (t1/2) was 87, 108, 159, 146.3, and 149 hours for 0.03, 0.1, 0.3, 1, and 3 mg/kg dose levels, respectively. The pharmacokinetics data from the Chinese clinical study is shown in FIG. 6B and Table 10, ADG106 achieved peak concentrations (Cmax) of 2.3, 11.2, 27.2, 62.9, 155 and 255 $\mu$g/L for 0.1, 0.5, 1.5, 3, 5 and 10 mg/kg dose levels. The AUC0-t values were 255, 1761, 4162, 10016, 20966, and 39658 $\mu$g/L·h for 0.1, 0.5, 1, 1.5, 3, 5, and 10 mg/kg dose levels (Table 10), respectively. The ADG106 terminal phase half-life (t1/2) was 3.7, 6.1, 7.5, 8.9, 7.2 and 5.5 days for 0.1, 0.5, 1.5, 3, 5, and 10 mg/kg dose levels, respectively.

Table 10. Pharmacokinetics Parameters of ADG106 in the Chinese clinical study.

| Parameter | Accelerated Titration Phase | Dose Escalation Phase | | | | |
|---|---|---|---|---|---|---|
| | 0.1 mg/kg | 0.5 mg/kg | 1.5 mg/kg | 3 mg/kg | 5 mg/kg | 10 mg/kg |
| Cmax ($\mu$g/L) | 2.3 | 11.2 | 27.2 | 62.9 | 155 | 255 |
| AUC(0-t) (mg/L*h) | 255 | 1761 | 4162 | 10016 | 20966 | 39658 |
| T1/2 (day) | 3.7 | 6.1 | 7.5 | 8.9 | 7.2 | 5.5 |
| CLz (mL/h/kg) | 0.37 | 0.28 | 0.33 | 0.25 | 0.22 | 0.23 |

**Immunogenicity**

Immunogenicity of ADG106 in subjects at dose levels of 0.03 mg/kg, 0.1 mg/kg, 0.3 mg/kg, 0.5mg/kg, 1 mg/kg, 1.5mg/kg, 3 mg/kg, 5 mg/kg, and 10 mg/kg were determined. Blood samples were collected as described in the PK analysis. The anti-drug-antibody (ADA) to ADG106 in human serum was measured using a validated Affinity Capture and Elution (ACE) based immunoassay. All samples were first analyzed for ADAs in a screening assay. Study samples with results below the screening cut-off were reported as negative for ADAs. In the event of a positive result in the screening assay, samples were analyzed in the confirmatory assay. All samples confirmed positive were reported as positive. The immunogenicity studies show that treatment-induced anti-drug antibodies were developed in about 20% of the patients tested. ADG106 treatment emergent or boosted ADA occurred in 5 of 23 Chinese patients tested so far.

**Update of US Phase 1 Study**

As of Nov 30, 2020, a total of 35 patients were treated with ADG 106 in 7 dose escalation cohorts (16 patients at 0.03, 0.1,0.3, 1, 3, 5, and 10 mg/kg), 2 flat dose cohorts (3 patients at 300 mg and 3 patients at 400 mg, Q3W), and 2 dose expansion cohorts (9 patients at 3 mg/kg and 4 patients at 300 mg, Q3W). DLT evaluations of the 7 dose escalation cohorts (ie, 0.03, 0.1, 0.3, 1, 3, 5, and 10mg/kg, Q3W) have been completed and 2 DLTs were observed: G2 hypoglossal nerve disorder at 5 mg/kg and G3 adrenal insufficiency at 10mg/kg cohorts.

Among the 35 patients, 24 (69%) patients discontinued ADG 106 treatment: 19 (54%) patients discontinued due to progression disease or clinical progression (including one 64-years-old male patient with Stage IV tongue cancer with multiple lung metastases, died of disease progression on study day 4), 3 (9%) due to adverse events (one patient at 1mg/kg due to un-related G3 tachycardia; one patient at 10mg/kg due to related G3 adrenal insufficiency and related G3 anemia; one patient at 5mg/kg, due to related G2 hypoglossal nerve disorder), and 2 (6%) decided by investigator (i.e., one patient at 0.3mg/kg due to increased tumor marker and sever pain; one patient at 5mg/kg due to disease encroaching on sacral nerves and causing pain). Currently 11 (31%) patients at 3 mg/kg expansion cohort, 300mg and 400mg flat dose cohorts are still on the study treatment.

Among the 35 patients, there were 7 patients with colon cancers, 4 with renal cell carcinoma, 3 with follicular lymphoma, 3 with HNSCC, 2 with esophageal carcinoma, 2 with anal carcinoma, and one patient each with breast cancer, lung fibroma, NSCLC, mesothelioma, GI cancer, cholangial cancer, pancreatic cancer, appendiceal cancer, sebaceous cancer, endometrial cancer, cervix cancer, ovarian cancer, Leiomyosarcoma, and Hodgkin lymphoma (14 cancer types with one patient each). The male to female ratio was 16:19; the median age was 61 years (range 25-82 years).

Among the 35 patients, median treatment duration was 2 cycles (range 1-10 cycles) and median total amount received was 450 mg (range 9-3075mg). Two patients experienced DLTs: one of the three patients in the 10mg/kg cohort (male/58 yr/colon cancer) experienced DLT of G3 adrenal insufficiency; one of the three patients in 5 mg/kg cohort (female/54 yr/ovarian cancer) experienced DLT of G2 hypoglossal nerve disorder. The most common adverse events (AEs, ≥10%, regardless of causality) were fatigue (29%), nausea (23%), decreased appetite (20%), vomiting (14%), peripheral edema (14%), arthralgia (14%), dehydration (14%), anemia (11%), dyspnea (11%), tumor pain (11%), and dizziness (11%). Most of these AEs were Grade 1 or 2, and there was only 1 un-related Grade 4 acute respiratory failure (due to disease progression, male/64 yr/tongue cancer, total 1 dose of study drug at 3mg/kg). Grade 3 AEs included fatigue (5/35 patients), decreased appetite (2/35 patients), anemia (2/35 patients), dyspnea (2/35 patients), vomiting (1/35 patients), and tumor pain (1/35 patients).

Most drug-related TEAEs were G1 or G2 and there was no G4 drug-related TEAEs. Grade 3 drug-related TEAEs included fatigue (3/35 patients), decreased appetite (1/35 patients), AST increase (1/35 patients), adrenal insufficiency (1/35 patients), anemia (1/35 patients), dyspnea (1/35 patients), and influenza like symptoms (1/35 patients). Across all dose cohorts of 35 patients treated, liver function tests showed: AST increased in 15 patients (43%, all grades; only 2 G3); ALT increased in 8 patients (23%, all grade, only 1 G3); total bilirubin increased in 2 patients (6%, 1 G3); albumin decreased in 20 patients (57%, all grade, no ≥G3); the other G3 liver function enzyme increase included ALP increase (2/35 patients) and GGT increase (3/35 patients). Only 1 G4 pre-dose GGT increase occurred in 1 patient. One 42-year-old female patient with colon cancer with multiple liver metastasis, after 1 dose of the study drug, discontinued the study treatment due to progression disease at study day 29. At the end of treatment visit on study day 29, the liver test showed both G3 AST increase and G3 total bilirubin increase; pre-dose and throughout the study G4 GGT increase was not clinically significant. Among 35 patients treated, 31 patients (89%) reported hemoglobin decrease (all grades, 2 with G3), 20 patients (57%) reported lymphocytes decrease (all grades, 7 with G3, 1 with pre-dose G4 lymphocytes decrease which was not clinically significant by PI assessment). There was no G3 or G4 decreases of leukocytes, platelets, and neutrophils.

A total of 17 SAEs (all causes) occurred in 13 patients and only 4 SAEs in 3 patients were related to the study treatment: G3 anemia, G3 adrenal insufficiency in male/58 yr patient with colon cancer at 10mg/kg cohort; G2 hypoglossal nerve disorder in female/54 yr patient with ovarian cancer at 5mg/kg; and G3 flu like symptoms in male/42 yr patient with anal cancer at 5mg/kg. Tumor shrinkage and decline in SUV scores on PET CT image was observed in one patient with stage IV Follicular Lymphoma (FL) who was refractory to multiple prior therapies including Rituximab.

Among the 35 patients, 23 had post-tumor scans available and best response were 12 patient with SD and 11 patient with PD. Disease control rate was 52% (12/23 patients) and there was no CR or PR. Tumor shrinkage occurred in 3 patients: one patient with follicular lymphoma had tumor size decreased 2%, one patient with endometrial carcinoma had tumor size decreased 5%, one patient with esophageal carcinoma had tumor size decreased 18% tumor shrinkage.

ADG106 demonstrated favorable safety and tolerability profiles at doses up to 10 mg/kg. Currently patients are enrolled at cohort expansion of 3 mg/kg and flat dose of 400mg, Q3W. Preliminary clinical activity was seen in 1 patient with follicular lymphoma, 1 patient with endometrial carcinoma, and 1 patient with esophageal carcinoma, which warrants further evaluation.

**Update of China Phase 1 Study**

As of November 30, 2020, 57 patients have been enrolled and 57 patients have been treated in the ADG106-1002 clinical study in China. Among these 57 patients, 14 had nasopharyngeal cancer, 13 had non-small cell lung cancer, 10 had Non-Hodgkin's lymphoma, 5 had adenoid cystic cancer, 4 had cervical cancer, and 11 had others. Most patients were male (38/57) and the median age was 50 yr (range: 21-72yr). Among the 57 patients enrolled, 18 patients enrolled in dose escalation study (0.1mg/kg, n=1; 0.5mg/kg, n=3; 1.5mg/kg, n=5; 3mg/kg, n=3; 5mg/kg, n=3; 10mg/kg, n=3) and 39 patients enrolled in dose expansion study (3mg/kg, n=11; 5mg/kg, n=28). 45 of the 57 (68.9%) patients discontinued ADG106 treatment (31 patients discontinued due to progression disease, 7 due to AEs, 5 due to withdrawal of consent, 1 due to death related to disease progression, and 1 due to other reason). 12 patients (3mg/kg, n=2; 5mg/kg, n=10) were still on the study treatment. The median treatment duration was 2 cycles (1-17 cycles).

Among 18 patients enrolled in dose escalation study, 1 DLT (Grade 4 neutrophil count decreased, 10mg/kg) was observed in 1 patient with mantle cell lymphoma. The most commonly reported TEAEs (≥ 20%) were anemia (26, 46%), proteinuria (22, 39%), C-reactive protein increase (20, 35%), aspartate aminotransferase increase (18, 32%), hypoalbuminaemia (17, 30%), hyponatraemia (17, 30%), asthenia (17, 30%), neutrophil count decrease (16, 28%), neutrophil count increase (16, 28%), alanine aminotransferase increase (16, 28%), white blood cell count decrease (15, 26%), constipation (14, 25%), rash (14, 25%), hypochloraemia (13, 23%), cough (13, 23%), white blood cell count increase (12, 21%), blood present in urine (12, 21%), pruritus (12, 21%), and platelet count increase (12, 21%). Most TEAEs were G1 or G2. 21 patients (35%) experienced G3 to 4 TEAEs; the most common were anemia, white blood cell count decrease, hyponatremia, and neutrophil count decrease. Across all dose cohorts of 57 patients, liver function tests showed: AST increased in 18 patients (32 %, all grades; 1 G3, no G4); ALT increased in 16 patients (28%, all grades; 1 G3, no G4); albumin decreased in 17 patients (30%, all grades; 1 G3, no G4). A total of 15 SAEs (all causes) occurred in 11 patients and 7 SAEs were related to the study treatment.

Among the 57 patients, 48 had post-tumor scans available and best response were 27 patients with SD, 20 patients with PD and 1 patient with PR. Disease control rate was 58%. Tumor shrinkage was observed in 10 patients, including 3 patients with nasopharyngeal carcinoma, 3 patients with peripheral T-cell lymphoma, 2 patients with adenoid cystic carcinoma, 1 patient with non-small cell lung cancer, and 1 patient with melanoma. 1 patient with nasopharyngeal carcinoma demonstrated partial response with about 40% tumor reduction in the target lesion.

### Example 3. Pharmacokinetics (PK)/Toxicokinetics (TK), and immunogenicity in cynomolgus monkeys with repeated ADG106 treatment

Blood samples for PK/TK analysis were collected at different time points in each dosing cycle. The concentrations of ADG106 were determined from the serum samples using a validated ELISA method, in which an anti-ADG106 idiotype mouse monoclonal antibody was used to coat ELISA microplates for capture, and an HRP-labeled goat anti-hIgG-Fc polyclonal antibody was used for detection. Mean serum concentrations of ADG106 in different dosage groups versus time were plotted. PK parameters were estimated using a non-compartmental method with WinNonlin.

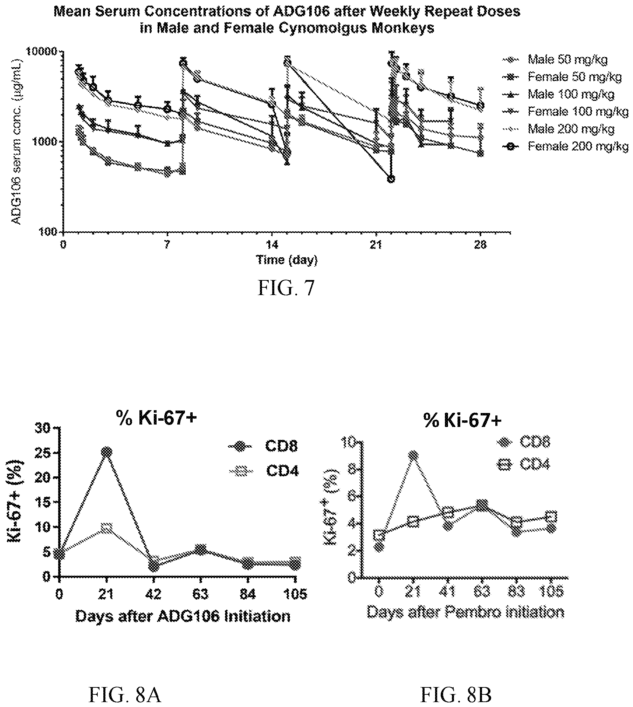

As shown in FIG.7, in cynomolgus monkeys, no gender-based differences were observed in the PK parameters in any of the dose levels tested. In all PK/TK studies, the total and peak systemic exposure to ADG106 increased dose-proportionally in males and females on all days when PK/TK where assessed. There were no observable adverse findings in GLP toxicity studies at doses up 200 mg/kg in monkey with a weekly repeat-dosing for one month.

Anti-drug-antibody (ADA) levels against ADG106 in monkey serum samples were measured using a validated electrochemiluminescence (ECL) assay. All samples were first analyzed for ADAs in a screening assay. Study samples with results below the screening cut-off were reported as negative for ADAs. In the event of a positive result in the screening assay, samples were analyzed in the confirmatory assay. All samples confirmed positive were reported as positive.

ADAs were present and correlated with effects of increased ADG106 clearance and reduced exposure in individual animals. These ADA positive titers and higher ADG106 clearances were mainly observed at 50 mg/kg (4/10 animals) and in 1/10 animals at 100 mg/kg, but not in animals in the 200 mg/kg treated group.

### Example 4. Biomarker Studies

### Immunological Effects of ADG106 Treatment

To study the immunological effects of ADG106 treatment, peripheral blood was collected prior to treatment initiation and at each ADG106 treatment cycle. T cell proliferation was examined by analyzing Ki-67 expression using flow cytometry analysis. Soluble CD137 (sCD137) levels in plasma were also examined using a validated MSD-based electrochemiluminescence assay.

As shown in FIG. 8A, in one patient (patient code R011), a four to five fold increase in the frequency of proliferating CD8+ T cells was observed following treatment with ADG106. The frequency of CD8+ T cells peaked at the first blood draw 21 days after ADG106 infusion with close to 25% of total CD8+ T cells expressing Ki-67. The frequency of Ki-67+ CD4+ T cells increased about one fold after ADG106 treatment. At about 40 days post treatment, the frequency of Ki-67+ CD8+ T and Ki-67+ CD4+ T cells decreased to baseline levels. Such effects are similar to what was observed in Pembrolizumab-treated patients (FIG. 8B). Peripheral CD4+ and CD8+ T cells demonstrated increased Ki67 expression in certain individuals treated with higher dose levels of ADG106 (FIGS. 9A-9B). FIGs. 10A-10B show CD137+CD4+ and CD137+CD8+ T cell populations in certain individuals treated with various levels of ADG106.

### Pharmacodynamics (PD)/Prognostic Biomarkers of ADG106

Blood samples were collected prior to ADG106 treatment and at various time points after treatment for exploratory biomarker studies. These biomarkers include serum levels of pro-inflammatory cytokines (TNFα, IFNγ, IL-2, IL-6, IL-10, etc.) and soluble CD137. Peripheral blood immune cell profiles, such as absolute cell counts for circulating T cells, natural killer (NK) cells, B cells, effector T cell subpopulations, and memory T cell subpopulations, were analyzed by flow cytometry using respective antibodies for each biomarker. The dynamic changes of peripheral T cell clones were analyzed through TCR sequencing. Expression of CD137 and its ligand, PD-L1, and/or tumor infiltrating lymphocytes, *etc.*, were also analyzed by immunohistochemistry (IHC) on archival tumor tissue or fresh biopsy tissue using validated methods.

As shown in FIG. 11, ADG106 treatment significantly increased the plasma levels of soluble CD137 (sCD137) in all patients tested, which suggests that ADG106 treatment induced CD137 expression in the patients. Additionally, as shown in FIGS. 12A-12B, sCD137 plasma levels increased more in patients with stable diseases than patients with progressive diseases, while membrane-bound CD137 (mCD137) plasma levels increased more in patients with progressive diseases than patients with stable disease after one cycle of ADG106 treatment. The increase of CD137 expression upon ADG106 treatment indicates that ADG106 engages the activation of the CD137 signaling pathway in CD8+ T cells. These results suggest that sCD137 and mCD137 could be used as prognostic biomarker for ADG106 treatment.

As shown in FIG. 13, Ki67+ CD8+ T-cells tended to increase more after one cycle of ADG106 treatment in patients who had stable diseases, in comparison to patients who had progressive diseases. Additionally, as shown in FIG. 15A, basal levels of CD8+ effector memory T ($T_{em}$) cells correlated with clinical outcome of ADG106 treatment, in which patients who achieved stable diseases after treatment had significantly lower pre-treatment levels of CD8+ $T_{em}$ cells than patients who had progressive diseases. After once cycle of ADG106 treatment, the levels of CD8+ $T_{em}$ cells increased in patients who achieved stable diseases compared to pre-treatment levels, while the levels of CD8+ $T_{em}$ cells decreased in patients who had progressive diseases compared to pre-treatment levels (FIGS. 15B-15C).

FIGS. 15A-15D show biomarker levels in a patient with angioimmunoblastic T cell lymphoma (Patient R017) who achieved 33% tumor shrinkage after four cycles of ADG106 treatment. The patient had increased proliferation (Ki67) and decreased mCD137 levels of CD8+ T-cells, and decreased Treg cells, the levels of CD8+ $T_{em}$ cells increased initially upon ADG 106 treatment.

## Example 5. ADG106 efficacy in L5178-R and L5178-S murine T cell lymphoma syngeneic models

To evaluate the *in vivo* anti-tumor efficacy of ADG106 in the treatment of T cell lymphoma, female DBA/2 mice were inoculated subcutaneously at the right flank with L5178-R or L5178-S murine T cell lymphoma tumor cells ($1 \times 10^5$) in 0.1 ml of PBS for tumor development. The animals were randomized and treatment started when tumor volumes reach 50-80 mm³, or on the day of cell inoculation (FIGs. 17A and 17C). Each group consisted of 8 tumor-bearing mice. Isotype control or ADG106 at 20mg/kg dose was administered by intraperitoneal injection once every 3 days. Tumor growth and animal body weight were monitored every 2-3 days.

As shown in FIGs. 17A and 17C, ADG106 has antitumor activity in the L5178-S T lymphoma model, whereas the L5178-R T lymphoma model is resistant to ADG106 treatment. Expression of CD137 ligand was examined in the two murine T lymphoma L5178-R and L5178-S cells. These lymphoma cells were stained with PE-labeled anti-CD137 ligand or isotype control antibody by incubation for 30-60 min on ice in dark. After washing off unbound antibodies, the samples were analyzed by flow cytometry. FIGs.17B and 17D show the staining patterns of isotype control (black) and anti-CD137 ligand antibodies (grey) in L5178-R and L5178-S cells, respectively. The results indicate that L-5178-R T lymphoma cells are positive for CD137 ligand expression, whereas L5178-S T lymphoma cells are negative for CD137 ligand expression.

In the two murine T cell lymphoma models, L5178-R is resistant while L5178-S is sensitive to ADG106 treatment. Notably, these two T cell lymphoma models differ in terms of the CD137 ligand expression status: L5178-R is positive whereas L5178-S is negative for CD137 ligand expression. As CD137 ligand is the natural agonist for CD137 and ADG106 works by the same mechanism to activate CD137 receptor, the resistance of L5178-R T lymphoma to ADG106 treatment is consistent with the hypothesis that tumors with overexpression of the CD137 ligand could have developed resistance to agonists that have similar mechanism of action in the same pathway, such as anti-CD137 antibodies like ADG106, during the course of tumorigenesis. Such data support that CD137 ligand expression status in tumors may be useful for stratifying patients in treatment with CD137 agonists.

## Example 6. ADG106 exposure in relation to total dose and determination of Recommended Phase 2 Dose (RP2D)

The US and China trials have completed dose escalation up to 10 mg/kg. As shown in FIG. 18, analysis of the PK data showed a linear relationship between ADG106 exposure and total doses (body weight times the dose in mg per kg). Analysis of drug exposure suggested the drug safety and efficacy data of ADG106 were closely related to the total dose rather than the dose per unit weight (mg per kg of body weight). ADG106 was well tolerated when the total dose was below 500 mg, thus a flat dose at or below 400 mg would have significant safety margin for the patient. As shown in FIGs. 9A-9B and 10A-10B, PD biomarkers Ki67 and mCD137 suggest an optimal dose of ADG106 at about 3-5 mg/kg. The clinical responses and exploratory PD analysis of the peripheral lymphocyte activation have shown that ADG106 is pharmacologically active at doses ≥1 mg/kg, optimally around 1.5, 3, 5 mg/kg range or around 300 mg in total dose within 50% of the range.

In summary, dose to 1mg/kg to 5 mg/kg seems well tolerated and efficacious as far as the total dose will not go beyond 500mg, preferably not beyond 400mg, or alternatively flat dose from 150 to 500mg is well tolerated and effective, these dose (approximately 10-50% above or below the planned dose) would be used as RP2D, (Recommended Phase 2 Dose) based on the observed clinical safety, pharmacology, and PK/PD data for single and combination use.

**Example 7. ADG106 in combination with immune checkpoint inhibitors in murine cancer models**

The following example describes *in vivo* therapeutic efficacy of the anti-CD137 antibody ADG106 in combination with various immune checkpoint inhibitors in murine cancer models.

**ADG106 in combination with atezolizumab**

C57BL/6 mice (n=10 per group, female, 6-8 weeks old) were inoculated subcutaneously with 3LL (JCRB) murine lung cancer cells, a Lewis lung cancer cell line. When tumors were established (75 mm$^3$), treatment began with isotype control antibody, ADG106 (10mg/kg), TECENTRIQ® (atezolizumab, 10 mg/kg), or the combination of ADG106 (10mg/kg) and TECENTRIQ® (atezolizumab, 10 mg/kg) by intraperitoneal injection twice a week for 3 weeks. Tumor growth was monitored twice weekly and reported as mean tumor volume ± SEM over time.

As shown in FIGs. 19A-19B, compared to the isotype control antibody, both ADG106 and TECENTRIQ® (atezolizumab) monotherapies showed anti-tumor activity, and the combination of ADG106 with TECENTRIQ® (atezolizumab) exhibited synergistic anti-tumor efficacy in the 3LL lung cancer model. These results indicate that ADG106 and TECENTRIQ® (atezolizumab) each as single agents could inhibit tumor growth at certain degrees. Combination of ADG106 and TECENTRIQ® (atezolizumab) further enhanced antitumor efficacy, leading to complete tumor regression in 7/10 mice, which suggests a synergistic effect between these two agents.

**ADG106 in combination with anti-PD-1 antibody**

C57BL/6 mice (n=8 per group, female, 6-8 weeks old) were transplanted subcutaneously with $5 \times 10^5$ Lewis lung cancer cells. After tumors were established (*i.e.,* having reached a volume of 75 mm$^3$), mice were treated with vehicle alone, anti-CD137 antibody ADG106 (5 mg/kg), anti-PD-1 antibody 2E5 (5 mg/kg), the combination of ADG106 (5 mg/kg) and anti-PD-1 antibody 2E5 (5 mg/kg) synchronously, anti-CD137 antibody ADG106 (5 mg/kg), then treated with anti-PD-1 antibody 2E5 (5 mg/kg) 7 days later, or treated with anti-PD-1 antibody 2E5 (5 mg/kg), then anti-CD137 antibody ADG106 (5 mg/kg) 7 days later, by intraperitoneal injection twice a week for 2 weeks. Tumor growth was monitored twice weekly and reported as mean tumor volume ± SEM over time.

As shown in FIGs. 20A-20B, compared to the isotype control antibody, both ADG106 and anti-PD-1 antibody monotherapies showed anti-tumor activity, and the combination of ADG106 with anti-PD-1 antibody synchronously exhibited synergistic anti-tumor efficacy in the Lewis lung cancer model. These results indicate that ADG106 and anti-PD-1 antibody 2E5 each as single agents could significantly inhibit tumor growth. Combination of ADG106 and anti-PD-1 antibody 2E5 further enhanced antitumor efficacy, leading to complete tumor regression, which suggests a synergistic effect between these two agents. On the other hand, compared to ADG106 or anti-PD1 antibody monotherapies, combination treatment with ADG106 and anti-PD-1 antibody administered 7 days apart didn't exhibit significant synergistic anti-tumor efficacy in the Lewis lung cancer model.

**ADG106 in combination with ADG116**

BALB/c mice (n=8 per group, female, 6-8 weeks old) were transplanted subcutaneously with $3 \times 10^5$ 4T1 breast cancer cells, a triple-negative mouse breast cancer cell line. After tumors were established (*i.e.,* having reached a volume of 96 mm$^3$), mice were treated with isotype control antibody, anti-CD137 antibody ADG106 (5 mg/kg), anti-CTLA-4 antibody ADG116 (2 mg/kg), or the combination of ADG106 (5 mg/kg) and ADG116 (2 mg/kg) by intraperitoneal injection twice a week for 2 weeks. Tumor growth was monitored twice weekly and is reported as mean tumor volume ± SEM over time.

As shown in FIGs. 21A-21B, compared to the isotype control antibody, both ADG106 and ADG116 monotherapies showed anti-tumor activity, and the combination of ADG106 with ADG116 exhibited synergistic anti-tumor efficacy in the 4T1 breast cancer model. These results indicate that ADG106 and ADG116 each as single agents could inhibit 4T1 tumor growth. Combination of ADG106 and ADG116 further enhanced antitumor efficacy, suggesting a synergistic effect between these two agents.

In summary, the results demonstrate that ADG106 can synergize with various immune checkpoint inhibitors to enhance antitumor efficacy in different mouse syngeneic cancer models.

**Example 8. ADG106 in combination with chemotherapeutic agents in murine cancer models**

The following example describes *in vivo* therapeutic efficacy of the anti-CD137 antibody ADG106 in combination with chemotherapeutic agent docetaxel in 4T1 breast cancer model mice, and ADG106 in combination with chemotherapeutic agent cisplatin in Lewis lung cancer model mice.

**ADG106 in combination with docetaxel**

BALB/c mice (n=8 per group, female, 6-8 weeks old) were transplanted subcutaneously with $3\times10^5$ 4T1 breast cancer cells. After tumors were established (*i.e.,* having reached a volume of 96 mm$^3$), mice were treated with vehicle alone, anti-CD137 antibody ADG106 (5 mg/kg, twice a week for 4 doses by intraperitoneal injection), Docetaxel (5 mg/kg, once a week for 3 doses by intravenous injection), or the combination of anti-CD137 antibody ADG106 (5 mg/kg, twice a week for 4 doses by intraperitoneal injection) and Docetaxel (5 mg/kg, once a week for 3 doses by intravenous injection). Tumor growth was monitored twice weekly and is reported as mean tumor volume $\pm$ SEM over time.
As shown in FIGs. 22A-22B, ADG106 and Docetaxel mono-therapies were well tolerated by mice and showed marginal anti-tumor activity. The combination of ADG106 with Docetaxel exhibited enhanced anti-tumor efficacy in the 4T1 breast cancer model. No obvious toxicity was observed during the study.

**ADG106 in combination with cisplatin**

C57BL/6 mice (n=8 per group, female, 6-8 weeks old) were transplanted subcutaneously with $5\times10^5$ Lewis lung cancer cells. After tumors were established (*i.e.,* having reached a volume of 75 mm$^3$), mice were treated with vehicle only, ADG106 (5 mg/kg, twice a week for 4 doses), Cisplatin (5 mg/kg, once a week for 3 doses), or the combination of ADG106 (5 mg/kg, twice a week for 4 doses) and Cisplatin (5 mg/kg, once a week for 3 doses) by intraperitoneal injection. Tumor growth was monitored twice weekly and reported as mean tumor volume $\pm$ SEM over time.
As shown in FIGs. 23A-23B, compared to the isotype control antibody, both ADG106 and cisplatin mono-therapies showed anti-tumor activity, and the combination of ADG106 with cisplatin exhibited synergistic anti-tumor efficacy in the Lewis lung cancer model. No obvious toxicity was observed during the study.

**ADG106 in combination with cisplatin and Paclitaxel**

C57BL/6 mice (n=7 per group, female, 6-8 weeks old) were transplanted subcutaneously with $5\times10^5$ Lewis lung cancer cells. After tumors were established (i.e., having reached a volume of 70 mm$^3$), mice were treated with vehicle only, ADG106 (5 mg/kg, twice a week for 4 doses), the combination of Cisplatin (5 mg/kg, once a week for 3 doses) and paclitaxel (10mg/kg, once a week for 3 doses), or the combination of ADG106 (2.5 mg/kg, twice a week for 4 doses), Cisplatin (5 mg/kg, once a week for 3 doses) and paclitaxel (10mg/kg, once a week for 3 doses), by intraperitoneal injection. Tumor growth was monitored twice weekly and reported as mean tumor volume $\pm$ SEM over time.
As shown in FIGs. 23C-23D, compared to the isotype control antibody and ADG106 monotherapy, the combination therapy with ADG106, Cisplatin and Paclitaxel did not exhibit synergistic anti-tumor efficacy in the Lewis lung cancer model. Combination of Cisplatin and Paclitaxel showed toxicity during the study.

**ADG106 in combination with cisplatin and Pemetrexed**

C57BL/6 mice (n=8 per group, female, 6-8 weeks old) were transplanted subcutaneously with $5\times10^5$ Lewis lung cancer cells. After tumors were established (i.e., having reached a volume of 120 mm$^3$), mice were treated with vehicle only, ADG106 (2 mg/kg, twice a week for 4 doses), the combination of Cisplatin (4 mg/kg, once a week for 3 doses) and Pemetrexed (200 mg/kg, once a week for 3 doses), or the combination of ADG106 (2 mg/kg, twice a week for 4 doses), Cisplatin (4 mg/kg, once a week for 3 doses) and Pemetrexed (200 mg/kg, once a week for 3 doses), by intraperitoneal injection. Tumor growth was monitored twice weekly and reported as mean tumor volume $\pm$ SEM over time.
As shown in FIGs. 23E-23F, compared to the isotype control antibody, and ADG106 monotherapy, the combination therapy of ADG106, Cisplatin and Pemetrexed did not exhibit the synergistic anti-tumor efficacy in the Lewis lung cancer model.
In summary, the results demonstrate that ADG106 can synergize with various chemotherapeutic agents to enhance antitumor efficacy in the Lewis lung mouse syngeneic cancer models.

**Example 9. ADG106 in combination with anti-CD20 antibody in a murine lung cancer model**

C57BL/6 mice (n=6 per group, female, 6-8 weeks old) were transplanted subcutaneously with $5\times10^5$ LLC-CD20

cells, a Lewis lung cancer cell line expressing CD20. After tumors were established (i.e., having reached a volume of 75 $mm^3$), mice were dosed intraperitoneally twice a week for 3 weeks, with isotype control antibody (10 mg/kg), ADG106 (10 mg/kg), anti-CD20 antibody Rituximab (10 mg/kg), or the combination of ADG106 (10 mg/kg) and Rituximab (10 mg/kg). Tumor growth was monitored twice weekly and reported as mean tumor volume $\pm$ SEM over time.

The results shown in FIGs. 24A-24B indicate that ADG106 or Rituximab each as single agents only weakly inhibited tumor growth in the LLC-CD20 tumor model, but combination of these two agents led to stronger antitumor efficacy, which suggests that ADG106 could enhance the antitumor activity of Rituximab.

**Example 10. ADG106 in combination with local radiation in a murine colon cancer model**

C57BL/6 mice (n=8 per group, female, 6-8 weeks old) were transplanted subcutaneously with $5\times10^5$ MC38 colon cancer cells. After tumors were established (i.e., having reached a volume of 75 $mm^3$), mice were treated twice a week for 3 weeks with isotype control antibody (10 mg/kg), high dose (20Gy) local radiation, ADG106 (10 mg/kg), or the combination of ADG106 (10 mg/kg) and high dose (20Gy) local radiation. Tumor growth was monitored twice weekly and reported as mean tumor volume $\pm$ SEM over time.

The results in FIGs. 25A-25B indicate that a single dose radiation on tumor significantly inhibited tumor growth, whereas ADG106 alone had marginal if any antitumor activity. Moreover, combination of these two treatments led to even stronger antitumor efficacy, suggesting that ADG106 could enhance the antitumor effect of radiation therapy in this colon cancer model.

Example 11. **Identification of NK cell proliferation** as a **pharmacodynamic biomarker in first-in-human phase 1 trial with ADG106**

Pre- and post-treatment peripheral blood samples from 31 patients in a phase 1 trial were collected for biomarker analyses. The TBNK flow cytometry assay was employed to measure the composition of T (CD3+, CD4+/CD8+ subsets), B (CD19+) and NK (CD16+/CD56+) cells in peripheral blood samples. NK cell levels were determined as percentages of CD45-1- lymphocytes and absolute cell counts.

The results shown in FIG. 26A demonstrate proliferation of NK cells in patients treated with ADG106 after the first cycle of treatment, the post-treatment NK cell levels (Post) were measured at Day 8/C1D8, Day 15/C1D15, and/or Day 21/C1D21 after the first treatment (Table 11). Among the 32 patients analyzed, 88% (28/32) patients showed an increase of the NK cell level in their peripheral blood, 6% (2/32) showed no change, and 6% (2/32) showed a decrease of the NK cell level upon the ADG106 treatment.

The results shown in FIG. 26B demonstrate dose-dependent stimulations of NK cell proliferation by ADG106 treatment at doses of 0.1mg/kg, 0.5mg/kg, and 1.5 mg/kg. No significant difference in NK cell proliferation effect was observed at doses higher than 1.5 mg/kg, suggesting that ADG106 might have saturated CD137 receptors on NK cells at doses $\geq$1.5 mg/kg.

The ADG106 phase 1 clinical trial enrolled patients regardless of their tumor types, including patients with NSCLC, NPC, lymphoma, CRC, HCC, melanoma, cervical cancer, etc., and the percentage of NK cells before and after the first dose of ADG106 was analyzed. As shown in Table 11, the effect of ADG106 on NK cell proliferation was not specific to the tumor type, but rather a universal phenomenon observed within the majority of the patients. Stimulation of NK cell proliferation lasted from Day 8 up until Day 15, and in some cases, until Day 21 after the first infusion (Table 11).

Table 11. Percentage of NK cells (CD3-CD36+/CD16+) before and after ADG106 treatment.

| Subject ID | 105-003 | 105-004 | 105-005 | 105-006 | 105-007 | 105-008 | S0103 | S0104 |
|---|---|---|---|---|---|---|---|---|
| Predose | 17.4 | 50 | 24.7 | 18.2 | 7.5 | 19.5 | 16.3 | 15.68 |
| Postdose | 23.1 | 55 | 35.4 | 19.5 | 14.7 | 25 | 18.81[#] | 16.65[#] |
| **Subject ID** | **S0105** | **S0106** | **S0107** | **S0109** | **S0110** | **S0112** | **S0113** | **S0127** |
| Predose | 32.95 | 3.68 | 12.67 | 24.11 | 16.93 | 21.7 | 36.14 | 25.04 |
| Postdose | 39.58[#] | 4.27[#] | 20.01[#] | 34.74[#] | 40.45[#] | 26.931[#] | 27,23[#] | 25.14 |
| **Subject ID** | **S128** | **S0130** | **S0131** | **S0132** | **S133** | **S0134** | **S0135** | **S0136** |
| Predose | 20.64 | 10.02 | 12.23 | 15.22 | 28.89 | 4.4 | 17.62 | 27.28 |
| Postdose | 28.38 | 11.18 | 22.03 | 35.85 | 36.13 | 8.25 | 22.79 | 46.22 |

(continued)

| Subject ID | S0137 | S0138 | S0139 | S0140 | S0142 | S0143 | S0144 | S0147 |
|---|---|---|---|---|---|---|---|---|
| Predose | 17.31 | 15.68 | 18.62 | 14.69 | 4.83 | 24.56 | 21 | 8.53 |
| Postdose | 21.21* | 15.01 | 14.06 | 34.54 | 11.84 | 32.43 | 22.19* | 10.14 |

Data not available at all time points, unmarked post-dose data were taken on C1D8; *: Post dose data taken on C1D15; #: Post dose data taken on C1D21.

NK cell proliferation is an indication for CD137 target engagement in cancer patients. The results shown in FIG. 27A indicate that the average baseline percentage of NK cells was lower in patients who had a stable disease (S) than those whose cancer progressed (P). The results shown in FIG. 27B indicate that patients with a stable disease (S) had a higher average percentage increase in NK cells compared to patients whose disease progressed (P) after treatment with ADG106.

In summary, the results demonstrate that NK cell proliferation could serve as a pharmacodynamic biomarker for anti-CD137 antibody treatment in cancer patients.

**Example 12. Predict patient response to anti-CD137 antibody treatment by measuring CD137 and CD137L expression levels**

To improve patient clinical responses, biomarker studies were conducted to explore predictive and pharmacodynamics (PD) biomarkers.

Soluble CD137 (sCD137)

sCD137 levels in blood samples from patients treated with ADG106 in the Phase I clinical trial were detected using Human 4-1BB/TNFRSF9 DuoSet ELISA (R&D SYSTEMS®, Cat#: DY838) and the expression levels were measured using a highly sensitive electrochemiluminescence Meso Scale Discovery (MSD) assay. The correlation of soluble CD137 (sCD137) expression levels with patients' clinical response to the drug treatment was analyzed.

The results in FIG. 28A show a significant correlation between nasopharyngeal cancer (NPC) patients' clinical outcome with changes in their soluble CD137 (sCD137) expression levels, which is expressed as ratios of induced changes in soluble CD137 levels (C2-C1) (*i.e.,* sCD137 level after treatment at time point C2 subtracted by sCD137 level before treatment at time point C1) to baseline sCD137 expression levels (C1) (*i.e.,* sCD137 level before treatment at time point C1). For patients with progressive disease (P), this ratio was about 3; for patients with stable disease (S), this ratio was about 7; for patients with partial response (PR), this ratio was about 16 (FIG. 28A). Similar trends were also observed in other tumor types. The results shown in FIG. 28B demonstrate dose-dependent induction of soluble CD137 expression by ADG106 treatment at doses of 3 mg/kg, 5 mg/kg, and 10 mg/kg. The results in FIGs. 28A-28B demonstrate that the ratios of induced changes in soluble sCD137 expression levels (C2-C1) to baseline sCD137 expression levels (C1) could serve as a good indicator for the selection of recommended phase 2 dose (RP2D).

CD137L and other biomarkers

A multiplex immunohistochemistry ("mIHC") panel was used to analyze formalin fixed and paraffin embedded (FFPE) tumor biopsy samples from patients in the Phase I clinical trial. Three biomarkers, CD137, CD137L, and PD-L1, were tested with this mIHC panel, and this mIHC panel was validated by a contract research organization. The anti-CD137L antibody TY23561 (see, e.g., PCT/CN2020/094371) was used to detect CD137L in the mIHC panel. Briefly, the tissue slides were heated in an oven for 30 min at 65°C, then stained with primary and secondary antibodies against CD137, CD137L, and PD-L1 using the preset staining program on the IHC stainer (Leica BOND® RX). Then, the tissue slides were mounted by proper side mounting media and the whole stained sections were scanned and imaged by the PERKINELMER 3.0 Imaging system. The scanned images of the patient sections were then analyzed using the INFORM® software package. The CD137L protein expression level was quantified as percentage of CD137L expression or CD137L (%), which is the number of CD137L positive cells divided by the number of all the nucleated cells. CD137L expression levels were measured in formalin-fixed paraffin-embedded (FFPE) tumor biopsy specimens of 24 patients treated with ADG106. The correlation of CD137L expression levels with patients' clinical response to the ADG106 treatment was analyzed.

The results in FIGs. 29A-29C indicate that patients expressing high levels of CD137L had better response to the ADG106 treatment. As shown in Table 12, one nasopharyngeal carcinoma (NPC) patient with a 43.78% CD137L expression level experienced a 40% tumor reduction after two cycles of ADG106 treatment (FIGs. 30A- 30B). Two lymphoma patients with 23.08% and 20.54% CD137L expression levels had 36% and 32% tumor shrinkage respectively after two cycle of treatment (FIG. 31). A NSCLC patient with a 40% CD137L expression level only showed moderate response (*i.e.*, Stable Disease), which may be due to the lower ADG106 dose administered to the patient during dose escalation (Table 12). The results in Table 12 also demonstrate a correlation between the ratio of induced change of CD137 protein expression levels to baseline CD137 levels and patients' clinical response. Low CD137 baseline levels also correlated with a progressive disease (PD) response. However, the correlation between the CD137L expression levels with patients' clinical response is stronger in comparison.

Table 12. Summary of CD137 and CD137L expression in ADG106 treated cancer patients

| Dose (mg/kg) | Response | Tumor type | CD137 | CD137L | Tumor reduction (%) |
|---|---|---|---|---|---|
| 0.1 | PD | NSCLC | 0.18% | 3.26% | 13.0 |
| 0.5 | SD | NSCLC | 0.03% | 40.00% | 8.1 |
| 0.5 | PD | NPC | 0.26%, | 3.41% | 23.7 |
| 0.5 | PD | NPC | 3.62% | 13.40% | 12.7 |
| 1.5 | SD | NPC | 0.00% | 0.60% | 21.6 |
| 1.5 | PD | NSCLC | 0.00% | 1.69% | 28 |
| 1.5 | PD | NPC | 0.06% | 0.72% | 54 |
| 3 | SD | ACC | 0.04% | 0.03% | 10 |
| 3 | SD | NSCLC | 0.27% | 3.46% | 24 |
| 3 | SD | ACC | 0.37% | 0.17% | 16 |
| 3 | SD | AITL | 21.88% | 23.08% | -33 |
| 3 | SD | Melanoma | 0.06% | 0.01% | -7 |
| 5 | PD | NPC | 0.03% | 6.76% | 21 |
| 10 | SD | NSCLC | 0.18% | 0.18% | 17 |
| 5 | PD | NSCLC | 0.03%, | 0.05% | 31 |
| 5 | SD | Lymphoma | 3.63% | 0.06% | 51 |
| 5 | SD | NSCLC | 0.15% | 2.06% | 8 |
| 5 | SD | Mucinous adenocarcinoma | 0.00% | 0.00% | 15 |
| 5 | N/A | NPC | 1.34% | 3.68% | NA |
| 5 | PR | NPC | 1.48% | 43.78% | -40 |
| 5 | NA | ACC | 0.01% | 0.01% | NA |
| 5 | SD | Lymphoma | 3.82% | 20.54% | -32 |
| 5 | NA | Lymphoma | 1.38% | 0.76% | NA |
| 5 | NA | NPC | 0.00% | 2.64% | NA |
| CD137L Expression Levels in Different Tumor Types | | | | | |

746 Tumor microarray samples covering 18 different tumor types were purchased from commercial sources. CD137L expression was measured using the mIHC assay. It was found that CD137L expression was the highest in breast cancer (Table 13 and FIG. 32). Given the unmet medical needs in the triple negative breast cancer (TNBC) treatment, breast cancer particularly TNBC warrant further investigation as targeted indication for ADG106 clinical trials.

Table 13. Summary of CD137L expression in 746 TMA tumor specimen

| Tumor types | CD137L expression |
|---|---|
| Melanoma (n=56) | 1.79% |
| NSCLC (n=50) | 2.00% |

(continued)

| Tumor types | CD137L expression |
|---|---|
| SCLC (n=70) | 14.29% |
| HNSCC (n=44) | 6.82% |
| BLC (n=50) | 10.00% |
| CRC-ADC (n=67) | 0.00% |
| STC (n=53) | 11.32% |
| ESC-SCC (n=36) | 5.56% |
| CXC (n=50) | 0.00% |
| HCC (n=44) | 6.82% |
| ccRCC (n=24) | 0.00% |
| UCC (n=9) | 0.00% |
| KIC (n=62) | 3.23% |
| ADC (n=31) | 9.68% |
| BRC (n=48) | 41.67% |
| AITL (n=12) | 0.00% |
| PTCL, NOS (n=28) | 3.57% |
| Cd1NK/T (n=12) | 0.00% |

CD137L expression was $7.8 \pm 17\%$ in SCLC (p=0.00025). There was no significant difference of CD137L expression between different grades of SCLC. The PDL1 expression was $1.3 \pm 0.31\%$ in normal tissue, and $6.1 \pm 15\%$ in SCLC (p=0.004). SCLC patients with low PD-L1 expression may be a potential indication for ADG106 monotherapy, while SCLC patients with high PD-L1 expression may benefit from ADG106 and anti-PD1 combination therapy (Table 14).

Table 14. Expression of CD137, CD137L and PD-L1 in SCLC samples

| Sample Type | Category | CD137 (%) | | | CD137L (%) | | | PD-L1 (%) | | |
|---|---|---|---|---|---|---|---|---|---|---|
| | | ave | std | P-value | ave | std | P-value | ave | std | P-value |
| Normal | normal (5) | 0.00 | 0.00 | - | 0.18 | 0.35 | - | 1.26 | 0.31 | - |
| SCLC | Total (70) | 0.20 | 0.55 | 0.40 | 7.82 | 17.45 | 0.000 | 6.05 | 14.65 | 0.004 |
| | I-II (39) | 0.2636 | 0.6511 | - | 5.87 | 12.58 | - | 6.14 | 14.40 | - |
| | II-III (30) | 0.1193 | 0.3755 | - | 10.6 1 | 22.41 | 0.15 | 6.13 | 15.42 | - |
| | IV (1) | 0 | - | - | 0 | - | - | 0.23 | - | - |

CD137L expression was lower than what was observed in the ADG106-1002 trial. The average expression of CD137L was $1.56 \pm 2.23\%$ in AITL, $0.69 \pm 0.89\%$ in ALCL, $1.43 \pm 2.01\%$ in EATL, $0.91 \pm 1.32\%$ in NK/T, and $2.28 \pm 4.80\%$ in PTCL, NOS (p=0.07).

**Example 13. Biomarker-guided Phase 2 clinical study on ADG106 treatment of cancer**

Based on the findings from the Phase 1 clinical study, the mIHC assay and/or an IHC assay for CD137L is used to guide patient stratification and selection for a Phase 2 clinical study. The Phase 2 study is a biomarker-guided study that investigates treatment efficacy among patients with advanced solid tumors or hematological malignancies. There are two arms in this study: the first arm involves monotherapy of ADG106, and the second arm involves the combination therapy of ADG106 and an anti-PD-1 antibody, Toripalimab (JUNSHI BIOSCIENCES).

Based on the preliminary clinical data discussed in Example 12, a 15-20% CD137L expression level is used as the initial cut-off value for designating patients as biomarker positive (*i.e.,* higher expression level than the cut-off value) versus biomarker negative (*i.e.,* lower expression level than the cut-off value). The CD137L expression level cut-off value is further validated with more patient data from a biomarker validation group. For easy implementation, a chromogenic IHC assay is developed and cross-validated with the fluorescence IHC assay described in Example 12. Either the chromogenic IHC assay or the fluorescence IHC assay may be used as a companion diagnostic test for ADG106 monotherapy, and the combination therapy of ADG106 and Toripalimab.

Part 1 of the Phase 2 study involves patient stratification. A first group of biomarker positive patients (biomarker (+), n=40) and a second group of biomarker negative patients (biomarker (-), n=80) are recruited. Both groups are further divided into two arms: patients in the first arm receive ADG106 monotherapy, and patients in the second arm receive a combination therapy of ADG106 and Toripalimab. Toripalimab is administered at 3 mg/kg every two weeks (Q2W). An interim analysis is performed after Part 1 of the Phase 2 study. If the overall response rate (ORR) is higher than 30% and/or the ORR is higher in biomarker (+) patients, Part 2 of the study is initiated.

Part 2 of the Phase 2 study involves patient pre-selection. In Part 2, only patients with positive biomarkers (biomarker (+), n=40) are recruited. These biomarker-positive patients are further divided into two arms: patients in the first arm receive ADG106 monotherapy, and patients in the second arm receive a combination therapy of ADG106 and Toripalimab.

The primary objectives of the Phase 2 study are to study the overall response rate (ORR) and duration of the response. The secondary objectives of the Phase 2 study are to study the progression free survival (PFS) rate and overall survival rate.

**Example 14. Population pharmacokinetic modeling and simulation for ADG106 Phase II dose selections as monotherapy or in combination with other drugs**

Non-compartmental Analysis (NCA) of the pharmacokinetic (PK) data of the Phase I clinical studies in US and China showed an approximately linear relationship between ADG106 exposure and total dose (body weight times the dose in mg per kg, or BW×mg/kg), including clinical data from 0.1 mg/kg to 10 mg/kg once-every-three-week (Q3W) intravenous (IV) dosing.

**Population pharmacokinetic modeling**

A one-compartment population pharmacokinetic (pop-PK) model was developed and has been shown to characterize the observed PK data reasonably well. The US trial ADG106-1001 included a mixture of BW-based and fixed dosing while the China trial ADG106-1002 employed only BW-based dosing. Thus, population PK modeling were conducted for each trial's data separately (e.g. cycle 1 PK for ADG106-1001 and cycle 1 and cycle 2 PK for ADG106-1002 due to data availability for all subjects). The result suggested that the Chinese and US patient populations have overall similar population estimates and inter-subject variability for drug clearance (CL) and volume of distribution (Vd), and therefore available PK data from cycle 1 and 2 Q3W dosing were combined to generate one set of PK parameter estimates for ADG106.

Population modeling was performed using Nonlinear Mixed Effects Modeling, NONMEM® software Version 7.5.0 (Icon Development Solutions, Ellicott City, MD, USA) (Beal S, Sheiner LB, Boeckmann A, Bauer RJ, NONMEM user's guides (1989-2009), Icon Development Solutions, Ellicott City, MD (2009).) with an INTEL® FORTRAN compiler and Perl-speaks-NONMEM® (PsN) (Lindbom L, Pihlgren P, Jonsson EN, PsN-Toolkit-a collection of computer intensive statistical methods for non-linear mixed effect modeling using NONMEM, Comput Methods Programs Biomed (2005) 79:241-257). The ADVAN13 subroutine and the first-order conditional estimation with interaction (FOCEI) method was applied. The objective function value computed by NONMEM® was used in a log-likelihood ratio test for the comparison of hierarchical models. The addition of a structural or variance parameter was considered statistically significant when the objective function value dropped by ≥3.84 (P < 0.05 for 1 degree of freedom). Inter-individual variability for both CL and Vd was modeled by an exponential error model, which assumes a log-normal distribution. The difference between the observed and model-predicted concentrations was modeled as an additive and proportional error. Goodness-of-fit plots and Visual Predictive Check (VPC) (FIG. 33) were used to evaluate model fitting and parameter estimations. As shown in FIG. 33, the pop-PK modeling fitting was reasonable with no indication of misspecification of the structural or error model.

Covariate analysis for the two population PK parameters (CL and Vd) was done for body weight (BW). BW appeared to be weakly (e.g. about 0.3-0.4 as the estimated exponent value for BW-based allometric scaling) positively correlated with CL and moderately (e.g. about 0.5-0.7 as the estimated exponent value for BW-based allometric scaling) positively correlated with volume of distribution. PK simulations suggested that fixed IV dosing using the formula "BW×mg/kg" resulted in overall similar PK variabilities when compared with BW-based dosing at the population level (e.g. mg/kg) for a virtual patient population with a pre-specified mean and standard deviation (SD). As an example, a set of normally distributed random BW with a mean of 60 kg and SD of 10% was generated for a virtual patient population (20 subjects). There was no significant difference in the simulated PK (except for one subject that has notably higher $C_{max}$ in the 180mg fixed dose) among the 20 virtual patients dosed at 3mg/kg BW-based dosing vs. 180mg fixed IV dosing (FIG.

34). To summarize, based on the population PK modeling and simulation findings from both the China and US trials, fixed IV dose is recommended for further dose optimization for single and combination use of ADG106.

## Simulation for ADG106 Phase II dose selections as monotherapy

Virtual patient population (e.g. with 100 subjects for each BW bin such as 60kg, 70kg, 80kg, 90kg and 100kg) simulations using the estimated PK parameters and their associated variabilities/residual errors suggested that 200mg Q3W fixed IV dose of ADG106 (e.g. about 2.9 mg/kg for a 70 kg patient) should be considered as the recommended Phase 2 dose (RP2D) for monotherapy if the observed median $C_{max}$ and $AUC_{0-21d}$ associated with $\geq$ G3 TEAEs and DLTs in both trials were benchmarked against the simulated individual $C_{max, \, cycle \, 1}$ and $AUC_{0-21d, \, cycle1}$ across the evaluated dose levels (FIG. 35). The 60 kg virtual patient population was shown as a representative lower-BW scenario (e.g. close to the mean BW in ADG106-1002 China trial), compared with higher BW populations (e.g. the mean BW in ADG106-1001 US trial was about 80 kg). This was used due to relatively higher exposures (e.g. $C_{max}$ and AUC) in lower BW patients as BW was identified as weak-to-moderate covariate for both PK parameters. Note that in both the US and China trials, $\geq$ G3 treatment related AEs were not observed in subjects dosed less than 3mg/kg, corresponding to a fixed dose of 210 mg for a 70kg patient. Of note, in the US trial, a flat Q3W IV dose of 300 mg and 400 mg ADG106 was shown to be safe and well tolerated, further supporting the proposed 200 mg flat IV dose as the Phase II monotherapy dose.

In addition, based on exploratory pharmacodynamic (PD) and biomarker analysis before and post ADG106 treatment (e.g. peripheral NK cell dynamics, sCD137 profiles, see, Examples 11-12), it appears that ADG106 is pharmacologically active as monotherapy at doses $\geq$1 mg/kg (e.g. about 70 mg for a 70kg patient), saturating around 1.5 to 3 mg/kg or around 100-200 mg in total dose for a 70 kg patient. Notably, the exposures achieved at these dose levels appear to be in general agreement with projected systemic ADG106 concentrations associated with robust in vivo efficacy in tumor-bearing mouse models (FIG. 36). In addition, based on virtual patient PK simulations, the recommended 200mg RP2D IV dose can likely result in exposures that are close to those observed for the 3 responders in the China trial (FIG. 35, open circles) even for heavier patients (e.g. BW=100kg). In summary, a flat Q3W IV dose of 200 mg is expected to be well tolerated and optimal as ADG106 RP2D monotherapy dose based on the totality of preclinical and clinical data to date and PK/PD evaluations.

## Simulation for ADG106 Phase II dose selections in combination with other drugs

To better optimize the dosing regimen of ADG106 in a combination therapy setting (e.g. when other drugs are to be administered as Regulatory Authority approved doses or PD-saturating doses), a 50mg flat IV dose of ADG106 is recommended as its starting dose due to both safety and efficacy considerations. To elaborate, there could be unquantified/uncertain combination effect with other MOAs, particularly with Immunooncology {IO} agents which could be potentially additive and/or synergistic for AEs. Furthermore, 50mg flat IV dose of ADG106 is projected to be within about 2-4 fold of the maximum efficacious and PD-saturating dose level of ADG106 as monotherapy, and therefore should allow for likely patient benefits in a combination setting due to potential combination effects for efficacy as well. Based on PK simulations, the mean simulated $C_{max}$ at 50mg fixed IV dose is about 18 $\mu$g/mL for a 60 kg BW patient population (FIG. 35) and it is associated with theoretically calculated systemic CD137 receptor occupancy (RO) of >90% using SPR-based binding affinity value, and this concentration range (e.g. $\geq$10 $\mu$g/mL) was shown to trigger robust T cell activation in vitro (with anti-CD3 antibody co-treatment). However, considering likely lower tissue distribution of antibodies, using a theoretical 10% of systemic concentration to represent the drug concentration at the site of action (e.g. tumour tissue), the mean simulated $C_{max}$ at 50 mg fixed IV dose is associated with theoretical local tissue CD137 RO of >70% and the calculated local tissue $C_{max}$ (e.g. about 1.8 $\mu$g/mL) is within the concentration range (e.g. 1-3 $\mu$g/mL) which triggered observable, yet not maximal T cell activation in vitro. In addition, 1 $\mu$g/mL of ADG106 saturated the activation of CD137 receptor signaling in a crosslinking-dependent manner in vitro (FIG. 37). Using NFkB signaling activation to assess the functional activity of the ADG106 by in vitro cellular reporter gene assay (FIG. 38), 1.8 $\mu$g/mL was shown to be >EC80 in the nonlinearly regressed concentration-effect curve. These observations and calculations further suggested that the 50 mg fixed IV dose of ADG106 may likely result in partial efficacy by itself in vivo and therefore is ideal as the starting dose to further assess the PK/PD relationship in a combination treatment setting in patients. Notably, at 50 mg fixed IV dose of ADG106, all subjects in all evaluated BW bin (e.g. 60-100 kg) had significantly (e.g. >2-4 fold) lower simulated individual $C_{max}$, cycle 1 and $AUC_{0-21d}$, cycle 1 than the observed lowest $C_{max}$ and $AUC_{0-21d}$ associated with $\geq$ G3 treatment related AEs and DLTs in both US and China trials (FIG. 35). This lends further justification for the 50 mg fixed dose of ADG106 to be recommended as the starting dose to be evaluated in combination treatment. The proposed intermediate (e.g. 100mg) and maximum total dose (e.g. 200mg as the RP2D monotherapy dose) of ADG106 would be further defined based on the emerging clinical safety, tolerability and PK/PD (e.g. biomarker and efficacy) data for various combination use.

**Example. 15 Phase Ib/II Study of ADG106 Combined with Anti-PD-1 Antibody (Toripalimab) in Patients with Advanced or Metastatic Solid and/or Hematological Malignancies**

This is a Phase Ib/II, open label, multicenter, sequential dose escalation study to evaluate the safety, tolerability, PK, and preliminary efficacy of ADG106 combined with Toripalimab in patients with advanced or metastatic solid tumor and/or hematological malignancies based on CD137L biomarker status in phase Ib. For Phase II, only patients with advanced/metastatic small cell lung cancer (SCLC), triple-negative breast cancer (TNBC), non-small cell lung cancer (NSCLC), nasopharyngeal cancer (NPC), and T-cell lymphoma (TCL) will be enrolled.

Several PD-1/PD-L1 targeted antibodies were approved for treatment of various cancers. Preclinicla studies demonstrated combined anti-PD-1 and anti-CD137 antibodies have brought positive outcomes for some cancer medication (Wei, PLoS ONE 2013). Toripalimab is a humanized IgG4 monoclonal antibody against PD-1 and approved for the treatment of melanoma by the China National Medical Products Administration (NMPA). It is anticipated that ADG106 combined with Toripalimab would increase efficacy of Toripalimab in solid tumors and hematological malignancies.

Phase I studies of ADG106 (both in US and China) have identified a predictive biomarker CD137L in FFPE tumor specimens of the patients treated with ADG106. A significant correlation between patient outcome with CD137L expression (IHC) was observed. Patients expressing high CD137L had better response to ADG106 treatment. Based on these findings in Phase 1 studies, the Phase II part of this phase Ib/II study is designed to use IHC assay to pre-select patients to further evaluate the safety and efficacy of ADG106 combined with Toripalimab in advanced and/or metastatic SCLC, TNBC, NSCLC, NPC, and TCL. It is expected that ADG106 (targeting CD137) combined with Toripalimab will further improved anti-cancer effectiveness synergistically or additively.

**Preclinical Studies**

ADG106 was intensively investigated in nonclinical studies. The pharmacodynamic (PD) studies presented indicate that ADG106 is specific and binds with low nanomolar affinity to CD137 on activated T cells with subsequent enhancement and proliferation of target T cells with pro-inflammatory responses. ADG106 was efficacious as a single agent in multiple syngeneic mouse models of cancers and tumors biopsies from some of these studies showed evidence of infiltrating T cells. The median effective dose (ED50) was determined to be approximately 1 mg/kg in the syngeneic mouse liver and breast cancer models. Combining ADG106 with anti-PD-1/anti-PD-L1 mAb or anti-CTLA4 mAb in a mouse lung and colorectal cancer model resulted in enhanced in vivo anti-tumor efficacy. Combinations of ADG106 with antiangiogenesis agents (VEGF receptor tyrosine kinase inhibitor), anti-CD20 mAb, or Bendamustine, could also lead to enhanced in vivo antitumor efficacy in different mouse tumor models. The rat single intravenous (IV) pharmacokinetic (PK) study showed a slow clearance (CL) with the half-life (T1/2) values ranged from 277 to 351 hours. In the entire monkey PK/toxicokinetics (TK) studies, higher anti-drug antibody (ADA) titers corresponded to shorter T1/2, faster CL, and lower systemic exposure. Despite the variability from the presence of ADA in the monkeys, these studies clearly demonstrated that PK characteristics of ADG106 in rats and monkeys were consistent to other fully human therapeutic monoclonal IgG antibodies in these nonhuman species. Despite high ADA titers in some monkeys, enough monkeys were exposed to significant levels of ADG106 to permit determination of a NOAEL and HNSTD of > 200 mg/kg in cynomolgus monkey.

*Preclinical Pharmacology*

The pharmacodynamic studies performed with ADG106 have demonstrated that ADG106 is specific and binds with high affinity to both recombinant expressed CD137 and CD137 on activated CD4+ and CD8+ T cells of multiple species. ADG106 binds with low nanomolar affinity to the activated cynomolgus monkey and human T cells. In monkey and human T cells, ADG106 binds stronger to activated CD8+ T cells than CD4+ T cells, suggesting CD137 expression is induced more in CD8+ T cells than in CD4+ T cells upon activation and that CD8+ T cells may be the main target cells in mediating the ADG106 functional and pharmacological activities. It was also demonstrated that ADG106 caused enhancement and proliferation of target T cells, with pro-inflammatory responses (IFN-$\gamma$ release and NF$\kappa$B dependent transcriptional signaling). This was expected given that CD137 is known to function as a co-stimulatory T cell receptor. ADG106 binds to rodent CD137 receptors with over 100-fold lower affinity but was able to activate recombinant human, monkey, mouse, and rat CD137 receptors with comparable nanomolar potency as determined in a functional cellular NF$\kappa$B reporter gene assay. Studies comparing ADG106 with 2 clinical reference anti-CD137 mAbs (urelumab and utomilumab) showed that all 3 mAbs bind with high affinity to activated human T cells and not to the naive T cells, and all can function as agonists for the human CD137 receptor following crosslinking in the cellular NF$\kappa$B reporter gene assay. However, only urelumab had the unique property to function as a human CD137 agonist without crosslinking. Furthermore, ADG106 also fully blocks the interaction of CD137 to the CD137 ligand (4-1BBL). This property might prevent the potential of dual independent activation of CD137 potentially accounting for its distinct pharmacology and toxicology profile among this class of therapeutic antibodies.

ADG106 treatment in tumor bearing mice led to T cell tumor infiltration. It is proposed that these cytotoxic T cells account for ADG106 single agent anti-tumor efficacy in multiple syngeneic mouse tumor models including *CT*-26 (colon carcinoma), H22 (liver cancer) and EMT6 (breast cancer). Combining ADG106 with anti-PD-1/anti-PD-L1 mAb, or anti-CTLA4 mAb was shown to enhance anti-tumor efficacy in different MC38 (colorectal cancer) and Lewis lung cancer syngeneic mouse cancer models. These studies highlight the potential of ADG106 as monotherapy and in combination therapy with other immune-oncology therapies for synergistic anti-tumor efficacy. Combinations of ADG106 with antiangiogenesis agents (VEGF receptor tyrosine kinase inhibitor), anti-CD20 mAb, or Bendamustine, could also lead to enhanced in vivo antitumor efficacy in different mouse tumor models. Further, ADG106 treatment was shown to induce the development of long-lasting anti-tumor memory T cell responses when complete responders in the CT26 colon cancer model were protected from further tumor engraftment and growth after being rechallenged with the same tumor cells.

ADG106 mediated immune complement and antibody-dependent cell-mediated cytotoxicity responses were assessed. Consistent with its IgG4 immunoglobin class, ADG106 was unable to bind C1q therefore, unlikely to induce complement-dependent cytotoxicity. Further, ADG106 has low binding affinity for human $Fc\gamma R1$ protein and hence unlikely to elicit antibody-dependent cell cytotoxicity or antibody-dependent cell-mediated phagocytosis. In human whole cell assays, ADG106 did not show detectable NK cell antibody-dependent cell-mediated cytotoxicity on activated human CD8+ T cells. ADG106 did not stimulate cytokine (IL-2, IL-4, IL-6, IL-10, TNF-$\alpha$, IFN-$\gamma$, IL-17) release in human blood ex vivo, suggesting that ADG106 has lower risk of triggering acute cytokine release syndrome in humans. While no stand-alone safety pharmacology studies were performed, electrocardiography, blood pressure, respiration and neurological examinations in cynomolgus monkeys were investigated as part of the Good Laboratory Practice (GLP) toxicology studies showing no potential adverse effects following repeat doses up to 200 mg/kg.

Tissue immunohistochemistry cross-reactivity studies with ADG106 were performed in rat, cynomolgus monkey, and human tissues ex vivo. Although, staining was observed in smooth muscle cells or vascular tunica media in both monkey and human tissues, these tissues are not reported to express CD137 and the binding was considered cross-reactivity or non- specific. Furthermore, with the cytoplasmic nature of the staining, no significant biological relevancy is anticipated. An additional safety study of the potential for in vitro hemolysis in rabbit blood demonstrated that there was no ADG106-related hemolysis at the concentrations up to 2.54 mg/mL tested. In addition, the absence of any recorded dose site reactions in the rat and cynomolgus monkey IV infusion studies also indicates that there is no local intolerance to ADG106. Although the toxicology studies performed with ADG1 06 did not reveal any adverse effects of treatment, the absence of in vivo functional PD response and toxicity might be expected given the specificity of ADG106 for CD137 on activated cells and relatively low expression of CD137 in healthy animals under basal conditions.

*Preclinical Pharmacokinetics*

Pharmacokinetics/TK of ADG106 and ADA were investigated in Sprague-Dawley rat and cynomolgus monkey single IV (bolus) PK studies and non-GLP (dose range finding) and GLP-compliant (pivotal studies) once weekly repeat dose IV infusion toxicity studies, with doses of 10, 30 or 100 mg/kg/dose in the rats and 10 to 200 mg/kg/dose in the monkeys. The PK/TK studies consistently demonstrated that there was no gender-based differences in any PK parameters at any dose level. In both rats and monkeys, the systemic exposure (AUC and C0/Cmax) increased in an approximately dose-proportional manner in both sexes as the dosage increased. The volume of distribution (Vd) values after a single dose of ADG106 demonstrated that ADG106 has limited distribution beyond the blood compartment in both rats and monkeys.

The rat single IV PK study showed a slow CL with the half-life (T1/2) values that ranged from 277 to 351 hours and CL from 0.00326 to 0.00444 mL/mg/kg, which is consistent with the high molecular weight of ADG106 as an IgG molecule. This slow CL would also account for the drug accumulation noted in the rat toxicology studies from Day 1 to Day 22, as increases in total systemic exposure (AUC0-last) to ADG106 were observed in the majority of treated groups. This slow CL was also established because only minimal ADA were detected sporadically in the rat PK/TK studies and when present these ADAs had no effect on any PK parameters.

In contrast, no clear drug accumulation was noted following repeat dosing in monkeys. Single IV dose of ADG1 06 in monkeys had a quicker CL than the rat with sex-averaged values for CL being 0.00360 to 0.00447 mL/min/kg on Day 1 and increased to between 0.0106 to 0.0362 mL/min/kg on Day 45. In general, the Day 45 administration of ADG106 had a faster CL, shorter T1/2, and lower AUC0-inf than was observed after the Day 1 administration. Similar findings were also noted in some individual animals in the monkey toxicity studies. In these studies, the ADG106 levels declined more rapidly following repeated dosing between Day 1 and Day 22, resulting in AUC0-last values in some animals being significantly lower than others in the same dose group. This corresponded to high ADA titers in these animals. Indeed, in the entire monkey PK/TK studies, the higher ADA titers corresponded to shorter T1/2, faster CL, and lower systemic exposure.

In conclusion, despite the variability from the presence of ADA in the monkeys, these studies clearly demonstrated that PK characteristics of ADG1 06 in rats and monkeys were consistent to other human IgG molecules. In both rats and monkeys, no sex-related differences in PK parameters were evident and the total and peak systemic exposures to ADG106 increased dose-proportionally. Repeated IV dosing resulted in ADG106 accumulation in rats although accumulation was not evident in monkeys with no or low ADA titers. The presence of high ADA titers corresponded to shorter T1/2, faster CL, and lower systemic exposure in affected individual monkeys following repeated dosing. The distribution, metabolism, excretion, and PK drug interactions of ADG106 would be considered analogous to other IgG antibodies.

*Preclinical Toxicology*

There were no observable adverse findings in the non-GLP or GLP toxicity studies in Sprague-Dawley rats and cynomolgus monkeys.

In the GLP-compliant toxicity study in Sprague-Dawley rats, once weekly dosing of ADG106 up to 29 days by IV infusion (over 30 minutes) at 10, 30, or 100 mg/kg/dose was well-tolerated and did not result in any ADG106 related changes. The no observed effect level (NOEL) and NOAEL for ADG106 were considered to be 100 mg/kg. At the NOAEL, the peak plasma concentration (Cmax) and area under the curve (AUC0-168h ) of ADG106 on Day 22 were 3230 $\mu$g/mL and 275000 h$\cdot\mu$g/mL for males and 2930 $\mu$g/mL and 200000 h$\cdot\mu$g/mL for females, respectively. In the GLP-compliant toxicity study in cynomolgus monkeys, once weekly dosing of ADG106 up to 29 days by IV infusion (over 1 hour) at 50, 100, or 200 mg/kg/dose was well tolerated and did not result in any ADG106-related changes. Based on the absence of adverse findings during this study, the NOEL and NOAEL for ADG106 were considered to be 200 mg/kg. At the NOAEL of 200 mg/kg, the $C_{max}$ and AUC0-168h of ADG1 06 on Day 22 were 5750 $\mu$g/mL and 442000 h$\cdot\mu$g/mL in males and 5630 $\mu$g/mL and 462000 h$\cdot\mu$g/mL in females, respectively. The ADAs were present in monkeys and correlated with effects of increased ADG106 CL and reduced AUC0-168h in individual animals. These ADA positive titers and higher ADG106 CLs were mainly observed at 50 mg/kg (4/10 animals) and in 1/10 animals at 100 mg/kg, but not in animals at 200 mg/kg. Most importantly, there were no significant ADA titers or related effects on ADG106 systemic exposure in the 200 mg/kg treated group whose data supports the established NOAEL in this study.

**Objectives and endpoints**

*Objectives*

The primary objectives of this study are: to assess the safety and tolerability of ADG106 combined with Toripalimab, in the patients with advanced or metastatic solid and/or hematological malignancies in Phase 1b; to validate stratification/pre-selection biomarkers for ADG106 alone or in the combination ADG106 with Toripalimab in patients with advanced/metastatic SCLC, TNBC, NSCLC, NPC, and TCL will be enrolled in Phase II; and to evaluate the preliminary anti-tumor activity of ADG106 combined with Toripalimab in CD137L biomarker positive and negative patients with advanced or metastatic SCLC, TNBC, NSCLC, NPC, and TCL will be enrolled in Phase II.

The secondary Objectives of this study are: to assess the pharmacokinetic (PK) profile of ADG106 and Toripalimab; and to assess the immunogenicity of ADG106 and Toripalimab.

The exploratory objectives of this study is to assess pharmacodynamic (PD) biomarkers and predictive biomarkers for ADG106 alone or in combination with Toripalimab.

*Endpoints*

Primary Endpoints:

- Determine DLT, MTD or MAD, and RP2D;
- Objective response rate (ORR), as assessed by RECIST version 1.1 or Lugano Classification;
- Safety endpoints include AEs, clinical laboratory results, vital signs, physical examination findings, and ECG results; and
- Cutoff values of biomarkers for patient selection.

Secondary Endpoints:

- Duration of response (DOR), Disease Control Rate (DCR), Progression-Free Survival (PFS), and Overall Survival (OS);
- PK endpoints include peak plasma concentration (Cmax), serum concentration at the end of a dosing interval (Ctrough), time to reach Cmax (Tmax), area under the curve from time zero to the last timepoint (AUC0-last), AUC from time zero to infinity (AUC0-inf), clearance (CL), and volume of distribution at steady state (Vss), elimination half-life (T1/2), as data permit; and

- Anti-drug antibodies (ADA) levels.

Exploratory Endpoints:

- Pharmacodynamic (PD) biomarkers (Peripheral blood and tissues) for ADG106 alone or in combination with Toripalimab;
- Serum proteins (cytokines, sCD137, sPD-L1, etc);
- Peripheral blood immune cell phenotyping (T cells, Tregs, etc);
- Peripheral immune cell genotyping (TCRseq, etc);
- Tissue biomarkers (CD137, CD137L, PD-L1, CD20, TIL); and
- Tissue genotyping (TMB, MSI, TCRseq, etc).

**Study design**

*Overall Study Design*

This is a phase Ib/II study. The Phase Ib portion will employ traditional 3+3 study design to determine Recommended Phase 2 Dose (RP2D) of ADG106 in combination with Toripalimab. Dose Escalation Level 1: ADG106 50mg, Q3W combined with Toripalimab 240mg, Q3W. Dose Escalation Level 2: ADG106 100mg, Q3W combined with Toripalimab 240mg, Q3W. Dose Escalation Level 2: ADG106 200mg, Q3W combined with Toripalimab 240mg, Q3W.

Patients must have advanced or metastatic solid or hematological malignancies. No biomarkers test required at dose escalation (Phase Ib). A cycle is defined as 21 days. Dose-Limiting Toxicities (DLT) will be observed in Cycle 1 (21 days). After 3-6 patients treated with ADG106 50mg, Q3W completed DLT observation, Safety Review Committee (SRC) will review all safety data and decide whether to escalate to ADG106 100mg, Q3W. Similarly, after 3-6 patients with ADG106 with 100mg and 200mg, Q3W completed DLT observation, the SRC will review all safety data and determine the RP2D of ADG106 for the combination regimen.

The Phase II portion is a two stage, randomized, biomarker-stratified trial of ADG106 combined with Toripalimab versus ADG106 in patients with advanced or metastatic SCLC, TNBC, NSCLC, NPC, and TCL that are either CD137L positive or negative.

In Stage 1, there are 2 arms (ADG106 combined with Toripalimab vs. ADG106 alone) randomized at 1:1 ratio for each of CD137L positive and negative cohorts independently. Patients with SCLC, TNBC, NSCLC, NPC, and TCL will be stratified by CD137L blood biomarkers.

Arm A: Patients with CD137L-positive advanced or metastatic SCLC, TNBC, NSCLC, NPC, and TCL, which includes: Cohort 1: ADG106 RP2D (200mg), Q3W, iv; and Cohort 2: ADG106 RP2D (TBD), Q3W plus Toripalimab 240mg, Q3W, iv.

Arm B: Patients with CD137L-negative advanced or metastatic SCLC, TNBC, NSCLC, NPC, and TCL, which includes: Cohort 1: ADG106 RP2D (200mg), Q3W, iv; and Cohort 2: ADG106 RP2D (TBD), Q3W plus Toripalimab 240mg, Q3W, iv.

The two arms in Stage 1 may start to enroll simultaneously. An interim analysis will be conducted by the Study Review Committee (SRC) when all planned patients at stage 1 completed at least one post-treatment tumor scan. If the analysis at the end of Stage 1 meets Go criteria (e.g., one arm achieves ORR 25% or difference between two arms 20%), the trial will proceed to Stage 2.

In Stage 2, CD137L positive patients with advanced/metastatic SCLC, TNBC, NSCLC, NPC, and TCL will be randomized at 1:1:1 ratio to: Arm C: ADG106 RP2D (200mg), Q3W, iv; Arm D: ADG106 RP2D (TBD), Q3W iv plus Toripalimab 240mg, Q3W, iv; or Arm E: Toripalimab 240mg, Q3W, iv.

ADG106 alone, ADG106 plus Toripalimab, Toripalimab can be dosed up to 1 year. If tumors have responded and treatment is required to continue more than 1 year, the Principal Investigator should discuss with the Sponsor and make decision. The treatment can continue until disease progression, intolerable toxicity, or withdrawal of consent.

Interactive Voice Response System (IVRS) will be used. The SRC (Study Review Committee) will review all efficacy as well as safety data at the planned interim analysis and then recommend whether the Stage 2 will start or not.

For the combination regimen, ADG106 should be intravenously administered first, then 30 minutes later, Toripalimab will follow to be administered intravenously thereafter.

The investigator may decide to continue treatment with the combination regimens beyond tumor progression as defined by RECIST or Lugano Classification. Patients withdrawn from the treatment due to intolerable adverse events requiring treatment discontinuation will be followed up until the adverse events return to Grade 0 or 1, became stable, or until the patient receives new treatment, whichever occurs first. In the absence of symptoms and signs of disease progression, patients with stable Eastern Cooperative Oncology Group (ECOG) status will be allowed to continue treatment despite initial radiographic progression. In this case, a repeat scan will be required preferably in 4 weeks, but no later than 6 weeks in order to confirm disease progression.

**Dose Selection Rationale**

For phase Ib, ADG106 50mg, 100mg and up to 200mg, Q3W IV dosing were selected based on the totality of data (eg, safety, tolerability, biomarker and efficacy), integrated PK/AE analysis including population modeling and simulation of FIH phase I study results of ADG106 monotherapy in China and US. Toripalimab, is to be administered as Regulatory Authority (China NMPA) approved doses in ADG106 combination regimens.

**Study population**

*Inclusion Criteria*

Each patient must meet the following criteria to be enrolled in the study:

1. 18 years of age or older and younger than 75 years of age;
2. Eastern Cooperative Oncology Group (ECOG) performance status $\leq 1$;
3. Histologically or cytologically documented, incurable or metastatic malignacies that is advanced (non-resectable) or recurrent and progressing since the last anti-tumor therapy and for which no recognized standard therapy exists or for which standard of care has been declined (with reason to be clearly documented). For phase II, only patients with advanced/metastatic SCLC, TNBC, NSCLC, NPC, and TCL will be enrolled;
4. For Phase II, a patient must have tumor tissue (archival or fresh biopsy if archived tumor tissues were not available) for IHC staining of biomarkers;
5. At least one measurable disease per RECIST v1.1 or Lugano Classification;
6. Adequate organ function as defined by the following criteria (without transfusional/growth factor support within 2 weeks):

    a. Absolute neutrophil count (ANC) $\geq 1000$ cells/$\mu$L;
    b. Platelet count $\geq 100,000$/$\mu$L;
    c. Hemoglobin $\geq 9.0$ g/dL;
    d. Serum aspartate transaminase (AST) and serum alanine transaminase (ALT) $\leq 3.0$ x upper limit of normal (ULN) ($\leq 5.0$ x ULN in patients with documented liver involvement);
    e. Total serum bilirubin $\leq 1.5 \times$ ULN (except for patients with documented Ciilbert's syndrome) (or $< 2 \times$ ULN for subjects with liver metastases); and
    f. Serum creatinine $\leq 1.5$ x ULN or creatinine clearance of $\geq 50$ mL/min.

7. International normalized ratio (INR) and activated partial thromboplastin time (aPTT) $\leq 1.5 \times$ ULN; patients on full-dose oral anticoagulation must be on a stable dose (minimum duration 14 days); if receiving warfarin, the patient must have an INR $\leq 3.0$ and no active bleeding (i.e., no bleeding within 14 days prior to first dose of study drug); patients on low molecular weight heparin will be allowed;
8. Willing to complete all scheduled visits and assessments at the institution administering therapy; and
9. Able to read, understand and provide written informed consent.

*Exclusion Criteria*

Patients who meet any of the following criteria cannot be enrolled:

1. Life expectancy < 3 months, as determined by the Investigator;
2. Treatment with any local or systemic antineoplastic therapy (including chemotherapy, hormonal therapy, radiation or immunotherapy, etc) within 3 weeks prior to first dose of the combination regimen, with the following exceptions:

    a) Hormonal therapy with gonadotropin-releasing hormone (GnRH) agonists or antagonists for prostate cancer,
    b) Hormone-replacement therapy or oral contraceptives; or
    c) Palliative radiotherapy for bone metastases or other non-target lesions $\geq 2$ weeks prior to first dose of the combination regimen.

3. Major trauma or major surgery, or unlnealed wound within 4 weeks prior to first dose of the combination regimen;

4. Adverse events (AEs) from prior anti-cancer therapy that have not resolved to Grade ≤ 1 except for alopecia. or irAE of immunotherapy resulting in permanent discontinuation;

5. Central nervous system disease involvement (but allow patients with prior brain metastases treated at least 4 weeks prior to the first dose of ADG1 06, ADG106-Toripalimab, Toripalimab that are clinically stable and do not require chronic corticosteroid treatment to be enrolled in the study), or prior history of NCI CTCAE Grade ≥ 3 drug-related CNS toxicity;

6. Clinically significant cardiac disease, such as:

   a. New York Heart Association Class III IV cardiac disease, including preexisting clinically significant ventricular arrhythmia, congestive heart failure or cardiomyopathy;
   b. Unstable angina pectoris ≤ 6 months prior to start date of the combination regimen;
   c. Acute myocardial infarction ≤ 6 months prior to start date of the combination regimen;
   d. Other clinically significant heart disease (e.g. ≥ Grade 3 uncontrolled hypertension or history of poor compliance with an antihypertensive regimen);
   e. Clinically significant valvular disease, cardiomegaly, ventricular hypertrophy, or cardiomyopathy; or
   f. Significant ECG abnormalities including 2nd degree (type II) or 3rd degree atrioventricular (AV) block.

7. Evidence of active uncontrolled viral, bacterial, or systemic fungal infection defined as requiring use of systemic antimicrobials within 2 weeks of enrollment, prophylactic therapy according to institutional protocols is acceptable;

8. Known positive test result for human immunodeficiency virus (HIV) (except the disease is clinically controlled) or acquired immune deficiency syndrome (AIDS);

9. Active hepatitis C virus (HCV) or hepatitis B virus (HBV) requiring treatment; patients who are positive for hepatitis B core antibody or hepatitis B surface antigen must have a Hepatitis B virus DNA titer less than 2500 copies/mL or 500 IU/mL; patients who are positive for hepatitis C antibody must have a HCV RNA below the lower detection limit of the assay; those who are PCR positive will be excluded;

10. Second primary malignancy that has not been in remission for greater than 3 years; exceptions that do not require a 3-year remission include: non-melanoma skin cancer, cervical carcinoma in situ on biopsy or squamous intraepithelial lesion on Papanicolaou (PAP) smear, localized prostate cancer (Gleason score < 6), or resected melanoma in situ; other localized, solid tumors in situ or other low risk cancers may also be exempt after discussion with the Sponsor Medical Monitor;

11. Patients with underlying hemoglobinopathies (e.g., thalassemia) will be excluded;

12. History (within the last 5 years) or risk of autoimmune disease (e.g., autoimmune thyroid disease, Bell's palsy, glomerulonephritis, Guillain-Barré syndrome, inflammatory bowel disease, multiple sclerosis, rheumatoid arthritis, Sjögren's syndrome, systemic lupus erythematosus, vascular thrombosis associated with antiphospholipid syndrome, vasculitis, or Wegener's granulomatosis);

13. Patients who have immunosuppressive medications or immunosuppressive doses of systemic corticosteroids (> 10 mg/day prednisone or equivalent) within 28 days prior the 1st dose of the study treatment. However, patients who received a short course of corticosteroids (e.g., pre-medication prior to a contrast CT) will be eligible for study entry;

14. Any serious underlying medical (e.g., pulmonary, renal, hepatic, gastrointestinal, or neurological) or psychiatric condition (e.g., alcohol or drug abuse, dementia or altered mental status) or any issue that would limit compliance with study requirements, impair the ability of the patient to understand informed consent, or that in the opinion of the investigator would contraindicate the patient's participation in the study or confound the results of the study;

15. Known hypersensitivity, allergies, or intolerance to immunoglobulins or to any excipient contained in ADG106 (see Investigator's Brochure) and Toripalimab (see product insert);

16. Pregnant, lactating, or breastfeeding;

17. Ability to become pregnant; however, female patients who have a negative pregnancy test before enrollment and agree to use two highly effective forms of contraception (oral, injected or implanted hormonal contraception and condom; intrauterine device and condom; diaphragm with spermicidal gel and condom) during the trial and for 90 days after the end of ADG106 treatment) are considered eligible;

18. Male patients with female partners of childbearing potential (unless they agree to take measures not to father children by using one form of highly effective contraception [condom plus spermicide] during the trial and for 90 days after the end of ADG106 treatment); men with pregnant or lactating partners should be advised to use barrier method contraception (for example, condom plus spermicidal gel) to prevent exposure to the fetus or neonate; or

19. Participation or plans to participate in another interventional clinical study while taking part in this protocol; participation in an observational study is acceptable.

*Patient Screening Procedures*

Written ICF signed must be obtained prior to any study-related procedures or request for archival tissue. Screening procedures will take place at the qualified clinical research centers. Demographic data to be recorded in the eCRFs include patients' gender, race, and date of birth. Patients' relevant, significant medical and surgical history, including malignancy history along with prior cancer treatments will be recorded. Following tests will be performed: weight, height and vital signs measurements, physical examinations, recording ECGs, obtaining clinical laboratory tests, etc. For phase II, tumor tissue samples (including archive or fresh biopsy) are collected and tested for the protocol required biomarker. All female patients of childbearing potential must have a urine or blood pregnancy test. In addition, female patients and male patients will be asked to practice a reliable medically accepted form of birth control during participation in the study. Patient's current medication schedule will be reviewed to ensure patient's safety during the treatment.

Additionally, the following laboratory tests and imaging procedures will be performed: laboratory tests: hematology, comprehensive serum chemistry panel, coagulations parameters, pregnancy test; endocrine tests: TSH and free T4, morning (AM) cortisol; virology screening for HBV, HCV, and HIV; MRI and/or CT scan tumor assessment; and tumor biopsy (mandatory if archived tumor tissues are not available).

For patient convenience, if an assessment was performed before signing ICF as part of the patient's routine clinical evaluation, it doesn't have to be repeated as long as the testing fulfills the study requirements and is performed within the allowed screening time window prior to the first dose of study drug. Retesting of abnormal screening values to reassess eligibility are allowed only once during the screening period. The last results obtained prior to the first dose of study drug will be used to determine eligibility. Laboratory results, including a pregnancy test, must be obtained within 3 days prior to first dosing. Serum pregnancy test will be obtained at screening and patients must have a negative serum pregnancy test (within 72 hours before the first investigational product administration). For the remainder of visits, a urine pregnancy test prior to dosing is acceptable.

**Study Intervention**

Study *drug*

ADG106 is a fully human ligand-blocking, agonistic anti-CD137 IgG4 mAb. Toripalimab is a humanized IgG4 monoclonal antibody against PD-1 and approved for the treatment of melanoma by the China National Medical Products Administration (NMPA).

The dose of ADG106 will be 50mg, 100mg, and up to 200mg for phase Ib part (dose escalation part). For phase II part, ADG106 will be dosed at its RP2D as monotherapy or in combination with Toripalimab. Toripalimab will be dosed at a fix 240mg, Q3W, iv for both phase Ib and phase II parts. ADG106 drug product will be administered as an intravenous infusion, as directed in the Pharmacy Manual.

ADG106 will be diluted in saline or 5% dextrose to appropriate concentration for infusion as described in the Pharmacy Manual. Diluted ADG106 in saline or 5% dextrose will be stored for ≤ 4 hours at room temperature or ≤ 24 hours at 2°C to 8°C prior to administration. Diluted ADG106 prepared in 100 mL IV bags will be administered via IV infusion with an in-line 0.22micron filter using a volumetric pump over an approximate 90-minute period (± 10% window). The infusion rate will begin at 0.5 mL/minute (30 mL/hour) for the first 30 minutes, with the subject being closely monitored for AEs. If no noteworthy event is observed during the first 30 minutes, the infusion rate will be increased to 1 mL/minute (60 mL/hour) for 15 minutes, with the subject being similarly monitored for AEs. If there are no safety issues during this period, the infusion rate will be increased to 2 mL/minute (120 mL/hour), with the subject being carefully monitored until administration is complete.

Subjects will be closely monitored for vital signs at predose, every 15 minutes during infusion (Cycle 1 Day 1), at the end of infusion, and at 2 hours and 6 hours after the end of infusion of ADG106 (± 10% window). After Cycle 3, the 6-hour timepoint for vital signs will be eliminated in the absence of any clinically significant events during the prior cycles. The ECG will be assessed within 30 minutes predose and at approximately 30 minutes after the end of infusion. At 6 hours observation period after the end of infusion (± 10% window) and as clinically indicated. The PI will continue to be on call for any medical events until the time of the next assessment; and if the subject needs to be admitted to the local emergency department during this time, the PI will be contacted immediately. If there are no safety issues after the first dose, subjects may receive subsequent ADG106 doses at an infusion rate of 2 mL/minute (120 mL/hour).

All diluted ADG106 doses prepared in a syringe will be administered from the syringe via IV infusion with an in-line 0.2micron filter using a volumetric pump over a period of 60 minutes (± 10%). Preparation of Toripalimab should follow the manufacturers instructions.

*Treatment Allocation, Administration and Premedication*

All potential study candidates will provide signed informed consent and will undergo screening procedures before participating in the study. The screening period is up to 28 days. This is an open label study, and there is no blinding or randomization in Phase Ib. Tumor tissues samples of qualified patients (not required for patients enrolled in Phase Ib portion of this study) will be tested for the CD137L biomarker before the initial study treatment dosing, then based on biomarkers (positive versus negative), a patient will be randomized to receive either single ADG106 or ADG106 combined with Toripalimab, or Toripalimab (phase II, stage 2 only; patients with SCLC, TNBC, NSCLC, NPC and TCL will be enrolled for Phase II) treatment.

For Phase II, eligible patients with SCLC, TNBC, NSCLC, NPC and TCL will randomly be assigned to either single ADG106 or ADG106 combined with Toripalimab based on their biomarker test results. Interactive voice response system (IVRS) will be used in the Phase II portion of this study. Each subject enrolled in this study will receive a unique subject number after signing the ICF. Subjects will be identified throughout the study by this unique subject number. Once a subject number has been assigned to a subject, it cannot be reassigned to other subjects. 2 arms in Stage 1 may start to enroll simultaneously.

For the ADG106-Toripalimab combination regimens, ADG106 should be intravenously infusion first, then 30 minutes later, Toripalimab will administered intravenously. Before administration, ADG106 will be diluted to appropriate volume and concentration and administered through 60-90 minutes $\pm$ 15 minutes IV infusion. Every effort should be made to target the total infusion time to be as close as possible to 60-90 minutes. However, given the variability of infusion pumps, a window of 15 minutes is permitted (i.e., infusion time is 60-90 ($\pm$ 15) minutes). The exact start and stop time of infusion, and any infusion interruptions for reaction(s) or other reason(s), should be carefully documented.

Patients will be closely monitored during treatment infusion and for at least 2 hours after the end of the infusion. All dose administrations will be performed in the clinical research center under the supervision of appropriately trained staff. Blood samples will not be collected from the same arm used for the IV infusion for at least 4 hours after administration.

*Duration of Treatment*

The treatment is scheduled to be Q3W for the first 4 cycles. However, if the study drug is beneficial, based on patient and investigator consent, the patient may continue to receive single ADG106, or ADG106 combination regimens, or Toripalimab, Q3W until disease progression or an intolerable toxicity, withdrawal of consent, or for a maximum treatment duration of 1 year of ADG106, or ADG106 combination regimen, or Toripalimab.

A treatment cycle is defined as one IV dose of the study drug(s) administered on day 1 every 21 days. During the study, patients will be evaluated for safety and toxicity, PK, immunogenicity, pharmacodynamics and efficacy per Schedule of Activities.

If tumors responded to ADG106 combination regimen and the combination regimen therapy required to continue more than 1year, Principal Investigator should discuss with the Sponsor and make decision. Investors will be strongly encouraged to involve hematologist in the management of patients with grade 3-4 anemia in whom hemolysis is suspected and patients with grade 2-4 hemolysis. If a patient in whom hemolysis is suspected will be monitored for immune system hyperreactivity, including antibodies that maybe directed against RBCs.

**Definition**

*Definition of Maximum Tolerated Dose*

The MTD is defined as the highest dose level of ADG106 in ADG106-Toripalimab combination regimen at which no more than 1 out of 6 patients experiences a DLT during the first cycle.

*Definition of Maximum Administered Dose*

If $\geq$2 out of 6 patients at the same dose level experience a DLT, then dose escalation is stopped, the MTD is exceeded, and this dose level is declared the MAD and the study might de-escalate to further titrate the MID. If MTD is not reached after completion of all dose levels during dose escalation, MAD will be 200mg of ADG106-Toripalimab combination regimen.

*Definition of Dose-limiting Toxicities (DLT)*

A DLT is defined as a toxicity (AE at least possibly related to ADG106, or or ADG106-Toripalimab) occurring during the DLT observation period (the initial 21 days) as defined below:

1. **Hematologic Toxicity:**

   a. Grade ≥4 hematologic toxicity.
   b. Grade ≥4 neutropenia of any duration, or Grade 3 neutropenia that lasts for more than 7 days or with documented infection.
   c. Grade ≥3 febrile neutropenia (ANC <1000/mm3 with a single temperature greater than 38.3°C or is higher than 38°C for >1 hour)
   d. Grade 4 thrombocytopenia or Grade 3 thrombocytopenia with bleeding.
   e. Grade ≥3 anemia that lasts more than 7 days.

2. **Non-hematologic toxicity:**

   a. Grade ≥3 non-hematologic toxicity (Except: Grade 3 rash, nausea, vomiting, and diarrhea if lasting <3 days with optimal medical management or Grade 3 fatigue less than 7 days).
   b. Any Grade 3 or Grade 4 non-hematologic related laboratory abnormality, if

      i. Requires medical intervention for the patient, or
      ii. Abnormal laboratory test results lead to hospitalization, or ≥Grade 3 abnormal laboratory results that last >72 hours despite appropriate replacement therapy.
      iii. Abnormal laboratory test results persist > 1 week.

   c. Any Grade ≥3 elevation of AST, ALT, or total bilirubin
   d. Any AST or ALT >3× ULN and concurrent total bilirubin >2× ULN, i.e. findings consistent with Hy's law or FDA definition of potential drug-induced liver injury.

3. Any Grade 5 toxicity (Except: if it is clearly not related to ADG106 or ADG106-Toripalimab, i.e. accidental death or death due to disease progression).
4. Delay of study therapy by any toxicity under "Criteria for withholding Study Drug" or ASCO irAE guideline for more than 3 weeks will be a DLT.
5. Other Grade of toxic reactions that require early termination of the trial after a discussion with the Investigator and the Sponsor.
6. Any toxicity that requires discontinuation under "Rules for Study Drug Discontinuation" or ASCO irAE guideline will be a DLT.

All AEs meeting the criteria described above should count as a DLT unless they can definitively be attributed to disease progression or other extraneous cause(s). Note: Grade 3 or 4 infusion-related reactions (IRR) are not DLTs. However, if a Grade 3 or 4 IRR occurs, the patient will need to stop the study treatment and be replaced with a new patient. If ≥2 patients in a treatment group have Grade 3 or 4 IRR, the dose level must be suspended, and the SRC will review the study's safety data to determine whether to continue further enrollment.

**Dose Modification and Management of Withholding, Resuming or Discontinuation of the Study Drug**

Intra-subject dose reduction of ADG106 in the combination regimens is not allowed. For any irAEs, ASCO guideline should be followed.
If significant toxicity occurs, dosing may be interrupted or delayed up to 6 weeks of scheduled therapy or permanently discontinued as described below. In the event of multiple toxicities, dosing management should be based on the worst toxicity observed. Patients are to be instructed to notify investigators at the first occurrence of any adverse symptom.
Generally, the following rules apply: for ADG106 combined with Toripalimab if an irAE occured, both ADG106 and Toripalimab should be discontinued. It's at Principal Investigators' discretion whether to resume treatment after irAE is controlled.

*Criteria for Withholding Study Drug*

Withholding criteria for this study are:

   1. Grade ≥3 non-cutaneous, non-laboratory, drug-related AEs.
   2. Any ≥ Grade 3 drug-related laboratory abnormalities with the following exceptions for lymphopenia and asymptomatic Grade 3 electrolyte abnormalities that can be resolved with replacement therapy within 72 hours and prior to dosing.

3. Any AEs, laboratory abnormalities, or intercurrent illness, which in the judgment of the investigators warrants interrupting or delaying the dose of study drug.

4. Increases in hepatic enzymes and/or bilirubin (Hy's Law):

   a) For patients without metastatic liver involvement:

   - AST or ALT Grade $\geq$ 3 (> 5 x ULN) AND concurrent total bilirubin 2 x ULN), or
   - Any AST or ALT > 10 x ULN.

   b) For patients with metastatic liver involvement:

   - Any AST or ALT > 10 x ULN.

5. Patients with Grade 4 anemia or hemolysis will permanently discontinue ADG106. Patients with Grade 3 hemolysis who recovered from the event and who have evidence of cinical benefit (per Investigator) will be discussed with the medical monitor. Selected patients may continue the treatment of ADG106.

For any patient in 4 above, the documentation of the time course of onset and resolution is essential and also to exclude potential causes of such hepatic abnormalities, including viral or drug or ethanol induced hepatitis, cholelithiasis, hemolysis, and other etiologies.

*Immune-Related Adverse Events*

Study treatment may be temporarily suspended for up to 6 weeks beyond the last dose if patients experience an irAE as defined below. If the study drug needs to be held due to irAE lasting 6 weeks and more from the last dose, then the study drug may be discontinued and the patient will be followed for safety and efficacy. If a patient must be tapered off steroids used to treat irAE, ADG106 may be held for 6 weeks until steroids are discontinued or reduced to prednisone dose (or dose equivalent) $\leq$ 10 mg/day. The acceptable length of interruption will depend on agreement between the Investigator, the sponsor's medical expert and the medical monitor.

*Withholding ADG106/Toripalimab for immune-related Adverse Events (irAE) (Follow ASCO Guideline)*

1. Immune-related hemolytic anemia: ADG1 06/Toripalimab will be withheld for Grade $\geq$ 2.
2. Immune-related pneumonitis: ADG106/Toripalimab will be withheld for Grade $\geq$ 1 with radiographic evidence of pneumonitis progression.
3. Immune-related colitis: *ADG106/Toripalimab* may be held temporarily and resumed if toxicity does not exceed Grade 1. At Grade $\geq$ 2 Investigator may consider permanently discontinuing ADG106/Toripalimab.
4. Immune-related hepatitis: ADG106/Toripalimab will be withheld for Grade $\geq$ 2 hepatitis based on liver enzyme elevations.
5. Immune-related endocrinopathies:

   a. Hypophysitis: Considering withholding *ADG106/Toripalimab* until patient is stabilized on replacement hormones for Grade 1 and Grade 2. For Grade 3-4 hypophysitis *ADG106/Toripalimab* should be withheld until patient is stabilized on replacement hormones.
   b. Thyroid disorders: ADG106/Toripaliamb will be withheld for Grade $\geq$ 3 hypo- or hyperthyroidism until symptoms resolve to baseline with appropriate supplementation or therapy.
   c. Diabetes: For Grade 2 diabetes, ADG106 may be withheld until glucose control is obtained. ADG106/Toripalimab will be held for Grade $\geq$ 3 hyperglycemia until glucose control is obtained on therapy with reduction of toxicity to G1 or less.

6. Inflammatory dermatitis: For Grade 1, continue ADG106/Toripaliamb. For Grade 2, consider holding *ADG106/Toripalimab* and monitor weekly for improvement. If not resolved, interrupt treatment until skin AE has reverted to grade 1. For Grade 3-4, ADG106/Toripalimab will be withheld and consult Dermatology on appropriateness of resuming.
7. Immune-related inflammatory arthritis: *ADG106/Toripalimab* will be withheld for Grade $\geq$ 2 inflammatory arthritis

until symptom control.

8. Immune-related nephritis: Temporarily holding of ADG106/Toripalimab will be considered at Grade 1-2 nephritis and consult nephrology. Permanently discontinue *ADG106/Toripalimab* for Grade 3-4 nephritis.

9. Immune-related myasthenia gravis (or gravis-like syndrome, myositis or muscle weakness), Guillain-Barré, meningoencephalitis: permanently discontinue *ADG106/Toripalimab* for any grade.

10. Ocular inflammatory toxicity: *ADG106/Toripalimab* will be withheld for Grade ≥ 2 ocular inflammatory toxicity, e.g. uveitis, episcleritis until after Ophthalmology consult.

11. Immune-related pancreatitis: *ADG106/Toripalimab* will be withheld for Grade ≥ 2 pancreatitis (based on amylase levels), or any grade of recurring pancreatitis.

12. Immune-related myocarditis or pericarditis - hold at Grade 1 and permanently discontinue ADG106/Toripalimab after Grade1.

13. Consider holding *ADG106/Toripalimab* for other irAE and lower Grade irAE per judgment of the Investigator and to be discussed with medical monitor or study director.

*Criteria to Resume Treatment (Follow ASCO Guideline)*

Patients may resume treatment with study drug(s) when the drug-related AEs resolve to Grade ≤1 except the following:

1 Patients may resume treatment in the presence of a Grade 2 fatigue or any grade of alopecia.

2 Patients who have not experienced a Grade 3 drug-related skin toxicity may resume treatment in the presence of Grade 2 skin toxicity under active treatment.

3 Drug-related pulmonary toxicity, diarrhea or colitis must have resolved or improved to baseline before treatment is resumed.

4 Drug-related endocrinopathies adequately controlled with only physiologic hormone replacement may resume treatment.

5 Treatment should be withheld in patients with an adverse event that poses a risk of major organ damage (e.g., DVT, PE, angina, COPD exacerbation).

*Rules for* Study *Drug Discontinuation* (*Follow ASCO Guieline)*

1. Discontinue treatment for any Grade 4 drug-related AEs or laboratory abnormality.

2. Discontinue treatment for inability to reduce corticosteroid dose to 10 mg or less of prednisone or equivalent per day within 8 weeks.

3. Grade 3 or 4 increases in liver enzymes and/or bilirubin. Patients with an AST or ALT ≥ 3×ULN and a concomitant increase in bilirubin > 2xULN should permanently discontinue study drug. All Grade 3-4 increases in liver enzymes and/or bilirubin should be discussed with the medical monitor or study director.

4. Discontinue treatment for any AE, laboratory abnormality, or intercurrent illness which, in the judgment of the investigator, presents a substantial clinical risk to the patient with continued investigational drug dosing.

**Concomitant medications**

Concomitant medications should be closely examined, and medications associated with a risk of hepatotoxicity should be modified (if possible) or their dose (e.g., acetaminophen) should be limited. All concomitant medications, particularly the use of medications associated with hepatotoxicity, should be discussed with the Medical Monitor prior to patient entry. Some concomitant medications (ConMeds) are allowed during the study to treat infusion-related reactions and other adverse events for symptomatic relief or management irAEs. Hormonal replacement therapy (e.g., for prostate cancer), anticoagulation therapy, oral contraceptives, denosumab, bisphosphonates and inhaled or topical corticosteroids/mineralocorticoids can be used or continue their use during the study at the discretion of the investigators.

Pretreatment with immunosuppressive agents is not allowed in the first cycle; however, it may be used in the second cycle or beyond with agreement from both the investigator and sponsor's medical monitor for management of adverse event that is clinical indicated for immunosuppressive therapy. Other use of ConMeds should be agreed upon by both the investigator and sponsor's medical monitor.

**Prohibited Medications/procedures**

Any other anti-cancer therapies, marketed or investigational, are prohibited during the study. Herbal or alternative medicines are not allowed. Immuno-stimulant agents like IFNs or IL2 are also prohibited during the study, and for 30 days after the last dose of study drug.

After Cycle 1, palliative radiation therapy (e.g., for bone metastases and other non-target lesions) and certain minor procedures/surgeries may be allowed with agreement between investigator and sponsor's medical monitor.

**Removal from Study Treatment and Off-Study Criteria**

Participation in this research study is completely voluntary. Patients are free to withdraw from this study at any time by informing the investigator. If a patient, for whatever reason is no longer appropriate to continue receiving study therapy, they will be notified and withdrawn from the study. Furthermore, if the patient is non-compliant (e.g., non-compliant with visits, concomitant medications) they will be withdrawn from the study and a replacement patient may be recruited.

Additionally, prior to removal from study, efforts must be made to have all patients complete a EOT visit assessment approximately 30 days following the last dose of study therapy.

*Criteria for removal* from *study* treatment

If patients are taken off study treatment, they will be followed until meeting the Off-Study criteria. When patients are off of protocol therapy, data will still be collected to meet protocol objectives.

Also, patients who complete study treatment will be followed for other study endpoints. Below are criteria for removal from study treatment: progressive disease, participant requests to be withdrawn from active therapy, unacceptable toxicity, investigator discretion, or positive pregnancy test.

*Off-Study Criteria*

Once a patient is taken off study, no further data can be collected. Off-study criteria are: patient requests to be withdrawn from study or follow up period; patient completed all protocol required safety follow-ups; death; or screen failure.

**Efficacy Assessments**

*Response Evaluation Criteria in Solid Tumors (RECIST) Version 1.1*

Although the clinical benefit of the study regimens has not yet been fully established, the intent of offering this treatment is to provide a possible therapeutic benefit, and thus the patient will be carefully monitored for tumor response, in addition to safety and emerging DLT.

A computed tomography (CT) scan (with oral/IV contrast unless contraindicated) or magnetic resonance imaging (MRI) of the tumor lesion(s), chest, abdomen, and pelvis will be performed at Baseline, and every 6 weeks (+/- 1 week) for the first 4 cycles. If treatment continues beyond 4 cycles, then assessments will be carried out every 9 weeks for the remaining treatment duration thereafter, until disease progression or death, or intolerable adverse events, or withdrawal of consent. The evaluation of efficacy will be based on tumor assessment by the investigator per RECIST v1.1. Lugano Classification will be used for tumor assessment of lymphoma.

Because of the potential for pseudo-progression, patients with radiographic progression in the absence of clinical deterioration, including worsening performance status as assessed by the Investigator, may continue to receive study treatment at the discretion of the investigator with the approval of the Sponsor Medical Monitor and an additional scan will be obtained 4-6 weeks later. If this subsequent scan shows disease progression, the patient will be discontinued from study treatment.

Treatment beyond initial RECIST-defined progression is permitted for patients with stable ECOG status who consent and do not have clinical symptoms and signs of disease progression to continue on treatment until the next imaging assessment. These patients will be re-evaluated for disease progression with restaging scans in 4 weeks, but no later than 6 weeks, after initial determination of progression.

Response and progression will be evaluated in this study using the Response Evaluation Criteria in Solid Tumors (RECIST) version 1.1 guideline(s). The following general principles must be followed:

1. To assess objective response, it is necessary to estimate the overall tumor burden at baseline to which subsequent measurements will be compared. All baseline evaluations should be performed as closely as possible to the beginning of treatment and never more than 28 days before beginning of treatment.

2. Measurable disease is defined by the presence of at least one measurable lesion. Note: Tumor lesions that are situated in a previously irradiated (or locally treated) area will be considered measurable, provided there has been clear imaging-based progression of the lesions since the time of radiation or local therapy.

3. All measurements should be recorded in metric notation by use of a ruler or calipers.

4. The same method of assessment and the same technique must be used to characterize each identified lesion at baseline and during follow-up.

Antitumor response patterns seen with immunotherapeutic agents may extend beyond the typical time course of responses seen with cytotoxic agents. Therefore, the investigator may decide to continue treatment with ADG106 combination regimen beyond tumor progression as defined by RECIST.

Once the specific criteria of RECIST defined disease progression are met, a repeat efficacy evaluation should be performed at the next per protocol scheduled assessment timepoint or earlier, if clinically necessary, (but no sooner than 4 weeks from the previous assessment) in order to confirm disease progression. If the disease progression is confirmed, then the patient may be discontinued. Otherwise, the investigator may decide to continue treatment.

While awaiting confirmation of disease progression, patients may continue to receive study drug, if their clinical status is considered to be stable by the investigator, based on the following criteria: absence of clinical signs and symptoms indicative of disease progression; clinical disease progression not requiring immediate therapeutic intervention; and/or no decline in ECOG performance status.

The decision to continue study treatment after the first evidence of disease progression is at the discretion of the investigator and requires that the patient agrees to this treatment plan, and that the shared decision to treat beyond progression is documented in source documents. Continuing treatment beyond confirmed disease progression will not be allowed.

*Recording of AEs*

AEs should be documented and recorded using NCI CTCAE v5.0. All AEs will be recorded once signing ICF until End of Study visit. Patients must be followed for AEs until all drug related toxicities have resolved or stabilized. SAEs will be reported after signing ICF until End of Study (EOS) visit.

*Blood Samples for PK evaluations*

Blood samples will be collected from all patients to determine the serum concentration of ADG106 and Toripalimab using a validated assay for PK analyses. Approximately 2 mL blood will be collected at each of the following time points for each antibody drug. Serum will be separated and stored for bioanalysis of antibody drug concentration with validated assay.

Cycle 1 and Cycle 4: pre-dose (within 30 minutes before dosing), EOI+10min, and after EOI 6 h + 30min, and days 8 and 15 after EOI. Cycle 2 and 3: pre-dose (within 30 minutes before dosing), EOI+10min. For the patients who continue the treatment after first 4 doses: pre-dose (within 30 minutes before dosing) and EOI+10min every two cycles. If possible, at EOT, and approximately 30 days. An additional blood sample should be collected from patients experiencing unexpected and/or serious AEs if possible.

The serum concentration of ADG106 and Toripalimab, PK parameters will be monitored more intensively during the first and fourth treatment cycle. The PK sampling time points may be adjusted based on cumulative data.

Non-compartmental analysis will be conducted using Phoenix WinNonlin Version 8.3. PK parameters include, but not limited to AUClast, AUCinf, Cmax, Tmax, t1/2, MRT, CL, Vd will be reported. Dose proportionality will be assessed as well.

*Immunogenicity Evaluations*

Blood samples for ADA analyses will be collected at pre-dose of Cycle 1 to 4, and at End of Study (EOS). For the patients who continue the treatment after the first 4 doses, the blood samples for ADA will be collected at pre-dose every two cycles. Approximately 3 mL blood for each antibody drug will be collected, and serum will be separated and stored for bioanalysis of anti-drug antibodies with validated assays. The incidence of antibodies against drug antibody will be summarized for all patients who received at least one administration of investigational drug. Neutralization activity will be evaluated if ADA is positive. Impact of ADAs on PK, efficacy/safety will be evaluated, if applicable.

*Pharmacodynamics and Predictive Biomarker Assessment*

Blood samples will be collected with time poinst listed below from all patients to determine relevant PD biomarkers quantitaively or qualitatively using a validated assay. Blood draw time points are as follows: Cycle 1: pre-dose (within 30 minutes before dosing) on day 1, and days 8 and 15. Cycles 2-4: pre-dose (within 30 minutes before dosing).

Pharmacodynamic biomarkers for ADG106 alone or in combination with Toripalimab will include but are not limited to: soluble proteins (sCD137, sPD-L1), peripheral immune cell subset profiling, tumor infiltrated lymphocytes, and pharmacogenomics markers both in peripheral blood and in tumor tissues (if available).

Tissue biopsies for stratification/preselection biomarker evaluation are mandatory for patients in enrolled in Phase II portion of this study, if archieved tumor tissue are not available before treatment but optional on treatment and at the end of treatment.

Patients may have available sufficient and adequate formalin fixed tumor tissue sample (e.g. 15 FFPE slides) preferably from a biopsy of a tumor lesion obtained either at the time of or after the diagnosis of advanced disease has been made and from a site not previously irradiated. Alternatively, patients may have a biopsy taken prior to entering the study to provide adequate tissue. Patients with biopsy accessible tumors may also undergo optional post-treatment tumor biopsies at Cycle 3 and at end of treatment. Patients will be given a separate, specific written consent. to provide baseline, on-treatment, and/or end of treatment biopsies. Biopsies at Cycle 3 should be collected after radiographic tumor scans scheduled for that cycle have been completed.

Biomarker sample analysis will be performed using validated procedures and methods as outlined in the Laboratory Manual. The Sponsor will supply complete written instructions for handling, processing, storage, and shipping of samples prior to study initiation.

**Safety Evaluations**

Safety assessments will be carried out during specified periods to include physical examination findings, vital signs, ECOG performance status, laboratory variables (e.g. hematology, coagulation tests, serum chemistry, urine tests and pregnancy test), ECG, and AEs. AEs are graded according to the CTCAE v5.0 by investigators. Study site personnel will be responsible for properly documenting and reporting AEs/SAEs.

The Study Review Committee (SRC) will consist of enrolling investigators and Sponsor representatives. SRC will review available safety, clinical activity, PK, and pharmacodynamic data and to determined MTD, MAD, and PR2D for phase Ib part. After stage 1 of phase II completed planned patient treatment, SRC will review all available data (safety and efficacy) and decide whether stage 2 to start or not. These decisions will be documented.

**Safety Reporting**

*Definitions*

An adverse event (AE) is defined as any reaction, side effect, or untoward event that occurs during the course of the clinical trial whether or not the event is considered related to the study drug or clinically significant. Checkpoint inhibition is associated with immune-related AEs (irAEs).

For this clinical trial, AEs will include any events reported by the patient, any new medical conditions or symptoms, any new abnormal findings on physical examination or laboratory evaluation. Additionally, any worsening of a pre-existing condition or abnormality will also be considered as an AE. All AEs must be recorded on eCRFs and must be followed until return to baseline or stabilized. Events that do not meet the definition of an AE include:

- Medical or surgical procedures (e.g., surgery, endoscopy, tooth extraction, transfusion); the condition that leads to the procedure is an AE;
- The cancer indication being studied, or expected progression of that cancer indication, even within 30 days of last dose and/or if serious criteria is met. Disease progression is to be recorded on specific tumor assessment pages rather than the AE page of the eCRF;
- Death or Disease Progression should not be recorded as a Serious Adverse Event or Adverse event. If an Adverse Event results in Death, enter the AE diagnosis that led to death with the outcome recorded as 'Fatal';
- Deterioration of a laboratory value, which is unequivocally due to disease progression, should also not be recorded as an Adverse Event;
- Pre-existing diseases or conditions present or detected before the start of study drug administration that do not worsen; and
- Situations where an untoward medical event has not occurred (e.g., planned hospitalization for an elective procedure, with elective defined as known or planned at the time of signing of the informed consent).

A serious adverse event is defined as any adverse experience that meets any of the following criteria:

- Results in death (death due to progressive disease will not be reported as a SAE for this study)
- Is life-threatening.
- Requires hospitalization or prolongation of existing hospitalization. Additionally, complications occurring during hospitalization are also considered AEs.
- Results in persistent or significant disability or incapacity (This definition is not intended to include experiences of relatively minor medical significance such as uncomplicated headache, nausea, vomiting, diarrhea, influenza, or accidental trauma (e.g., sprained ankle) which may interfere or prevent everyday life functions but do not constitute a substantial disruption.);
- Important medical events that may not result in death, be life-threatening, or require hospitalization may be considered serious when, based upon appropriate medical judgment, they may jeopardize the patient or patient and may require medical or surgical intervention to prevent one of the outcomes listed in this definition.
- May have resulted in a congenital anomaly or birth defect.

The following are NOT considered SAEs in this clinical study:

- A visit to the emergency room, observation or admission to a "Short Stay Area" for ambulatory care, or other hospital department less than 24 hours that does not result in admission (unless considered an "important medical event" or "life-threatening").
- Elective surgery planned prior to signing study consent.
- Medical/surgical hospital admission for purpose other than remedying an ill health state and was planned prior to entry into the study. Appropriate documentation is required in these cases.
- Routine health assessment requiring hospital admission for baseline/trending of health status (e.g., routine colonoscopy).
- Hospital admission encountered for another life circumstance that carries no bearing on health status and requires no medical/surgical intervention (e.g., lack of housing, economic inadequacy, care-giver respite, family circumstances, administrative).
- Hospital administration or other medical occurrences (such as prolonged hospitalization or death) for adverse events due to the malignant disease under study (including associated signs and symptoms of disease progression). Changes in disease are reported separately.
- Hospital admission for study biopsies.

Any AE caused by a drug is considered an adverse reaction.

**End of Treatment (EOT) and End of Study (EOS) visit**

An End of Treatment (EOT) visit will be conducted within 30-day of the last dose. An End of Study (EOS) visit should be completed within 30 days of EOT visit. EOT and EOS visits may be conducted in the same day.

**Study Statistical Design**

The study is a phase Ib/II study. Phase Ib is a traditional 3+3 dose escalation design and patients with advanced/metastatic solid or hematologic malignancies will be treated. There are 3 dose escalation cohorts. Phase II is 2 stage, randomized design with one interim analysis. Only patients with advanced/metastatic SCLC, TNBC, NSCLC, NPC and TCL will be enrolled in Phase II. In Stage 1 there are 2 arms (ADG106 combined with Toripalimab vs. ADG106) randomized at 1:1 ratio for each of biomarker positive and negarive cohorts independently. If the analysis at the end of Stage 1, it meets Go criteria (e.g. one arm achieves ORR $\geq$ 25% or difference between two arms $\geq$ 20%), the trial will proceed to Stage 2. In Stage 2, three arms (ADG106 combined with Toripalimab vs. Toripalimab vs. ADG106) will be randomized to biomarker positive cohort at 1:1:1 ratio.

At the end of Stage 1, an interim analysis will be conducted to evaluate efficacy based on Go criteria: any single arm achieves 25% response rate in biomarker positive cohort; or the difference in ORR between two arms is greater or equal to 20%.

There are two stages for this 2 stage multi-arm randomized study. In Stage 1, it is designed to independently evaluate efficacy of ADG106 combined with Toripalimab versus ADG106 alone in 2 patient cohorts: one cohort included 48 patients who are biomarker positive; the other cohort included 48 patients who are biomarker negative. See Figure 1.2-1 for study schema. 48 patients will be randomized (1:1) to receive ADG106 combined with Toripalimab or ADG106 within each cohort. Within each cohort, there will be an interim analysis for efficacy at the end of Stage 1 and futility monitoring when the half of total sample size, i.e. patients, completes at least one post-treatment tumor assessment. In Stage 2, 72 subjects with three treatment arms (ADG106 combined with Toripalimab versus ADG106 verus Toripalimab) will be randomized within single cohort for patients who are biomarker positive at 1:1:1 randomization ratio.

The efficacy endpoint ORR will be monitored for futility using the 2-arm Bayesian optimal phase 2 (BOP2) design to assess treatment effect between ADG106 combined with Toripalimab versus ADG106. Specifically, let $n$ denote the interim sample size and $N$ denote the maximum sample size. Let $p_{con}$ denote the probability of response in control treatment, $p_{exp}$ denote the probability of response in experimental treatment. The null hypothesis is $H_0: p_{exp} \leq p_{con}$, under which the experimental arm is deemed as unacceptable, with respective to the control. The following Bayesian rule are employed to make a go/no-go decision: (Futility stopping) stop enrolling patients and claim that the experimental arm is unacceptable if

$$Pr(p_{exp} > p_{con} | data) < \lambda(\frac{n}{N})^{\alpha},$$

where $\lambda$=0.91 and $\alpha$=0.89 are design parameters optimized to maximize the power under $H_1: p_{con} = 0.1$ and $p_{exp} = 0.35$, while controlling the type I error rate at 0.1 under $p_{con} = p_{exp} = 0.1$. This optimization is performed assuming a vague prior Beta (0.15,0.85) for $p_{con}$ and a vague prior Beta (0.3,0.7) for $p_{con}$. The above decision rule corresponds to the stopping boundaries in Table 12 and yields a statistical power of 0.95.

Table 12: Optimized stopping boundaries

| Interim (no. in control) | Interim (no. in experimental) | No. response in control | Stop for futility if no. of response in experimental - no. of response in control <= |
|---|---|---|---|
| 12 | 12 | 0 | Never |
| 12 | 12 | 1 | -1 |
| 12 | 12 | 2~11 | -2 |
| 12 | 12 | 12 | -1 |
| 24 | 24 | 0 | Never |
| 24 | 24 | 1~24. | -1 |
| 36 | 36 | 0~2 | 0 |
| 36 | 36 | 3~11 | 1 |
| 36 | 36 | 12~23 | 2 |
| 36 | 36 | 24~33 | 1 |
| 36 | 36 | 34~36 | 0 |
| 48 | 48 | 0 | 0 |
| 48 | 48 | 1 | 2 |
| 48 | 48 | 2~3 | 3 |
| 48 | 48 | 4~6 | 4 |
| 48 | 48 | 7~12 | 5 |
| 48 | 48 | 13~29 | 6 |
| 48 | 48 | 30~36 | 5 |
| 48 | 48 | 37~40 | 4 |
| 48 | 48 | 41~43 | 3 |
| 48 | 48 | 44~45 | 2 |
| 48 | 48 | 46~47 | 1 |

(continued)

| Interim (no. in control) | Interim (no. in experimental) | No. response in control | Stop for futility if no. of response in experimental - no. of response in control <= |
|---|---|---|---|
| 48 | 48 | 48 | 0 |

Based on Table 12, the interim futility monitoring is performed when the number of enrolled patients reaches 24, 48, 72. When the total number of patients reaches the maximum sample size of 96, we reject the null hypothesis and conclude that the experimental arm is acceptable, compared to to the control, if the futility stopping boundary is not crossed. The go/no-go criteria in Table 12 are non-binding. Table 13 below are the operating characteristics of the design based on 10000 simulations using the BOP2 application.

Table 13: Operating characteristics

| Scenario | Pr (Eff) for Control | Pr (Eff) for Experimental | Early Futility Stopping (%) | Claim Promising (%) | Average Sample Size |
|---|---|---|---|---|---|
| 1 | 0.1 | 0.25 | 14.31 | 72.47 | 89.4 |
| 2 | 0.1 | 0.35 | 2.64 | 94.89 | 94.6 |
| 3 | 0.2 | 0.35 | 19.26 | 62.35 | 87.0 |
| 4 | 0.2 | 0.35 | 19.26 | 62.35 | 87.0 |
| 5 | 0.2 | 0.40 | 10.85 | 78.78 | 90.7 |

Interim Analysis at the end of Stage 1.

At the end of Stage 1 an interim analysis will be conducted by Study Review Committee (SRC) to evaluate Cohort 1 (Biomarker positive) and Cohort 2 (Biomarker negative) independently after all patients in the Stage 1 complete at least one post-treatment tumor assessment. The interim analysis will be conducted according to the following Go criteria assuming futility is not reached based on Table 12 above. If futility is reached, the cohort will stop the enrollment. If futility is not met, then the cohort will continue enrollment until reaching n=48.

The interim analysis will determine whether the enrollment for Stage 2 will begin based on Go criteria as below: a) any single arm achieves 25% response rate in biomarker positive cohort; or b) the difference in ORR between two arms is greater or equal to 20%.

At the end of Stage 2, there will be two primary efficacy treatment comparisons: a) ADG106 combined with Toripalimab vs. ADG106; and b) ADG106 combined with Toripalimab vs. Toripalimab. The secondary comparison is a) Toripalimab vs. ADG106.

For Combo and ADG106 arms, the data from Stage 1 and Stage 2 will combined at the final analysis. The above hypothesis a) and b) will be test with multiplicity adjustment using Dunnett's correction to conforl family-wise error rate (FWER). P-values will be adjusted as well using the same correction. Due to the complexitiy of this two-stage study design, a simulation program has been written to calculate approximate power estimations with fixed sample size and stages. The operating characteristics of the study including the number of subjects needed and the power are obtained through clinical trial simulations. In the simulation only biomarker positive cohort is considered as it is only cohort possibly having Stage 2 component.

Basic assumptions are: Objective Response Rate (ORR) for ADG106 combined with Toripalimab, ADG106 and Toripalimab, are $\pi$combo = 0.35, $\pi$adg106 = 0.10 and $\pi$antiPD1 =0.25 respective. The total alpha =0.1; In Stage 1 there are 2 arms (24 patients earch arm) (ADG106 combined with Toripalimab vs. ADG106) randomized at 1:1 ratio for each of biomarker positive and negarive cohorts independently. If the analysis at the end of Stage 1, if no futility rule defined in Table 12 is reached and it meets Go criteria (e.g., one arm achieves ORR $\geq$ 25% or difference between two arms $\geq$ 20%), the trial will proceed to Stage 2. In Stage 2, 72 subjects with three treatment arms (ADG106 combined with Toripalimab versus ADG106 verus Toripalimab) will be randomized within single cohort for patients who are biomarker positive at 1:1:1 randomization ratio.

Table 14: Power, futility, and interim reaching Go Probabilities (simulation number=100000)

| ORR Assumptions | Stage 1 | | Stage 2 | | | |
|---|---|---|---|---|---|---|
| | Futility Probability At Stage 1 | Reach Go at the interim | Power for testing Combo vs. ADG106[a] | Power for testing Combo vs. anti PD1[b] | Power for testing antiPD1 vs. ADG106[c] | Futility Probability At Stage 2 |
| $\pi_{combo} = 0.35$, $\pi_{adg106} = 0.10$ $\pi_{antiPD1} = 0.25$ | 0.009 | 0.843 | 0.693 | 0.070 | 0.284 | 0.088 |
| $\pi_{combo} = 0.25$, $\pi_{adg106} = 0.10$ $\pi_{antiPD1} = 0.25$ | 0.054 | 0.529 | 0.284 | 0.008 | 0.163 | 0.176 |
| $\pi_{combo} = 0.40$, $\pi_{adg106} = 0.10$ $\pi_{antiPD1} = 0.25$ | 0.004 | 0.926 | 0.845 | 0.150 | 0.313 | 0.045 |
| $\pi_{combo} = 0.40$, $\pi_{adg106} = 0.15$ $\pi_{antiPD1} = 0.25$ | 0.014 | 0.886 | 0.657 | 0.140 | 0.120 | 0.107 |
| $\pi_{combo} = 0.10$, $\pi_{adg106} = 0.10$ $\pi_{antiPD1} = 0.10$ | 0.402 | 0.046 | 0.004 | 0.000 | 0.000 | 0.041 |
| $\pi_{combo} = 0.20$, $\pi_{adg106} = 0.20$ $\pi_{antiPD1} = 0.20$ | 0.428 | 0.164 | 0.008 | 0.002 | 0.002 | 0.135 |
| Note: a) and b) are primary comparisons defined above and c) is secondary comparison | | | | | | |

Table 15: Power, futility, and interim reaching Go Probabilities without Dunett's correction (simulation number=100000)

| ORR Assumptions | Stage 1 | | Stage 2 | | | |
|---|---|---|---|---|---|---|
| | Futility Probability At Stage 1 | Reach Go at the interim | Power for testing Combo vs. ADG106[a] | Power for testing Combo vs. antiPD 1[b] | Power for testing antiPD1 vs. ADG106[c] | Futility Probability At Stage 2 |
| $\pi_{combo} = 0.35$, $\pi_{adg106} = 0.10$ $\pi_{antiPD1} = 0.25$ | 0.009 | 0.843 | 0.731 | 0.123 | 0.324 | 0.088 |
| $\pi_{combo} = 0.25$, $\pi_{adg106} = 0.10$ $\pi_{antiPD1} = 0.25$ | 0.054 | 0.529 | 0.329 | 0.017 | 0.177 | 0.176 |
| $\pi_{combo} = 0.40$, $\pi_{adg106} = 0.10$ $\pi_{antiPD1} = 0.25$ | 0.004 | 0.926 | 0.867 | 0.233 | 0.361 | 0.045 |
| $\pi_{combo} = 0.40$, $\pi_{adg106} = 0.15$ $\pi_{antiPD1} = 0.25$ | 0.014 | 0.886 | 0.714 | 0.221 | 0.170 | 0.107 |
| $\pi_{combo} = 0.10$, $\pi_{adg106} = 0.10$ $\pi_{antiPD1} = 0.10$ | 0.402 | 0.464 | 0.005 | 0.001 | 0.001 | 0.041 |
| $\pi_{combo} = 0.20$, $\pi_{adg106} = 0.20$ | 0.428 | 0.164 | 0.008 | 0.002 | 0.002 | 0.135 |

(continued)

| ORR Assumptions | Stage 1 | | Stage 2 | | | |
|---|---|---|---|---|---|---|
| | Futility Probability At Stage 1 | Reach Go at the interim | Power for testing Combo vs. ADG106[a)] | Power for testing Combo vs. antiPD 1[b)] | Power for testing antiPD1 vs. ADG106[c)] | Futility Probability At Stage 2 |
| $\pi_{antiPD1}$ =0.20 | | | | | | |
| Note: a) and b) are primary comparisons defined above and c) is secondary comparison | | | | | | |

The simulations in Table 14 and Table 15 above indicate under the null hypothesis the Type 1 error is under control. Descriptive statistics will be used to summarize data including baseline patient characteristics, treatment with ADG106 alone or ADG106 combination regimens, safety variables and preliminary efficacy. Categorical or nominal variables will be summarized by frequency and percentage. Continuous variables will be summarized using standard summary statistics. Where appropriate, 95% confidence intervals around point estimates will be presented.

Biomarker data will be summarized by actual dose level at each protocol scheduled timepoint using either descriptive statistics or frequency tabulations, as appropriate for the type of data. For stratification/selection markers, ROC analysis of responder group and non-responder group will be performed to establish and validate the cutoff value for the biomarkers. The patient response (ORR, PFS, OS) will be compared between the biomarker positive and negative groups.

**Data Analysis**

*Analysis Population*

Pharmacokinetic, efficacy, and safety analyses will be based on the safety-evaluable population, defined as all patients who have received at least one dose of ADG106, ADG106-Toripalimab combination regimen, or Toripalimab.

*PK*

PK parameters will include, but not be limited to, AUC, Cmax, Ctrough, t1/2, CL, and Vss. The concentration-time data will be summarized by descriptive statistics (n, mean, standard deviation, coefficient of variation, median, minimum, maximum, and geometric mean) according to dosing cohort and time of the study. PK parameters will be estimated using a non-compartmental method with Phoenix WinNonlin (Certara, USA). Individual and mean plasma concentration of ADG106 or Toripalimab versus time will be tabulated and plotted by dose level. Dose proportionality and drug antibody accumulation may be evaluated.

A population PK-based modeling approach may also be applied for PK characterization and PK covariate analyses. The steady state PK profile may be projected based on the population PK model. Population PK and PD data may be analyzed using modeling approaches and may also be pooled with data from other studies to investigate any association between investigational drug exposure and biomarkers or significant safety endpoints. Alternative dosing approach, e.g., body size-based or fixed dosing, may be evaluated using a population PK/PD approach. The results of these analyses, if performed, may be reported separately.

*Immunogenicity*

All samples will first be analyzed for ADAs in a screening assay. Study samples with results below the screening cut-off will be reported as negative for ADAs. In the event of a positive result in the screening assay, samples will be analyzed in the confirmatory assay. All samples confirmed positive will be reported as positive and may be analyzed for presence of neutralizing antibodies (NAbs).

The incidence of ADA will be summarized for all patients who received at least one administration of investigational drug. Impact of ADAs on PK, efficacy/safety of investigational drug will be evaluated, if applicable.

*Pharmacodynamics*

Biomarker data will be summarized by actual dose level at each protocol scheduled timepoint using either descriptive statistics or frequency tabulations, as appropriate for the type of data.

*Stratification/preselection biomarkers*

ROC analysis of responder group and non-responder group will be performed to establish and validate the cutoff value for the biomarkers. The Patient response (ORR, PFS, OS) will be compared between the biomarker positive and negative groups.

*Safety Analysis*

Any patient receiving any amount of single dose of investigational study regimen will be included in the summaries and listings of safety data. Safety and tolerability profile will be characterized by following criteria: type and frequency of therapy; start date, stop date, severity, relationship, expectedness, outcome, and relationship of AE to study drug; and relationship of study therapy and laboratory abnormalities.

Adverse events will be classified using the MedDRA classification system. The severity of the toxicities will be graded according to the NCI CTCAE version 5.0.

In all summaries, emphasis will be placed on treatment emergent adverse events (TEAEs), namely, those with initial onset or that worsen in severity after the first dose of investigational study regimen. AEs will be summarized by the frequency of patients experiencing TEAEs corresponding to body systems and MedDRA preferred term and by worst NCI CTCAE (version 5.0) grade. Summaries will also be provided for TEAEs, namely, those judged by the investigator to be related or likely related to investigational drug.

Adverse events resulting in discontinuation of investigational study regimen or withdrawal from the study, Grade 3 or higher, serious adverse events, and deaths on-study will be tabulated. All DLTs will be reported and the MTD will be identified if so, reached during the study.

CMP, CBC with diff, U/A, coagulation laboratory data, ECGs, will be summarized by cycle. The hematologic and chemistry laboratory results will be graded according to the NCI CTCAE version 5.0 severity grade. For the parameters which severity grade does not exist in the NCI CTCAE version 5.0, the frequency of patients with a laboratory abnormality meeting pre-specified criteria for the local or central laboratory will be summarized. The changes in ECG readings from baseline will be summarized using descriptive statistics by nominal time point.

Other safety data collected will be listed and summarized using descriptive statistics as appropriate. Notable values may be flagged. Notable / abnormal values for safety data will be further specified in the SAP and will be used for shift tables.

# SEQUENCE LISTING

**SEQ ID NO: 1 human CD137 amino acid sequence**
MGNSCYNIVATLLLVLNFERTRSLQDPCSNCPAGTFCDNNRNQICSPCPPNSFSSAGGQ
RTCDICRQCKGVFRTRKECSSTSNAECDCTPGFHCLGAGCSMCEQDCKQGQELTKKGCK
DCCFGTFNDQKRGICRPWTNCSLDGKSVLVNGTKERDVVCGPSPADLSPGASSVTPPAP
AREPGHSPQIISFFLALTSTALLFLLFFLTLRFSVVKRGRKKLLYIFKQPFMRPVQTTQ
EEDGCSCRFPEEEEGGCEL

**SEQ ID NO: 2 ADG106 HVR-H1**
FSLSTGGVGVGWI

**SEQ ID NO: 3 ADG106 HVR-H2**
LALIDWADDKYYSPSLKSRL

**SEQ ID NO: 4 ADG106 HVR-H3**
ARGGSDTVIGDWFAY

**SEQ ID NO: 5 ADG106 HVR-L1**
RASQSIGSYLA

**SEQ ID NO: 6 ADG106 HVR-L2**
DASNLETGV

**SEQ ID NO: 7 ADG106 HVR-L3**
YCQQGYYLWT

**SEQ ID NO: 8 ADG106 VH**
EVQLVESGGGLVQPGGSLRLSCAASGFSLSTGGVGVGWIRQAPGKGLEWLALIDWADDK
YYSPSLKSRLTISRDNSKNTLYLQLNSLRAEDTAVYYCARGGSDTVIGDWFAYWGQGTL
VTVSS

**SEQ ID NO: 9 ADG106 VL**
DIQLTQSPSSLSASVGDRVTITCRASQSIGSYLAWYQQKPGKAPKLLIYDASNLETGVP
SRFSGSGSGTDFTLTISSLQPEDFATYYCQQGYYLWTFGQGTKVEIKR

**SEQ ID NO: 10 ADG106 Heavy Chain**
EVQLVESGGGLVQPGGSLRLSCAASGFSLSTGGVGVGWIRQAPGKGLEWLALIDWADDK
YYSPSLKSRLTISRDNSKNTLYLQLNSLRAEDTAVYYCARGGSDTVIGDWFAYWGQGTL
VTVSSASTKGPSVFPLAPCSRSTSESTAALGCLVKDYFPEPVTVSWNSGALTSGVHTFP
AVLQSSGLYSLSSVVTVPSSSLGTKTYTCNVDHKPSNTKVDKRVESKYGPPCPPCPAPE
FLGGPSVFLFPPKPKDTLMISRTPEVTCVVVDVSQEDPEVQFNWYVDGVEVHNAKTKPR

EEQFNSTYRVVSVLTVLHQDWLNGKEYKCKVSNKGLPSSIEKTISKAKGQPREPQVYTL
PPSQEEMTKNQVSLTCLVKGFYPSDIAVEWESNGQPENNYKTTPPVLDSDGSFFLYSRL
TVDKSRWQEGNVFSCSVMHEALHNHYTQKSLSLSLGK

**SEQ ID NO: 11 ADG106 Light Chain**

DIQLTQSPSSLSASVGDRVTITCRASQSIGSYLAWYQQKPGKAPKLLIYDASNLETGVP
SRFSGSGSGTDFTLTISSLQPEDFATYYCQQGYYLWTFGQGTKVEIKRTVAAPSVFIFP
PSDEQLKSGTASVVCLLNNFYPREAKVQWKVDNALQSGNSQESVTEQDSKDSTYSLSST
LTLSKADYEKHKVYACEVTHQGLSSPVTKSFNRGEC

**SEQ ID NO: 12 ADG10059 HVR-H1**

YSITSGHYWAWI

**SEQ ID NO: 13 ADG10059 HVR-H2**

VSSISGYGSTTYYADSVKGRF

**SEQ ID NO: 14 ADG10059 HVR-H3**

ARGGSDAVLGDWFAY

**SEQ ID NO: 15 ADG10059 HVR-L1**

RASQGIGSFLA

**SEQ ID NO: 16 ADG10059 HVR-L2**

DASNLETGV

**SEQ ID NO: 17 ADG10059 HVR-L3**

YCQQGYYLWT

**SEQ ID NO: 18 ADG10059 VH**

EVQLVESGGGLVQPGGSLRLSCAASGYSITSGHYWAWIRQAPGKGLEWVSSISGYGSTT
YYADSVKGRFTISRDNSKNTLYLQLNSLRAEDTAVYYCARGGSDAVLGDWFAYWGQGTL
VTVSS

**SEQ ID NO: 19 ADG10059 VL**

DIQLTQSPSSLSASVGDRVTITCRASQGIGSFLAWYQQKPGKAPKLLIYDASNLETGVP
SRFSGSGSGTDFTLTISSLQPEDFATYYCQQGYYLWTFGQGTKVEIKR

**SEQ ID NO: 20 ADG10059 Heavy chain**

EVQLVESGGGLVQPGGSLRLSCAASGYSITSGHYWAWIRQAPGKGLEWVSSISGYGSTT
YYADSVKGRFTISRDNSKNTLYLQLNSLRAEDTAVYYCARGGSDAVLGDWFAYWGQGTL
VTVSSASTKGPSVFPLAPCSRSTSESTAALGCLVKDYFPEPVTVSWNSGALTSGVHTFP
AVLQSSGLYSLSSVVTVPSSSLGTKTYTCNVDHKPSNTKVDKRVESKYGPPCPPCPAPE
FLGGPSVFLFPPKPKDTLMISRTPEVTCVVVDVSQEDPEVQFNWYVDGVEVHNAKTKPR

EEQFNSTYRVVSVLTVLHQDWLNGKEYKCKVSNKGLPSSIEKTISKAKGQPREPQVYTL
PPSQEEMTKNQVSLTCLVKGFYPSDIAVEWESNGQPENNYKTTPPVLDSDGSFFLYSRL
TVDKSRWQEGNVFSCSVMHEALHNHYTQKSLSLSLGK

**SEQ ID NO: 21 ADG10059 Light chain**
DIQLTQSPSSLSASVGDRVTITCRASQGIGSFLAWYQQKPGKAPKLLIYDASNLETGVP
SRFSGSGSGTDFTLTISSLQPEDFATYYCQQGYYLWTFGQGTKVEIKRTVAAPSVFIFP
PSDEQLKSGTASVVCLLNNFYPREAKVQWKVDNALQSGNSQESVTEQDSKDSTYSLSST
LTLSKADYEKHKVYACEVTHQGLSSPVTKSFNRGEC

**SEQ ID NO: 22 AG10058 HVR-H1**
FSLSTSGVGVGWI

**SEQ ID NO: 23 AG10058 HVR-H2**
LALIDWDDDKYYSPSLKSRL

**SEQ ID NO: 24 AG10058 HVR-H3**
ARGGSDTVLGDWFAY

**SEQ ID NO: 25 AG10058 HVR-L1**
RASQSVSPYLA

**SEQ ID NO: 26 AG10058 HVR-L2**
DASSLESGV

**SEQ ID NO: 27 AG10058 HVR-L3**
YCQQGYSLWT

**SEQ ID NO: 28 AG10058 VH**
EVQLVESGGGLVQPGGSLRLSCAASGFSLSTSGVGVGWIRQAPGKGLEWLALIDWDDDK
YYSPSLKSRLTISRDNSKNTLYLQLNSLRAEDTAVYYCARGGSDTVLGDWFAYWGQGTL
VTVSS

**SEQ ID NO: 29 AG10058 VL**
DIQLTQSPSSLSASVGDRVTITCRASQSVSPYLAWYQQKPGKAPKLLIYDASSLESGVP
SRFSGSGSGTDFTLTISSLQPEDFATYYCQQGYSLWTFGQGTKVEIKR

**SEQ ID NO: 30 AG10058 Heavy chain**
EVQLVESGGGLVQPGGSLRLSCAASGFSLSTSGVGVGWIRQAPGKGLEWLALIDWDDDK
YYSPSLKSRLTISRDNSKNTLYLQLNSLRAEDTAVYYCARGGSDTVLGDWFAYWGQGTL
VTVSSASTKGPSVFPLAPCSRSTSESTAALGCLVKDYFPEPVTVSWNSGALTSGVHTFP
AVLQSSGLYSLSSVVTVPSSSLGTKTYTCNVDHKPSNTKVDKRVESKYGPPCPPCPAPE
FLGGPSVFLFPPKPKDTLMISRTPEVTCVVVDVSQEDPEVQFNWYVDGVEVHNAKTKPR

EEQFNSTYRVVSVLTVLHQDWLNGKEYKCKVSNKGLPSSIEKTISKAKGQPREPQVYTL
PPSQEEMTKNQVSLTCLVKGFYPSDIAVEWESNGQPENNYKTTPPVLDSDGSFFLYSRL
TVDKSRWQEGNVFSCSVMHEALHNHYTQKSLSLSLGK

**SEQ ID NO: 31 AG10058 Light chain**
DIQLTQSPSSLSASVGDRVTITCRASQSVSPYLAWYQQKPGKAPKLLIYDASSLESGVP
SRFSGSGSGTDFTLTISSLQPEDFATYYCQQGYSLWTFGQGTKVEIKRTVAAPSVFIFP
PSDEQLKSGTASVVCLLNNFYPREAKVQWKVDNALQSGNSQESVTEQDSKDSTYSLSST
LTLSKADYEKHKVYACEVTHQGLSSPVTKSFNRGEC

**SEQ ID NO: 32 HVR-H1 Formula (I)**
X1TFX2X3YX4IHWV, wherein X1 is F or Y, X2 is S or T, X3 is G, N, or S, and X4 is A, G, or W

**SEQ ID NO: 33 HVR-H1 Formula (II)**
YSIX1SGX2X3WX4WI, wherein X1 is S or T, X2 is H or Y, X3 is H or Y, and X4 is A, D, G, N, S, or T

**SEQ ID NO: 34 HVR-H1 Formula (III)**
FSLSTX1GVX2VX3WI, wherein X1 is G or S, X2 is A or G, and X3 is A, G, S, or T

**SEQ ID NO: 35 HVR-H2 Formula (IV)**
LALIDWX1X2DKX3YSX4SLKSRL, wherein X1 is A, D, or Y, X2 is D or G, X3 is R, S, or Y, and X4 is P or T

**SEQ ID NO: 36 HVR-H2 Formula (V)**
IGX1IYHSGX2TYYX3PSLKSRV, wherein X1 is D or E, X2 is N or S, and X3 is N or S

**SEQ ID NO: 37 HVR-H2 Formula (VI)**
VSX1ISGX2GX3X4TYYADSVKGRF, wherein X1 is A, G, S, V, or Y, X2 is A, D, S, or Y, X3 is D, G, or S, and X4 is S or T

**SEQ ID NO: 38 HVR-H3 Formula (VII)**
ARX1GX2X3X4VX5GDWFX6Y, wherein X1 is E or G, X2 is E or S, X3 is D or T, X4 is A, T, or V, X5 is A, I, L, T, or V, and X6 is A, D, or G

**SEQ ID NO: 39 HVR-L1 Formula (VIII)**
X1ASQX2X3X4X5X6X7X8, wherein X1 is Q or R, X2 is D, G, or S, X3 is I or V, X4 is G, R, S, or T, X5 is P, R, S, or T, X6 is A, D, F, S, V, or Y, X7 is L or V, and X8 is A, G, or N

**SEQ ID NO: 40 HVR-L2 Formula (IX)**
X1ASX2X3X4X5GX6, wherein X1 is A or D, X2 is N, S, or T, X3 is L or R, X4 is A, E, or Q, X5 is S or T, and X6 is I or V

**SEQ ID NO: 41 HVR-L3 Formula (X)**
YCQQX1YX2X3X4T, wherein X1 is A, G, S, or Y, X2 is Q, S, or Y, X3 is I, L, T, or Y, and X4 is I, S, V, or W

**SEQ ID NO: 42 HVR-L3 Formula (XI)**
YCX1QX2X3X4X5PX6T, wherein X1 is E or Q, X2 is P, S, or Y, X3 is D, L, S, T, or Y, X4 is D, E, H, S, or T, X5 is D, L T, or W, and X6 is L, P, R, or V

**SEQ ID NO: 43 sCD137 amino acid sequence**

LQDPCSNCPAGTFCDNNRNQICSPCPPNSFSSAGGQRTCDICRQCKGVFRTRKEC
SSTSNAECDCTPGFHCLGAGCSMCEQDCKQGQELTKKGCKDCCFGTFNDQKRGI
CRPWTNCSLDGKSVLVNGTKERDVVCGPSPADLSPGASSVTPPAPAREPGHSPQ

**SEQ ID NO: 44 CD137L nucleic acid sequence**
Atggaatacgcctctgacgcttcactggaccccgaagccccgtggcctcccgcgccccgcgctcgcgcctgccgcgtactgc
cttgggccctggtcgcggggctgctgctgctgctgctgctcgctgccgcctgcgccgtcttcctcgcctgccctgggccgtgtc
cggggctcgcgcctcgcccggctccgcggccagcccgagactccgcgagggtcccgagctttcgcccgacgatcccgccg
gcctcttggacctgcggcagggcatgtttgcgcagctggtggcccaaaatgttctgctgatcgatgggcccctgagctggtaca
gtgacccaggcctggcaggcgtgtccctgacggggggcctgagctacaaagaggacacgaaggagctggtggtggccaag
gctggagtctactatgtcttctttcaactagagctgcggcgcgtggtggccggcgagggctcaggctccgtttcacttgcgctgca
cctgcagccactgcgctctgctgctggggccgccgccctggctttgaccgtggacctgccacccgcctcctccgaggctcgga
actcggccttcggtttccagggccgcttgctgcacctgagtgccggccagcgcctgggcgtccatcttcacactgaggccaggg
cacgccatgcctggcagcttacccagggcgccacagtcttgggactcttccgggtgacccccgaaatcccagccggactccctt
caccgaggtcggaa

**SEQ ID NO: 45 CD137L amino acid sequence**
MEYASDASLD PEAPWPPAPR ARACRVLPWA LVAGLLLLLL LAAACAVFLA
CPWAVSGARA SPGSAASPRL REGPELSPDD PAGLLDLRQG MFAQLVAQNV
LLIDGPLSWY SDPGLAGVSL TGGLSYKEDT KELVVAKAGV YYVFFQLELR
RVVAGEGSGS VSLALHLQPL RSAAGAAALA LTVDLPPASS EARNSAFGFQ
GRLLHLSAGQ RLGVHLHTEA RARHAWQLTQ GATVLGLFRV TPEIPAGLPS
PRSE

**SEQ ID NO: 46 PD-L1 amino acid sequence**
MRIFAVFIFMTYWHLLNAFTVTVPKDLYVVEYGSNMTIECKFPVEKQLDLAALIV
YWEMEDKNIIQFVHGEDLKVQHSSYRQRARLLKDQLSLGNAALQITDVKLQDA
GVYRCMISYGGADYKRITVKVNAPYNKINQRILVVDPVTSEHELTCQAEGYPKA
EVIWTSSDHQVLSGKTTTTNSKREEKLFNVTSTLRINTTTNEIFYCTFRRLDPEEN
HTAELVIPELPLAHPPNERTHLVILGAILLCLGVALTFIFRLRKGRMMDVKKCGIQ
DTNSKKQSDTHLEET

**SEQ ID NO: 47 Ki67 amino acid sequence**

```
MWPTRRLVTIKRSGVDGPHFPLSLSTCLFGRGIECDIRIQLPVVSKQHCKIEIHEQEAI
LHNFSSTNPTQVNGSVIDEPVRLKHGDVITIIDRSFRYENESLQNGRKSTEFPRKIREQ
EPARRVSRSSFSSDPDEKAQDSKAYSKITEGKVSGNPQVHIKNVKEDSTADDSKDSVAQ
GTTNVHSSEHAGRNGRNAADPISGDFKEISSVKLVSRYGELKSVPTTQCLDNSKKNESP
FWKLYESVKKELDVKSQKENVLQYCRKSGLQTDYATEKESADGLQGETQLLVSRKSRPK
SGGSGHAVAEPASPEQELDQNKGKGRDVESVQTPSKAVGASFPLYEPAKMKTPVQYSQQ
QNSPQKHKNKDLYTTGRRESVNLGKSEGFKAGDKTLTPRKLSTRNRTPAKVEDAADSAT
KPENLSSKTRGSIPTDVEVLPTETEIHNEPFLTLWLTQVERKIQKDSLSKPEKLGTTAG
QMCSGLPGLSSVDINNFGDSINESEGIPLKRRRVSFGGHLRPELFDENLPPNTPLKRGE
APTKRKSLVMHTPPVLKKIIKEQPQPSGKQESGSEIHVEVKAQSLVISPPAPSPRKTPV
ASDQRRRSCKTAPASSSKSQTEVPKRGGRKSGNLPSKRVSISRSQHDILQMICSKRRSG
ASEANLIVAKSWADVVKLGAKQTQTKVIKHGPQRSMNKRQRRPATPKKPVGEVHSQFST
GHANSPCTIIIGKAHTEKVHVPARPYRVLNNFISNQKMDFKEDLSGIAEMFKTPVKEQP
QLTSTCHIAISNSENLLGKQFQGTDSGEEPLLPTSESFGGNVFFSAQNAAKQPSDKCSA
SPPLRRQCIRENGNVAKTPRNTYKMTSLETKTSDTETEPSKTVSTANRSGRSTEFRNIQ
KLPVESKSEETNTEIVECILKRGQKATLLQQRREGEMKEIERPFETYKENIELKENDEK
MKAMKRSRTWGQKCAPMSDLTDLKSLPDTELMKDTARGQNLLQTQDHAKAPKSEKGKIT
KMPCQSLQPEPINTPTHTKQQLKASLGKVGVKEELLAVGKFTRTSGETTHTHREPAGDG
KSIRTFKESPKQILDPAARVTGMKKWPRTPKEEAQSLEDLAGFKELFQTPGPSEESMTD
EKTTKIACKSPPPESVDTPTSTKQWPKRSLRKADVEEEFLALRKLTPSAGKAMLTPKPA
GGDEKDIKAFMGTPVQKLDLAGTLPGSKRQLQTPKEKAQALEDLAGFKELFQTPGHTEE
LVAAGKTTKIPCDSPQSDPVDTPTSTKQRPKRSIRKADVEGELLACRNLMPSAGKAMHT
PKPSVGEEKDIIIFVGTPVQKLDLTENLTGSKRRPQTPKEEAQALEDLTGFKELFQTPG
HTEEAVAAGKTTKMPCESSPPESADTPTSTRRQPKTPLEKRDVQKELSALKKLTQTSGE
TTHTDKVPGGEDKSINAFRETAKQKLDPAASVTGSKRHPKTKEKAQPLEDLAGLKELFQ
TPVCTDKPTTHEKTTKIACRSQPDPVDTPTSSKPQSKRSLRKVDVEEEFFALRKRTPSA
GKAMHTPKPAVSGEKNIYAFMGTPVQKLDLTENLTGSKRRLQTPKEKAQALEDLAGFKE
LFQTRGHTEESMTNDKTAKVACKSSQPDPDKNPASSKRRLKTSLGKVGVKEELLAVGKL
TQTSGETTHTHTEPTGDGKSMKAFMESPKQILDSAASLTGSKRQLRTPKGKSEVPEDLA
GFIELFQTPSHTKESMTNEKTTKVSYRASQPDLVDTPTSSKPQPKRSLRKADTEEEFLA
FRKQTPSAGKAMHTPKPAVGEEKDINTFLGTPVQKLDQPGNLPGSNRRLQTRKEKAQAL
EELTGFRELFQTPCTDNPTTDEKTTKKILCKSPQSDPADTPTNTKQRPKRSLKKADVEE
EFLAFRKLTPSAGKAMHTPKAAVGEEKDINTFVGTPVEKLDLLGNLPGSKRRPQTPKEK
AKALEDLAGFKELFQTPGHTEESMTDDKITEVSCKSPQPDPVKTPTSSKQRLKISLGKV
GVKEEVLPVGKLTQTSGKTTQTHRETAGDGKSIKAFKESAKQMLDPANYGTGMERWPRT
PKEEAQSLEDLAGFKELFQTPDHTEESTTDDKTTKIACKSPPPESMDTPTSTRRRPKTP
LGKRDIVEELSALKQLTQTHTDKVPGDEDKGINVFRETAKQKLDPAASVTGSKRQPRT
PKGKAQPLEDLAGLKELFQTPICTDKPTTHEKTTKIACRSPQPDPVGTPTIFKPQSKRS
LRKADVEEESLALRKRTPSVGKAMDTPKPAGGDEKDMKAFMGTPVQKLDLPGNLPGSKR
WPQTPKEKAQALEDLAGFKELFQTPGTDKPTTDEKTTKIACKSPQPDPVDTPASTKQRP
KRNLRKADVEEEFLALRKRTPSAGKAMDTPKPAVSDEKNINTFVETPVQKLDLLGNLPG
SKRQPQTPKEKAEALEDLVGFKELFQTPGHTEESMTDDKITEVSCKSPQPESFKTSRSS
KQRLKIPLVKVDMKEEPLAVSKLTRTSGETTQTHTEPTGDSKSIKAFKESPKQILDPAA
SVTGSRRQLRTRKEKARALEDLVDFKELFSAPGHTEESMTIDKNTKIPCKSPPPELTDT
ATSTKRCPKTRPRKEVKEELSAVERLTQTSGQSTHTHKEPASGDEGIKVLKQRAKKKPN
```

PVEEEPSRRRPRAPKEKAQPLEDLAGFTELSETSGHTQESLTAGKATKIPCESPPLEVV
DTTASTKRHLRTRVQKVQVKEEPSAVKFTQTSGETTDADKEPAGEDKGIKALKESAKQT
PAPAASVTGSRRRPRAPRESAQAIEDLAGFKDPAAGHTEESMTDDKTTKIPCKSSPELE
DTATSSKRRPRTRAQKVEVKEELLAVGKLTQTSGETTHTDKEPVGEGKGTKAFKQPAKR
KLDAEDVIGSRRQPRAPKEKAQPLEDLASFQELSQTPGHTEELANGAADSFTSAPKQTP
DSGKPLKISRRVLRAPKVEPVGDVVSTRDPVKSQSKSNTSLPPLPFKRGGGKDGSVTGT
KRLRCMPAPEEIVEELPASKKQRVAPRARGKSSEPVVIMKRSLRTSAKRIEPAEELNSN
DMKTNKEEHKLQDSVPENKGISLRSRRQNKTEAEQQITEVFVLAERIEINRNEKKPMKT
SPEMDIQNPDDGARKPIPRDKVTENKRCLRSARQNESSQPKVAEESGGQKSAKVLMQNQ
KGKGEAGNSDSMCLRSRKTKSQPAASTLESKSVQRVTRSVKRCAENPKKAEDNVCVKKI
RTRSHRDSEDI

**SEQ ID NO: 48 ADG116 HVR-H1**
YSISSGYHWSWI

**SEQ ID NO: 49 ADG116 HVR-H2**
LARIDWDDDKYYSTSLKSRL

**SEQ ID NO: 50 ADG116 HVR-H3**
ARSYVYFDY

**SEQ ID NO: 51 ADG116 HVR-L1**
RASQSVRGRFLA

**SEQ ID NO: 52 ADG116 HVR-L2**
DASNRATGI

**SEQ ID NO: 53 ADG116 HVR-L3**
YCQQSSSWPPT

**SEQ ID NO: 54 ADG116 VH**
EVQLVESGGGLVQPGGSLRLSCAASGYSISSGYHWSWIRQAPGKGLEWLARIDW
DDDKYYSTSLKSRLTISRDNSKNTLYLQLNSLRAEDTAVYYCARSYVYFDYWG
QGTLVTVSS

**SEQ ID NO: 55 ADG116 VL**
DIQLTQSPSSLSASVGDRVTITCRASQSVRGRFLAWYQQKPGKAPKLLIYDASNR
ATGIPSRFSGSGSGTDFTLTISSLQPEDFATYYCQQSSSWPPTFGQGTKVEIKR

**SEQ ID NO: 56 Atezolizumab HVR-H1**
GFTFSDSWIH

**SEQ ID NO: 57 Atezolizumab HVR-H2**

WISPYGGSTYYADSVKG

**SEQ ID NO: 58 Atezolizumab HVR-H3**
RHWPGGFDY

**SEQ ID NO: 59 Atezolizumab HVR-L1**
RASQDVSTAVA

**SEQ ID NO: 60 Atezolizumab HVR-L2**
SASFLYS

**SEQ ID NO: 61 Atezolizumab HVR-L3**
QQYLYHPAT

**SEQ ID NO: 62 Atezolizumab VH**
EVQLVESGGGLVQPGGSLRLSCAASGFTFSDSWIHWVRQAPGKGLEWVAWISPY
GGSTYYADSVKGRFTISADTSKNTAYLQMNSLRAEDTAVYYCARRHWPGGFDY
WGQGTLVTVSS

**SEQ ID NO: 63 Atezolizumab VL**
DIQMTQSPSSLSASVGDRVTITCRASQDVSTAVAWYQQKPGKAPKLLIYSASFLY
SGVPSRFSGSGSGTDFTLTISSLQPEDFATYYCQQYLYHPATFGQGTKVEIKRTV

**SEQ ID NO: 64 2E5 HVR-H1**
TYYIS

**SEQ ID NO: 65 2E5 HVR-H2**
YINMGSGGTNYNEKFK

**SEQ ID NO: 66 2E5 HVR-H3**
IGYFDY

**SEQ ID NO: 67 2E5 HVR-L1**
RSSQSLLDSDGGTYLY

**SEQ ID NO: 68 2E5 HVR-L2**
LVSTLGS

**SEQ ID NO: 69 2E5 HVR-L3**
MQLTHWPYT

**SEQ ID NO: 70 2E5 VH**
QVQLVQSGAEVKKPGSSVKVSCKASGFTFTTYYISWVRQAPGQGLEYLGYINMG
SGGTNYNEKFKGRVTITADKSTSTAYMELSSLRSEDTAVYYCAIIGYFDYWGQG

TMVTVSS

**SEQ ID NO: 71 2E5 VL**
DVVMTQSPLSLPVTLGQPASISCRSSQSLLDSDGGTYLYWFQQRPGQSPRRLIYL
VSTLGSGVPDRFSGSGSGTDFTLKISRVEAEDVGVYYCMQLTHWPYTFGQGTKL
EIK

**SEQ ID NO: 72 First epitope of anti-CD137L**
MEYASDASLDPEAPWPPAPRARACRVLP

**SEQ ID NO: 73 Second epitope of anti-CD137L**
MEYASDASLDPEAPWPPAPRARA

**SEQ ID NO: 74 TY23561 HVR-H1**
FSLSTSGVGVSWI

**SEQ ID NO: 75 TY23561 HVR-H2**
LALIDWAGDKYYSPSLKSRL

**SEQ ID NO: 76 TY23561 HVR-H3**
ARYGYSSYALDY

**SEQ ID NO: 77 TY23561 HVR-L1**
RASQSVRGSYLA

**SEQ ID NO: 78 TY23561 HVR-L2**
AASTLQSGV

**SEQ ID NO: 79 TY23561 HVR-L3**
YCQQYSSLWT

**SEQ ID NO: 80 TY23561 VH**
EVQLVESGGGLVQPGGSLRLSCAASGFSLSTSGVGVSWIRQAPGKGLEWLALIDWAGDK
YYSPSLKSRLTISRDNSKNTLYLQLNSLRAEDTAVYYCARYGYSSYALDYWGQGTLVTV
SS

**SEQ ID NO: 81 TY23561 VL**
DIQLTQSPSSLSASVGDRVTITCRASQSVRGSYLAWYQQKPGKAPKLLIYAASTLQSGVP
SRFSGSGSGTDFTLTISSLQPEDFATYYCQQYSSLWTFGQGTKVEIKR

**SEQ ID NO: 82 Toripalimab HVR-H1**
DYEMH

**SEQ ID NO: 83 Toripalimab HVR-H2**
VIESETGGTAYNQKFKG

**SEQ ID NO: 84 Toripalimab HVR-H3**
EGITTVATTYYWYFDV

**SEQ ID NO: 85 Toripalimab HVR-L1**
RSSQSIVHSNGNTYLE

**SEQ ID NO: 86 Toripalimab HVR-L2**
KVSNRFS

**SEQ ID NO: 87 Toripalimab HVR-L3**
FQGSHVPLT

**SEQ ID NO: 88 Toripalimab VH**
QGQLVQSGAEVKKPGASVKVSCKASGYTFTDYEMHWVRQAPIHGLEWIGVIESETGGTA
YNQKFKGRVTITADKSTSTAYMELSSLRSEDTAVYYCAREGITTVATTYYWYFDVWGQG
TTVTVSS

**SEQ ID NO: 89 Toripalimab VL**
DVVMTQSPLSLPVTLGQPASISCRSSQSIVHSNGNTYLEWYLQKPGQSPQLLIYKVSNR
FSGVPDRFSGSGSGTDFTLKISRVEAEDVGVYYCFQGSHVPLTFGQGTKLEIK

**ASPECTS OF THE INVENTION**

1. A method of treating a cancer in a subject, comprising administering to the subject an effective amount of an anti-CD137 antibody that specifically binds to an extracellular domain of human CD137, wherein the antibody binds to one or more amino acid residues selected from the group consisting of amino acid residues 51, 53, 62-73, 83, 89, 92, 95-104 and 112-116 of SEQ ID NO: 1, and wherein the anti-CD137 antibody is administered at a dose of no more than 500 mg.

2. A method of treating a cancer in a subject, comprising administering to the subject an effective amount of an anti-CD137 antibody that specifically binds to an extracellular domain of human CD137, wherein the antibody binds to one or more amino acid residues selected from the group consisting of amino acid residues 51, 53, 62-73, 83, 89, 92, 95-104 and 112-116 of SEQ ID NO: 1, and wherein the anti-CD137 antibody is administered at a dose of no more than 10mg/kg.

3. A method of treating a cancer in a subject, comprising administering to the subject an effective amount of an anti-CD137 antibody that specifically binds to an extracellular domain of human CD137, wherein the antibody binds to one or more amino acid residues selected from the group consisting of amino acid residues 51, 53, 62-73, 83, 89, 92, 95-104 and 112-116 of SEQ ID NO: 1, and wherein the cancer is resistant or refractory to a prior therapy.

4. The method of aspect 3, wherein the prior therapy is treatment with an anti-CD20 antibody.

5. The method of aspect 4, wherein the anti-CD20 antibody is rituximab.

6. A method of treating a cancer in a subject, comprising administering to the subject an effective amount of an anti-CD137 antibody that specifically binds to an extracellular domain of human CD137, wherein the antibody binds to one or more amino acid residues selected from the group consisting of amino acid residues 51, 53, 62-73, 83, 89, 92, 95-104 and 112-116 of SEQ ID NO: 1; and wherein the subject has a high level in one or more biomarkers selected from the group consisting of total CD137, membrane bound CD137 (mCD137), CD137 ligand (CD137L), and PD-L1 and/or a low level of CD8+ effector memory T ($T_{em}$) cells or natural killer (NK) cells compared to a reference level.

7. A method of treating a cancer in a subject, comprising: (a) administering to the subject an effective amount of an anti-CD137 antibody that specifically binds to an extracellular domain of human CD137, wherein the antibody binds to one or more amino acid residues selected from the group consisting of amino acid residues 51, 53, 62-73, 83, 89, 92, 95-104 and 112-116 of SEQ ID NO: 1; and (b) subsequently determining a level of one or more biomarkers selected from the group consisting of total CD137, membrane bound (mCD137), soluble CD137 (sCD137), CD137L, Ki67, CD8+ effector memory T ($T_{em}$) cells, regulatory T ($T_{reg}$) cells, and NK cells in a sample of the subject.

8. The method of aspect 7, wherein an increased level of one or more biomarkers selected from the group consisting of total CD137, sCD137, Ki67, CD137L, NK cells and CD8 $T_{em}$ cells, and/or a decreased level of one or more biomarkers selected from the group consisting of mCD137 and $T_{reg}$ cells after administration of the anti-CD137 antibody compared to the level of the one or more biomarkers before administration of the anti-CD137 antibody indicates that the subject may benefit from the administration of the anti-CD137 antibody.

9. The method of aspect 7 or 8, wherein the sample has an increased level of one or more biomarkers selected from the group consisting of total CD137, sCD137, Ki67, CD137L, NK cells and CD8 $T_{em}$ cells, and/or a decreased level of one or more biomarkers selected from the group consisting of mCD137 and $T_{reg}$ cells after administration of the anti-CD137 antibody compared to the level of the one or more biomarkers before administration of the anti-CD137 antibody, the method further comprises administering to the subject an effective amount of the anti-CD137 antibody.

10. A method of providing a prognosis for a subject who has been administered with an effective amount of an anti-CD137 antibody that specifically binds to an extracellular domain of human CD137, wherein the antibody binds to one or more amino acid residues selected from the group consisting of amino acid residues 51, 53, 62-73, 83, 89, 92, 95-104 and 112-116 of SEQ ID NO: 1; the method comprising determining a level of one or more biomarkers selected from the group consisting of total CD137, membrane bound (mCD137), soluble CD137 (sCD137), Ki67, CD137L, NK cells, CD8+ effector memory T ($T_{em}$) cells, and regulatory T ($T_{reg}$) cells in a sample of the subject, wherein an increased level of one or more biomarkers selected from the group consisting of total CD137, sCD137, Ki67, CD137L, NK cells and CD8 $T_{em}$ cells, and/or a decreased level of one or more biomarkers selected from the group consisting of mCD137 and $T_{reg}$ cells after administration of the anti-CD137 antibody compared to the level of the one or more biomarkers before administration of the anti-CD137 antibody identifies the subject as having a high likelihood of responding to the anti-CD137 antibody treatment.

11. The method of any one of aspects 6-10, wherein the level of one or more biomarkers comprise a level of total CD137.

12. The method of any one of aspects 6-11, wherein the level of one or more biomarkers comprises a level of sCD137 in a plasma sample.

13. The method of any one of aspects 6-12, wherein the level of one or more biomarkers comprises a level of mCD137 on CD8$^+$ T cells.

14. The method of any one of aspects 6-13, wherein the level of one or more biomarkers comprises a level of Ki67 on CD8$^+$ T cells.

15. The method of aspect 13 or 14, wherein the CD8$^+$ T cells are tumor infiltrating T cells.

16. The method of any one of aspects 6-15, wherein the level of one or more biomarkers comprises a level of CD137L.

17. The method of any one of aspects 5-16, wherein the level of one or more biomarkers comprises a level of NK cells in a blood sample.

18. The method of any one of aspects 11 and 13-16, wherein the sample is a tumor biopsy sample.

19. The method of aspect 18, wherein level of the one or more biomarkers is detected by immunohistochemistry (IHC).

20. The method of any one of aspects 1-19, wherein the cancer is solid cancer.

21. The method of any one of aspects 1-20, wherein the cancer is selected from the group consisting of colon cancer, breast cancer, lung cancer, esophageal cancer, endometrial cancer, gastrointestinal cancer, cholangiocarcinoma, nasopharyngeal cancer (NPC), adenoid cystic carcinoma (ACC), melanoma, mesothelioma, mantle cell lymphoma, T cell lymphoma, anal cancer, head and neck cancer, and appendiceal and sebaceous cancer.

22. The method of any one of aspects 1-19, wherein the cancer is a liquid cancer.

23. The method of aspect 22, wherein the cancer is non-Hodgkin's lymphoma.

24. A method of treating a cancer in a subject, comprising administering to the subject an effective amount of an anti-CD137 antibody that specifically binds to an extracellular domain of human CD137, wherein the antibody binds to one or more amino acid residues selected from the group consisting of amino acid residues 51, 53, 62-73, 83, 89, 92, 95-104 and 112-116 of SEQ ID NO: 1, and wherein the cancer is selected from the group consisting of follicular lymphoma, T cell lymphoma and ACC.

25. The method of aspect 24, wherein the cancer is follicular lymphoma.

26. The method of aspect 24 or 25, wherein the cancer is T cell lymphoma.

27. The method of aspect 26, wherein the cancer is angioimmunoblastic T-cell lymphoma (AITL) or Peripheral T-cell lymphoma (PTCL).

28. The method of aspect 24, wherein the cancer is ACC.

29. A method of treating a lung cancer in a subject, comprising administering to the subject: (a) an effective amount of an anti-CD137 antibody that specifically binds to an extracellular domain of human CD137, wherein the antibody binds to one or more amino acid residues selected from the group consisting of amino acid residues 51, 53, 62-73, 83, 89, 92, 95-104 and 112-116 of SEQ ID NO: 1; and (b) an effective amount of an immune checkpoint inhibitor.

30. A method of treating a breast cancer in a subject, comprising administering to the subject: (a) an effective amount of an anti-CD137 antibody that specifically binds to an extracellular domain of human CD137, wherein the antibody binds to one or more amino acid residues selected from the group consisting of amino acid residues 51, 53, 62-73, 83, 89, 92, 95-104 and 112-116 of SEQ ID NO: 1; and (b) an effective amount of an immune checkpoint inhibitor.

31. The method of aspect 30, wherein the breast cancer is triple-negative breast cancer.

32. The method of any one of aspects 29-31, wherein the immune checkpoint inhibitor is an anti-PD-L1 antibody.

33. The method of any one of aspects 29-31, wherein the immune checkpoint inhibitor is an anti-PD-1 antibody.

34. The method of aspect 33, wherein the anti-PD-1 antibody is toripalimab.

35. The method of any one of aspects 29-31, wherein the immune checkpoint inhibitor is an anti-CTLA-4 antibody.

36. A method of treating a lung cancer in a subject, comprising administering to the subject: (a) an effective amount of an anti-CD137 antibody that specifically binds to an extracellular domain of human CD137, wherein the antibody binds to one or more amino acid residues selected from the group consisting of amino acid residues 51, 53, 62-73, 83, 89, 92, 95-104 and 112-116 of SEQ ID NO: 1; and (b) an effective amount of a chemotherapeutic agent.

37. A method of treating a breast cancer in a subject, comprising administering to the subject: (a) an effective amount of an anti-CD137 antibody that specifically binds to an extracellular domain of human CD137, wherein the antibody binds to one or more amino acid residues selected from the group consisting of amino acid residues 51, 53, 62-73, 83, 89, 92, 95-104 and 112-116 of SEQ ID NO: 1; and (b) an effective amount of a chemotherapeutic agent.

38. The method of aspect 37, wherein the breast cancer is triple-negative breast cancer.

39. The method of any one of aspects 36-38, wherein the chemotherapeutic agent is docetaxel.

40. The method of any one of aspects 36-38, wherein the chemotherapeutic agent is cisplatin.

41. A method of treating a lung cancer in a subject, comprising administering to the subject: (a) an effective amount of an anti-CD137 antibody that specifically binds to an extracellular domain of human CD137, wherein the antibody binds to one or more amino acid residues selected from the group consisting of amino acid residues 51, 53, 62-73, 83, 89, 92, 95-104 and 112-116 of SEQ ID NO: 1; and (b) an effective amount of an anti-CD20 antibody.

42. The method of aspect 41, wherein the anti-CD20 antibody is rituximab.

43. A method of treating a colon cancer in a subject, comprising administering to the subject: (a) an effective amount of an anti-CD137 antibody that specifically binds to an extracellular domain of human CD137, wherein the antibody binds to one or more amino acid residues selected from the group consisting of amino acid residues 51, 53, 62-73, 83, 89, 92, 95-104 and 112-116 of SEQ ID NO: 1; and (b) an effective amount of a radiation therapy.

44. The method of any one of aspects 1-43, wherein the anti-CD137 antibody is administered at a dose of about 50 mg to about 400 mg.

45. The method of aspect 44, wherein the anti-CD137 antibody is administered at a dose of 50 mg, 100 mg, 200 mg, 300 mg or 400 mg.

46. The method of any one of aspects 1-45, wherein the anti-CD137 antibody is administered at a dose of about 0.1 mg/kg to about 10 mg/kg.

47. The method of aspect 46, wherein the anti-CD137 antibody is administered at a dose of about 3 mg/kg to about 8 mg/kg.

48. The method of aspect 47, wherein the anti-CD137 antibody is administered at a dose of about 3mg/kg or about 5 mg/kg.

49. The method of any one of aspects 1-48, wherein the anti-CD137 antibody is administered intravenously.

50. The method of any one of aspects 1-49, wherein the anti-CD137 antibody is administered about once every three weeks.

51. The method of any one of aspects 1-50, wherein the subject receives at least 2 cycles of treatment with the anti-CD137 antibody.

52. The method of any one of aspects 1-51, wherein the cancer is advanced-stage cancer.

53. The method of any one of aspects 1-52, wherein the cancer is metastatic cancer.

54. The method of any one of aspects 1-53, wherein the cancer is resistant or refractory to a prior therapy.

55. The method of aspect 54, wherein the prior therapy is selected from the group consisting of viral gene therapy, immunotherapy, targeted therapy, radiation therapy, and chemotherapy.

56. The method of any one of aspects 1-55, wherein the anti-CD137 antibody is cross-reactive with a CD137 polypeptide from at least one non-human species selected from the group consisting of cynomolgus monkey, mouse, rat and dog.

57. The method of any one of aspects 1-56, wherein the anti-CD137 antibody binds to amino acid residues 51, 63-67, 69-73, 83, 89, 92, 98-104 and 112-114 of SEQ ID NO: 1.

58. The method of any one of aspects 1-57, wherein the anti-CD137 antibody comprises a heavy chain variable region (VH) and a light chain variable region (VL), wherein the VH comprises a HVR-H1 comprising the amino acid sequence of SEQ ID NO: 2, a HVR-H2 comprising the amino acid sequence of SEQ ID NO: 3, and a HVR-H3 comprising the amino acid sequence of SEQ ID NO: 4; and wherein the VL comprises a HVR-L1 comprising the amino acid sequence of SEQ ID NO: 5, a HVR-L2 comprising the amino acid sequence of SEQ ID NO: 6, and a HVR-L3 comprising the amino acid sequence of SEQ ID NO: 7.

59. The method of aspect 58, wherein the VH comprises the amino acid sequence of SEQ ID NO: 8, and/or the VL comprises the amino acid sequence of SEQ ID NO: 9.

60. The method of aspect 59, wherein the antibody comprises a heavy chain and a light chain, and wherein the heavy chain comprises the amino acid sequence of SEQ ID NO: 10, and/or the light chain comprises the amino acid sequence of SEQ ID NO: 11.

61. The method of any one of aspects 1-57, wherein the anti-CD137 antibody comprises a VH and a VL, wherein the VH comprises a HVR-H1 comprising the amino acid sequence of SEQ ID NO: 12, a HVR-H2 comprising the amino acid sequence of SEQ ID NO: 13, and a HVR-H3 comprising the amino acid sequence of SEQ ID NO: 14; and wherein the VL comprises a HVR-L1 comprising the amino acid sequence of SEQ ID NO: 15, a HVR-L2 comprising the amino acid sequence of SEQ ID NO: 16, and a HVR-L3 comprising the amino acid sequence of SEQ ID NO: 17.

62. The method of aspect 61, wherein the VH comprises the amino acid sequence of SEQ ID NO: 18, and/or the VL comprises the amino acid sequence of SEQ ID NO: 19.

63. The method of aspect 62, wherein the antibody comprises a heavy chain and a light chain, wherein the heavy chain comprises the amino acid sequence of SEQ ID NO: 20, and/or the light chain comprises the amino acid sequence of SEQ ID NO: 21.

64. The method of any one of aspects 1-57, wherein the anti-CD137 antibody comprises a VH and a VL, wherein the VH comprises a HVR-H1 comprising the amino acid sequence of SEQ ID NO: 22, a HVR-H2 comprising the amino acid sequence of SEQ ID NO: 23, and a HVR-H3 comprising the amino acid sequence of SEQ ID NO: 24; and wherein the VL comprises a HVR-L1 comprising the amino acid sequence of SEQ ID NO: 25, a HVR-L2 comprising the amino acid sequence of SEQ ID NO: 26, and a HVR-L3 comprising the amino acid sequence of SEQ ID NO: 27.

65. The method of aspect 64, wherein the VH comprises the amino acid sequence of SEQ ID NO: 28, and/or the VL comprises the amino acid sequence of SEQ ID NO: 29.

66. The method of aspect 65, wherein the antibody comprises a heavy chain and a light chain, wherein the heavy chain comprises the amino acid sequence of SEQ ID NO: 30, and/or the light chain comprises the amino acid sequence of SEQ ID NO: 31.

67. The method of any one of aspects 1-66, wherein the anti-CD137 antibody comprises a human IgG4 Fc region.

68. The method of aspect 67, wherein the human IgG4 Fc region comprises an S241P mutation, wherein numbering is according to Kabat.

69. The method of any one of aspects 1-68, wherein the subject is a human subject.

70. The method of any one of aspects 1-69, further comprising administering to the subject a therapeutically effective amount of at least one additional therapeutic agent.

**Claims**

1. An anti-CD137 antibody for use in a method of treating a cancer in a human subject, the method comprising administering to the subject an effective amount of the anti-CD137 antibody, wherein the anti-CD137 antibody specifically binds to an extracellular domain of human CD137, wherein the anti-CD137 antibody comprises a heavy chain variable region (VH) and a light chain variable region (VL), wherein:

a) the VH comprises a HVR-H1 comprising the amino acid sequence of SEQ ID NO: 2, a HVR-H2 comprising the amino acid sequence of SEQ ID NO: 3, and a HVR-H3 comprising the amino acid sequence of SEQ ID NO: 4; and wherein the VL comprises a HVR-L1 comprising the amino acid sequence of SEQ ID NO: 5, a HVR-L2 comprising the amino acid sequence of SEQ ID NO: 6, and a HVR-L3 comprising the amino acid sequence of SEQ ID NO: 7;

b) the VH comprises a HVR-H1 comprising the amino acid sequence of SEQ ID NO: 12, a HVR-H2 comprising the amino acid sequence of SEQ ID NO: 13, and a HVR-H3 comprising the amino acid sequence of SEQ ID NO: 14; and wherein the VL comprises a HVR-L1 comprising the amino acid sequence of SEQ ID NO: 15, a HVR-L2 comprising the amino acid sequence of SEQ ID NO: 16, and a HVR-L3 comprising the amino acid sequence of SEQ ID NO: 17; or

c) the VH comprises a HVR-H1 comprising the amino acid sequence of SEQ ID NO: 22, a HVR-H2 comprising the amino acid sequence of SEQ ID NO: 23, and a HVR-H3 comprising the amino acid sequence of SEQ ID NO: 24; and wherein the VL comprises a HVR-L1 comprising the amino acid sequence of SEQ ID NO: 25, a HVR-L2 comprising the amino acid sequence of SEQ ID NO: 26, and a HVR-L3 comprising the amino acid sequence of SEQ ID NO: 27and wherein the anti-CD137 antibody is administered at a dose of no more than 500 mg.

2. The anti-CD137 antibody for use in claim 1, wherein the cancer is solid cancer.

3. The anti-CD137 antibody for use in claim 2, wherein the cancer is selected from the group consisting of colon cancer, breast cancer, lung cancer, esophageal cancer, endometrial cancer, gastrointestinal cancer, cholangiocarcinoma, nasopharyngeal cancer (NPC), adenoid cystic carcinoma (ACC), melanoma, mesothelioma, mantle cell lymphoma, T cell lymphoma, anal cancer, head and neck cancer, and appendiceal and sebaceous cancer.

4. The anti-CD137 antibody for use in claim 1, wherein the cancer is a liquid cancer.

5. The anti-CD137 antibody for use in claim 4, wherein the cancer is non-Hodgkin's lymphoma.

6. The anti-CD137 antibody for use in any one of claims 1-5, wherein the anti-CD137 antibody is administered at a dose of about 50 mg to about 400 mg.

7. The anti-CD137 antibody for use in claim 6, wherein the anti-CD137 antibody is administered at a dose of 50 mg, 100 mg, 200 mg, 300 mg, or 400 mg.

8. The anti-CD137 antibody for use in any one of claims 1-7, wherein the subject has a body weight of no more than 100kg.

9. The anti-CD137 antibody for use in any one of claims 1-8, wherein:

   a) the anti-CD137 antibody is administered intravenously;
   b) the anti-CD137 antibody is administered about once every three weeks; and/or
   c) the subject receives at least 2 cycles of treatment with the anti-CD137 antibody.

10. The anti-CD137 antibody for use in any one of claims 1-9, wherein the cancer is:

   a) advanced-stage cancer;
   b) metastatic cancer; and/or
   c) resistant or refractory to a prior therapy, optionally wherein the prior therapy is selected from the group consisting of viral gene therapy, immunotherapy, targeted therapy, radiation therapy, and chemotherapy.

11. The anti-CD137 antibody for use in any one of claims 1-10, wherein the anti-CD137 antibody is cross-reactive with a CD137 polypeptide from at least one non-human species selected from the group consisting of cynomolgus monkey, mouse, rat and dog.

12. The anti-CD137 antibody for use in claim 1, wherein:

   a) the VH comprises the amino acid sequence of SEQ ID NO: 8, and the VL comprises the amino acid sequence of SEQ ID NO: 9;
   b) the VH comprises the amino acid sequence of SEQ ID NO: 18, and the VL comprises the amino acid sequence of SEQ ID NO: 19; or
   c) the VH comprises the amino acid sequence of SEQ ID NO: 28, and the VL comprises the amino acid sequence of SEQ ID NO: 29.

13. The anti-CD137 antibody for use in claim 12, wherein the antibody comprises a heavy chain and a light chain, and wherein:

   a) the heavy chain comprises the amino acid sequence of SEQ ID NO: 10, and the light chain comprises the amino acid sequence of SEQ ID NO: 11;
   b) the heavy chain comprises the amino acid sequence of SEQ ID NO: 20, and the light chain comprises the amino acid sequence of SEQ ID NO: 21; or
   c) the heavy chain comprises the amino acid sequence of SEQ ID NO: 30, and the light chain comprises the amino acid sequence of SEQ ID NO: 31.

**14.** The anti-CD137 antibody for use in any one of claims 1-13, wherein the anti-CD137 antibody comprises a human IgG4 Fc region, optionally wherein the human IgG4 Fc region comprises an S241P mutation, wherein numbering is according to Kabat.

**15.** The anti-CD137 antibody for use in any one of claims 1-14, wherein the method further comprises administering to the subject a therapeutically effective amount of at least one additional therapeutic agent.

| structure region | | | | | | | | | | | | | | CRD1 | | | | | | | | | |
|---|---|---|---|---|---|---|---|---|---|---|---|---|---|---|---|---|---|---|---|---|---|---|---|
| number | 23 | 24 | 25 | 26 | 27 | 28 | 29 | 30 | 31 | 32 | 33 | 34 | 35 | 36 | 37 | 38 | 39 | 40 | 41 | 42 | 43 | 44 | 45 |
| 6BWV(CD137-CD137L) 5 Å | | | | D | P | C | S | N | C | P | A | G | T | F | C | D | N | N | R | N | Q | I | C |
| 6BWV(CD137-CD137L) 4.5 Å | | | | D | P | C | S | N | C | P | A | G | T | F | C | D | N | N | R | N | Q | I | C |
| 6BWV(CD137-CD137L) 4 Å | | | | D | P | C | S | N | C | P | A | G | T | F | C | D | N | N | R | N | Q | I | C |
| CD137-ADG106 5 Å | S | L | Q | D | P | C | S | N | C | P | A | G | T | F | C | D | N | N | R | N | Q | I | C |
| CD137-ADG106 4.5 Å | S | L | Q | D | P | C | S | N | C | P | A | G | T | F | C | D | N | N | R | N | Q | I | C |
| CD137-ADG106 4 Å | S | L | Q | D | P | C | S | N | C | P | A | G | T | F | C | D | N | N | R | N | Q | I | C |
| 6A3W(CD137-utomilumab) 5 Å | | | | D | P | C | S | N | C | P | A | G | T | F | C | D | N | N | R | N | Q | I | C |
| 6A3W(CD137-utomilumab) 4.5 Å | | | | D | P | C | S | N | C | P | A | G | T | F | C | D | N | N | R | N | Q | I | C |
| 6A3W(CD137-utomilumab) 4 Å | | | | D | P | C | S | N | C | P | A | G | T | F | C | D | N | N | R | N | Q | I | C |
| 6MHR(CD137-urelumab) 5 Å | | | Q | D | P | C | S | N | C | P | A | G | T | F | C | D | N | N | R | N | Q | I | C |
| 6MHR(CD137-urelumab) 4.5 Å | | | Q | D | P | C | S | N | C | P | A | G | T | F | C | D | N | N | R | N | Q | I | C |
| 6MHR(CD137-urelumab) 4 Å | | | Q | D | P | C | S | N | C | P | A | G | T | F | C | D | N | N | R | N | Q | I | C |

FIG. 1A

| structure region | CRD2 | | | | | | | | | | | | | | | | | | | | | | | | | | | |
|---|---|---|---|---|---|---|---|---|---|---|---|---|---|---|---|---|---|---|---|---|---|---|---|---|---|---|---|---|
| number | 46 | 47 | 48 | 49 | 50 | 51 | 52 | 53 | 54 | 55 | 56 | 57 | 58 | 59 | 60 | 61 | 62 | 63 | 64 | 65 | 66 | 67 | 68 | 69 | 70 | 71 | 72 | 73 |
| 6BWV(CD137-CD137L) 5 Å | S | P | C | P | P | N | S | F | S | S | A | G | G | Q | R | T | C | D | I | C | R | Q | C | K | G | V | F | R |
| 6BWV(CD137-CD137L) 4.5 Å | S | P | C | P | P | N | S | F | S | S | A | G | G | Q | R | T | C | D | I | C | R | Q | C | K | G | V | F | R |
| 6BWV(CD137-CD137L) 4 Å | S | P | C | P | P | N | S | F | S | S | A | G | G | Q | R | T | C | D | I | C | R | Q | C | K | G | V | F | R |
| CD137-ADG106 5 Å | S | P | C | P | P | N | S | F | S | S | A | G | G | Q | R | T | C | D | I | C | R | Q | C | K | G | V | F | R |
| CD137-ADG106 4.5 Å | S | P | C | P | P | N | S | F | S | S | A | G | G | Q | R | T | C | D | I | C | R | Q | C | K | G | V | F | R |
| CD137-ADG106 4 Å | S | P | C | P | P | N | S | F | S | S | A | G | G | Q | R | T | C | D | I | C | R | Q | C | K | G | V | F | R |
| 6A3W(CD137-utomilumab) 5 Å | S | P | C | P | P | N | S | F | S | S | A | G | G | Q | R | T | C | D | I | C | R | Q | C | K | G | V | F | R |
| 6A3W(CD137-utomilumab) 4.5 Å | S | P | C | P | P | N | S | F | S | S | A | G | G | Q | R | T | C | D | I | C | R | Q | C | K | G | V | F | R |
| 6A3W(CD137-utomilumab) 4 Å | S | P | C | P | P | N | S | F | S | S | A | G | G | Q | R | T | C | D | I | C | R | Q | C | K | G | V | F | R |
| 6MHR(CD137-urelumab) 5 Å | S | P | C | P | P | N | S | F | S | S | A | G | G | Q | R | T | C | D | I | C | R | Q | C | K | G | V | F | R |
| 6MHR(CD137-urelumab) 4.5 Å | S | P | C | P | P | N | S | F | S | S | A | G | G | Q | R | T | C | D | I | C | R | Q | C | K | G | V | F | R |
| 6MHR(CD137-urelumab) 4 Å | S | P | C | P | P | N | S | F | S | S | A | G | G | Q | R | T | C | D | I | C | R | Q | C | K | G | V | F | R |

| structure region | CRD2 | | | | | | | | | | | | |
|---|---|---|---|---|---|---|---|---|---|---|---|---|---|
| number | 74 | 75 | 76 | 77 | 78 | 79 | 80 | 81 | 82 | 83 | 84 | 85 | 86 |
| 6BWV(CD137-CD137L) 5 Å | T | R | K | E | C | S | S | T | S | N | A | E | C |
| 6BWV(CD137-CD137L) 4.5 Å | T | R | K | E | C | S | S | T | S | N | A | E | C |
| 6BWV(CD137-CD137L) 4 Å | T | R | K | E | C | S | S | T | S | N | A | E | C |
| CD137-ADG106 5 Å | T | R | K | E | C | S | S | T | S | N | A | E | C |
| CD137-ADG106 4.5 Å | T | R | K | E | C | S | S | T | S | N | A | E | C |
| CD137-ADG106 4 Å | T | R | K | E | C | S | S | T | S | N | A | E | C |
| 6A3W(CD137-utomilumab) 5 Å | T | R | K | E | C | S | S | T | S | N | A | E | C |
| 6A3W(CD137-utomilumab) 4.5 Å | T | R | K | E | C | S | S | T | S | N | A | E | C |
| 6A3W(CD137-utomilumab) 4 Å | T | R | K | E | C | S | S | T | S | N | A | E | C |
| 6MHR(CD137-urelumab) 5 Å | T | R | K | E | C | S | S | T | S | N | A | E | C |
| 6MHR(CD137-urelumab) 4.5 Å | T | R | K | E | C | S | S | T | S | N | A | E | C |
| 6MHR(CD137-urelumab) 4 Å | T | R | K | E | C | S | S | T | S | N | A | E | C |

FIG. 1B

| structure region | | | | | | | | | | | CRD3 | | | | | | | | | | |
|---|---|---|---|---|---|---|---|---|---|---|---|---|---|---|---|---|---|---|---|---|---|
| number | 87 | 88 | 89 | 90 | 91 | 92 | 93 | 94 | 95 | 96 | 97 | 98 | 99 | 100 | 101 | 102 | 103 | 104 | 105 | 106 | 107 | 108 |
| 6BWV(CD137-CD137L) 5 Å | D | C | T | P | G | F | H | C | L | G | A | G | C | S | M | C | E | Q | D | C | K | Q |
| 6BWV(CD137-CD137L) 4.5 Å | D | C | T | P | G | F | H | C | L | G | A | G | C | S | M | C | E | Q | D | C | K | Q |
| 6BWV(CD137-CD137L) 4 Å | D | C | T | P | G | F | H | C | L | G | A | G | C | S | M | C | E | Q | D | C | K | Q |
| CD137-ADG106 5 Å | D | C | T | P | G | F | H | C | L | G | A | G | C | S | M | C | E | Q | D | C | K | Q |
| CD137-ADG106 4.5 Å | D | C | T | P | G | F | H | C | L | G | A | G | C | S | M | C | E | Q | D | C | K | Q |
| CD137-ADG106 4 Å | D | C | T | P | G | F | H | C | L | G | A | G | C | S | M | C | E | Q | D | C | K | Q |
| 6A3W(CD137-utomilumab) 5 Å | D | C | T | P | G | F | H | C | L | G | A | G | C | S | M | C | E | Q | D | C | K | Q |
| 6A3W(CD137-utomilumab) 4.5 Å | D | C | T | P | G | F | H | C | L | G | A | G | C | S | M | C | E | Q | D | C | K | Q |
| 6A3W(CD137-utomilumab) 4 Å | D | C | T | P | G | F | H | C | L | G | A | G | C | S | M | C | E | Q | D | C | K | Q |
| 6MHR(CD137-urelumab) 5 Å | D | C | T | P | G | F | H | C | L | G | A | G | C | S | M | C | E | Q | D | C | K | Q |
| 6MHR(CD137-urelumab) 4.5 Å | D | C | T | P | G | F | H | C | L | G | A | G | C | S | M | C | E | Q | D | C | K | Q |
| 6MHR(CD137-urelumab) 4 Å | D | C | T | P | G | F | H | C | L | G | A | G | C | S | M | C | E | Q | D | C | K | Q |

| structure region | | CRD3 | | | | | | | | |
|---|---|---|---|---|---|---|---|---|---|---|
| number | 109 | 110 | 111 | 112 | 113 | 114 | 115 | 116 | 117 | 118 |
| 6BWV(CD137-CD137L) 5 Å | G | Q | E | L | T | K | K | G | C | K |
| 6BWV(CD137-CD137L) 4.5 Å | G | Q | E | L | T | K | K | G | C | K |
| 6BWV(CD137-CD137L) 4 Å | G | Q | E | L | T | K | K | G | C | K |
| CD137-ADG106 5 Å | G | Q | E | L | T | K | K | G | C | K |
| CD137-ADG106 4.5 Å | G | Q | E | L | T | K | K | G | C | K |
| CD137-ADG106 4 Å | G | Q | E | L | T | K | K | G | C | K |
| 6A3W(CD137-utomilumab) 5 Å | G | Q | E | L | T | K | K | G | C | K |
| 6A3W(CD137-utomilumab) 4.5 Å | G | Q | E | L | T | K | K | G | C | K |
| 6A3W(CD137-utomilumab) 4 Å | G | Q | E | L | T | K | K | G | C | K |
| 6MHR(CD137-urelumab) 5 Å | G | Q | E | L | T | K | K | G | C | K |
| 6MHR(CD137-urelumab) 4.5 Å | G | Q | E | L | T | K | K | G | C | K |
| 6MHR(CD137-urelumab) 4 Å | G | Q | E | L | T | K | K | G | C | K |

FIG. 1C

EP 4 559 477 A2

| structure region | | | | | | | | | | | | | | | | | | | | | | | CRD4 |
|---|---|---|---|---|---|---|---|---|---|---|---|---|---|---|---|---|---|---|---|---|---|---|---|
| number | 119 | 120 | 121 | 122 | 123 | 124 | 125 | 126 | 127 | 128 | 129 | 130 | 131 | 132 | 133 | 134 | 135 | 136 | 137 | 138 | 139 | 140 | 141 | 142 |
| 6BWV(CD137-CD137L) 5 Å | D | C | S | F | G | T | F | N | D | Q | K | R | G | I | C | R | P | W | T | D | C | S | L | D |
| 6BWV(CD137-CD137L) 4.5 Å | D | C | S | F | G | T | F | N | D | Q | K | R | G | I | C | R | P | W | T | D | C | S | L | D |
| 6BWV(CD137-CD137L) 4 Å | D | C | S | F | G | T | F | N | D | Q | K | R | G | I | C | R | P | W | T | D | C | S | L | D |
| CD137-ADG106 5 Å | D | C | S | F | G | T | F | N | D | Q | K | R | G | I | C | R | P | W | T | D | C | S | L | D |
| CD137-ADG106 4.5 Å | D | C | S | F | G | T | F | N | D | Q | K | R | G | I | C | R | P | W | T | D | C | S | L | D |
| CD137-ADG106 4 Å | D | C | S | F | G | T | F | N | D | Q | K | R | G | I | C | R | P | W | T | D | C | S | L | D |
| 6A3W(CD137-utomilumab) 5 Å | D | C | C | F | G | T | F | N | D | Q | K | R | G | I | C | R | P | W | T | N | C | S | L | D |
| 6A3W(CD137-utomilumab) 4.5 Å | D | C | C | F | G | T | F | N | D | Q | K | R | G | I | C | R | P | W | T | N | C | S | L | D |
| 6A3W(CD137-utomilumab) 4 Å | D | C | C | F | G | T | F | N | D | Q | K | R | G | I | C | R | P | W | T | N | C | S | L | D |
| 6MHR(CD137-urelumab) 5 Å | D | C | C | F | G | T | F | N | D | Q | K | R | G | I | C | R | P | W | T | N | C | S | L | D |
| 6MHR(CD137-urelumab) 4.5 Å | D | C | C | F | G | T | F | N | D | Q | K | R | G | I | C | R | P | W | T | N | C | S | L | D |
| 6MHR(CD137-urelumab) 4 Å | D | C | C | F | G | T | F | N | D | Q | K | R | G | I | C | R | P | W | T | N | C | S | L | D |

| structure region | CRD4 | | | | | | | | | | | | | | | | | | | | | | |
|---|---|---|---|---|---|---|---|---|---|---|---|---|---|---|---|---|---|---|---|---|---|---|---|
| number | 143 | 144 | 145 | 146 | 147 | 148 | 149 | 150 | 151 | 152 | 153 | 154 | 155 | 156 | 157 | 158 | 159 | 160 | 161 | 162 | 163 | 164 | 165 |
| 6BWV(CD137-CD137L) 5 Å | G | K | S | V | L | V | Q | G | T | K | E | R | D | V | V | C | G | P | | | | | |
| 6BWV(CD137-CD137L) 4.5 Å | G | K | S | V | L | V | Q | G | T | K | E | R | D | V | V | C | G | P | | | | | |
| 6BWV(CD137-CD137L) 4 Å | G | K | S | V | L | V | Q | G | T | K | E | R | D | V | V | C | G | P | | | | | |
| CD137-ADG106 5 Å | G | K | S | V | L | V | Q | G | T | K | E | R | D | V | V | C | G | P | | | | | |
| CD137-ADG106 4.5 Å | G | K | S | V | L | V | Q | G | T | K | E | R | D | V | V | C | G | P | | | | | |
| CD137-ADG106 4 Å | G | K | S | V | L | V | Q | G | T | K | E | R | D | V | V | C | G | P | | | | | |
| 6A3W(CD137-utomilumab) 5 Å | G | K | S | V | L | V | N | G | T | K | E | R | D | V | V | C | G | P | | | | | |
| 6A3W(CD137-utomilumab) 4.5 Å | G | K | S | V | L | V | N | G | T | K | E | R | D | V | V | C | G | P | | | | | |
| 6A3W(CD137-utomilumab) 4 Å | G | K | S | V | L | V | N | G | T | K | E | R | D | V | V | C | G | P | | | | | |
| 6MHR(CD137-urelumab) 5 Å | G | K | S | V | L | V | N | G | T | K | E | R | D | V | V | C | G | P | S | P | E | N | L |
| 6MHR(CD137-urelumab) 4.5 Å | G | K | S | V | L | V | N | G | T | K | E | R | D | V | V | C | G | P | S | P | E | N | L |
| 6MHR(CD137-urelumab) 4 Å | G | K | S | V | L | V | N | G | T | K | E | R | D | V | V | C | G | P | S | P | E | N | L |

FIG. 1D

Time on Treatment and Duration of Response

FIG. 2

FIG. 3

EP 4 559 477 A2

FIG. 4

119

FIG. 5

Mean Serum Concentrations of ADG106 in Cycle 1

Legend: 0.03mg/kg, 0.1mg/kg, 0.3mg/kg, 1mg/kg, 3mg/kg, 5mg/kg, 10mg/kg

FIG. 6A

Mean serum concentrations of ADG106 (China)

Legend: 0.1 mg/kg, 0.5 mg/kg, 1.5 mg/kg, 3 mg/kg, 5 mg/kg, 10 mg/kg

FIG. 6B

**Mean Serum Concentrations of ADG106 after Weekly Repeat Doses in Male and Female Cynomolgus Monkeys**

FIG. 7

FIG. 8A

FIG. 8B

FIG. 9A

FIG. 9B

FIG. 10A

FIG. 10B

FIG. 11

## sCD137

FIG. 12A

mCD137

FIG. 12B

FIG. 13

FIG. 14A

FIG. 14B

FIG. 14C

FIG. 15A

FIG. 15B

FIG. 15C

FIG. 15D

FIG. 16A

FIG. 16B

FIG. 17A

FIG. 17B

FIG. 17C

FIG. 17D

FIG. 18

FIG. 19A

FIG. 19B

FIG. 20A

FIG. 20B

FIG. 21A

FIG. 21B

FIG. 22A

FIG. 22B

FIG. 23A

FIG. 23B

Lewis Cancer Model (C57BL/6)

Tumor Volume ($mm^3$)

**Legend:**
- Vehicle, BIW × 4 doses
- ADG106 (mIgG1), 2.5mg/kg, BIW × 4 doses
- Cisplatin, 5mg/kg, QW × 3 doses + Paclitaxel, 10mg/kg, QW × 3 doses
- ADG106 (mIgG1), 2.5mg/kg, BIW × 4 doses + Cisplatin, 5mg/kg, QW × 3 doses + Paclitaxel, 10mg/kg, QW × 3 doses

Days after the Start of Dosing

FIG. 23C

Lewis cancer model (C57BL/6)
Vehicle, BIW × 4 doses

Lewis cancer model (C57BL/6)
ADG106 (mIgG1), 2.5 mg/kg, BIW × 4 doses

Lewis cancer model (C57BL/6)
Cisplatin, 5 mg/kg + Paclitaxel, 10 mg/kg, QW × 3 doses

Lewis cancer model (C57BL/6)
ADG106 (mIgG1), 2.5 mg/kg, BIW × 4 doses
Cisplatin, 5 mg/kg + Paclitaxel, 10 mg/kg, QW × 3 doses

FIG. 23D

Lewis Cancer Model (C57BL/6)

Vehicle, BIW x 4 doses

ADG106 (mIgG1), 2mg/kg, BIW x 4 doses

Cisplatin, 4mg/kg, QW x 3 doses
+ Pemetrexed, 200mg/kg, QW x 3 doses

ADG106 (mIgG1), 2mg/kg, BIW x 4 doses
+ Cisplatin, 4mg/kg, QW x 3 doses
+ Pemetrexed, 200mg/kg, QW x 3 doses

FIG. 23E

FIG. 23F

FIG. 24A

FIG. 24B

EP 4 559 477 A2

FIG. 25A

FIG. 25B

FIG. 26A

FIG. 26B

basal N K _ r e s p o n s e

FIG. 27A

Δ % N K _ r e s p o n s e

FIG. 27B

FIG. 28A

FIG. 28B

FIG. 29A

FIG. 29B

Pre-dose: 7.44cm    C2(Day 42) : 3.28 cm

FIG. 30A

Pre-dose: 3.88 cm    C2(Day 42) : 3.26 cm

FIG. 30B

FIG. 31

FIG. 32

**Visual Predictive Check**
**Observations vs. Time (Run 0)**

FIG. 33

FIG. 34

**Simulated C$_{max,cycle\ 1}$ (BW=60kg) vs. Observed C$_{max,cycle\ 1}$**

**Simulated AUC$_{0-21d,cycle\ 1}$ (BW=60kg) vs. Observed AUC$_{0-21d,cycle\ 1}$**

FIG. 35

FIG. 36

FIG. 37

NFκB Reporter Activation in 293T cells
expressing human CD137

NFκB reporter activation in 293T cells
expressing monkey CD137

NFκB reporter activation in 293T cells
expressing mouse CD137

NFκB reporter activation in 293T cells
expressing rat CD137

FIG. 38

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- CN 2020090073 W
- CN 2020094278 W
- CN 2020115795 W
- WO 0348731 A
- WO 2006129163 A
- US 20060153808 A
- WO 2002053596 A
- WO 2002055106 A
- WO 2009079335 A
- WO 2003040170 A
- WO 2003015711 A
- WO 2004016805 A
- WO 2009022215 A
- US 20070117809 A
- CN 107840887 A
- US 10066013 B2
- WO 2019149281 A
- US 8119129 B
- WO 0114424 A
- WO 9842752 A
- WO 0037504 A
- US 6207156 B
- WO 2001014424 A
- WO 2000037504 A
- US 8017114 B
- US 7879984 B
- WO 2005044859 A
- WO 2004035607 A
- WO 2005103081 A
- WO 2004056312 A
- WO 2007031875 A
- WO 2015095410 A
- WO 2019105468 A1
- CN 2020094371 W
- US 20190055314 A1
- WO 2019036855 A1
- WO 2019037711 A1
- US 5677425 A
- US 6933368 B
- US 6291158 B
- US 6582915 B
- US 6593081 B
- US 6172197 B
- US 6696245 B
- EP 0368684 A
- EP 0616640 A
- WO 05035572 A
- WO 04101790 A
- WO 04081026 A
- WO 04058821 A
- WO 04003019 A
- WO 03002609 A
- WO 2007059782 A
- US 5994619 A
- US 4399216 A
- US 4634665 A
- US 5179017 A
- WO 8704462 A
- WO 8901036 A
- EP 338841 A
- WO 2019037711 A

### Non-patent literature cited in the description

- **WAN**. *Immunological Reviews*, 2009, vol. 229, 192-215
- **BROLL**. *Amer. J. Clin. Pathol.*, 2001, vol. 115 (4), 543-549
- **SEAMAN**. *Cancer Cell*, 2007, vol. 11, 539-554
- **DRENKARD**. *FASEB J*, 2007, vol. 21, 456-463
- **OLOFSSON**. *Circulation*, 2008, vol. 117, 1292-1301
- **CROFT**. *Nat Rev Immunol*, 2009, vol. 9, 271-285
- **LYNCH**. *Immunol Rev*, 2008, vol. 22, 277-286
- **VINAY**. *J Mol Med*, 2006, vol. 84, 726-736
- **YVONNE CHEN et al.** Selection and Analysis of an Optimized Anti-VEGF Antibody: Crystal Structure of an Affinity-matured Fab in Complex with Antigen. *J. Mol. Biol.*, 1999, vol. 293, 865-881
- **KABAT et al.** *J. Biol. Chem.*, 1977, vol. 252, 6609-6616
- **KABAT et al.** Sequences of proteins of immunological interest. U.S. Dept. of Health and Human Services, 1991
- **CHOTHIA et al.** *J. Mol. Biol.*, 1987, vol. 196, 901-917
- **AL-LAZIKANI B. et al.** *J. Mol. Biol.*, 1997, vol. 273, 927-948

- **MACCALLUM et al.** *J. Mol. Biol.*, 1996, vol. 262, 732-745
- **ABHINANDAN** ; **MARTIN**. *Mol. Immunol.*, 2008, vol. 45, 3832-3839
- **LEFRANC M.P. et al.** *, Dev. Comp. Immunol.*, 2003, vol. 27, 55-77
- **HONEGGER** ; **PLÜCKTHUN**. *J. Mol. Biol.*, 2001, vol. 309, 657-670
- **EHRENMANN F. et al.** *Nucleic Acids Res.*, 2010, vol. 38, D301-D307
- **ADOLF-BRYFOGLE J. et al.** *Nucleic Acids Res.*, 2015, vol. 43, D432-D438
- Fundamental Immunology. Raven Press, 1989
- **PEARSON**. *Methods Enzymol.*, 1990, vol. 183, 63-98
- **PEARSON**. *Methods Mol. Biol.*, 2000, vol. 132, 185-219
- **ALTSCHUL et al.** *J. Mol. Biol.*, 1990, vol. 215, 403-410
- **ALTSCHUL et al.** *Nucleic Acids Res.*, 1997, vol. 25, 3389-3402
- **WARD et al.** *Nature*, 1989, vol. 341, 544-546
- Epitope Mapping Protocols in Methods in Molecular Biology. 1996, vol. 66
- **SAMBROOK** ; **RUSSELL**. Molecular Cloning, A Laboratory Approach. Cold Spring Harbor Press, 2001
- **AUSUBEL et al.** Current Protocols in Molecular Biology. John Wiley & Sons, 2002
- **HARLOW** ; **LANE**. Antibodies: A Laboratory Manual. Cold Spring Harbor Laboratory Press, 1990
- Immunology-A Synthesis. Sinauer Associates, 1991
- **HURWITZ et al.** *Proc Natl Acad Sci USA*, 1998, vol. 95 (17), 10067-10071
- **CAMACHO et al.** *J Clin Oncology*, 2004, vol. 22 (145)
- **MOKYR et al.** *Cancer Res*, 1998, vol. 58, 5301-5304
- **FORBES, S.A. et al.** *Nucleic Acids Res.*, 2011, vol. 39
- Manual of Histological Staining Method of the Armed Forces Institute of Pathology. The Blakston Division McGraw-Hill Book Company
- The Armed Forces Institute of Pathology Advanced Laboratory Methods in Histology and Pathology. Armed Forces Institute of Pathology, American Registry of Pathology, 1994
- **CHEUK ATC et al.** *Cancer Gene Therapy*, 2004, vol. 11, 215-226
- **BIRD et al.** *Science*, 1988, vol. 242, 423-426
- **HUSTON et al.** *Proc. Natl. Acad. Sci. USA*, 1988, vol. 85, 5879-5883
- **KABAT, E. A. et al.** equences of Proteins of Immunological Interest. NIH Publication, 1991
- **TOMLINSON, I. M. et al.** J. Mol. Biol.. 1992, vol. 227, 776-798
- **COX, J. P. L et al.** *Eur. J. Immunol.*, 1994, vol. 24, 827-836
- **HOLLIGER P. et al.** *Proc. Natl. Acad. Sci. USA*, 1993, vol. 90, 6444-6448
- **POLJAK R. J. et al.** *Structure*, 1994, vol. 2, 1121-1123
- **KOHLER** ; **MILSTEIN**. *Nature*, 1975, vol. 256, 495
- **GREEN et al.** *Nature Genetics*, 1994, vol. 7, 13-21
- **HARLOW** ; **LANE**. Antibodies: A Laboratory Manual. Cold Spring Harbor Press, 1990
- **ACHIM KNAPPIK et al.** Fully Synthetic Human Combinatorial Antibody Libraries (HuCAL) Based on Modular Consensus Frameworks and CDRs Randomized with Trinucleotides. *J. Mol. Biol.*, 2000, vol. 296, 57-86
- **MICHAEL J. FELDHAUS et al.** Flow-cytometric isolation of human antibodies from a non-immune Saccharomyces cerevisiae surface display library. *Nat Biotechnol*, 2003, vol. 21, 163-170
- **KABAT et al.** Sequences of Proteins of Immunological Interest. NIH Publication, 1991
- **MCCAFFERTY et al.** *Nature*, 1990, vol. 348, 552-554
- Gene Expression Technology. **GOEDDEL**. Methods in Enzymology. Academic Press, 1990, vol. 185
- **TAKEBE, Y. et al.** *Mol. Cell. Biol.*, 1988, vol. 8, 466-472
- **URLAUB** ; **CHASIN**. *Proc. Natl. Acad. Sci. USA*, 1980, vol. 77, 4216-4220
- **KAUFMAN** ; **SHARP**. *J. Mol. Biol.*, 1982, vol. 159, 601-621
- NONMEM user's guides (1989-2009). **BEAL S** ; **SHEINER LB** ; **BOECKMANN A** ; **BAUER RJ**. Icon Development Solutions. Ellicott City, 2009
- **LINDBOM L** ; **PIHLGREN P** ; **JONSSON EN**. PsN-Toolkit-a collection of computer intensive statistical methods for non-linear mixed effect modeling using NONMEM,. *Comput Methods Programs Biomed*, 2005, vol. 79, 241-257
- **WEI**. *PLoS ONE*, 2013